# EUROPEAN PATENT APPLICATION

(11) **EP 3 678 044 A2**
(43) Date of publication of application: **08.07.2020**
(21) Application number: 19212296.8
(22) Date of filing: 24.12.2008
(51) Int. Cl.: G06K 7/08, G07F 7/10

(54) **CARDS AND DEVICES WITH MAGNETIC EMULATORS FOR COMMUNICATING WITH MAGNETIC STRIPE READERS AND APPLICATIONS FOR THE SAME**

(30) Priority: 24.12.2007 US 16491; 07.02.2008 US 26846; 11.02.2008 US 27807; 15.07.2008 US 81003; 05.08.2008 US 86239; 20.08.2008 US 90423; 16.09.2008 US 97401; 09.11.2008 US 112766; 23.11.2008 US 117186; 02.12.2008 US 119366 P; 08.12.2008 US 120813; 19.12.2008 US 339045; 19.12.2008 US 33904108; 19.12.2008 US 33904208; 19.12.2008 US 33904308; 19.12.2008 US 33904608; 19.12.2008 US 33904808; 19.12.2008 US 33905108; 19.12.2008 US 33905408; 19.12.2008 US 33905508; 19.12.2008 US 33905808; 19.12.2008 US 33906108; 19.12.2008 US 33906508; 19.12.2008 US 33906608; 19.12.2008 US 33906708; 19.12.2008 US 33906908; 19.12.2008 US 33907108; 19.12.2008 US 33907408; 19.12.2008 US 33907808; 19.12.2008 US 33908108; 19.12.2008 US 33908208; 19.12.2008 US 33908508; 19.12.2008 US 33908608; 19.12.2008 US 33909008; 19.12.2008 US 33909108; 19.12.2008 US 33909208; 19.12.2008 US 33909308; 19.12.2008 US 33909408; 19.12.2008 US 33909508; 19.12.2008 US 33909708; 19.12.2008 US 33909808; 19.12.2008 US 33910108; 19.12.2008 US 33910208; 19.12.2008 US 33910308
(62) Divisional of application: 08865573.3
(71) Applicant: Dynamics Inc., Pittsburgh, PA 15232 (US)
(72) Inventor: MULLEN, Jeffrey D., Pittsburgh, PA Pennsylvania PA15232 (US); CLOUTIER, Bruce, Pittsburgh, PA Pennsylvania 15644 (US); YEN, Philip, Cupertino, CA California CA 95014 (US); LAMBETH, David, Pittsburgh, PA Pennsylvania 15215 (US); KELLOGG, Ryan, Pittsburgh, PA Pennsylvania 15203-2195 (US); REUTZEL, Will, Pittsburgh, PA Pennsylvania 15206-4523 (US)
(74) Representative: Rooney, John-Paul

(57) **Abstract**

A card (12400) comprising a dynamic magnetic communications device for communicating a track of magnetic stripe data to a magnetic stripe reader, wherein said dynamic magnetic communications device communicates said track of magnetic stripe data serially a button (12411); wherein the card and button are configured to provide two bundles of payment information serially to a magnetic stripe reader via the dynamic magnetic communications device, and information is included in the discretionary fields of both bundles of payment information indicative of a split order.

## Description

### Background of the Invention

This invention relates to payment cards such as credit cards.

### Summary of the Invention

A card is provided with a dynamic number. For example, a payment card, such as credit card, is provided with a dynamic payment card number, such as a dynamic credit card number. A dynamic credit card number may, for example, change based on time or use.

A portion of a dynamic credit card number may be static. Accordingly, for example, the credit card number may change but particular digits of the dynamic credit card number may remain constant. For example, a dynamic credit card number may be provided such that the beginning one or more digits (e.g., first six digits) are static.

As such, the beginning one or more digits of a number may be representative of the type of card (e.g., a dynamic credit card) as well as other information. Such information may include, for example, routing information such that at least some of the digits of a number may be communicated to remote servers according to the static routing information.

For example, the beginning digit of numerous dynamic credit cards may have the same static one or more digits (e.g., begining digits). For example, the beginning digits of an American Express dynamic credit card may take the form of "399." The first digit (e.g., "3") may be representative of the card manufacturer (e.g., "American Express"). The second and third digits (e.g., "99") may be representative of the type of card (e.g., a dynamic credit card).

A magnetic emulator may be provided on a card (e.g., a credit or debit card). Such a magnetic emulator may include one or more wires that are able to produce an electromagnetic field that is operable to be read by, for example, a magnetic stripe reader. The magnetic emulator may be provided about a magnetic stripe and may be utilized to produce an electromagnetic field such that, for example, a magnetic stripe reader may seamlessly read a portion of the magnetic stripe, then read fields generated from the magnetic emulator, then read another portion of the magnetic stripe. In doing so, for example, only particular information related to an account (e.g., all or a portion of a credit card number) may be communicated via a magnetic emulator and, as such, may be operable to be changed.

A magnetic emulator may be placed adjacent to a magnetic stripe such that the first data read by a magnetic stripe data is provided by the magnetic emulator. Similarly, numerous magnetic emulators may be provided about one or more magnetic stripes. For example, a magnetic emulator may be provided at different locations on different tracks of a magnetic stripe. Magnetic emulators may share, for example, similar control circuitry. Magnetic emulators may communicate data directly to a read-head of a card reader without the presence of a magnetic medium. A card may be provided with one or more emulators and without a magnetic stripe.

A magnetic encoder may be provided, for example, to change the state of magnetic filaments located on a magnetic stripe such that information may be changed. A magnetic encoder may write information on a magnetic material configured for that magnetic encoder. Accordingly, for example, a card (e.g., a security card) may be provided with a magnetic stripe having one set of attributes (e.g., one coercivity) and another magnetic stripe, configured for use with a magnetic encoder, having a different set of attributes (e.g., a different coercivity).

An identification name may be printed on the front of a card (e.g., a credit, debit, or security card). This name may be unique. Accordingly, for example, no two credit cards may be fabricated that utilize the same identification name. For example, instead of providing a card with an identification name of "Christopher Mullen," that card may include an identification name of "Christopher-Mullen-201." As such, a user on a website may utilize the identification name in a field representative of "name on card"/"name of account holder." Such an identification name may also be communicated to a magnetic stripe reader via a magnetic stripe or magnetic emulator located on a credit card.

A batch of credit card numbers may be partitioned such that, for example, two users having the same name may not, for example, have a dynamic credit card associated with the same particular partition. For example, suppose the first three numbers of a dynamic credit card number are static (e.g. "391"). The first digit (e.g., "3") may be representative of a credit card manufacturer (e.g., "American Express."). The second digit (e.g., "9") may be representative of the type of credit card (e.g., a dynamic credit card). The third digit (e.g., "1") may be representative of the partition. Accordingly, a credit card verification process may be able to recognize a partition. Using this scheme, for example, ten partitions may be provided (e.g., "390-399"). As such, ten people using the exact same name may be provided with a dynamic credit card that utilizes this scheme and each could be provided with a different partition.

More than one display may be utilized on a card, such as an identification card or credit card. A single controller (e.g., a processor) and a clock may be utilized to drive such displays. Similarly, each display may be provided with its own controller and clock. The clock may supply timing signals to such controllers. Accordingly, each controller may be provided with a different type of coding. For example, one controller may utilize one coding scheme and another controller may utilize another coding scheme. As such a dynamic number, such as a dynamic credit card number, may be provided by one or more displays run by different coding schemes. If one coding scheme is compromised by a thief, such an additional coding scheme would provide additional security. Similarly, a single controller may provide multiple coding schemes to different portions of a dynamic number (e.g., a dynamic credit or debit card number).

A dynamic number may change periodically. A dynamic number may change based on a time period. This time period may be displayed on a display. For example, the time period may be a portion of a dynamic number. Accordingly, a transmitted dynamic number may include the information as to what time period, or time periods, the dynamic number is associated with.

A dynamic code may be provided. A dynamic code may be provided on its own display or on a display providing other information (e.g., dynamic card number). Such a dynamic code may be, for example, a dynamic security code. Accordingly, for example, a dynamic security code may be utilized with a dynamic card number to authorize a payment transaction. Such a dynamic code may also be communicated through a magnetic emulator or encoder. Alternatively, for example, one dynamic code may be communicated visually (e.g., for online transactions) and a different dynamic code may be communicated magnetically (e.g., for in-store transactions). Such codes may change based on time or use. One or more buttons may be provided to change a particular dynamic number or all dynamic numbers. For example, a button may be utilized to change a dynamic card number and a different button may be utilized to change a dynamic security code for online use. A single button may be provided, for example, that changes all dynamic numbers (e.g., a card number provided on a display, an online security code provided on the same or a different display, and an in-store code communicated magnetically to a magnetic stripe reader). An in-store code and an online security code may be, for example, the same code. Different tracks of data may, for example, have different security codes.

A card, or other device, is provided with a magnetic emulator operable to communicate with a magnetic stripe reader. The emulator may be operable to communicate a block of information serially such that a reader may sense the emulator and the information may be transmitted through the emulator serially. Accordingly, the emulator may comprise of, for example, a single wire or coil and may transmit bits one-by-one at high frequencies such that all information in a block is transmitted to a magnetic stripe reader while the read head of the reader is in the proximity of the single wire or coil. An emulator may also be provided in a parallel configuration such that multiple bits of data are emulated at the same time. For example, a parallel emulator may include 3000 regions to emulate 3000 bits simultaneously while a serial emulator may include a single region to communicate the 3000 bits in rapid succession.

A card, or other device, having a magnetic emulator may take the form of, for example, a credit card, debit card, security card, and/or any other type of card. Accordingly, the dynamic information may be a dynamic credit card number, a dynamic debit card number, a dynamic security number, or any other type of dynamic number. A display may be provided to display the data, or a portion of the data, communicated through an emulator or additional data. For example, a one type of security code may be displayed on a display and another type of security code may be communicated through a magnetic emulator.

A payment card, such as a credit card, may be provided that includes a display. All, or a portion of, a payment card number (e.g., a credit card) may, for example, be changed periodically and displayed on the display. Similarly, this changed information may be emulated via a parallel or serial emulator. A magnetic encoder may also be utilized to erase and write information to a magnetic medium.

A parallel or serial emulator may be located next to one or more magnetic stripe segments (e.g., sandwiched between two magnetic stripe segments). A magnetic stripe may be utilized to transmit static information such that power is conserved. For example, if the beginning bits of a data block must take a particular form (e.g., start bits followed by user identification information) then this information may be embodied as a magnetic stripe. A serial or parallel emulator may then be provided to communicate the remaining information of the block (e.g., dynamic credit card number).

Numerous types of structures may be utilized to determine when a read head of a magnetic stripe reader is reading, or is about to read, a magnetic stripe or a dynamic magnetic communications device (e.g., a magnetic emulator or encoder). Such structures may be utilized to turn a magnetic emulator, such as a serial magnetic emulator, ON. By only turning an emulator ON when the emulator is in the proximity of a magnetic stripe reader, power may be conserved. For example, a button may be provided on a card, or other device, such that a user may provide manual input to instruct the card, or other device, to turn an emulator ON. Alternatively, for example, one or more sensors may be provided to determine the presence of a read-head of a magnetic stripe reader. For example, a hall-effect sensor may be provided to detect a magnetic field's interaction with a read-head, a circuit may be provided to detect the presence of a conductive material, and/or a circuit may be provided to detect the capacitance of a particular material. Alternatively still, for example, the swiping motion of a card may be detected via one or more inertial sensors such as accelerometers and/or gyroscopes. Upon the initiation of turning an emulator ON, the emulator may be driven through a routine (e.g., repeatedly emulating the same block of information serially for a period of time or for a number of data transmissions). A card, or other device, may include, for example, a magnetic stripe section, followed by a read head detector, followed by a serial or parallel encoder. A second magnetic stripe section may follow the serial or parallel encoder (e.g., and another read head detector may precede the encoder to determine when the read head is not reading the encoder).

An emulator may be fabricated using a PCB printing technique. Such a technique may provide the emulator on, for example, a PCB board (e.g. FR4 board). Any additional components may be fabricated on the flexible PCB board. For example, a processor, display, and emulator may be fabricated on the same flexible PCB board. Such a flexible PCB board may be coupled to a flexible battery and hot-film laminated or cold laminated (e.g., injection molding) to form a card. The flexible PCB board and flexible battery may be placed into any housing of any device.

A card is provided, such as a credit card or security card, that may transmit information to a magnetic stripe reader via a dynamic magnetic communications device such as a magnetic emulator or a magnetic encoder. The emulator may transmit the information serially, for example, in order to reduce the amount of circuitry needed to emulate a particular block of information (e.g., payment information).

One or more buttons may be included on the card. Buttons may be included, for example, to provide a control interface to navigate through various options of the card. Additionally, coding schemes may be selected via buttons. Furthermore, a card may be locked until a private number is entered into a card or a number may only be generated (e.g., displayed and/or emulated) once a particular private number is entered into a card. Such a number may be, for example, a dynamic credit card, security card, and/or debit card number or other number (e.g., security code).

A card, or other device, having a magnetic emulator may take the form of, for example, a credit card, debit card, and/or security card. Accordingly, the dynamic information may be a dynamic credit card number, a dynamic debit card number, and/or a dynamic security number. A display may be provided to display the data, or a portion of the data, communicated through an emulator. In this manner, a credit card may be provided that includes a display. All, or a portion of, a credit card number may, for example, be changed periodically and displayed on the display. Similarly, this changed information may be emulated via a parallel or serial emulator or other dynamic magnetic communications device (e.g., a magnetic encoder).

A dynamic magnetic communications device (e.g., magnetic emulators and/or encoders) may be located next to one or more magnetic stripe segments (e.g., sandwiched between two magnetic stripe segments from a birds-eye perspective of a card). A magnetic stripe may be utilized to transmit static information such that power is conserved. For example, if the beginning bits of a data block must take a particular form (e.g., start bits followed by user identification information) then this information may be embodied as a magnetic stripe. A serial or parallel emulator or encoder may then be provided to communicate the remaining information of the block (e.g., dynamic credit card number).

Numerous types of structures may be utilized to determine when a read-head of a magnetic stripe reader is reading, or is about to read, a magnetic stripe or dynamic magnetic communications device. Such structures may be utilized to turn a magnetic emulator, ON and OFF. By only turning an emulator ON when the emulator is in the proximity of a magnetic stripe reader, power may be conserved. For example, a button may be provided on a card, or other device, such that a user may provide manual input to instruct the card, or other device, to turn an emulator ON.

A card is provided, such as a credit card or security card, that may transmit information to a magnetic stripe reader via a magnetic emulator. The magnetic emulator may be, for example, a circuit that emits electromagnetic fields operable to electrically couple with a read-head of a magnetic stripe reader such that data may be transmitted from the circuit to the magnetic stripe reader. The emulator may be operated serially such that information is transmitted serially to a magnetic stripe reader. Alternatively, for example, portions of a magnetic emulator may emit different electromagnetic fields at a particular instance such that the emulator is operated to provide physically parallel, instantaneous data. Alternatively still, a magnetic medium may be provided and a circuit may be provided to change the magnetic properties of the magnetic medium such that a magnetic stripe reader is operable to read information written on the magnetic medium.

A processor may be provided on a card, or other device, that controls a magnetic emulator. The processor may be configured to operate the emulator such that the emulator transmits serial or parallel information. Particularly, the processor may decouple portions of an emulator from one another such that different portions of the emulator may transmit different information (e.g., transmit data in a parallel operation). The processor may couple portions of an emulator together (or drive the portions together) such that all portions of the emulator transmits the same information (e.g., transmit data in a serial operation). Alternatively, the processor may drive a portion of the emulator to transmit data using one method (e.g., serially) while the processor drives another portion of the emulator using a different method (e.g., in parallel).

The processor may drive an emulator through a switching circuit. The switching circuit may control the direction and magnitude of current that flows through at least a portion of an emulator such that the switching circuit controls the direction and magnitude of the electromagnetic field created by at least that portion of the emulator. An electromagnetic field may be generated by the emulator such that the emulator is operable to electrically couple with a read-head from a magnetic stripe reader without making physical contact with the read-head. Particularly, for example, an emulator that is driven with increased current can be operable to couple with the read-head of a magnetic stripe reader even when placed outside and within the proximity of (e.g., 0.25 inches) the read-head.

A magnetic emulator may be operated to electrically couple, and transmit data to, devices other than a magnetic stripe reader. For example, a magnetic emulator may be operated to electrically couple, and transmit data to, a device using a Radio Frequency IDentification (RFID) protocol. Accordingly, a processor may drive the emulator at a frequency and magnitude in order to electrically couple with a read-head of a magnetic stripe reader and then drive the emulator at a different frequency and a different magnitude in order to electronically couple with an RFID reader.

A processor may receive information from a magnetic stripe reader detector and/or an RFID receiver detector. A processor may detect, for example, the presence of a read-head of a magnetic stripe reader by receiving signals from a magnetic stripe reader detector and, in response, the processor may drive a magnetic emulator in a manner that allows the emulator to couple with the magnetic stripe reader. The processor may also detect, for example, the presence of and RFID receiver by receiving signals from an RFID receiver detector and, in response, the processor may drive a magnetic emulator in a manner that allows the emulator to couple with the RFID receiver. More than one emulator may be provided on a card or other device and a processor may drive such emulators in a variety of different manners.

A circuit may be provided on a credit card that is operable to receive data from a magnetic stripe encoder and/or an RFID transmitter. Such a circuit may electrically couple with an RFID transmitter and/or magnetic stripe encoder and deliver information to a processor. In this manner, a card, or other device, may communicate bi-directionally with a device.

An emulator may communicate with a magnetic stripe reader outside of, for example, the housing of a magnetic stripe reader. Accordingly, for example, the emulator may be provided in devices other than cards sized to fit inside of the reading area of a magnetic stripe reader. In other words, for example, the emulator may be located in a device that is thicker than a card - yet the emulator can still communicate with one or more read-heads located in a magnetic stripe reader. Such a device may be, for example, a security token, a wireless communications device, a laptop, a Personal Digital Assistant (PDA), a physical lock key to a house and/or car, or any other device.

Dynamic information may be provided by a processor located on the card, or other device, and communicated through a magnetic emulator. Such dynamic information may, for example, change based on time. For example, the dynamic information may be periodically encrypted differently. One or more displays may be located on a card, or other device, such that the dynamic information may be displayed to a user through the display. Buttons may be provided to accept input from a user to, for example, control the operation of the card or other device.

Dynamic information may include, for example, a dynamic number that is used as, or part of, a number for a credit card number, debit card number, payment card number, and/or payment verification code. Dynamic information may also include, for example, a student identification number or medical identification number. Dynamic information may also, for example, include alphanumeric information such that a dynamic account name is provided.

Magnetic emulation circuits may be provided that generate electromagnetic fields. The emulation circuits may have active regions operable to be read by a read-head of a magnetic stripe reader. The emulation circuits may also have, for example, non-active regions that are not operable to be read by a read-head of a magnetic stripe reader. Multiple emulation circuits may be provided on different layers such that the active regions of multiple emulation circuits provide a read-head of a magnetic stripe reader continuous visibility to active regions while a card is swiped.

Magnetic emulation circuits may extend across multiple tracks. However, the areas of such magnetic emulation circuits that extended to undesired tracks may be configured to be invisible to the read-heads for those tracks. For example, a magnetic emulator may produce magnetic fields that are not oriented properly to be picked up by unintended read-head(s) but that are oriented properly to be picked up by intended read-head(s).

Read-head detectors may be provided to determine, for example, when a card is being swiped and/or when a read-head is located over a particular portion of a card (e.g., a magnetic emulation circuit). A magnetic emulation circuit may be provided as, for example, a coil. Portions of such a coil may be utilized to detect a read-head while in other portions of the coil may be utilized to communicate information electromagnetically to a read-head. Accordingly, a coil may be utilized to detect a read-head and, after a read-head is detected, the coil may be utilized to, for example, serially transmit information to a magnetic stripe reader.

Dynamic information may include, for example, a dynamic number that is used as, or part of, a number for a credit card number, debit card number, payment card number, and/or payment verification code. Dynamic information may also include, for example, a student identification number or medical identification number. Dynamic information may also, for example, include alphanumeric information such that a dynamic account name is provided.

Magnetic emulation circuits may be provided that generate electromagnetic fields. The emulation circuits may have active regions operable to be read by a read-head of a magnetic stripe reader. The emulation circuits may also have, for example, non-active regions that are not operable to be read by a read-head of a magnetic stripe reader. Multiple emulation circuits may be provided on different layers such that the active regions of multiple emulation circuits provide a read-head of a magnetic stripe reader continuous visibility to active regions while a card is swiped. A coil may include return paths that may be able to, for example, transmit information to a read-head but may communicate information using electromagnetic fields in an opposite direction than the primary paths (e.g., active regions) of a coil such that a reader may not be able discern a set of when the reader picks up part of the information from a return path followed by part of the information from a primary path (or vice versa).

Magnetic emulation circuits may extend across multiple tracks. However, the areas of such magnetic emulation circuits that extended to undesired tracks may be configured to be invisible to the read-heads for those tracks. For example, a magnetic emulator may produce magnetic fields that are not oriented properly to be picked up by unintended read-head(s) but that are oriented properly to be picked up by intended read-head(s).

Read-head detectors may be provided to determine, for example, when a card is being swiped and/or when a read-head is located over a particular portion of a card (e.g., a magnetic emulation circuit). A magnetic emulation circuit may be provided as, for example, a coil. Portions of such a coil may be utilized to detect a read-head while in other portions of the coil may be utilized to communicate information electromagnetically to a read-head. Accordingly, a coil may be utilized to detect a read-head and, after a read-head is detected, the coil may be utilized to, for example, serially transmit information to a magnetic stripe reader.

A read-head detector, or an array of read-head detectors, may be able to, for example, determine the type of reader that the card entered into. For example, a read-head detector array may determine, for example, when a motorized reader was utilized, an insertion reader was utilized, or a user-swipe reader was utilized. Such information may be stored and communicated to a remote storage device (e.g., a remote database). This stored information may be utilized to combat, for example, card cloning. For example, if a particular number of cards (e.g., 10 more) that made consecutive purchases from a machine (e.g., an ATM) detected more than one reader, then, for example, the system may make an autonomous determination that an illegal cloning device was located on front of that ATM machine. If, for example, multiple cards use a restaurant point-of-sale terminal and determine that multiple readers were used then, for example, a computer can make an autonomous determination that cloning may have occurred at the restaurant.

Cards may be swiped through the same reader multiple times for a number ofreasons (e.g., mis-swipes). However, over a number of cards (e.g., 100), instances of cloning may become apparent. Additionally, for example, information about swipes that happened outside of a transaction (e.g., the period from which the card is active after an appropriate unlocking code is entered) may be transmitted to detect instances where a magnetic emulator was turned ON and read by a reader, but no transaction was received by a processing/authorization facility. Such information may be utilized to, for example, provide an alert that the user may have encountered, and tried to use, a fake ATM machine.

Multiple magnetic emulators may be coupled in series. Multiple magnetic emulators, or arrays of magnetic emulators, may be controlled independently. Emulators may be assigned zones and may be utilized to communicate information on a zone-by-zone basis. In doing so, for example, emulators that include coils with return paths may place those return paths in other zones. Accordingly, the primary paths for an emulator may be included in a zone to communicate information when that zone is activated. When other zones are activates, the return paths of the emulator may not interfere with the primary paths of other emulators that are attempting to communicate information. Read-head detectors may be utilized, for example, to provide information to a processor so that the processor may make a determination as to what zone, or zones, should be activated to communicate information at any given time.

Magnetic emulators, such as magnetic emulators that include coils, may be fabricated on multiple layers of either flexible or rigid printed circuit board. Accordingly, a coil may be fabricated over multiple layers. Materials may be placed in the interior of these coils to assist the coil in communicating information to a read-head. For example, two PCB layers may be utilized. The top layer may be utilized for one set of paths (e.g., primary paths) and the bottom layer may be utilize for another set of paths (e.g., return paths).

A material may be sandwiched between the two layers to assist in reducing the effect of the electromagnetic fields from one set of coil segments on the side of the material opposite that set of coil segments. Such an interior material may be insulated such that the material does not short the coil segments. Additionally, such an interior material may be chosen, for example, such that the material does not saturate when the coil is conducting current. The coil and material may run, for example, along the location of a track of magnetic data for a payment card.

A material may be placed and/or printed on a PCB layer and sandwiched between two other PCB layers. These two other layers may each include coil segments and vias. The middle layer may also include vias such that the material is fabricated to be located in the center of the coil. The material may take a cylindrical, rectangular, square, or any type of shape. Four layers may also be utilized, where the coil segments are printed on a surface of the exterior layers and one or more materials are printed and/or placed on/between the interior layers. A material may be a magnetic material, ferromagnetic material, ferrimagnetic material, or any type of material. For example, copper may be printed on a PCB layer and plated with a material (e.g., nickel, iron, chrome, tin, gold, platinum, cobalt, zinc, alloys). A material, for example, may have a relative permeability multiple times greater than the permeability of a vacuum. A material, for example, may have a permeability of 2 to 25,000 N/A^2. A material may include, for example, a permalloy, iron, steel, ferrite, nickel or any other material. A material may be an alloy such as a nickel-iron alloy. Such a nickel-iron alloy may include, for example, nickel (e.g., 75-85%), iron, copper, molybdenum and may be placed through one or more annealing processes. Annealing may occur before and/or after the material is placed/printed on a layer of material (e.g., a PCB layer or other layer). A similar and/or different material may be placed either above and/or below a portion, or the entire, set of paths on a layer for a coil. Accordingly, for example, a material may be placed in the interior of a coil as well as along a side of the coil.

A read-head detector, or an array of read-head detectors, may be able to, for example, determine the type of reader that the card entered into. For example, a read-head detector array may determine, for example, when a motorized reader was utilized, an insertion reader was utilized, or a user-swipe reader was utilized. Such information may be stored and communicated to a remote storage device (e.g., a remote database). This stored information may be utilized to combat, for example, card cloning. For example, if a particular number of cards (e.g., 10 more) that made consecutive purchases from a machine (e.g., an ATM) detected more than one reader, then, for example, the system may make an autonomous determination that an illegal cloning device was located on front of that ATM machine. If, for example, multiple cards use a restaurant point-of-sale terminal and determine that multiple readers were used then, for example, a computer can make an autonomous determination that cloning may have occurred at the restaurant.

A material may be sandwiched between the two layers to assist in reducing the effect of the electromagnetic fields from one set of coil segments on the side of the material opposite that set of coil segments. Such an interior material may be insulated such that the material does not short the coil segments. Additionally, such an interior material may be chosen, for example, such that the material does not saturate when the coil is conducting current. The coil and material may run, for example, along the location of a track of magnetic data for a payment card. Accordingly, a coil may be fabricated so that the coil wraps around an interior material.

A material may be placed and/or printed on a PCB layer and sandwiched between two other PCB layers. These two other layers may each include coil segments and vias. The middle layer may also include vias such that the material is fabricated to be located in the center of the coil. The material may take a cylindrical, rectangular, square, or any type of shape. Four layers may also be utilized, where the coil segments are printed on a surface of the exterior layers and one or more materials are printed and/or placed on/between the interior layers. A material may be a magnetic material, ferromagnetic material, ferrimagnetic material, or any type of material. For example, copper may be printed on a PCB layer and plated with a material (e.g., nickel, iron, chrome, tin, gold, platinum, cobalt, zinc, allows). A material, for example, may have a relative permeability multiple times greater than the permeability of a vacuum. A material, for example, may have a relative permeability of 2 to 25,000. A material may include, for example, a permalloy, iron, steel, ferrite, nickel or any other material. A material may be an alloy such as a nickel-iron alloy. Such a nickel-iron alloy may include, for example, nickel (e.g., 75-85%), iron, copper, molybdenum and may be placed through one or more annealing processes. Annealing may occur before and/or after the material is placed/printed on a layer of material (e.g., a PCB layer or other layer). A similar and/or different material may be placed either above and/or below a portion, or the entire, set of paths on a layer for a coil. Accordingly, a material may be placed in the interior of a coil as well as along a side of the coil.

Gift cards may be purchased at a store and loaded into a dynamic card. For example, a person may go to a store, purchase a card (at which point the card is activated for use), and give the card to a friend. The friend may then scratch off a removable surface on the gift card to review a dynamic loading code. The friend may utilize user interfaces on the card (e.g., buttons) to load the credit card into the card. Thus, the friend may throw away the gift card but may utilize his/her dynamic card as the gift card. Particularly, the user may utilize a user interface (e.g., a button) on the card to signal to the card that the gift card should be utilized instead of, for example, the user's payment card (e.g., credit card data). Accordingly, the card may communicate the gift card information via a magnetic emulator to a magnetic stripe reader. A processor may provide this information to an RFID antenna located on the card as well as an IC chip located on the card incase, for example, the user utilizes a smartcard or RFID reader located at the point-of-sale.

A user may scratch off a removable surface of a gift card (or other type of card such as a pre-paid payment card) to reveal a code. This code may be entered online at a website that manages the user's payment card (e.g., the user's credit card). The gift card may then attach to the user's credit card. Thus, for example, the user may utilize his/her credit card at a store but the money may be drawn from the gift card. Alternatively, the user may, after making a purchase at a store, go onto his/her payment card's account website and view his/her gift cards. After making a purchase, the user may request a credit refund and may select his/her debit card to be utilized in its place. A period of time may be associated with this. For example, a user may be configured to only be able to utilize gift cards after a purchase for the user's current billing cycle or within a month of purchase.

A user may utilize a user interface on a card or other device (e.g., a mobile telephonic device or other mobile device) to select that a gift card be utilized. The user's payment card information (e.g., debit card information) may be communicated, but the interaction with the user interface may result in additional/different discretionary data being sent from the point-of-sale device through a routing server to an authorization server. One of these servers may look at the discretionary data and may utilize this data to route the information to a different authorization server (e.g., gift card authorization server) or may utilize the information at the authorization server to authorize the information differently (e.g., authorize the gift card purchase). If the amount purchased exceeds the gift card then the payment card may be automatically charged the remaining balance or the cashier may be prompted that the gift card transaction went through at the remaining gift card balance and was applied to the transaction such that the cashier can request additional payment from the customer. Accordingly, a user may enter a simplified code into his/her card order to load his/her payment card with a gift card. For example, a user may enter a code between 16 and 19 digits into a website associated with the user's payment card to attach the gift card to the user's account but may enter a shorter code (e.g., 4-6 characters) into his/her card such that a button is associated with adding/changing discretionary data transmitted by a card (or other data transmitted by a magnetic emulator).

A code that is added to a card may, for example, include data with what button should be utilized activate the transmission of a gift card or other information. Other information may include, for example, the name of a store, the expiration date of the gift card, and the amount of the gift card. For example, a user interface on a card may take the form of a mechanical button. A display may be located next to the button. A code may be entered that causes the display to display "WALMART" every time the button associated with the display is pressed. Accordingly, a user can see what gift cards are associated with what buttons at any given time. Displays may be, for example, LCD, electronic ink, or any other types of displays. Codes may be generated to delete cards after they are utilized. User interfaces may be associated with deleting gift cards from a user's card.

A global payment card is provided. A global card may, for example, allow a user to utilize the same payment account (e.g., credit account) but may transmit differently structured data depending on the country (e.g., the payment network) the user is located in. For example, suppose Japan includes a nineteen digit payment card number while a U.S. payment card number is fifteen or sixteen digits in length. Accordingly, a user may be provided with a nineteen Japanese number and a sixteen U.S. number when the user is provided with a payment account. The user may be issued with a card that includes a button for Japan. The card may be provided a default such that, for example, a particular country's data structure is utilized by default. Accordingly, a user may utilize the card in the U.S. without changing behavior. However, for example, when the user travels to Japan, the user may interact with a user interface (e.g., a button) such that the Japanese data structure with the Japanese payment information is utilized. Accordingly, a magnetic emulator may be provided to transmit data to a magnetic stripe reader. A processor may also provide the data to an IC chip operable to be read by a smartcard reader and/or an RFID antenna operable to be read by a contactless RFID reader.

Displays may be provided near user interfaces or other structures. For example, a display may be provided next to an LED. Cards may be programmed during manufacturing so that these displays may display particular information. Accordingly, for example, the same card architecture may be utilized to provide a number of different types of cards. A user may utilize user interfaces (e.g., mechanical or capacitive interfaces) to change the function of the display. For example, codes may be entered to reconfigure the displays. Alternatively, for example, a user may utilize buttons to select information to be displayed on displays associated with user interfaces. A code may associate a name of a store with a button and/or a dollar amount. For example, a display may be configured to read "Target $50." Information may be entered manually, but also may be received by a card. For example, a user may swipe a card a second time through a magnetic stripe reader and receive information via a magnetic emulator. This received information may be utilized to update information on the card (e.g., the balance of a gift card, credit account, and/or debit account). Information may also be received by an RFID antenna and/or IC chip located on a card and in communication with a central processor (or distributed processors). For example, transaction information (e.g., list of past transactions, stores where transactions occurred, amounts of transactions) and account information (e.g., balance information, bill information, amount due information) may be communicated to the card and displayed on one or more displays.

A dynamic card may be manufactured in a variety of ways. For example, a dynamic card may be printed onto a flexible material (e.g., a flexible polymer). Multiple layers of this material may be bonded together to form a multiple layer flexible structure. This multiple layer structure may be laminated (e.g., via hot and/or cold lamination) to form a card. The card may be programmed before or after lamination. A card may be programmed via a direct connection between a programmer and one or more contacts on a card. A card may be programmed via a capacitive, optical, or inductive communication via a communication link between a programmer and one or more components (e.g., a contact) on a card. Accordingly, for example, a card may be laminated and capacitively, optically, or inductively programmed. After programming, a processor on the card may be signaled to burn-out its programming communication channel(s) such that no further programming may occur. A portion of the card may not be laminated. Accordingly, a programmer may connect to this non-laminated portion of the card. The non-laminated portion of the card may be laminated after programming. Alternatively, for example, the non-laminated portion of the card may be cut after programming (e.g., and after the processor burns-out its programming ports so the processor cannot be further programmed).

Additional external communication devices may be provided on a card. For example, a USB port or Wi-Fi antenna may be provided on a card. Such additional external communication devices may, for example, allow a user to communicate with stationary computer, laptop, or other device. Such communication devices may, for example, be utilized to load gift cards, or other information (e.g., transactional or account information) from a laptop to a card or other device.

Displays may be provided near user interfaces or other structures. For example, a display may be provided next to an LED. Cards may be programmed during manufacturing so that these displays may display particular information. Accordingly, for example, the same card architecture may be utilized to provide a number of different types of cards. A user may utilize user interfaces (e.g., mechanical or capacitive interfaces) to change the function of the display. For example, codes may be entered to reconfigure the displays. Alternatively, for example, a user may utilize buttons to select information to be displayed on displays associated with user interfaces. A code may associate a name of a store with a button and/or a dollar amount. For example, a display may be configured to read "Target $50." Information may be entered manually, but also may be received by a card. For example, a user may swipe a card a second time through a magnetic stripe reader and receive information via a magnetic emulator. This received information may be utilized to update information on the card (e.g., the balance of a gift card, credit account, and/or debit account). Information may also be received by an RFID antenna and/or IC chip located on a card and in communication with a central processor (or distributed processors). For example, transaction information (e.g., list of past transactions, stores where transactions occurred, amounts of transactions) and account information (e.g., balance information, bill information, amount due information) may be communicated to the card and displayed on one or more displays.

A dynamic card may be manufactured in a variety of ways. For example, a dynamic card may be printed onto a flexible material (e.g., a flexible polymer). Multiple layers of this material may be bonded together to form a multiple layer flexible structure. This multiple layer structure may be laminated (e.g., via hot, warm and/or cold lamination) to form a card. The card may be programmed before or after lamination. A card may be programmed via a direct connection between a programmer and one or more contacts on a card. A card may be programmed via a capacitive, optical, or inductive communication via a communication link between a programmer and one or more components (e.g., a contact) on a card. Accordingly, for example, a card may be laminated and capacitively, optically, or inductively programmed. After programming, a processor on the card may be signaled to burn-out its programming communication channel(s) such that no further programming may occur. A portion of the card may not be laminated. Accordingly, a programmer may connect to this non-laminated portion of the card. The non-laminated portion of the card may be laminated after programming. Alternatively, for example, the non-laminated portion of the card may be cut after programming (e.g., and after the processor burns-out its programming ports so the processor cannot be further programmed).

Additional external communication devices may be provided on a card. For example, a USB port or Wi-Fi antenna may be provided on a card. Such additional external communication devices may, for example, allow a user to communicate with stationary computer, laptop, or other device. Such communication devices may, for example, be utilized to load gift cards, or other information (e.g., transactional or account information) from a laptop to a card or other device. A card is provided that includes a light sensor such that information can be communicated to a card via light (e.g., via a light transmitted from a TV or website).

Information that is transmitted to a magnetic stripe read-head can be changed by a card. The information can be changed based on software that is preloaded into a card. Similarly, the information can be determined, at least in part, by a user of the card. Accordingly, a user of a card may enter information into a card via user interfaces in order to change at least part of the information transmitted through a magnetic-stripe reader, via a magnetic stripe read-head, to a remote payment card processing server.

A user may command a card to communicate particular information to obtain a variety of functionalities. For example, a user may be required to perform a variety of actions at a point-of-sale (POS) magnetic stripe reader. Such actions may require that user to spend a particular amount of time. Accordingly, such a user may perform these activities before reaching the POS device. The user's decisions may be communicated through a POS reader output device such as a magnetic emulator/encoder, RFID antenna, and/or IC chip. Accordingly, in doing so, a user may decrease the time the user spends at a POS device. Accordingly, the time it takes to complete a transaction at a POS device can be significantly reduced.

User interfaces, such as capacitive or mechanical buttons, may be included on a card. One or more buttons may be associated with one or more tip amounts. Accordingly, for example, a user may press a particular button and a corresponding percentage may be communicated to a POS reader. Accordingly, a remote server may complete a transaction for the full-amount of the purchase (e.g., total cost, tax, and tip). The remote server may also pre-authorize the transaction for this full-amount such that a user can, for example, easily change the tip if desired. In determining the amount of the tip on the payment card, a user may increase the speed of a transaction as the user may not have to perform any math himself/herself. The user may instead be presented with a receipt that notes the desired tip as well as the total amount that includes the tip. Such a total operation may be performed in numerous locations. For example, a POS reader may recognize the inclusion of tip information in an card output signal (e.g., an RFID, IC chip, and/or magnetic stripe signal).

The POS reader may then perform the associated functions. Alternatively, for example, software located on a cash-register (e.g., a restaurant's cash register) may receive the information from the POS reader and may notice that tip information was included in a card output signal. Accordingly, for example, a card may be branded with indicia corresponding to a particular restaurant chain (e.g., TGIF, Red Robin, or Applebee's) and software may be added to the POS readers and/or cash registers associated with that particular restaurant chain.

A remote server may receive payment information provided to a payment card reader by a payment. This information may include data fields (e.g., discretionary data fields). A remote server may recognize that the received payment information includes user-defined data such as, for example, tip information. Accordingly, the remote server may perform additional processing steps based on this user-defined data. For example, the remote server may determine a tip amount based on received tip information and may authorize a payment card transaction for the amount. The remote server may communicate information back to a POS device indicative of the determined tip amount and/or total authorized amount.

A card may include buttons indicative of particular tip amounts. Alternatively, a card may include numerical buttons and a button indicative of a tip. Accordingly, a user may determine any tip amount that can be defined by the numerical buttons. Furthermore, the numerical buttons may be utilized for executing functionality other than functionality that corresponds to determining and providing tips.

A card is provided in which a user can enter his/her Personal Identification Number (PIN) into the card using one or more user interfaces. This PIN may be communicated in an output signal from a card (e.g., a signal from an RFID antenna, IC Chip, or magnetic emulator/encoder). Accordingly, for example, a user can enter his/her PIN into a card while waiting in a line for an ATM machine. The user can communicate this PIN from the card to the ATM machine using a reader output device on the card. The ATM machine may, for example, recognize that a valid PIN was received from the card and may provide the user with a welcome screen instead of a screen requesting the entry of a PIN.

Similarly, PIN-based purchases may be made where a user enters his/her PIN on a card instead of enters his/her PIN on a POS device. Furthermore, a user may utilize an on-card PIN entry instead of, for example, an on-receipt signature. Accordingly, for example, a user may purchase a meal at a restaurant. A waitress may present the user with a check. A user may press a button associated to providing an on-card PIN. The user may also press a button associated to a particular tip percentage. Accordingly, for example, the waitress may take possession of the card and may swipe the card through his/her POS device. The PIN may be utilized in lieu of a signature such that the total (including tip) is immediately authorized). The waitress may then, for example, present a receipt to a user that confirms that a financial transaction was completed, that an on-card PIN-based authentication was utilized, and the total amount including a line item for a tip amount.

A system is provided in which a user may utilize a PIN entry instead of a signature to complete a signature-based transaction. The PIN may be, for example, passed-through from a card to a remote server using an output device (e.g., RFID antenna, IC chip, magnetic emulator/encoder). The server may recognize that a card desires utilizing an on-card PIN instead of a signature by looking at a particular character or characters of discretionary data. Furthermore, the server may recognize that a card is one that can perform an on-card PIN functionality by, for example, looking at a particular character or characters of transmitted information. For example, a server may determine that a card includes an on-card PIN functionality by, for example, looking at a number of digits of a user's payment card number (e.g., first six digits of a credit card number) and comparing this data to a corresponding list of partial payment card numbers that include an on-card functionality. Accordingly, if a card is determined to have an on-card PIN functionality (or any pre-POS functionality or other functionality) then the server may look at other characters of the received data (e.g., a particular discretionary data field) to determine the user's execution of the function. Data received by a server may, for example, include the PIN number that a user entered into a card. Alternatively, data received by the server may include information representative of the user entering in the correct PIN onto the card. Accordingly, for example, a card may receive a PIN and determine that the PIN is correct. The card may then send a particular character (e.g., a "1") in a particular discretionary data location to the remote server.

A card with pre-ATM functionality is provided in order to expedite the ATM process when a user is provided with the ability to physically interact with the ATM machine. Accordingly, a user may perform ATM activities on his/her card so that when the user physically interacts with an ATM machine, the time of the interaction is reduced. A card is provided with a button that allows a user to designate that the user desires to withdrawal a particular amount of cash from an ATM. Accordingly, for example, the user may enter in his/her PIN onto a card while waiting in a line to use a particular ATM. The user can also utilize the user interfaces of the card to denote that the user desires a particular amount (e.g., $100) of cash from a particular account of the user's (e.g., checking). Accordingly, a card may communicate this information to an ATM when a user swipes or inserts his/her card into the ATM. Accordingly, a user may be prompted with a confirmation screen to confirm the user's pre-ATM card actions. In doing so, a user may decrease the amount of time he/she spends at an ATM machine.

A user may also be provided with a button that corresponds to a user-defined set of actions. For example, such a button may be indicative of taking a particular amount of money out of a checking account while transferring a particular amount of money from a savings account to that checking account, and not requiring a receipt from the ATM. The user may, for example, determine the actions that are associated with this button through an online website for a particular bank or card issuer. Accordingly, for example, when a user presses the button and the information is communicated to a reader (e.g., at an ATM), the remote server may retrieve information indicative of the actions the users desired to perform when the button was pressed. Accordingly, for example, users may customize his/her card via a website.

Additionally, for example, a user may reprogram his/her card with new code on a website. For example, a user may select a particular set of actions for a particular button on a website and the website may direct the user to hold his/her card to a display screen of the user's computer. A portion of the display screen may then flash light to the card. The card may include light sensors to determine characteristics of the flashing light. In doing so, information may be communicated from a website to a card.

A card is provided in which a user can perform pre-cashier checkout activities on his/her card while waiting in a checkout line. For example, a user may enter his/her PIN into a card for a PIN-based card payment as well as select, utilizing one or more user interfaces on the card, a variety of checkout options such as a particular amount of desires cash-back. The user may also determine, for example, that the user does or does not desire a receipt and that the user desires to utilize a particular type of payment (e.g., a credit payment from a credit account). The user's desired decisions may be communicated to a cashier via a cash register connected to a payment card/device reader.

A card is provided that includes a user interface associated with determining whether a user desires a receipt for a particular transaction. In doing so, for example, the information associated with the user's decision on whether the user desires a receipt can be communicated through a payment card reader. In doing so, a cashier may, for example, not need to ask a user whether the user desires a receipt - thus decreasing the time of interaction between the user and the cashier.

Coupons can be loaded into cards in a variety of ways. For example, a user may utilize computer interfaces on a card in order to load a coupon. For example, a user may be provided with a code and may enter that code into his/her card. Coupons, or other information, may also be communicated to a card via a television commercial or show. For example, a commercial for a product may include flashing indicia in a corner of the commercial. A user may hold his/her card up to the corner of the commercial and the information (e.g., coupon) may be communicated to the card. For example, a card may encrypt information based on an encryption algorithm. If this integrity of the algorithm is compromised, a commercial can be provided on national or regional television such that information can be communicated to cards that would change the encryption algorithm the card uses to encrypt data the card provides to payment card readers.

A card is provided that includes buttons associated with items. For example, a card may include a button associated with a particular type of drink (e.g., cola) and/or a particular types of snack (e.g., potato chips). A user that is waiting in line at a vending machine may, for example, press the button on his/her card associated with a particular item that the user desires to purchase. Accordingly, a user may swipe his/her card through a payment card magnetic stripe reader and the vending machine may receive payment information in addition to item ordering information. Accordingly, the vending machine may receive the desired order information, execute the order (e.g., vend the user a bottle of cola), and complete a payment transaction based on the amount of the ordered item and the payment card information provided by the payment card to the vending machine.

Displays may be provided near user interfaces or other structures. For example, a display may be provided next to an LED. Cards may be programmed during manufacturing so that these displays may display particular information. Accordingly, for example, the same card architecture may be utilized to provide a number of different types of cards. A user may utilize user interfaces (e.g., mechanical or capacitive interfaces) to change the function of the display. For example, codes may be entered to reconfigure the displays. Alternatively, for example, a user may utilize buttons to select information to be displayed on displays associated with user interfaces. A code may associate a name of a store with a button and/or a dollar amount. For example, a display may be configured to read "Target $50." Information may be entered manually, but also may be received by a card. For example, a user may swipe a card a second time through a magnetic stripe reader and receive information via a magnetic emulator. This received information may be utilized to update information on the card (e.g., the balance of a gift card, credit account, and/or debit account). Information may also be received by an RFID antenna and/or IC chip located on a card and in communication with a central processor (or distributed processors). For example, transaction information (e.g., list of past transactions, stores where transactions occurred, amounts of transactions) and account information (e.g., balance information, bill information, amount due information) may be communicated to the card and displayed on one or more displays.

A dynamic card may be manufactured in a variety of ways. For example, a dynamic card may be printed onto a flexible material (e.g., a flexible polymer). Multiple layers of this material may be bonded together to form a multiple layer flexible structure. This multiple layer structure may be laminated (e.g., via hot, warm and/or cold lamination) to form a card. The card may be programmed before or after lamination. A card may be programmed via a direct connection between a programmer and one or more contacts on a card. A card may be programmed via a capacitive, optical, or inductive communication via a communication link between a programmer and one or more components (e.g., a contact) on a card. Accordingly, for example, a card may be laminated and capacitively, optically, or inductively programmed. After programming, a processor on the card may be signaled to burn-out its programming communication channel(s) such that no further programming may occur. A portion of the card may not be laminated. Accordingly, a programmer may connect to this non-laminated portion of the card. The non-laminated portion of the card may be laminated after programming. Alternatively, for example, the non-laminated portion of the card may be cut after programming (e.g., and after the processor burns-out its programming ports so the processor cannot be further programmed).

Additional external communication devices may be provided on a card. For example, a USB port or Wi-Fi antenna may be provided on a card. Such additional external communication devices may, for example, allow a user to communicate with stationary computer, laptop, or other device. Such communication devices may, for example, be utilized to load gift cards, or other information (e.g., transactional or account information) from a laptop to a card or other device. A card is provided that includes a light sensor such that information can be communicated to a card via light (e.g., via a light transmitted from a TV or website).

Components of card may be oriented in a number of ways in order to increase the whimsical and festive nature of a card. Furthermore, the orientation of components may increase the functionality of a card. For example, buttons may be placed on the left-side of a card, but left-handed and right-handed users may naturally operate such buttons differently. Furthermore, buttons may be provided on the center of the card in order to increase the operational similarity of the buttons between left and right-handed users.

Numerous components may be placed on the front or back of a card or other device (e.g., a mobile telephonic device). For example, any number of displays, user interfaces such as buttons, sources of light such as LEDs, vibrational devices, sources of audible signals, microphones, power generating devices, sources of electrical energy, RFID antennas, processors, IC chips, or magnetic communication devices (e.g., magnetic emulators and encoders) may be provided on a card.

Indicia may be provided on the front and back of a card in order to increase the whimsical and festive nature of the card. Such indicia may also increase the functionality of a card. For example, a button may include a written letter (e.g., "A") and multiple numbers (e.g., "0" and "1"). Accordingly, a user may be issued with a letter or numerical PIN number and may utilize the same set of buttons regardless of whether the user received a numerical PIN or a letter-based PIN. In doing so, for example, a ten digit PIN may be represented by less than 10 buttons.

A loyalty-based payment application may be provided on a card. For example, a user may earn reward points when that user purchases an item using the payment card. In this manner, a user may earn reward points based on the type of item, the dollar amount of the item, and/or the time during which the item was purchased. A remote server may receive information indicative of reward points that a particular user has earned. This remote server may also transmit information back to a card (e.g., via a payment card reader). Such information may include the total amount of reward points that have been earned by a particular reader. The total amount of reward points may be stored on a payment card and displayed to a user via a display located on the card automatically after each transaction and/or as a result of manual input.

A user may provide manual input to a card in order to instruct the card to pay for a purchase using reward points. A purchase may be subsequently authorized in a variety of ways. For example, a flag may be placed in payment information that is communicated through a payment card reader that is indicative of a user's desire to utilize reward points for payment. Such a flag may take the form of a particular character or set of characters in a particular location of payment information. A remote server may then, for example, look for a particular character, or set of characters, in received payment information to determine whether payment is desired to be made by reward points. Alternatively, for example, a different payment account number may be communicated when reward points are desired and a remote server may utilize this different payment account number to authorize a payment transaction. Payment information that is communicated may be encrypted in a variety of ways. For example, all or part of the payment information may be encrypted for each transaction, which may be determined via manual input or read-head detectors, or based on time.

Multiple different types of rewards may be earned on a card. For example, a user may be provided with the option of earning reward points, airline miles, receive cash-back, or donating a purchase-based value to charity. In this manner, a card may be provided with a set of buttons where each button corresponds to a different type of reward. Additional buttons may be provided for additional functionalities (e.g., the entry of a user's Personal Identification Number). Before a purchase, a user may select the type of rewards that the user would like to earn for the purchase. Data corresponding to the selection may be provided in payment information communicated to a payment card reader. Such rewards information may take many forms. For example, data indicative of the selection may be provided as discretionary data. Alternatively, for example, a different account number may be communicated for each type of reward.

Payment information may be communicated in a variety of ways. For example, information indicative of the type of reward that is desired or the form of payment may be communicated via an IC chip, RFID antenna, and magnetic emulator or encoder. Payment information may be structured differently for each type of communication and, similarly, may include overlapping as well as different data. For example, data indicative of the type of reward desired may be provided as discretionary data in both track 1 and track 2 of a magnetic emulator. However, for example, data indicative of the type of reward desired may be provided as a different account number for a transaction based off an RFID signal from an RFID antenna. Data may be stored on a memory and constructed by a processor such that the payment information may delivered via a reader communication device.

A magnetic emulator may produce an electromagnetic field that is operable to be read by a magnetic stripe reader. Such a magnetic emulator may include a coil. Current may be provided through such a coil such that an electromagnetic signal is produced. Material may be placed inside the coil with a permeability that results in an increase of the electromagnetic signal about the exterior of the coil. Such a material may be, for example, a soft-magnetic material (e.g., a permalloy). Such a soft-magnetic material may not be able to, for example, be permanently magnetized.

A magnetic emulator having a coil with a soft-magnetic interior may be fabricated in a printed circuit board process (e.g., using an FR4 board material). In doing so, for example, the electromagnetic field located about the exterior of the coil may be increased by the presence of the soft-magnetic interior.

Magnetostrictive materials may be provided about the interior of a coil. Magnetostrictive materials may mechanically distort in response to a magnetic field. This mechanical distortion may, in turn, affect the magnetic field. Magnetic emulators are provided with coils having magnetostrictive interior materials. An aperture may be cut into a printed circuit board layer such that the magnetostrictive material is operable to mechanically distort within the aperture while in a multiple layer printed circuit board.

Non-magnetostrictive material may be utilized as an interior material for a coil operable to communicate data to a magnetic stripe reader. Such a non-magnetostrictive material may have zero parts per million of magnetostrictive elements or may have a low amount of magnetostriction (e.g., less than 200 parts per million).

A permanent magnet may be placed about a magnetic emulator. For magnetic emulators that include one or more coils, a permanent magnet may be placed about the interior, or exterior, of one of the coils. Such a permanent magnetic may provide, for example, a bias magnetic field that may increase the amount of electromagnetic field present about the exterior of a coil. A bias field may also be created, for example, via a coil (e.g., a coil about a magnetic emulator).

A magnetic emulator may include one or more coils that include both a soft-magnetic material and a permanent magnet.

### Brief Description of the Drawings

The principles and advantages of the present invention can be more clearly understood from the following detailed description considered in conjunction with the following drawings, in which the same reference numerals denote the same structural elements throughout, and in which:
FIG. 1 is an illustration of cards constructed in accordance with the principles of the present invention;
FIG. 2 is an illustration of cards constructed in accordance with the principles of the present invention;
FIG. 3 is an illustration of cards constructed in accordance with the principles of the present invention;
FIG. 4 is an illustration of cards constructed in accordance with the principles of the present invention;
FIG. 5 is an illustration of cards constructed in accordance with the principles of the present invention;
FIG. 6 is an illustration of a card constructed in accordance with the principles of the present invention;
FIG. 7 is an illustration of a card constructed in accordance with the principles of the present invention;
FIG. 8 is an illustration of a personal electronic device constructed in accordance with the principles of the present invention;
FIG. 9 is an illustration of cards constructed in accordance with the principles of the present invention;
FIG. 10 is an illustration of cards constructed in accordance with the principles of the present invention;
FIG. 11 is an illustration of cards constructed in accordance with the principles of the present invention;
FIG. 12 is an illustration of emulators constructed in accordance with the principles of the present invention;
FIG. 13 is an illustration of process topologies constructed in accordance with the principles of the present invention;
FIG. 14 is an illustration of cards constructed in accordance with the principles of the present invention;

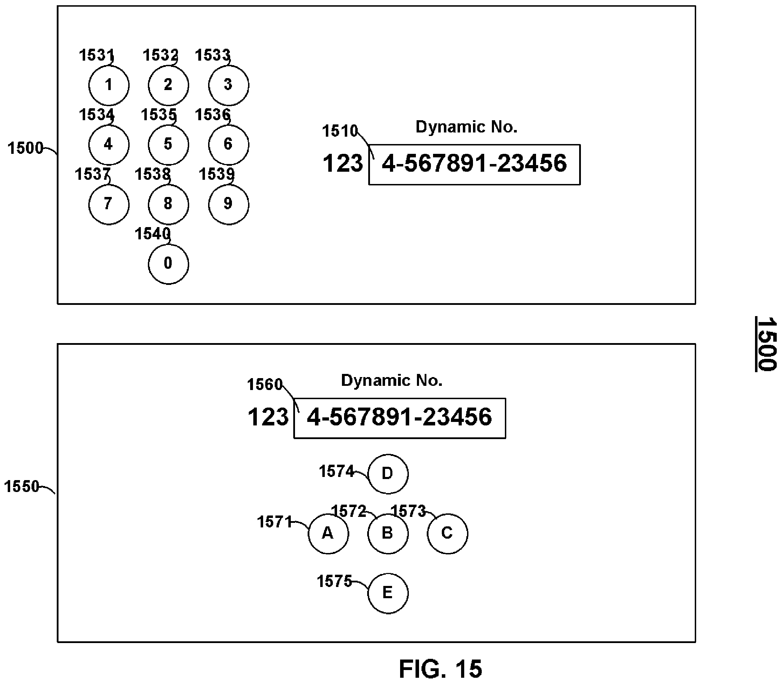

FIG. 15 is an illustration of cards constructed in accordance with the principles of the present invention;

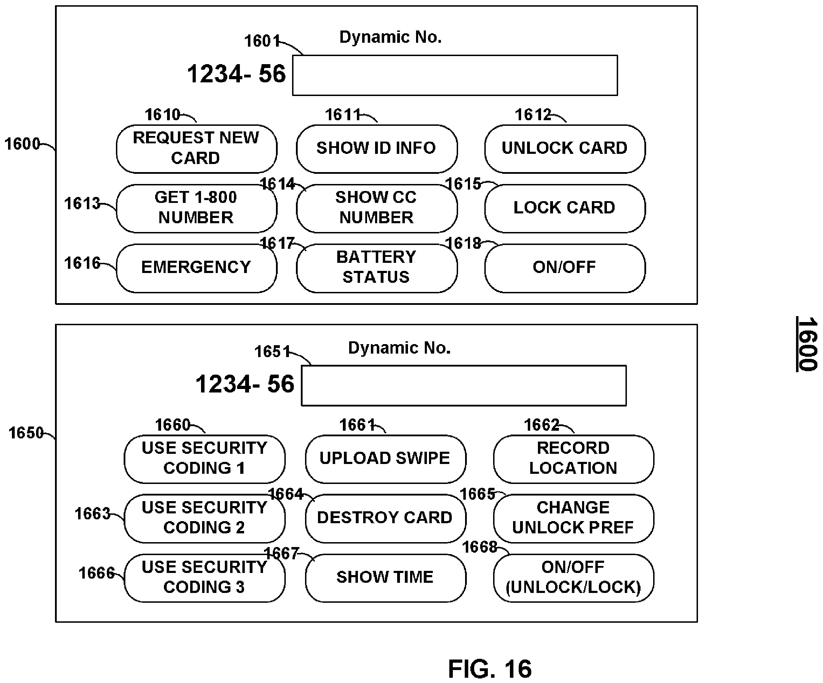

FIG. 16 is an illustration of cards constructed in accordance with the principles of the present invention;
FIG. 17 is an illustration of cards constructed in accordance with the principles of the present invention;
FIG. 18 is an illustration of process topologies constructed in accordance with the principles of the present invention;
FIG. 19 is an illustration of cards constructed in accordance with the principles of the present invention;
FIG. 20 is an illustration of a personal electronic device constructed in accordance with the principles of the present invention;
FIG. 21 is an illustration of cards constructed in accordance with the principles of the present invention;
FIG. 22 is an illustration of cards constructed in accordance with the principles of the present invention;
FIG. 23 is an illustration of cards constructed in accordance with the principles of the present invention;

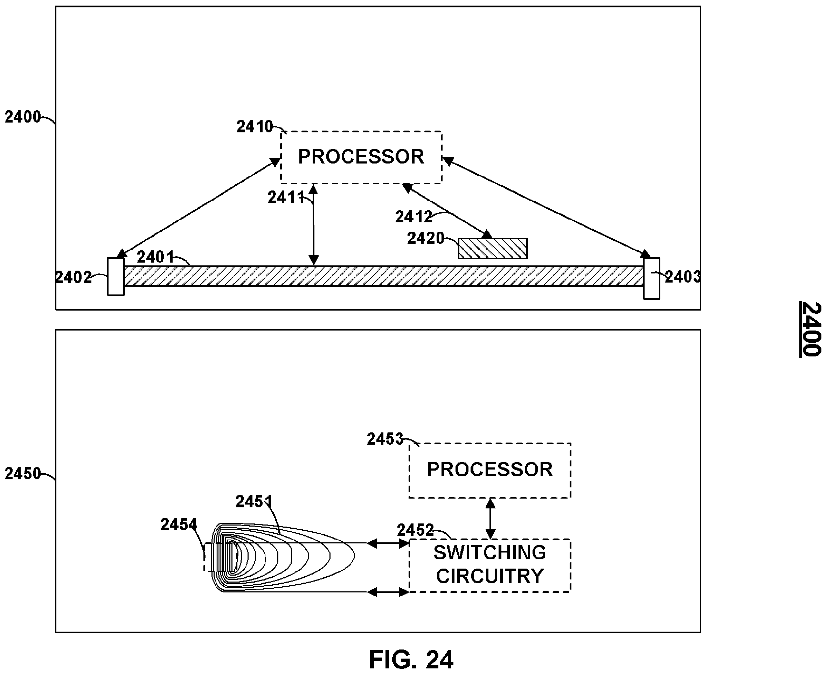

FIG. 24 is an illustration of cards constructed in accordance with the principles of the present invention;
FIG. 25 is an illustration of process flow charts constructed in accordance with the principles of the present invention;
FIG. 26 is an illustration of the electrical coupling between a card and a reader constructed in accordance with the principles of the present invention;
FIG. 27 is an illustration of the electrical coupling between a card and a reader constructed in accordance with the principles of the present invention;

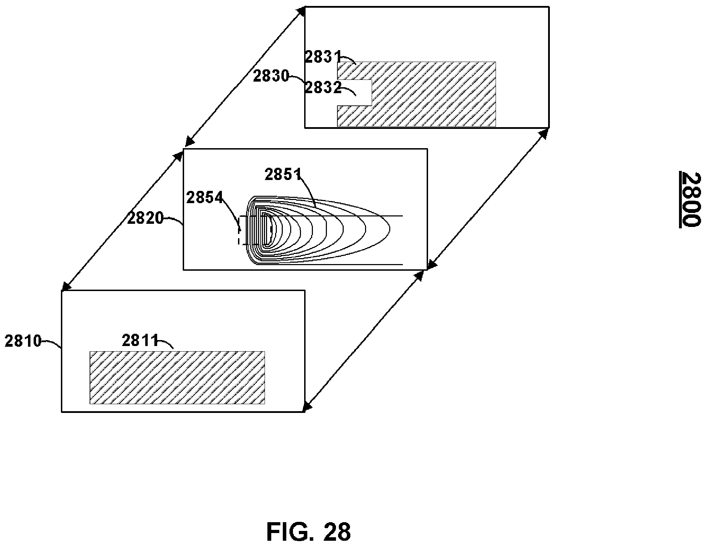

FIG. 28 is an illustration of magnetic shielding in accordance with the principles of the present invention;
FIG. 29 is an illustration of process flow charts constructed in accordance with the principles of the present invention;
FIG. 30 is an illustration of a personal electronic device constructed in accordance with the principles of the present invention;
FIG. 31 is an illustration of a card constructed in accordance with the principles of the present invention;
FIG. 32 is an illustration of a circuit constructed in accordance with the principles of the present invention;
FIG. 33 is an illustration of a circuit constructed in accordance with the principles of the present invention;
FIG. 34 is an illustration of a circuit constructed in accordance with the principles of the present invention;
FIG. 35 is an illustration of a circuit constructed in accordance with the principles of the present invention;
FIG. 36 is an illustration of a circuit constructed in accordance with the principles of the present invention;
FIG. 37 is an illustration of a circuit constructed in accordance with the principles of the present invention;
FIG. 38 is an illustration of a circuit and flow charts constructed in accordance with the principles of the present invention;
FIG. 39 is an illustration of a circuit and flow charts constructed in accordance with the principles of the present invention;
FIG. 40 is an illustration of a card constructed in accordance with the principles of the present invention;
FIG. 41 is an illustration of a circuit constructed in accordance with the principles of the present invention;
FIG. 42 is an illustration of a card constructed in accordance with the principles of the present invention;
FIG. 43 is an illustration of a circuit constructed in accordance with the principles of the present invention;
FIG. 44 is an illustration of a circuit constructed in accordance with the principles of the present invention;
FIG. 45 is an illustration of a circuit constructed in accordance with the principles of the present invention;
FIG. 46 is an illustration of a circuit constructed in accordance with the principles of the present invention;
FIG. 47 is an illustration of a circuit constructed in accordance with the principles of the present invention;
FIG. 48 is an illustration of a circuit constructed in accordance with the principles of the present invention;
FIG. 49 is an illustration of a circuit constructed in accordance with the principles of the present invention;

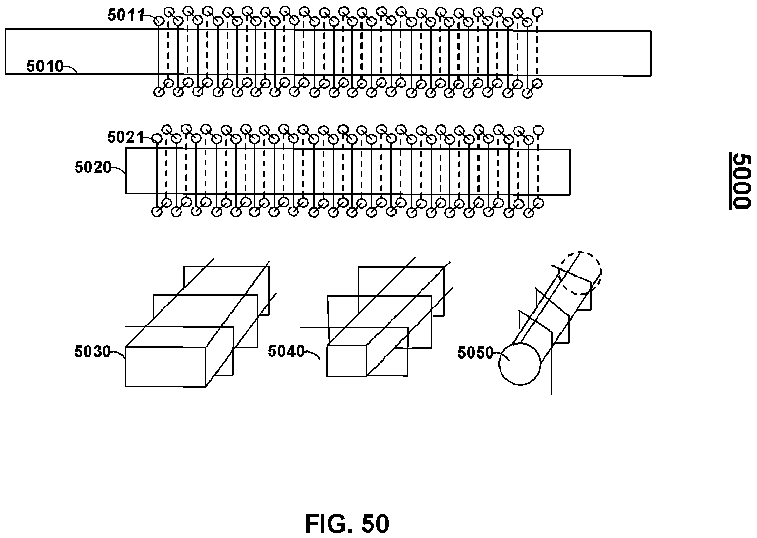

FIG. 50 is an illustration of a circuit constructed in accordance with the principles of the present invention;
FIG. 51 is an illustration of a card constructed in accordance with the principles of the present invention;
FIG. 52 is an illustration of a card constructed in accordance with the principles of the present invention;
FIG. 53 is an illustration of a cards constructed in accordance with the principles of the present invention;
FIG. 54 is an illustration of a webpage constructed in accordance with the principles of the present invention;
FIG. 55 is an illustration of a language scheme constructed in accordance with the principles of the present invention;
FIG. 56 is an illustration of process flow charts constructed in accordance with the principles of the present invention;
FIG. 57 is an illustration of process flow charts constructed in accordance with the principles of the present invention;
FIG. 58 is an illustration of cards constructed in accordance with the principles of the present invention;
FIG. 59 is an illustration of cards constructed in accordance with the principles of the present invention;
FIG. 60 is an illustration of cards constructed in accordance with the principles of the present invention;
FIG. 61 is an illustration of cards constructed in accordance with the principles of the present invention;
FIG. 62 is an illustration of a card and a payment process constructed in accordance with the principles of the present invention;
FIG. 63 is an illustration of a payment card constructed in accordance with the principles of the present invention;
FIG. 64 is an illustration of a payment card with multiple user interfaces constructed in accordance with the principles of the present invention;
FIG. 65 is an illustration of a payment card constructed in accordance with the principles of the present invention;
FIG. 66 is an illustration of a card and a portion operable to receive a written signature constructed in accordance with the principles of the present invention;
FIG. 67 is an illustration of a payment process and a graphical user interface constructed with the principles of the present invention;
FIG. 68 is an illustration of graphical user interfaces constructed in accordance with the principles of the present invention;
FIG. 69 is an illustration of a payment card constructed in accordance with the principles of the present invention;
FIG. 70 is an illustration of a flow chart of a payment process and a graphical user interface associated with the principles of the present invention;
FIG. 71 is an illustration of a flow chart of a payment process and a communications interface constructed in accordance with the principles of the present invention;
FIG. 72 is an illustration of a card constructed in accordance with the principles of the present invention;
FIG. 73 is an illustration of a card that includes user interfaces for ordering items constructed in accordance with the principles of the present invention;

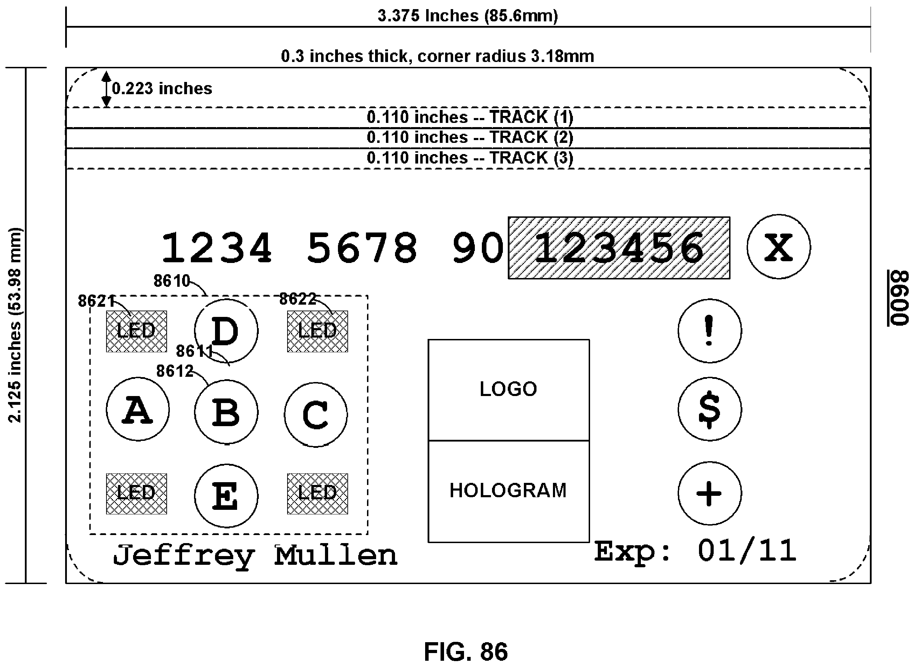

FIGS. 74-116 are illustrations of cards constructed in accordance with the principles of the present invention;
FIG. 117 is an illustrations of a card and a personal electronic device constructed in accordance with the principles of the present invention;
FIG. 118 is an illustrations of a card and a payment process constructed in accordance with the principles of the present invention;
FIG. 119 is an illustration of a card constructed in accordance with the principles of the present invention;
FIG. 120 is an illustration of a card constructed in accordance with the principles of the present invention;
FIG. 121 is an illustration of a card constructed in accordance with the principles of the present invention;
FIG. 122 is an illustration of a card constructed in accordance with the principles of the present invention;
FIG. 123 is an illustration of a card constructed in accordance with the principles of the present invention;
FIG. 124 is an illustration of a card constructed in accordance with the principles of the present invention;
FIG. 125 is an illustration of a card constructed in accordance with the principles of the present invention;
FIG. 126 is an illustration of a card constructed in accordance with the principles of the present invention;
FIG. 127 is an illustration of a webpage constructed in accordance with the principles of the present invention;
FIG. 128 is an illustration of a webpage constructed in accordance with the principles of the present invention;
FIG. 129 is an illustration of a card constructed in accordance with the principles of the present invention;
FIG. 130 is an illustration of a card constructed in accordance with the principles of the present invention;
FIG. 131 is an illustration of payment flow charts constructed in accordance with the principles of the present invention;
FIG. 132 is an illustration of a network topology constructed in accordance with the principles of the present invention;
FIG. 133 is an illustration of cards constructed in accordance with the principles of the present invention;
FIGS. 134 is an illustration of a personal electronic device constructed in accordance with the principles of the present invention.
FIG. 135 is an illustration of control signals and magnetic stripe reader sense signals constructed in accordance with the principles of the present invention;
FIG. 136 is an illustration of coils constructed in accordance with the principles of the present invention;
FIG. 137 is an illustration of a card manufacturing process constructed in accordance with the principles of the present invention;
FIG. 138 is an illustration of a card manufacturing process constructed in accordance with the principles of the present invention;
FIG. 139 is an illustration of cards constructed in accordance with the principles of the present invention;
FIG. 140 is a flow chart constructed in accordance with the principles of the present invention;
FIG. 141 is an illustration of a card constructed in accordance with the principles of the present invention;
FIG. 142 is an illustration of a card constructed in accordance with the principles of the present invention;
FIG. 143 is an illustration of control signals constructed in accordance with the principles of the present invention
FIG. 144 is a schematic of a drive circuit constructed in accordance with the principles of the present invention; and
FIG. 145 is an illustration of a card constructed in accordance with the principles of the present invention.

### Detailed Description of the Invention

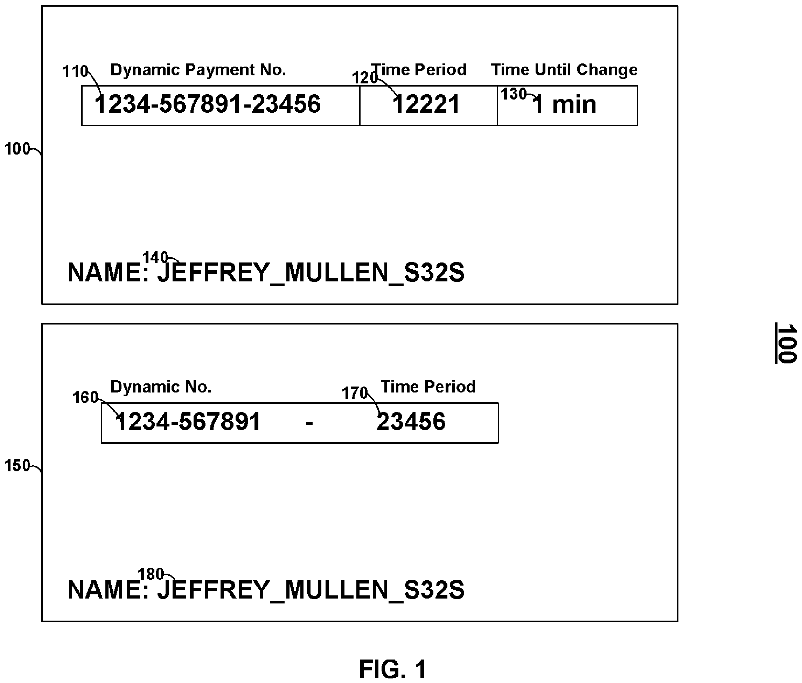

FIG. 1 shows card 100 that may include a display that displays dynamic credit card number 110, time period 120, and time until change information 130.

Identification information 140 may also be included on card 100. Identification information 140 may be permanently provided on card 100 or may be displayed on a display (e.g., the display that displays dynamic credit card number 110). For example, identification information 140 may be printed onto card 100 or embossed on card 100.

Dynamic credit card number 110 may be changed periodically. Additionally, a number may be changed right after a user enters a number into an online checkout process - but before that online checkout process completes. Accordingly, a particular number of dynamic credit card number 110 may, for example, be valid during time period 120 in which the particular number was generated. Dynamic numbers may, or may not, be repeated. The numbers that are valid for a particular period of time may be, for example, a numbers generated in adjacent time slots (e.g., the time slot or time slots before and after the generation of the displayed number).

A display may be bi-stable or non bi-stable. A bi-stable display may consume electrical energy to change the information displayed on the bi-stable display but may not consume electrical energy to maintain the display of that information. A non bi-stable display may consume electrical energy to both change and maintain information on the non bi-stable display. A display driving circuit may be provided, for example, for a bi-stable display (or a non bi-stable display). Such a display driving circuit may step-up a supply voltage (e.g., 1-5 volts) to a larger voltage (e.g., 6-15 volts) such that a bi-stable display may change displayed information. A controller (e.g., a processor) may be utilized to control such a display driving circuit.

Persons skilled in the art will appreciate that a particular number may be verified by a verification system (e.g., a credit card verification server) during a time period not associated with the particular number. In such instances, for example, the particular number may be transmitted along with the time period in which the particular number was generated. Accordingly, the particular number may be verified as being generated during the time period by the verification server. In instances of fraud, the cost of fraud may be transferred to the entity that provided the particular number and time period. In this manner, the liability for fraud may be transmitted to the entity that stores the particular number and time period for later use. In doing so, entities may be encouraged to, for example, only transmit current dynamic credit card numbers. The cost of fraud may be transferred, for example, to the association or bank that issued the card.

A website may include, for example, a text box that would allow a user to provide timing information 120. A different coding scheme may also be associated with each, or with a group of, different time periods. A credit card verification system may include a timing circuit that is in sync with a timing circuit on card 100 such that both the verification system and card 100 are aware of the current time period of card 100. A time period may be, for example, on the order of minutes, hours, or days. For example, a time period may be approximately 36 hours in duration.

Time until change information 130 may be provided on a display and may be representative of the amount of time remaining before number 110 changes.

Identification information 140 may be provided on card 100. Identification information 140 may be provided such that each card 100 has different identification information 140. Accordingly, even though to users may have the same name, each user may have different identification information 140. In doing so, a user may enter in identification information 140 on a text box at an online store and a verification system may, for example, be able to identify a particular user based on identification information 140. A number may include one or more digits representative of information that may be utilized in an identification process. For example, two digits may be representative of a partition. Users having the same name may be assigned different partitions. Accordingly, the name on a card may be associated with the name of a person. People with the same name may have the same identification information. However, such people with the same name may be recognized by having different identification digits. Two identification digits in a credit card number may allow for 100 partitions. Similarly, identification information may be provided in a dynamic credit card number. The digits for identification may change location each period or particular groups of periods as well as may be coded differently each period or particular groups of periods. Identification digits may be static (e.g., printed on a credit card or statically displayed so the digits do not change). In printing a static number of a card, for example, the amount of power needed to display a credit card number may be reduced.

Persons skilled in the art will appreciate, for example, that at least the first six digits of payment card number may remain static and may be utilized to route at least the rest of the payment card number to the appropriate verification network. Portions of remaining digits may remain static and may be utilized for identification while other remaining digits may be dynamic.

Card 150 may be provided and may include a display that includes a dynamic number with portion 160 that corresponds to a credit card number and portion 170 that corresponds to a time period. Persons skilled in the art will appreciate that other portions may be provided in such a number or that a particular portion may include particular information. For example, user identification information may be utilized as part of portion 160. Persons skilled in the art will appreciate that a static number may be permanently provided on a card (e.g., via printing or embossing). Alternatively, for example, a display may display a particular digit or digits time the display displays a card number. Persons skilled in the art will appreciate that a display may be configured to display numerical data or alphanumerical data. A display may also be configured to display other indicia (e.g., the image of a battery and its remaining life).

Persons skilled in the art will appreciate that portions 160 and 170 may take the form of a credit card number (e.g., a fifteen digit number that is transmitted as a credit card number to a credit card verification server). Additionally, any digit or digits of a dynamic credit card number may be static. For example, at least the first digit may be static. In doing so, for example, a credit card verification system may be able to identify a credit card number as being a dynamic credit card number.

A credit card number with three digits allocated for a time period associated with the generated number may, for example, be sufficient to provide enough time periods for a card's life. For example, suppose a time period is associated with a day. Three digits would provide for 1000 days of operation. As a card may be provided with a magnetic emulator or magnetic stripe encoder, additional information may be transmitted to a card verification system. For example, user identification information and time period information not embodied as a credit card number may be transmitted to a remote credit card verification system via a local magnetic stripe reader.

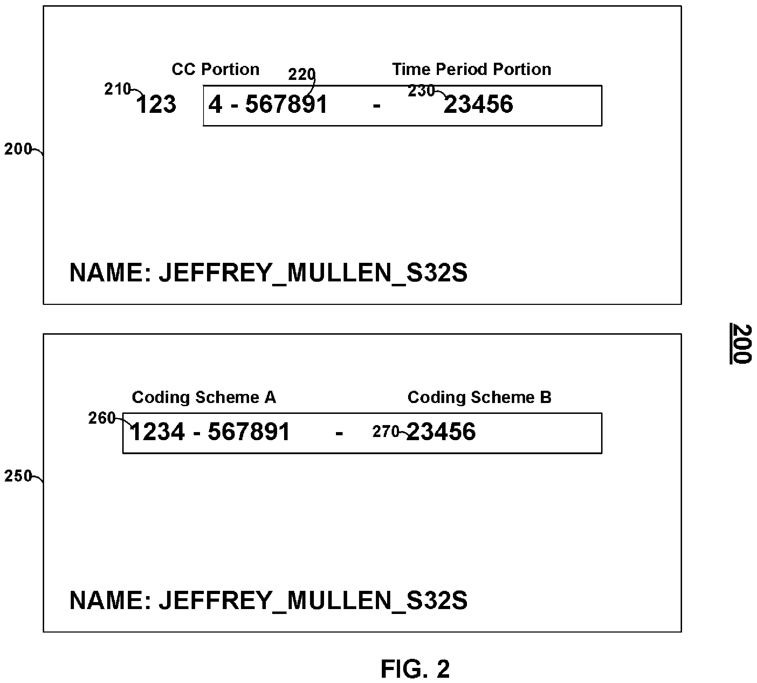

FIG. 2 shows card 200 that includes a credit card number having static portion 210, displayed portion 220, and displayed portion 230. Static portion 123 may be utilized such that a credit card verification system is able to recognize that a credit card number is a dynamic credit card such as an American Express dynamic credit card. Portion 220 may be dynamic and may periodically change. In doing so, the amount of fraud associated with card number theft may be reduced. Portion 230 may be representative of a time period for portion 220. Accordingly, identification of a number being a dynamic card number, a time stamp, and a dynamic number may all be transmitted as a 15-digit credit card number. Persons skilled in the art will appreciate that a payment card number may have different lengths. For example, a payment card number may be 19 digits in length or 16 digits in length. Persons skilled in the art will appreciate that a time period, for example, does not have to be transmitted. Systems may, for example, assume that a card is synchronized to a verification server. Similarly, transmission of a time stamp may be optional. As such, for example, a time stamp may be transmitted when one-click shopping is utilized by a particular online store, but a time stamp may not be needed, for example, when a purchase is made and a credit card number is entered for immediate processing.

Card 250 includes a number, which may be utilized as a credit card number, which includes portion 260 and 270. Portion 260 may include, for example digits that may be representative of the type of number displayed (e.g., a dynamic credit card number). Portion 260 may also include a dynamic number that may be utilized by a credit card processing system to a make a purchase on credit. Portion 270 may, for example, include digits associated with a time period for a dynamic credit card number. Portions 260 and 270 may be coded by different coding procedures. Accordingly, if the integrity of one procedure is compromised, a dynamic credit card may still provide security if, for example, the integrity of the other coding procedure is still intact.

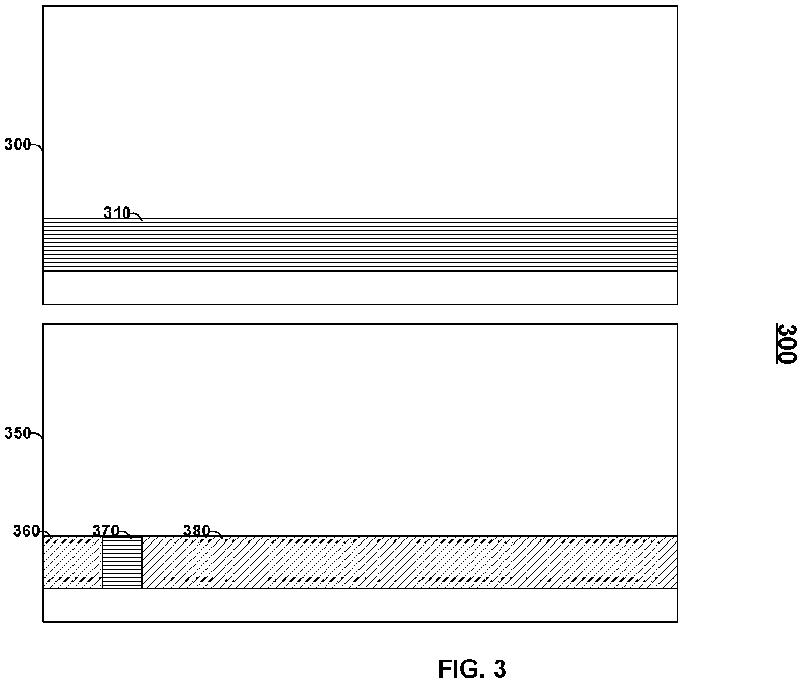

FIG. 3 shows credit card 300 may include structure 310 which may be, for example, a dynamic magnetic communications device such as a magnetic stripe encoder or a magnetic emulator. A magnetic stripe encoder may, for example, erase and re-write information to a magnetic stripe. A magnetic emulator may, for example, generate electromagnetic fields that are able to be read by a magnetic stripe reader.

Persons skilled in the art will appreciate that a button may be included that may provide a variety of capabilities. For example, a button, when pressed, may cause a magnetic stripe encoder to write a number (e.g., the credit card number generated at the time the button is pressed) to the magnetic stripe. This number may also be displayed on a display. As per another example, a button, while pressed, may cause a magnetic emulator to turn ON and emulate fields. A magnetic emulator or encoder may comprise a number of wires. A current may be flowed through the wires at particular strengths and polarities to generate fields capable of, for example, writing to a magnetic stripe or being read by a magnetic stripe reader.

Card 350 may include magnetic stripe portions 360 and 380 as well as magnetic encoder or emulator 370. Persons skilled in the art will appreciate that only a particular portion of information that needs to be transmitted may need to change. Accordingly, a dynamic encoder or emulator does not, for example, need to be utilized to emulate or encode all of the transmitted information. An encoder and/or emulator may be utilized, for example, to communicate a dynamic credit card number or a portion of a dynamic credit card number to a credit card reader. Similarly, an encoder and/or emulator may be utilized, for example to communicate a time period, dynamic feedback (e.g., whether the battery is LOW or the card has been breached by a thief), or identification information.

A rechargeable battery may be provided such that, for example, the card may be inserted into a device capable of recharging the rechargeable battery.

A magnetic credit card stripe may have multiple tracks. One or more magnetic encoders and/or emulators may be utilized to communicate information on one or more (e.g., all) tracks). Persons skilled in the art will appreciate that different tracks may be provided with a different bit density (e.g., bits-per-inch). Accordingly, the spacing of wires on emulators/encoders may be different depending on the type of track the emulator/encoder is attempting to emulate/encode.

An encoder and/or emulator may be placed adjacent to a magnetic stripe or underneath a magnetic stripe. For example, a particular portion of a particular track may be cut-out from a multiple track magnetic stripe and an emulator may be placed in this cut-out portion on a card. A magnetic encoder and/or emulator may be placed, for example, before a magnetic stripe, after a magnetic stripe, or with a magnetic stripe.

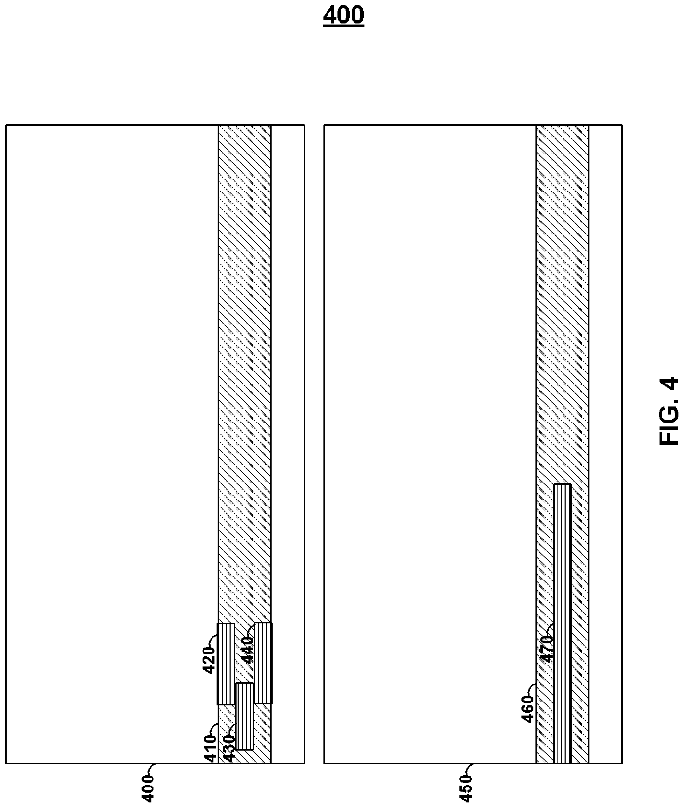

FIG. 4 shows card 400 that may include magnetic stripe 410, magnetic emulator 420, magnetic emulator 430, and magnetic emulator 440. Magnetic emulators 420, 430, and 440 may, for example, be utilized to emulate similar information, but on different tracks of a multiple track magnetic stripe. Similarly, magnetic emulators 420, 430, and 440 may be placed on different horizontal portions of their respective tracks so that each emulator provides similar information on each track. For example, suppose that a middle track includes a higher bit density then exterior tracks (e.g., in a three-track embodiment). Then, for example, magnetic emulator 430 may be located in a different location then emulators 420 and 440. Similarly, the two exterior tracks in a three-track magnetic stripe may be have a higher bit density then a middle track. Additionally, for example, different tracks may have data formatted in different ways. Accordingly, similar information on each track may be located in different areas due to formatting.

The exterior tracks in a three-track configuration (e.g., tracks 1 and 3) may, for example, be provided at 210 bits per inch. The middle track in such a configuration (e.g., track 2) may be provided at 75 bits per inch. Persons skilled in the art will appreciate that, for example, a magnetic stripe encoder may be utilized instead of a magnetic emulator. Magnetic stripe encoders may, for example, be placed under a portion of a magnetic stripe. Persons skilled in the art will appreciate that a payment card may be provided with any number of tracks. For example, a payment card may be provided with two tracks (e.g., tracks 1 and 2). Different tracks may include similar data (e.g., card numbers) as well as different data. For example, one track (e.g., track 1, which may be the track closest to the bottom of the card) may include name information while another track (e.g., track 2, which may be located above track 1).

Card 450 may include magnetic stripe 460 and magnetic encoder and/or emulator 470. Magnetic stripe 460 may have three tracks of data. As such, card 450 may include multiple tracks (e.g., three) and magnetic encoder and/or emulator 470 may, for example, form a portion of one of these tracks. The rest of the tracks may, for example, be provided as a magnetic stripe without a magnetic encoder/emulator. A single track may include, for example, any number of magnetic emulators and/or magnetic encoders.

For example, a single track may include a magnetic emulator (or encoder) at the beginning and end of a track such that the middle of the track is provided by a magnetic stripe. Similarly, a single track may include a magnetic emulator (or encoder) at, for example, just the beginning of a track. Similarly still, a single track may include a magnetic emulator (or encoder) at, for example, just the end of a track. A magnetic encoder or emulator located at an end of a magnetic stripe may, for example, communicate a string of a particular bit (e.g., zeros) to aid a magnetic reader's reading of magnetic stripe information. A magnetic stripe reader may be configured to, for example, determine the rate at which bits are being communicated by looking at a string of zeros provided on a card before, and after, payment card information. Persons skilled in the art will appreciate that a card may provide magnetic stripe information either in a forward configuration or backwards configuration. For example, a magnetic emulator, or encoder, may provide payment information in reverse order. Accordingly, a payment number may be communicated in payment information from its least significant digit to its most significant digit instead of from, for example, its most significant digit to its least significant digit. Persons skilled in the art will appreciate that magnetic stripe data may, for example, be provided as characters. Numerical data may be, accordingly, described in such magnetic stripe data as characters. In such instances, for example, a particular character of data may be utilized to just describe numerical data. Routing servers may, for example, look for such numerical data. Persons skilled in the art will appreciate that additional states may be provided in characters that are not used. Such additional states may be utilized to communicate, for example, additional information while still communicating numerical data. For example, a character that includes twenty or more states may be utilized to describe a digit as well as an extra bit of data. The first and eleventh character may correspond, for example, to the same digit (e.g., "0"). However, for example, the selection of the first and eleventh states may be utilized to transmit additional information. Such additional information may be, for example, a piece of the timing information or information indicative of a coding scheme used. Accordingly, a remote server (e.g., a routing or verification server) may be able to extract both digit information as well as additional information for a character associated with a digit under a particular data structure (e.g., an American Express credit card number format) and communicate these pieces of data to another server or different servers. The inclusion of additional data may be provided, for example, for any character of a data structure that has a number of states greater than the number of states used to describe its corresponding information under that structure. Different tracks of data may include, for example, different types of characters that utilize a different number of states. For example, a track (e.g., track 1) may include 7-bit characters while a different track (e.g., track 2) may include 5-bit characters. Characters as well as tracks of data may include, for example parity bits as well as different types of sentinels (e.g., start and end sentinels). A track of data may be provided with a character for a Longitudal Redundancy Check (LRC). A processor may encrypt, for example, entire tracks of data based on time or use. A processor may encrypt, for example, one or all tracks of data provided on a card.

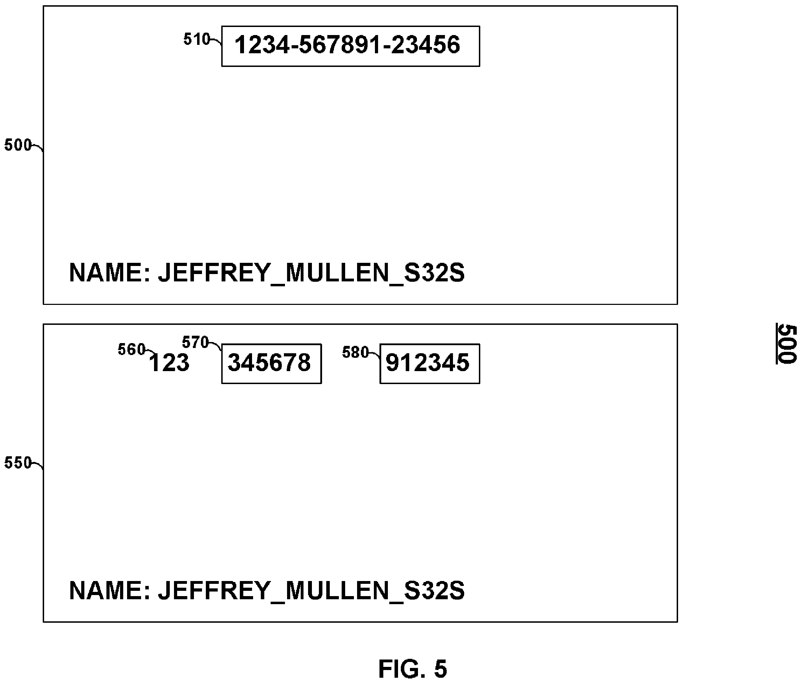

FIG. 5 shows card 500 that includes display 510. Display 510 may, for example, be located near the top of a card 500. Display 510 may also be, for example, provided in the middle of the top of card 500 or off-set from the middle of card 500 (e.g., either left or right of the middle). In doing so, for example, the amount of space beneath the display may be maximized. Accordingly, the size of particular components may be increased. For example, a battery may be placed beneath display 510 and the location of a display at the top of card 500 may allow for a battery of increased size. By increasing the size of a battery, the life of card 500, without a recharge, may be increased. One or more batteries may be utilized in card 500.

Card 550 may include display 570 and 580 that may provide part of a credit card number. Static information 560 may also be utilized as part of that credit card number. Displays 570 and 580 may each be operated by a different microprocessor. Each of displays 570 and 580 may operate under different coding procedures (provided by the different processors). A single processor may be utilized to provide the different coding procedures. Displays 570 and 580, and the associated processors, may be clocked by a single clock and may be powered by a single battery. Persons skilled in the art will appreciate that any number of processors, clocks, or any other structure may be utilized in card 550. Displays 570 and 580 may, for example, be located near the top of card 550.

Persons skilled in the art will appreciate that any information utilized in any type of payment transaction (e.g., credit card transaction) may be displayed on a display and communicated to a reader via an emulator or encoder. For example, a user's zip code may be provided on a dynamic credit card on a display as a dynamic number that changes with time. This zip code may be representative of, for example, a time stamp. In this manner, for example, a user may communicate a time stamp to a credit card verification system by entering in the dynamic time stamp into a text box located on the payment stage of an online store.

Persons skilled in the art will also appreciate that a fifteen digit number may be sufficient to provide a large amount of data. For example, a first portion of digits may identify a credit card as a dynamic credit card (e.g., digits 1 and 2). A second portion may identify a user (e.g., digits 3-9). A third portion may be representative of a security code that changes with respect to time (e.g., digits 10-13). A fourth portion may be representative of a time-stamp (e.g., digits 14 and 15). Any portion (e.g., the first portion and second portion) may be static and, as such, may be printed on a credit card. A time stamp may, for example, cycle through and restart at 0. Accordingly, for example, a two digit time stamp may start at 00, end at 99, and then restart at 00. If such a time stamp changes every day, the chances that a time-stamp is not synchronized with a server may be relatively low. Persons skilled in the art will appreciate that environmental characteristics such as temperature may affect the operation of a clock and may introduce delay. Accordingly, the time stamp may be utilized, for example, to confirm that the credit card is in synchronization with a verification system.

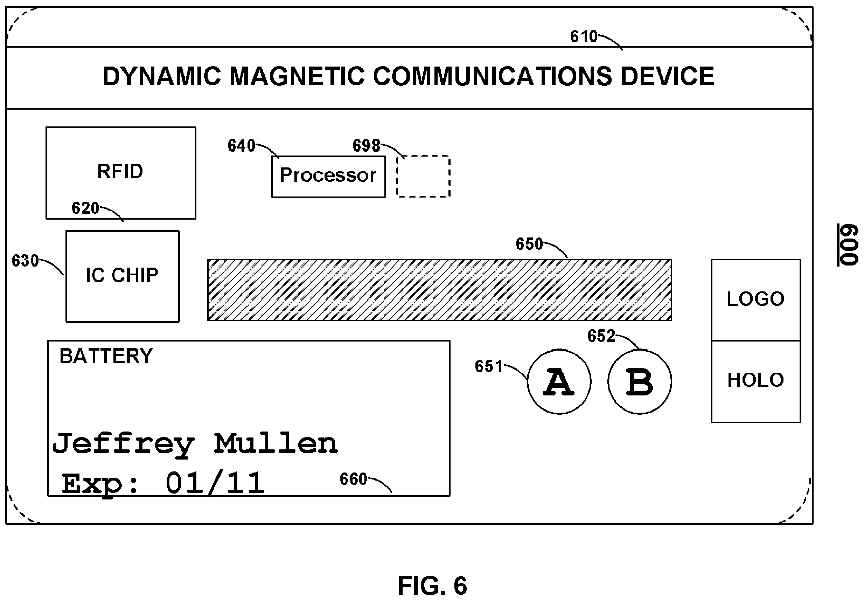

FIG. 6 shows card 600 that may include, for example, one or more IC chips 630 (e.g., EMV chips), RFID antennas 620, processors 640, displays 650, dynamic magnetic communications devices 610 (e.g., magnetic encoders and/or magnetic emulators), batteries 660, and buttons 651 and 652. Additionally circuitry 698 may be provided and may include, for example, one or more oscillators or additional circuitry. Persons skilled in the art will appreciate that button 651 may, for example, be utilized by a user to select one encryption algorithm for a number displayed on display 650 while button 652 may be utilized by a user to select a different encryption algorithm. Persons skilled in the art will appreciate that the components of card 600 may be provided on either surface of a card (e.g., a front or rear surface of the card) or inside of a card. A logo (e.g., of a card issuer) and logo may be provided on either surface of a card.

A button, such as button 651, may be utilized, for example, to display a number. Such a number may be, for example, encrypted from a secure number based on time or use. For example, one-time use numbers (e.g., a payment number or code) may be retrieved from a list of numbers on memory each time button 651 is pressed and displayed on display 650. A processor may only go through each number once on a list. A registration process may be provided in which a user may be requested to enter in a sequence of numbers such that a remote server may validate the card and learn where in a sequence of a list a card currently resides. Numbers may be repeated on a list or may only occur once on a list. All of the numbers available by the length of the number may be utilized by the list or only a portion of the numbers available by the length of the number may be provided by the list. A secret number may be encrypted on a card and a verification server may also have knowledge of this secret number. Accordingly, the remote server may perform the same encryption function as the card on the secret number and verify that the resultant encrypted number is the same as the resultant encrypted number on a card. Alternatively, for example, the remote server may decrypt the received encrypted number to determine the authenticity of the encrypted number and validate an activity (e.g., validate a security access request or a purchase transaction).

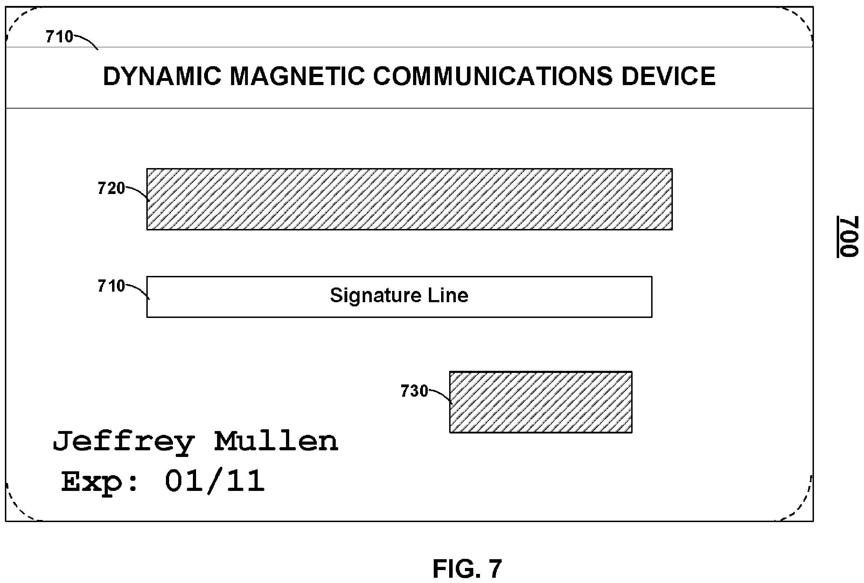

FIG. 7 shows card 700 that may include, for example, signature area 710 that may include a material operable to receive marks from a pen (e.g., a signature). Card 700 may also include, for example, displays 720 and 730. Display 720 may, for example, display a payment number while display 730 displays a security code (e.g., for online purchase authentication). Display 720 as well as display 730 may be utilized on the same side as, for example, dynamic magnetic communications device 710.

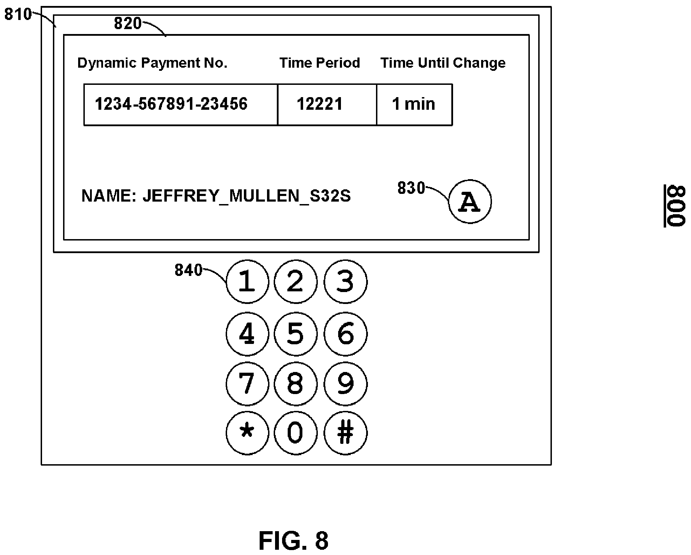

FIG. 8 shows personal electronic device 800 which may be, for example, a portable telephonic device, portable media player, or any type of electronic device. Persons skilled in the art will appreciate that the functionality of a card may be provided on a personal device and displayed through a graphical user interface. Personal electronic device 800 may include, for example, user inputs 840 and display 810. Virtual card 820 may be displayed on display 820. Display 820 may be a touch-sensitive display such that, for example, virtual button 830 may be provided on virtual card 820. Persons skilled in the art will appreciate that cards may be provided as virtual cards and a user may interact with such virtual cards in order to provide a variety of functions. Personal electronic device 800 may communicate to a card reader such as, for example, an RFID reader.

Persons skilled in the art will appreciate that a dynamic magnetic communications device (e.g., a magnetic emulator or magnetic encoder) may be fabricated, either completely or partially, in silicon and provided as a silicon-based chip. Other circuitry (e.g., driving circuitry) may also be fabricated on such a silicon-based chip. A processor, such as a processor for controlling a magnetic communications device, may be, for example, a programmable processor having on-board programmable non-volatile memory (e.g., FLASH memory), volatile memory (e.g., RAM), as well as a cache. Firmware as well as payment information (e.g., dynamic numbers) may be, for example, communicated from a programming device to a processor's on-board programmable non-volatile memory (e.g., a FLASH memory) such that a card may provide a variety of functionalities. Such a processor may also have one or more power-saving operating modes, in which each operating mode turns OFF a different set of circuitry to provide different levels of power consumption. One or more power-savings modes may turn OFF, for example, one or more clocking circuitry provided on a processor. An Application-Specific Integrated Circuit (ASIC) may also be included in a card or other device to provide, for example, processing, dynamic magnetic communications, as well as driving capabilities.

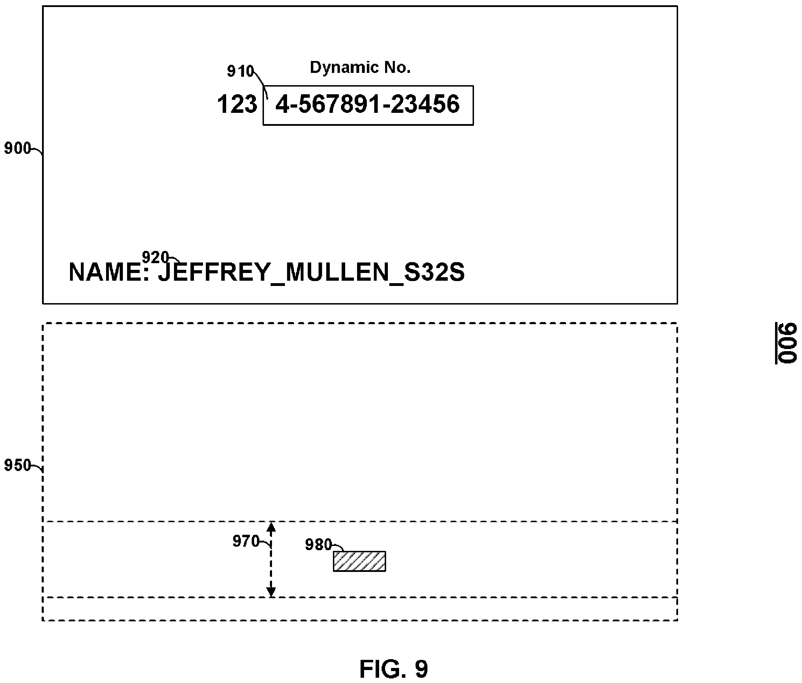

FIG. 9 shows card 900 that may include a display that displays dynamic number 910, which may be utilized, for example, as part of a credit card number (e.g., with a static portion of a credit card number that proceeds dynamic number 910). Persons skilled in the art will appreciate that a dynamic number may take any forms such as, for example, a dynamic credit card number, a dynamic verification code number, and/or a dynamic security code number. For example, card 100 may include a dynamic credit card number and a dynamic verification code (e.g., a 15 digit credit card number and a 4 digit verification code).

Identification information 920 may be provided on card 900. Accordingly, for example, a dynamic number may be provided for a particular period of time according to a coding scheme for that particular period of time. Thus, the identification information, time, and dynamic information may be transmitted via manual entry (e.g., through an online store) or via a magnetic emulator (e.g., through a magnetic stripe reader). A remote server may receive such information and verify whether the dynamic information is correct for particular identification information and a particular period of time.

Card 950 is provided and may include magnetic emulator 960 instead of magnetic stripe 970. Persons skilled in the art will appreciate that magnetic emulator 960 may be embedded behind a magnetic stripe or may be located next to a magnetic stripe. Magnetic emulator 960 may take many forms. For example, magnetic emulator 960 may include any number of emulation segments (e.g., one or more wires or coils) to emulate a particular bit or number of bits of information.

Emulator 960 may include a single emulation segment and may communicate a block of information serially by communicating bits at a high data transmission rate. Accordingly, a serial emulator may be provided.

Emulator 960 may include multiple emulation segments - each of which may, for example, simultaneously emulate a different bit of information. Accordingly, a parallel emulator may be provided.

Emulator 960 may include multiple emulation segments - each of which may, for example, emulate the same bit of information. Accordingly, a serial emulator may be provided. Such a serial emulator may, for example, allow for a larger area to be read by a reader. In doing so, for example, a read head may be located over emulator 160 for a longer period of time such that more information may be read by a read-head for a particular period of time.

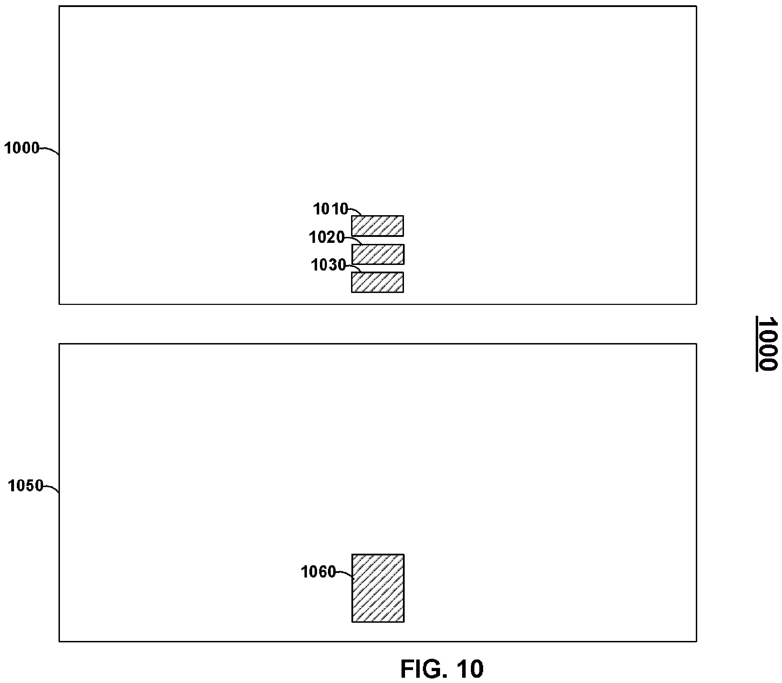

FIG. 10 shows card 1000, which may include any number of emulators. For example, card 1000 may include emulator 1010, 1020, and 1030. Each of emulators 1010, 1020, and 1030 may, for example, be a serial emulator. Such emulators may be vertically aligned or vertically staggered. Persons skilled in the art will appreciate that by providing multiple emulators, multiple tracks of a magnetic stripe may be simultaneously emulated.

A magnetic stripe reader may, for example, determine information on a magnetic stripe by detecting the frequency of changes in magnetic fields (e.g., flux transversals). A particular frequency of flux transversals may correlate to, for example, a particular information state (e.g., a logic "1" or a logic "0"). Accordingly, for example, a magnetic emulator may change the direction of an electromagnetic field at particular frequencies in order to communicate a different state of information (e.g., a logic "1" or a logic "0").

Persons skilled in the art will appreciate that a magnetic emulator may electromagnetically communicate information serially by changing the magnitude of an electromagnetic field with respect to time. As such, for example, a current in a single direction may be provided through a magnetic emulator in order for that magnetic emulator to generate an electromagnetic field of a single direction and a particular magnitude. The current may then be removed from the magnetic emulator such that, for example, the electromagnetic field is removed. The creation of a presence of an electromagnetic field, and the removal of that electromagnetic field, may be utilized to communicate information to, for example, a magnetic stripe reader. A magnetic stripe reader may be configured to read, for example, the change in flux versus time and may associate an increase in an electromagnetic field (e.g., creation of a field) as one flux transversal and a decrease (e.g., removal of a field) as another transversal. In doing so, for example, driving circuitry (not shown) may be provided which, in turn, controls when current is provided to a magnetic emulator. The timing of magnetic flux transversals, as determined by a magnetic stripe reader, may be utilized by that reader to determine whether a logic one ("1") or logic zero ("0") was communicated. Accordingly, a driving circuit may change the frequency of when current is supplied and removed from a magnetic emulator in order to communicate a logic one ("1") or a logic zero ("0").

A driving circuit may, for example, change the direction of current supplied to a magnetic emulator to increase the amount of change in an electromagnetic field magnitude for a period of time. In doing so, for example, a magnetic stripe reader may more easily be able to discern overall changes in an electromagnetic field and, as such, may more easily be able to discern information. As such, for example, a driving circuit may increase the magnitude of an electromagnetic field by providing negative current, decrease the amount of negative current until no current is provided and provide an increasing positive current in order to provide a large swing in the magnitude of an electromagnetic field. Similarly, a driving circuit may switch from providing one amount of negative current (or positive current) to one amount of positive current (or negative current).

Persons skilled in the art will appreciate that a string of a particular bit of data (e.g., a string of logic zeros "Os") may be communicated before as well as after information is communicated through a magnetic emulator. A magnetic stripe reader may utilize such data, for example, to determine base timing information such that the magnetic stripe reader has a timing reference that the reader can utilize to assist in determining timing changes of perceived flux transversals. Accordingly, for example, a magnetic emulator may send data at different overall frequencies and a magnetic stripe reader may be able to reconfigure itself to receive data at such overall frequencies. Information may be encoded using, for example, Frequency/Double Frequency (F2F) encoding such that magnetic stripe readers may perform F2F decoding.

A processor may control one or more emulators by, for example, controlling the direction of the current supplied through one or more segments of an emulator. By changing the direction of current through a region, for example, the direction of an electromagnetic field may be changed. Similarly, a processor may control one or more emulators by, for example, controlling the change in magnitude of current supplied through one or more segments of an emulator. As such, for example, a processor may increase the magnitude of current as well as decrease the magnitude of current supplied through an emulator. A processor may control the timing of such increases and decreases in current such that a magnetic emulator may, for example, communicate F2F encoded information.

Card 250 may be provided and may include emulator 260. Emulator 260 may be configured so that it can be read by more than one read heads. For example, emulator 260 may be configured so that it can be read by three read heads. Accordingly, one region may be utilized to communicate the same information across all three read heads simultaneously. Emulator 260 may, for example, cycle through transmitting information from each track such that all tracks are communicated serially. Such tracks may be communicated with bits identifying each track such that processing connected to each read-head can determine the information desired to be communicated to each particular read-head. As such, a magnetic stripe reader may be configured to receive the multiple tracks (e.g., tracks 1 and 2) through each read-head such that the magnetic stripe reader receives four tracks of data. The magnetic stripe reader may then, for example, utilize identification bits (e.g., start sentinels) in each track to identify the received track. Similarly, for example, the magnetic stripe reader may be configured to recognize that multiple instances of the same track were received and only forward a single instance of each received track to a processor. Persons skilled in the art will appreciate that different tracks may be communicated with the same identification bits. Accordingly, for example, a magnetic stripe reader may be configured to determine the identity of tracks by performing additional computations. For example, the magnetic stripe reader may be configured to check all of the information sent in those tracks and, if the information is the same, a single instance may be provided to subsequent processing. Additionally, for example, the magnetic stripe reader may be configured to determine the length of tracks with the same identification bits (e.g., start sentinels) to determine whether different tracks were communicated. Similarly, a single emulator that sequentially communicates multiple different tracks of information to a single read-head may, for example, change the timing of each communicated track such that processing coupled to the single read-head discerns just the single track that the read-head expected to receive.

Persons skilled in the art will appreciate that a magnetic stripe on a card may be of a particular density having a fixed amount of bits. A serial emulator, for example, may communicate more than this fixed amount of bits by, for example, increasing the rate at which bits are serially communicated. A serial emulator may, for example, communicate a format code that a reader may utilize to discern the length of the communicated information. For example, a serial emulator may communicate a track of information that is greater than, approximately, 750 bits in length, 1,000 bits in length, or 2,000 bits in length. Any number of bits may define, for example, a character (e.g., 4-bit characters, 5-bit characters, 6-bit characters, 7-bit characters, or 8-bit characters).

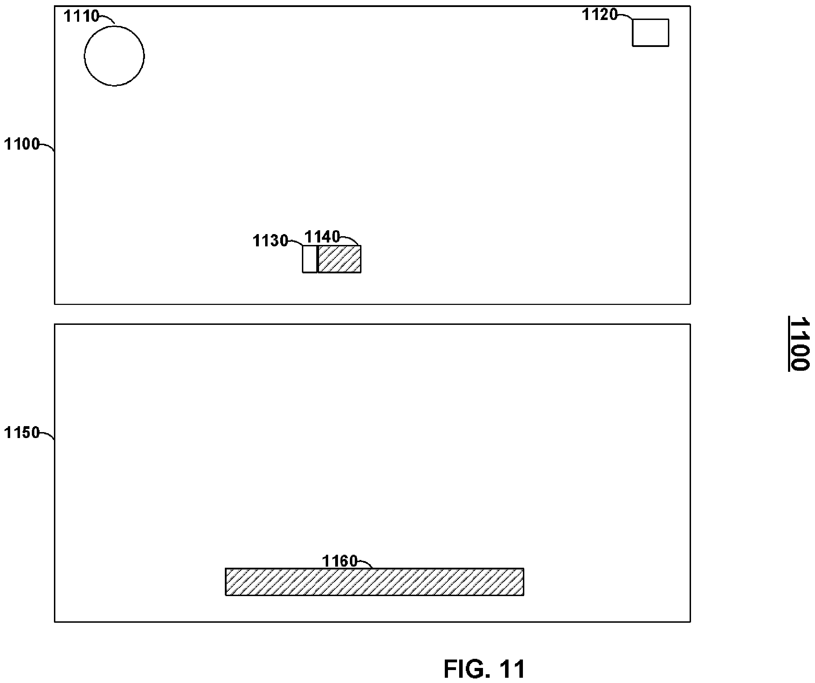

FIG. 11 shows card 1100 that may include structures to determine when card 1100 is being read by a magnetic stripe reader. Card 1100 may include magnetic emulator 1140. For example, card 1100 may include button 1110 that may be pressed in order to initiate an emulator such as emulator 1140. Emulator 340 may be configured to turn ON while button 1110 is pressed. Alternatively, for example, emulator 1140 may be configured to turn ON when button 1110 is pressed for a particular period of time, to transmit a particular sequence of data (e.g., information including a number that changes with every button press), and/or a particular number of repetitions of a sequence of the same data. Persons skilled in the art will appreciate that if a button press of button 1110 is associated with turning emulator 1140 ON for a particular period (e.g., more than 1 second but less than 5 seconds) of time then, for example, emulator 1140 may only turn ON for a relatively short period of time (or not at all) when button 1110 is accidently pressed down for a relatively long period of time. Inertial movement sensor 1120 may be included to detect a swipe in order to initiate one or more emulators. Inertial movement sensor 1120 may include, for example, any number of accelerometers and/or gyroscopes. Read-head sensor 1130 may be included to detect when a read head of a magnetic stripe reader is about to pass over, or is passing over, emulator 1140. Persons skilled in the art will appreciate that multiple read head sensors 1130 may be located (e.g., aligned vertically) adjacent to emulator 1140 as different sensors may detect different types of readers. Additionally, for example, one or more sensors may be placed on the other side of emulator 1140 such that emulator 1140 is sandwiched between sensors. In this manner, for example, emulator 1140 may be triggered regardless of the direction that card 1100 is swiped. Sensors may include, for example, hall-effect sensors, capacitive sensors, and/or conductive material sensors. Conductive material sensors may detect conductive material by closing an electrical loop that uses the conductive material to close the electrical loop.

Card 1150 may include emulator 360. Emulator 1160 may extend, for example, along the majority of the length of card 1150 (or approximately all of card 1150). Emulator 1160 may be a parallel emulator or, for example, may be a serial emulator. For example, emulator 1160 may be a coil such that a single bit is emulated by emulator 1160 at a time. Such an emulator 1180 may, for example, switch bits being emulated at a high rate so that a read-head of a magnetic stripe reader can receive a large amount of information while the read-head of the magnetic stripe reader is located over emulator 1160.

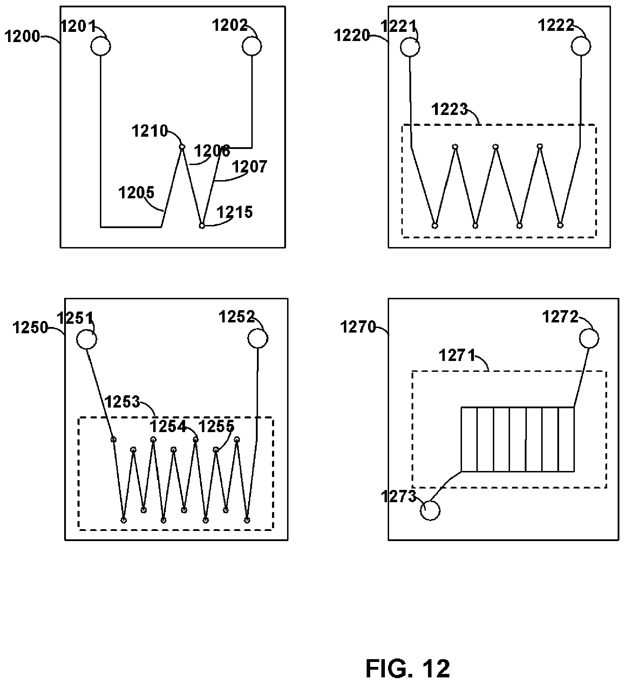

FIG. 12 shows emulators 1200, 1220, 1250, and 1270. Emulator 1200 may include, for example, contacts 1201 and 1202 that may provide a current through conductive segments 1205, 1206, and 1207 in a particular direction. Particularly, for example, a voltage may be applied across contacts of a particular polarity to provide a current in a particular direction (given the particular resistance of the emulator). Driving circuitry may be coupled to one or more contacts for providing a particular current (e.g., of a particular direction and/or particular magnitude). Information may be communicated by, for example, supplying current to and emulator, and removing the current supply from the emulator, in a fashion that results in communicating information to a magnetic stripe reader through electromagnetic fields provided by the emulator. Additional circuitry may be provided to control different attributes of an electrical signal provided to conductive segments 1205, 1206, and 1207. For example, a transistor may be provided to assist with controlling the magnitude of a current that reaches conductive segments 1205, 1206, and 1207.

Emulator 1200 may be fabricated, for example, using any fabrication technique such as a printed circuit board fabrication technique (e.g., utilizing FR4). Via 1210 and via 1215 may be included to electrically couple conductive segments 1205 and 1207 to conductive segment 1206. Accordingly, for example, conductive segment 1206 may be provided at a different height then conductive segment 1205 and conductive segment 1207 (e.g., with respect to a base). For example, conductive segments 1205 and 1207 may be provided one surface of a material while conductive segment 1206 is provided on another surface of a material. Segment 1205 and 1207 may be located closer to the reverse side of a card while segment 1206 may be located closer to the obverse side of a card (or vise versa). Accordingly, for example, wire segments 1205, 1206, and 1207 may take on a three-dimensional shape and particular segments (e.g., wire segment 1206) may be closer to a particular surface than other segments. Persons skilled in the art will appreciate that segment 406 may be angled (e.g., with respect to a top or bottom edge of a card) or may be in parallel (with respect to a top or bottom edge of a card). An emulator may include multiple instances of emulator 1200 coupled in, for example, a series configuration. Conductive segments on the same surface of a material may be, for example, spaced uniformly on that surface. Accordingly, for example, a coil may be provided as an emulator with numerous turns and a current may be provided through that emulator such that an electromagnetic field is generated that is operable to be sensed by a magnetic stripe reader.

Persons skilled in the art will appreciate that numerous vias and line segments may be provided such that conductive segments are provided at several heights. Thus, for example, a coil may be fabricated on a multiple layer board. Emulator 1200 may be utilized, for example, to transmit information serially. For example, the direction of the electromagnetic field created by line segments 1205, 1206, and 1207 may be changed. Control circuitry may, in turn, change the direction of the electromagnetic field (e.g., by changing the direction of the current) at different frequencies such that a reader configured to detect the frequency of field reversals (e.g., using F2F decoding) can receive information. As such, emulator 1200 may communicate multiple bits of data serially by utilizing line segments 1205, 1206, and 1207. Any number of line segments may be added. Additionally, for example, only a single conductive segment may be provided. Furthermore, multiple instances of emulator 1200 may be placed next to each other and may be separately controlled. In doing so, for example, multiple, independent electromagnetic fields may be controlled such that different information may be emulated simultaneously (e.g., in parallel).

Conductive segments may, for example, be printed on a board (e.g., a flexible PCB board) in a conductive material (e.g., a metal). Similarly, vias may be provided and filled to provide conductive interconnects. Multiple layers may be printed to provide a three-dimensional PCB. Persons skilled in the art will appreciate, however, that an emulator may be provided on a single layer with any number of conductive segments (e.g., one or more than one).

Emulator 1220 may be provided and may include conductive segment array 1223. Person skilled in the art will appreciate that conductive segment array 1223 may be fabricated in multiple layers to form a coil. Accordingly, the coil may be provided with a current of a particular direction and may generate an associated electromagnetic field across the coil. Additionally, for example, current may be provided and removed from emulator 1220 to communicate information. In extending the length of an array (e.g., adding more segments or increasing the space of segments), the amount of time a read head is operable to read information from an array may be increased.

Emulator 1250 may be provided and may include contacts 1251 and 1252 to provide a current (e.g., a current of a particular direction) to conductive segment array 1253. Persons skilled in the art will appreciate that conductive segments in array 1253 may be coupled to vias that are not horizontally aligned with one another. Accordingly, the vias may be staggered. In doing so, for example, the conductive segments may be spaced closer together as vias may be spaced closer together. For example, by staggering the lengths of line segments, vias 1254 and 1255 may be able to be provided at larger densities. In turn, a coil may be provided with an increased number of turns.

Emulator 1270 may be provided and may include array 471 controlled by contacts 1272 and 1273. Vertical conductive segments may, for example, be controlled together or may be controlled independently (e.g., by having a separate pair of contacts for each vertical conductive segment). Components may be provided on each segment in order to, for example, provide current through each segment. For example, resistors may be added to each segment. The resistors of each segment may be different. For example, the resistance of each segment may be configured to be substantially equal. Multiple emulators may be utilized on a structure (e.g., a card or other device) to communicate to, for example, communicate to different read heads or the same read head.

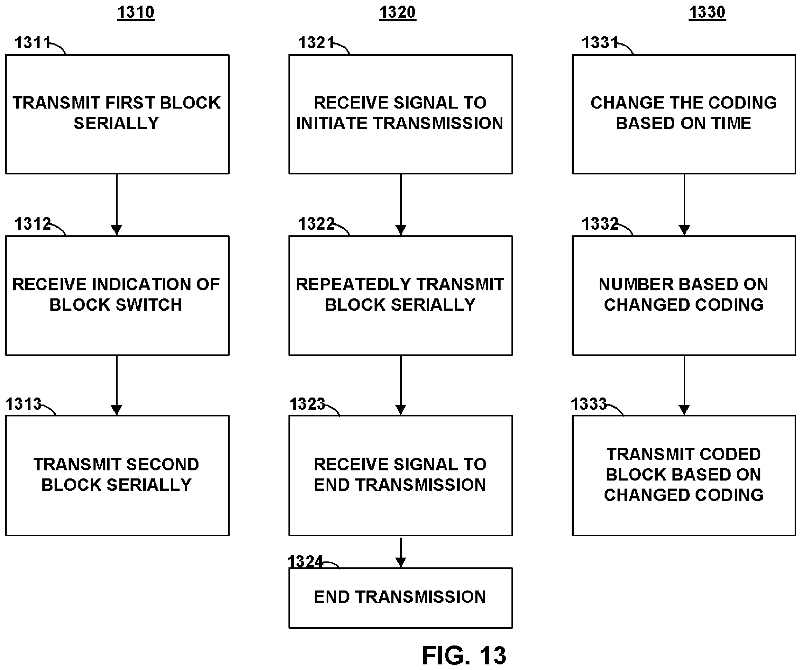

FIG. 13 shows flow charts 1310, 1320, and 1330. Flow chart 1310 may be utilized in a card or any other device (e.g., a token or personal electronic device). Step 510 may be utilized to transmit a first block of data (e.g., track 1 credit card information). Step 512 may then receive an indication to switch the data block that is being communicated. Such a switch may be done autonomously through processing logic or manually through a manual interface such as a button. Step 1313 may provide a second block of data (e.g., track 2 credit card information). In this manner, for example, an emulator may provide different types of data in different types of formats and at different data block lengths.

Flow chart 1320 may be provided and may include step 1321, in which a signal is received to initiate a transmission. Such a transmission may take the form of an emulation and may be triggered autonomously through software (e.g., the detection of a read head) or manually through a manual interface (e.g., one or more buttons). Step 1322 may initiate and repeatedly send a block of data serially. Step 1323 may initiate and a signal may be received to end a transmission. Such a signal may be provided, for example, autonomously through software (e.g., the detection of a read-head by a second detector circuit after an emulator) or manually through a manual interface (e.g., on or more buttons. Transmission may be ended in step 1324.

Persons skilled in the art will appreciate that a read-head detector may be utilized to cause a magnetic emulator to, for example, transmit a block of information serially (e.g., payment information) once each time the read-head detector senses a read-head. Alternatively, for example, a read-head detector may be utilized to cause a magnetic emulator to, serially transmit the same block of information repeatedly a particular number of times or for a particular period of time. Pauses may be introduced between transmissions of the block of information (e.g., payment information). Any dynamic magnetic communications device (e.g., one or more emulators and/or encoders) may be utilized based on the readings of one or more read-head detectors.

Flow chart 1330 may be provided. Step 1331 may, for example, change the coding of a number based on time (e.g., may code a credit card number differently with respect to time). Accordingly, a number may be changed based on the changed coding in step 532. The coded block may be transmitted in step 533. Persons skilled in the art will appreciate that a number (e.g., a dynamic number) may be provided visual via a display as well as magnetically via an emulator. Such a visual and emulation display may occur simultaneously. A different manual interface may be utilized (e.g., a different button) to turn a display ON or to turn an emulator ON.

Persons skilled in the art will appreciate that a particular magnetic stripe track (e.g., track 2 of a payment card such as a credit card) may have a particular amount of information at a particular density and in a particular format. For example, an emulator may transmit 40 characters, where each character is represented by 5 bits, by transmitting 200 bits. A magnetic stripe may include a track with, for example, 400 magnetic regions that may represent a maximum of approximately 400 flux reversals. Persons skilled in the art will appreciate that, furthering this example, with serial transmission, all 400 flux reversals (or more) may be transmitted by a single region. If start bits are utilized in a data block, then, for example, the data block may be repeatedly sent and resent and be properly utilized by a reader. For example, if a reader picks up a serial transmission in the middle of a transmission, the reader may not recognize the start bits and may wait until a start bits are received. Thus, an emulator may be driven such that it can, for example, send a data block approximately at least twice (e.g., approximately 800 associated flux reversals if a block is associated with 400 flux reversals) while a read-head of a magnetic stripe reader is operable to communicate with an emulator (e.g., the read-head is located over the emulator). In doing so, for example, an emulator may be able to transmit a block regardless of when a read head starts reading a block.

A magnetic stripe reader may, for example, be configured such that it is able to sense approximately at least 30,000 flux changes per second. Accordingly, an emulator region may transmit, for example, two blocks of information (e.g., of 400 transversals) to such a reader in approximately 0.0266 seconds. If, for example, the region is approximately 1mm wide, the user may be operable to swipe at approximately .037 meters/second (approximately 1.5 inches/second) and the information may be communicated to a reader. If, for example, the region is approximately 5mm wide, the user may swipe at approximately 7.5 inches/second and the information may be communicated to a reader. Persons skilled in the art will appreciate that larger currents may be utilized to drive larger regions. Persons skilled in the art will appreciate that users may be able to swipe at a variety of speeds, and may change the speed of the swipe while a card is being read by a reader, and an emulator may still properly transmit information via emulation. An emulator may be provided, for example, such that a user may swipe at speeds up to at least approximately 0.5, 1, 5, 10 feet/second for a particular reader (e.g., a reader operable of reader 30,000 flux changes a second).

Persons skilled in the art will appreciate that a number of manufacturing techniques may be utilized. An emulator may provide a uniform field for a particular distance about the surface of a card, but may be able to rapidly change the direction of the field. A coil may be provided using a two-layer or several-layer PCB techniques. Persons skilled in the art will appreciate that the width of a conductive segment (e.g., a wire trace) may be approximately 0.003 inches (or larger) and the diameter of vias connecting layers may be approximately 0.008 inches (or larger). Additionally, for example, a pattern of concentric rectangles may be utilized to produce a desired field in serial (or in parallel, for example, if multiple are utilized) transmission scheme. A single trace may also be split into multiple parallel traces that collective generate a desired field pattern.

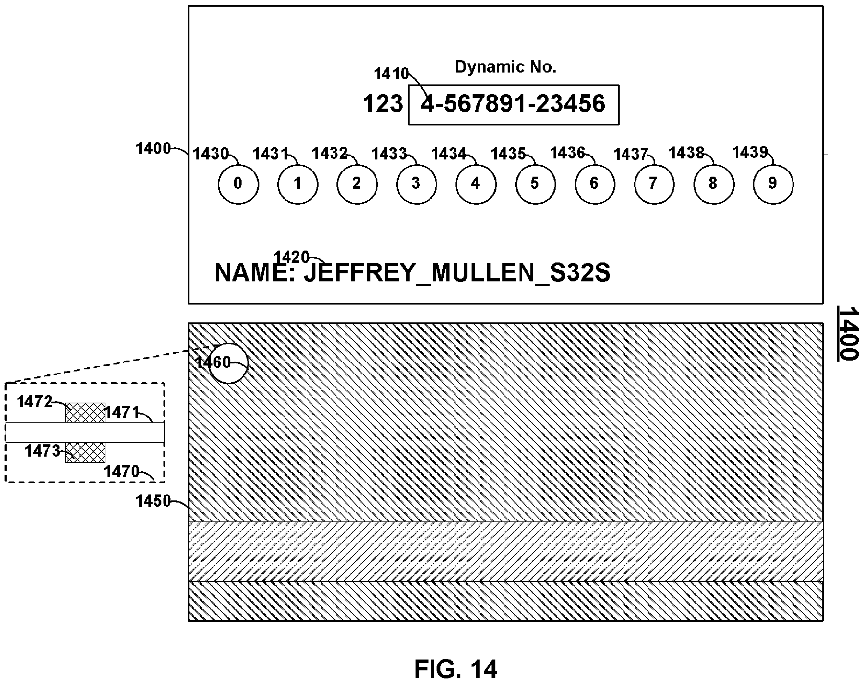

FIG. 14 shows card 1400 that may include a display that may display dynamic number 1410, which may be utilized, for example, as a credit card number or as part of a credit card number (e.g., with a static portion of a credit card number that proceeds dynamic number 1410). Persons skilled in the art will appreciate that a dynamic number may take any forms such as, for example, a dynamic credit card number, a dynamic verification code number, and/or a dynamic security code number. For example, card 1400 may include a dynamic credit card number and a dynamic verification code (e.g., a 15 digit credit card number and a 4 digit verification code or a 16 digit credit card number and a 3 digit code).

Identification information 1420 may be provided on card 1400. Accordingly, for example, a dynamic number may be provided for a particular period of time according to a coding scheme for that particular period of time. Thus, the identification information, time, and dynamic information may be transmitted via manual entry of that information (e.g., through a payment information input process on an online store) or via a magnetic emulator (e.g., through an in-store magnetic stripe reader). A remote server may receive such information and verify whether the dynamic information is correct for particular identification information and a particular period of time. A remote server may look at particular parts of a payment number (e.g., a static portion of a payment card number) and may determine whether another part of that payment number (e.g., a dynamic number) is valid for that particular part for a particular period of time. A number, or portion of a number, may be changed based on use (e.g., as a result of a user pressing a button or a read-head detector determining the presence of a magnetic stripe read-head).

Input buttons 1430-1439 may be provided such that manual input may be received and processed by card 100. Manual input buttons 1430-1439 may be utilized in a variety of ways. For example, an individual may be issued with a private personal identification number (PIN) to turn the card ON and/or to activate a feature. Thus, buttons 1430-1439 may be utilized to confirm that the individual issued the card is utilizing the card and its various features. In doing so with a credit card, for example, the amount of fraud associated with physical card theft may be minimized. Accordingly, a dynamic credit card number may be generated (e.g., coded) upon successful entry of an appropriate PIN. Additionally, for example, manual input keys 1430-1439 may be used to navigate through a list of options or initiate features. For example, button 1430 may turn card 1400 ON/OFF. Button 1431 may turn display 1410 ON/OFF. Button 1432 may turn an emulator located on card 100 ON/OFF. Persons skilled in the art will appreciate that a credit card number may be coded based on time and transmitted with an identification number to a verification server. In turn, the verification server may decode the number based on time and identification number to verify, for example, a credit card transaction.

Card 1450 may include button 1460 which may, for example, be in the form of an aperture. For example the aperture may be defined in material 171 and may include sensors 1472 to 1473 to determine if a user presses around the aperture. Accordingly, a person pinching the aperture with two fingers may cause an electrical connection between sensors 1472 and 1473 via the skin of two fingers touching via the aperture. Accordingly, for example, pinching may result in the recognition of the activation of a button while just touching one side may not cause the activation of a button. In doing so, the number of times a button may become active by accident (e.g., while in a user's wallet) may be decreased.

Card 1500 includes buttons 1531-1540. Buttons 1531-1540 may be aligned vertically or horizontally (e.g., with respect to the bottom of a card)or, for example, substantially in a block or circle.

Card 1550 may include buttons 1571-1575, which may be aligned, for example, in the shape of a directional up-down/left-right pad with a centralized button. Accordingly, buttons 1571-1575 may be used to navigate through a list of options. Accordingly, for example, display 1560 may include multiple lines of alphanumeric text and buttons 1571-1575 may be used to navigate through the test. Additionally, a personal identification code may be provided and may be entered via buttons 1517-1575 (e.g., 'A-B-B-D-E-A' may be entered to turn the card ON or turn a feature ON.

Card 1600 may be included with buttons 310-319. Buttons 1610-1619 may also be associated with digits 0-8, respectively. Another button may be added and associated with, for example, digit 9 such that a digit-based keypad is provided. A digit may be pressed multiple times in succession such that alphanumeric data may be entered. Button 1610 may be utilized to request a new card. In pressing button 1610, or any button, information representative of this request may be displayed so that the information may be entered online or transmitted through a reader via a magnetic emulator. The receipt of such information may cause the desired action to occur (e.g., a new card may be sent).

Button 1611 may be pressed to display and/or emulate identification information associated with the user of the card (or allow a user to LOGIN/LOGOUT of the card so that multiple users can utilize the card). Button 312 may be used to unlock the card. For example, button 312 may be pressed, then a personal identification code may be entered, then button 312 may be pressed again. If the correct personal identification code was entered, for example, then a feature (e.g., card unlocking) may occur. A process may, for example, include determining if button 312 is pressed and the entrance of a correct personal identification code without, for example, determining a subsequent entry of button 312. Such a process may, for example, allow a user to expedite entry of a personal identification code. If a user enters an incorrect personal identification code, for example, nothing may happen or the user may be prompted, via the display, to reenter the code. After a particular amount of time waiting for the next manual input for a code, the processor may return to looking for the first manual input representative of a correct code (e.g., after 5 seconds). After an incorrect code is received, a processor may return to looking for the entry of the first manual input representative of a correct code (e.g., the first button of an appropriate code). Moreover, for example, a particular number of codes entered in error may permanently lock the card or may lock the card until a period of time has passed (e.g., 5 minutes). Button 313 may be added to present the 1800 number for the card on the display. Button 314 may be utilized to show, as well as magnetically emulate the a dynamic number (e.g., the dynamic credit card number for a period of time and for a particular person). Button 1615 may be utilized for to lock a card. Button 1616 may be utilized to transmit, for example, an emergency alert such as an alert that the card is about to be stolen or someone is in trouble (and may, for example, be transmitted upon swiping of a card). Button 1617 may be utilized to display/magnetically emulate battery status. Button 318 may be utilized to turn the card ON/OFF. Persons skilled in the art will appreciate that, for example, additional information (e.g., alerts, battery information, card replacement requests) may be communicated as discretionary data in communicated payment information or may be communicated in a separate information transmission. Similarly, such information may be embedded in non-discretionary data information in communicated payment information.

Card 1650 may be utilized. Buttons 1660-1668 may be provided. Buttons 1660, 1663, and 1666 may each be associated with a different coding scheme. Pressing button 1660, 1663, and 1666 may cause a number (e.g., credit card or security number) to be generated differently. Thus, for example, a company may issue security cards and may associate different button switch different levels of security or may rotate between the coding schemes or may allow for a new coding scheme to be used if a coding scheme is compromised. Button 1661 may be utilized to upload information at upload locations (e.g., upload new software). Accordingly, circuitry may be included to receive information from a swipe.

Button 1664 may be utilized to destroy a card (e.g., burn out components). Persons skilled in the art will appreciate that the functions of various buttons may be triggered autonomously upon particular determinations of a processor. For example, a processor may determine that someone is trying to break through the casing of a component (e.g., a memory) and may autonomously burn out components or perform other tasks (e.g., erase memory) as a result of the determinations. A processor may write information to a memory when the processor detects an fraudulent attack on a card by, for example, erasing a portion of data (e.g., payment card number(s)), erasing all of the data, or changing the data (e.g., replace a payment card number with a number indicative of a fraudulent activity) on a memory.

Button 1667 may be utilized to show time (e.g., the current time) on a display. A clock may be provided on card 1650 such that time may be kept. Such a clock may be provided with its own battery such that the clock may continue to keep track of time even when, for example, a processor is OFF. Persons skilled in the art will appreciate that a card may be ON when the card is delivered to a user but that a processor may be in a hibernation mode. Accordingly, for example, an ON/OFF button (or an unlock code) may wake that processor out of such a hibernation mode.

Button 1663 may be utilized to record (e.g., store in memory), display on a display, and communicate through a dynamic magnetic communications device (e.g., a magnetic emulator or encoder) a location of the card or a history of locations of a card. Accordingly, for example, a locating device (e.g., GPS receiver) may be provided on a card. A transmitter may be provided that may communicate a signal that multiple remote receivers may receive (e.g., mobile phone base stations) such that the location of a card may be determined (e.g., via a triangulation process).

Button 1665 may be utilized to change the unlocking preferences (e.g., change a personal identification code). For example, a user may be prompted to enter the user's current personal identification code, then be prompted to enter the user's new personal identification code, and then be prompted to confirm entry of the user's new personal identification code. If the two new personal identification codes match, then, for example, the personal identification code for a user may be changed. A card may be provided with a default personal identification code. Button 1668 may be utilized on turn a card ON/OFF.

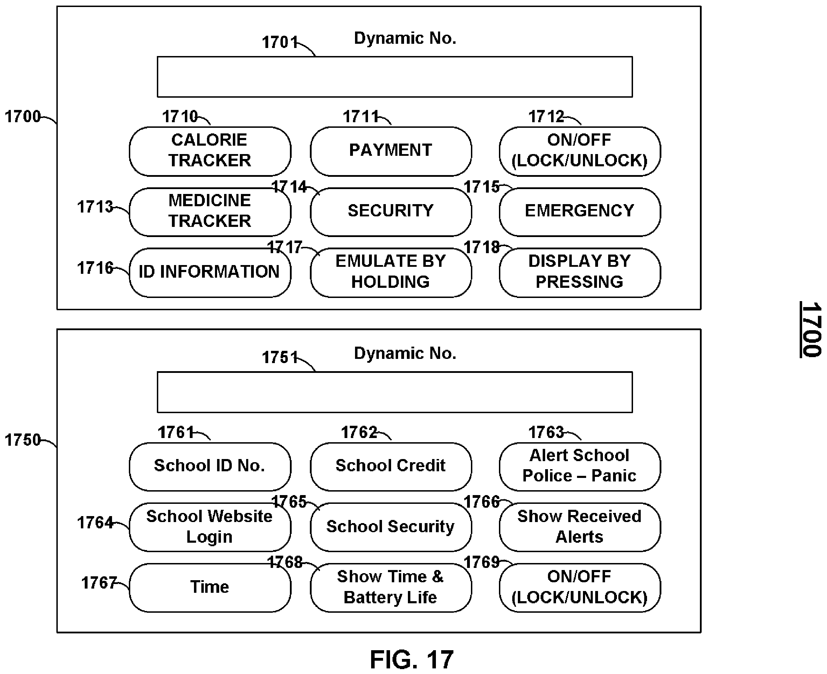

FIG. 17 shows card 1700 which may include buttons 1710-1718. Button 1710 may include a calorie tracker such that a user can enter in calories he/she eats per day. Thus, whenever a card is swiped via a magnetic stripe reader, or otherwise communicates data to a card reader or device, the calorie information may be entered into a database which can be utilized to populate a webpage (e.g., a calorie tracker webpage).

Button 1713 may be utilized as a medicine tracker (e.g., to track the type and number of pills taken). Information may be displayed on display 1701 that a user may enter such that the information may be associated with information entered by a user. For example, display 401 may provide an alphanumeric word "A342F2432S" that may be associated with, for example, 100 calories for breakfast and 300 calories for lunch on December 12, 2007. This word may be entered on a website such that the information associated with the word may be used to populate the website (e.g., the calorie tracker).

Button 1716 may be used to display identification information (e.g., name and phone number of card user). Accordingly, for example, someone that finds card 1700 may press button 1716 to determine the owner of card 1716 as well as other information (e.g., phone number and email address).

Button 1711 may be used for payment. Accordingly, for example, a payment number may be displayed on a display (along with additional payment data such as a payment security code). A dynamic magnetic communications device (or other device operable to communicate to a card reader) may also transmit information that includes such a payment number and additional payment data.

Button 1714 may be used for security (e.g., an online login). Accordingly, for example, a user may press button 1714 and may be provided with a code (e.g., an access security code) such that the user may enter particular portions of a website (e.g., a webpage associated with a user's banking account). Such an access code may be displayed to a user such that a user may enter the code into a keypad at a lock such that the lock is opened upon received of the correct code. Similarly, such an access security code may be, for example, communicated via a dynamic magnetic communications device (e.g., a magnetic emulator or magnetic encoder) as well as other reader communications devices (e.g., RFIDs and IC chips such as EMV chips). Such codes may change based on time or based on use (e.g., every time button 1714 is pressed by a user).

Button 1717 may be used to magnetically emulate information by holding button 1716 such that data may be communicated via a magnetic emulator to a magnetic stripe reader. For example, button 1710 may be pressed and then button 1717 may be pressed to emulate information associated with a calorie tracker. Button 1712 may be used, for example, to turn card 400 ON/OF and/or UNLOCK/LOCK card 1700. Button 1715 may be utilized as an emergency alert (e.g., a panic button). Accordingly, for example, a student may press emergency button 415 and swipe his/her card into a magnetic stripe reader and the appropriate authorities (e.g., police) may be alerted of the magnetic stripe reader, and its location, from which an emergency was initiated (and the identity of the person that initiated an emergency. In this manner, a police button, firefighter button, and ambulance button may be utilized.

Alternatively, for example, a doctor button, a nurse button, or a food button may be utilized for hospital cards. Button 1718 may be utilized to display information on display 1701 while the button is pressed. Accordingly, for example, calorie tracker 410 may be utilized and then button 418 may be pressed to display information associated with calorie tracker 410.

FIG. 17 shows card 1750 that may include, for example, buttons 1761-1769. Button 1761 may be used to display, as well as magnetically communicate via a magnetic emulator to a magnetic stripe reader, school identification information. Button 462 may be utilized to display, as well as communicate through a dynamic magnetic communications device, school credit information. Button 464 may be utilized to display, as well as communicate through a dynamic magnetic communications device, website login information. Button 1767 may be utilized to display emulate, for example, time information. Button 1766 may be utilized to show alerts that are received. For example, a receiver may be included in a card that may receive wireless alerts. Accordingly, for example, students may be alerted of a school-related risk/danger/information (e.g., bomb threat, fire, or school cancelled due to snow) and may be shown this information via display 1751. Button 1751 may be utilized to show, for example, the most recent alert and/or scroll through alerts. Button 1769 may be utilized, for example, to turn card 1750 ON/OFF and/or UNLOCK/LOCK card 1750.

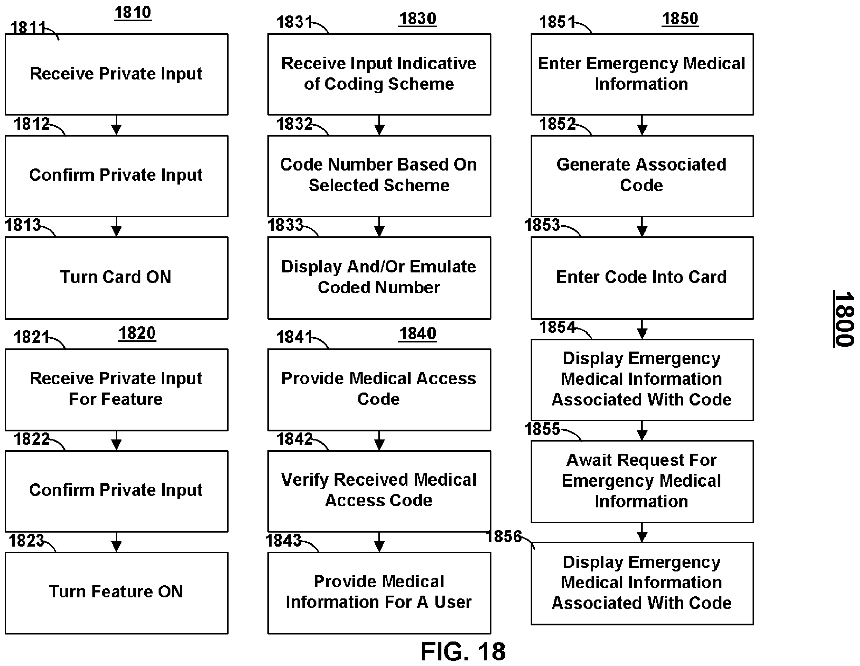

FIG. 18 shows flow charts 1810, 1820, and 1830. Flow chart 1810 may include, for example, step 1811, in which private input (e.g., private identification information) is received. This information may be confirmed, for example, in step 1812. Additionally, confirmation of the correct private number may turn a card ON (e.g., allow information to be displayed/emulated) in step 1813.

Flow chart 1820 may be included. Step 1821 may be provided, in which, for example, private input is received for a particular feature. This input may be confirmed in step 1822. Accordingly, for example, a feature may be turned ON in step 1823. Flow chart 1830 may be included. Step 1831 may be provided, in which input indicative of a particular coding scheme is received. A number (e.g., website login and/or credit card) may be coded or generated (e.g., from a hash table associated with a particular input) in step 1823. The coded, generated, and/or retrieved information may be displayed and/or communicated through a magnetic emulator in step 1833.

Flow chart 1840 may be provided and may be utilized, for example, in conjunction with a medical card and medical information retrieval system. For example, medical information may be stored on the memory of a card. Such medical information may be, for example, a user's height, weight, eye color, blood type, previous medical conditions, previous medications taken, current medical conditions, current medications taken, allergies, doctor contact information, as well as contact information for an emergency contact person.

Persons skilled in the art will appreciate that a user may control access to the user's medical information by, for example, keeping the medical information in his/her pocket and under his/her control at all times. (e.g., similar to the protection afforded to car keys and house keys). In the case of an emergency (e.g., a car accident), first responders may look for the user's medical card in order to gain access to the user's medical information. Such a medical card may take the form of, for example, an identification card (e.g., a driver's license or passport). A sticker may be placed on a card or device (e.g., a mobile telephone or identification card) stating that a user has a medical card in his/her wallet (e.g., as well as the location of the card such as on the left-hand side of the wallet). A medical card may, for example, be taken by a first responder and may display a passcode for the responder to enter onto a website in order for the responder to obtain the user's medical information. Identification information may be permanently displayed on the card (e.g., printed or embossed) and this identification may be entered into a website along with a user. Instructions for accessing the medical information may be printed or embossed on a card or other device. Such an access security code may, for example, change based on time or use (e.g., press of a particular button or particular buttons). A first responder may be prompted by a website, for example, to enter in a responder's username and password such that the responder can be identified as a responder that may access the medical information of a user. Medical information stored on a remote server may include, for example, pictures (e.g., of a birth certificate and bodily parts at various times), x-rays, medical reports, as well as any other type of medical information. A medical card may also store such images and other data.

Flow chart 1840 may include, for example, a card (or other device) providing a medical access code in step 1841. Step 1842 may be included, in which a medical access code is verified (e.g., on a remote server). Step 1843 may be provided, in which medical information for a user may be provided as the result of the verification of a correct access code. An access code may be, for example, a five, six, seven, or eight digit code (e.g., "834699"). Persons skilled in the art will appreciate that a card may include, for example, medical information. Such medical information may be displayed, for example, by a user pressing a particular button. The information may be scrolled left/right as well as up/down using the same button or additional buttons. For example, a first line of data may be "Blood Type: B" and a second line of data that can be scrolled down to using a button may be "Allergies: None"

Flow chart 1850 may be provided. Step 1851 may be provided, in which a user may go to a website or a graphical user interface on a device and enter in his/her emergency medical information. Such information may be, for example, pre-populated with the websites prior knowledge as to the user's emergency medical information. Such information may be changed by a user. The entry of medical information may take many forms. For example, the entry of medical information may be done through the selection of options. For example, a user may be provided with a list of allergies and may select those allergies that apply to a user. A user may then, for example, generate a code in step 1852. Such a code may be, for example, associated with the particular combination of selections that user made. A user may then, for example, enter this code into his/her medical card using buttons on that medical card. In this manner, the medical card may include data on a memory that may recognize the code and may display, at a user's request, the medical information associated with that code (e.g., step 1854). Accordingly, for example, a user may customize and update his/her payment card without having to connect the user's payment card to a computer (e.g., via a USB port). A card may wait for a request for emergency medical information (e.g., step 1855) and may provide the emergency medical information as a result of receiving the request (e.g., step 1856).

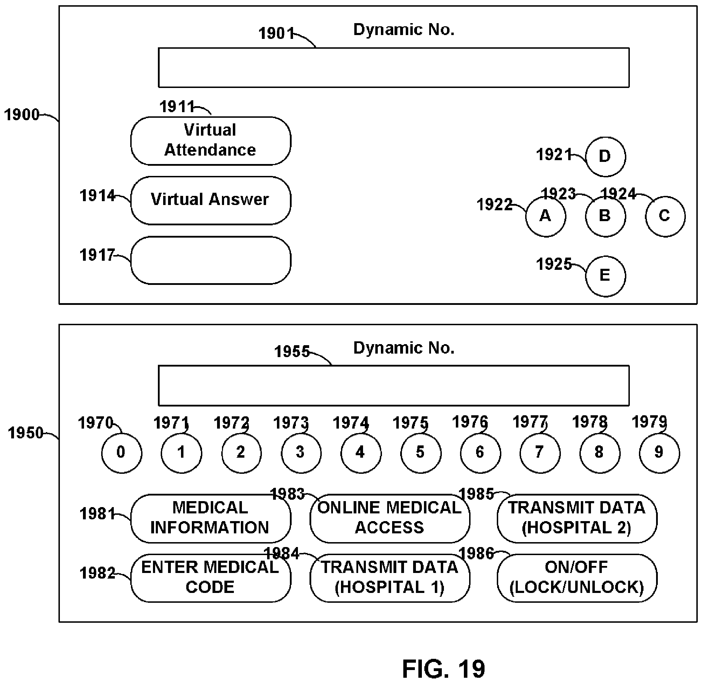

FIG. 19 shows card 1900 which may include buttons 1911, 1914, and 1917. Button 1911 may be utilized for virtual attendance. A user may press button 1911 and transmit identification information (e.g., either wirelessly or via a magnetic emulator) to a server such that attendance may be recorded. Similarly, button 1914 may be utilized to provide a virtual answer to a question. For example, button 1914 may be pressed, a button associated with answer "B" may be pressed, button 1914 may be pressed again, and then a card may be swiped and information associated with the answer transmitted (e.g., via a magnetic emulator) to a server for further processing. Buttons 1921-625 may be utilized, for example, to enter responses into a card so that the responses may be displayed visually or communicated via a magnetic emulator.

Card 1950 may be provided with buttons 1970-679 and 1981-1986. Button 1981 may be utilized, for example, to display medical information on a display of card 1950. Button 1982 may be utilized, for example, to prompt a processor on card 1950 that a code associated with medical information is about to be entered. Button 1983 may be utilized, for example, to provide (e.g., via a display) a code for accessing a user's online medical record. Button 1984 may be utilized, for example, to communicate information (e.g., insurance information) in one format to a particular hospital that accepts that format. Button 1985 may be utilized, for example, to communicate the same information (e.g., the same insurance information) in a different format to a different hospital that accepts that different format. Button 1986 may be utilized to turn a card ON/OFF.

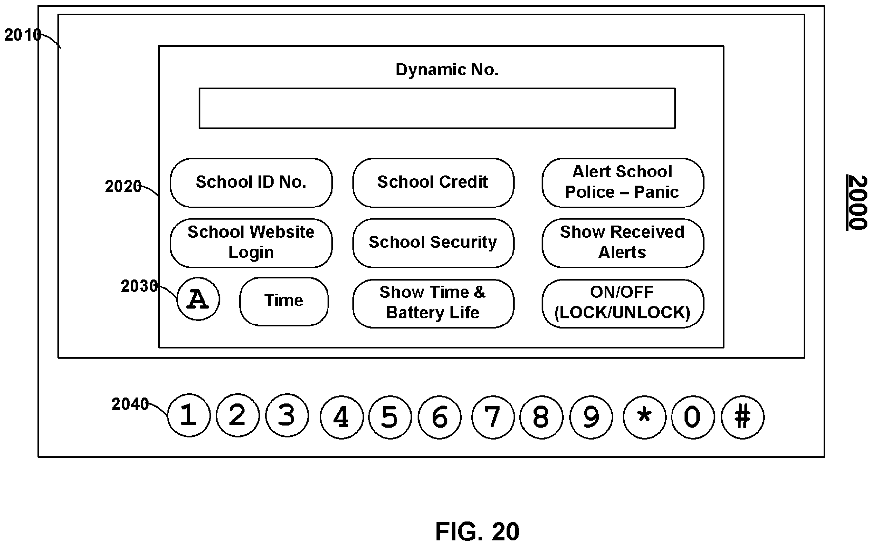

FIG. 20 shows personal electronic device 2000 which may be, for example, a portable telephonic device, portable media player, or any type of electronic device. Persons skilled in the art will appreciate that the functionality of a card may be provided on a personal device and displayed through a graphical user interface. Personal electronic device 2000 may include, for example, user inputs 2040 and display 2010. Virtual card 2020 may be displayed on display 2020. Display 2020 may be a touch-sensitive display such that, for example, virtual button 2030 may be provided on virtual card 2020. Persons skilled in the art will appreciate that cards may be provided as virtual cards and a user may interact with such virtual cards in order to provide a variety of functions. Personal electronic device 2000 may communicate to a card reader such as, for example, an RFID reader.

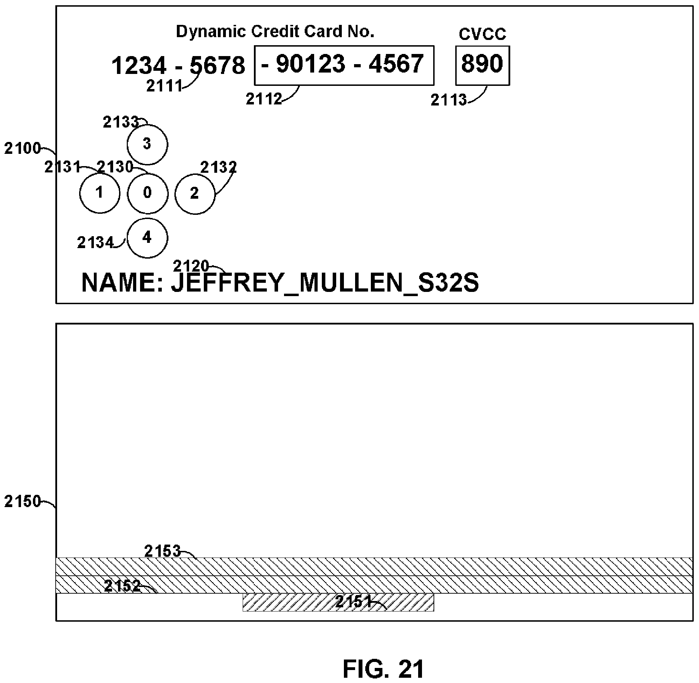

FIG. 21 shows card 2100 that includes printed information 2111 and 2120, displays 2112 and 2113, and buttons 2130-2134. Card 2100 may be, for example, a payment card such as a credit card, debit card, and/or gift card. Payment information, such as a credit/debit card number may be provided as static information 2111, dynamic information 2112 and/or 2113, or any combination thereof.

For example, a particular number of digits of a credit card number (e.g., the last 3 digits) may be provided as dynamic information. Such dynamic information may be changed periodically (e.g., once every hour). Information may be changed via, for example, encryption. Software may be provided at, for example, the payment verification servers that verifies the dynamic information for each period of time such that a payment can be validated and processed for a particular user. A user may be identifies using, for example, static information that is used to form a credit card number or other static information (e.g., information 2120). Additionally, identification information may be derived (e.g., embedded) in dynamic information. Persons skilled in the art will appreciate that a credit card number may have, for example, a length of 15 or 16 digits. A credit card number may also have a length of up to 19 digits. A verification code may be used with some payment systems and such a verification code may be provided statically on the card or may be provided as dynamic information. Such a verification code may be provided on a second display located on, for example, the front or rear surface of card 2100. Alternatively, a verification code may be displayed on the same display as other dynamic information (e.g., dynamic information 2112). A display may be, for example, a flexible electronic ink display. Such a flexible electronic ink display may, for example, utilize power to change displayed information, but may not utilize power to display information after the information is changed.

Card 2150 may be provided. Card 2150 may include static magnetic stripe tracks 2153 and 2152. A magnetic emulator may be provided as device 2151. Device 2151 may be operable to electrically couple with a read-head of a magnetic stripe reader. Persons skilled in the art will appreciate that a read-head housing of a magnetic stripe reader may be provided with one, two, or three active read-heads that are operable to each couple with a separate magnetic track of information. A reader may also have more than one read-head housing and each read-head housing may be provided with one, two, or three active read-heads that are operable to each couple with a separate magnetic track of information. Such read-head housings may be provided different surfaces of a magnetic stripe reader. For example, the read-head housings may be provided on opposite walls of a trough sized to accept payment cards. Accordingly, the devices on the opposite sides of the trough may be able to read a credit card regardless of the direction that the credit card was swiped.

A magnetic emulator may be provided and may be positioned on card 2150 such that when card 2150 is swiped through a credit card reader, the magnetic emulator passes underneath, or in the proximity of, a read-head for a particular magnetic track. An emulator may be large enough to simultaneously pass beneath, or in the proximity of, multiple read-heads. Information may be transmitted, for example, serially to one or more read-heads. Information from different tracks of data may also be transmitted serially and the magnetic stripe reader may determine the different data received by utilize the starting and/or ending sentinels that define the information for each track. A magnetic emulator may also transmit a string of leading and/or ending zeros such that a magnetic reader may utilize such a string of zeros to provide self-clocking. In doing so, for example, information may be transmitted serially at high speeds to a magnetic stripe reader. For example, credit card information may be transmitted to a magnetic stripe reader at speeds up to, and greater than, 30Khz).

Different emulators may be provided, and positioned, on card 2150 to each couple with a different read-head and each emulator may provide different track information to those different read-heads. Read-head detectors may be utilized to detect when a read-head is over an emulator such that an emulator is controlled by a processor to operate when a read-head detector detects the appropriate presence of a read-head. In doing so, power may be saved. Additionally, the read-head detector may detect how many read-heads are reading the card and, accordingly, only communicate with the associated emulators. In doing so, additional power may be conserved. Accordingly, an emulator may be utilized to communicate dynamic information to a magnetic stripe reader. Such dynamic information may include, for example, dynamic payment card information that changes based on time.

A static magnetic stripe may be provide to transmit data for one or more tracks to a magnetic strip reader where dynamic information is not desired. Card 2150, for example, may include static magnetic track 2153 and static magnetic track 2152. Information on static magnetic tracks 2152 and 2153 may be encoded via a magnetic stripe encoder. Device 2151 may include an emulator such that dynamic information may be communicated through emulator 2151. Any combination of emulators and static magnetic tracks may be utilized for a card or device.

One or more batteries, such as flexible lithium polymer, batteries may be utilized to form card 2100. Such batteries may be electrically coupled in a serial combination to provide a source of power to the various components of card 2100. Alternatively, separate batteries may provide power to different components of card 2100. For example, a battery may provide power to a processor and/or display of card 2100, while another battery provides a source of energy to one or more magnetic emulators of card 2100. In doing so, for example, a processor may operate even after the battery that supplies power to an emulator completely discharges. Accordingly, the processor may provide information to another component of card 2100. For example, the processor may display information on a display to indicate to a user that the magnetic emulator is not longer operational due to power exhaustion. Batteries may be, for example, rechargeable and contacts, or other devices, may be provided on card 2100 such that the battery may be recharged.

Buttons (e.g., buttons 2130-2134) may be provided on a card. Such buttons may allow a user to manually provide information to a card. For example, a user may be provided with a personal identification code (e.g., a PIN) and such a personal identification code may be required to be manually inputted into a card using the buttons in order for the card to operate in a particular manner. For example, the use of a magnetic emulator or the use of a display may require a personal identification code.

By dynamically changing a portion of a user's credit card number, for example, credit card fraud is minimized. By allowing the dynamic information to displayed visually to a user, and changed magnetically on a card, user behavior change is minimized (with respect to a credit card with completely static information). By requiring the use of a personal identification code, the fraud associated with lost or stolen credit cards is minimized. Fraud associated with theft/loss is minimized as third party users do not know the personal identification code needed to operate particular aspects of a credit card with dynamic information.

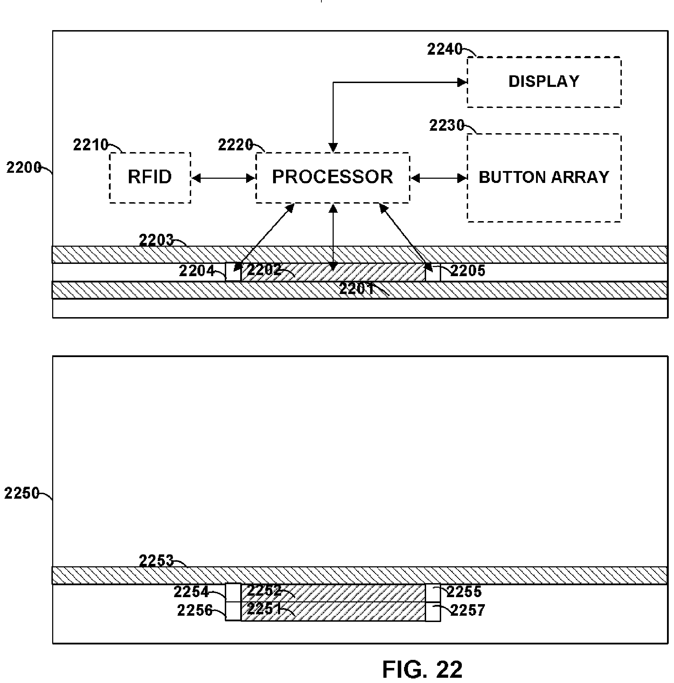

FIG. 22 shows card 2200. Card 2200 may include, for example, static magnetic stripe track 2203, static magnetic stripe track 2201, and magnetic emulator 2202 sandwiched between read-head detectors 2204 and 2205. A read-head detector may, for example, be provided as a circuit that detects, for example, changes in capacitance or mechanical coupling to a conductive material. Processor 2220 may be provided to, for example, receive information from read-head detectors 2204 and 2205 and control emulator 2202. Persons skilled in the art will appreciate that processor 2220 may cause a current to flow through a coil of emulator 2202 in a different direction to produce different electromagnetic fields. The transitions between the different electromagnetic fields may be sensed by a magnetic stripe reader as information. Accordingly, a magnetic emulator may transmit data serially while a read-head is electrically coupled with a magnetic reader.

RFID antenna 2210 may be provided on card 2200. Such an RFID antenna may be operable to transmit information provided by processor 2220. In doing so, for example, processor 2220 may communicate with an RFID device using RFID antenna 2210 and may communicate with a magnetic stripe reader using magnetic emulator 2204. Both RFID antenna 2210 and magnetic emulator 2204 may be utilized to communicate payment card information (e.g., credit card information) to a reader. Processor 2240 may also be coupled to display 2240 such that dynamic information can be displayed on display 2240. Button array 2230 may also be coupled to processor 2220 such that the operation of card 2200 may be controlled, at least in part, by manual input received by button array 2230.

Card 2250 may be provided and may include static magnetic track 2253, magnetic emulators 2251 and 2252, and magnetic read-heads 2254-2257). Persons skilled in the art will appreciate that static magnetic track 2253 may be a read-write track such that information may be written to magnetic track 2253 from a magnetic stripe reader that includes a head operable to magnetically encode data onto a magnetic track. Information may be written to magnetic track 2253 as part of a payment process (e.g., a credit card or debit card transaction). Persons skilled in the art will appreciate that a static magnetic track may include a magnetic material that includes ferromagnetic materials that provide for flux-reversals such that a magnetic stripe reader can read the flux-reversals from the static magnetic track. Persons skilled in the art will also appreciate that a magnetic emulator may communicate information that remains the same from payment card transaction to payment card transaction (e.g., static information) as well as information that changes between transactions (e.g., dynamic information).

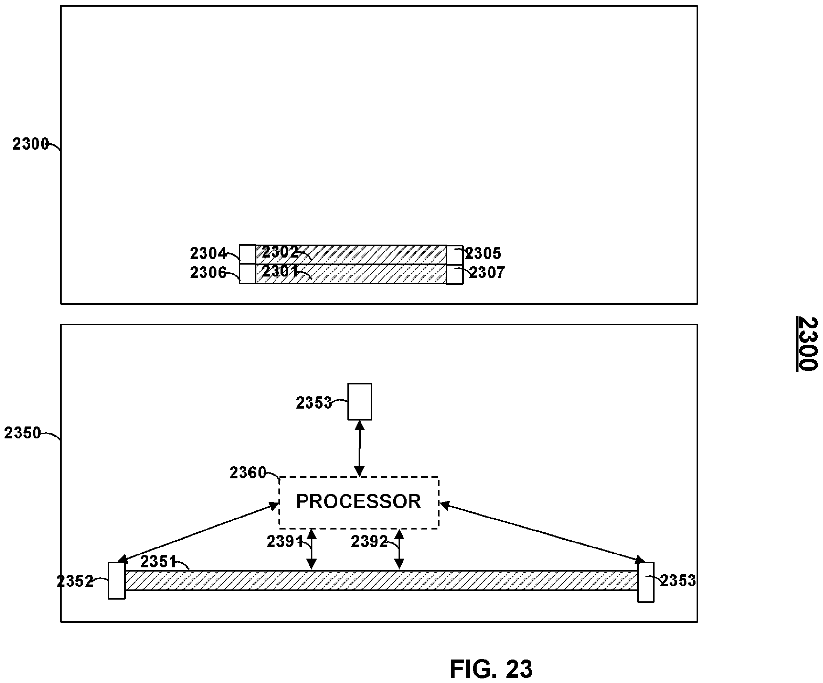

FIG. 23 shows card 2300 that may include magnetic encoders 2302 and 2302 without, for example, a static magnetic track. Read-head detectors 2304-2307 may also be provided. Persons skilled in the art will appreciate that a magnetic reader may include the ability to read two tracks of information (e.g., may include at least two read-heads). All of the information needed to perform a financial transaction (e.g., a credit/debit card transaction) may be included on two magnetic tracks. Alternatively, all of the information needed to perform a financial transaction (e.g., a gift card transaction) may be included on one magnetic track. Accordingly, particular cards, or other devices, may include the ability, for example, to only transmit data associated with the tracks that are needed to complete a particular financial transaction. Persons skilled in the art will appreciate that for systems with three tracks of information, the bottom two tracks may be utilized for credit card information. Persons skilled in the art will also appreciate that a secure credit card transaction may be provided by only changing, for example, one of two magnetic tracks utilized in a credit card transaction (for those transactions that utilize two tracks). Accordingly, one track may be a static magnetic track constructed from a magnetic material and the other track may be provided as a magnetic emulator. Persons skilled in the art will also appreciate that numerous additional fields of data may be provided on a magnetic track in addition to a credit card number (or a security code). Dynamic information may be provided in such additional fields in order to complete a particular financial transaction. For example, such additional dynamic information may be numbers (or characters), encrypted with time and synced to software, at a validating server, operable to validate the encrypted number for a particular period of time.

Card 2350 includes processor 2360. RFID field detector 2353 may provide information to processor 2350. Additionally, magnetic stripe detectors may provide information to processor 2350. An RFID receiver may produce an electromagnetic field that an RFID antenna is operable to electrically couple with and communicate information to. An RFID receiver may act as a source of electrical power to an RFID antenna. Such a power may be harvested (e.g., via RFID 2210 of FIG. 22) to charge a rechargeable battery of a card or other device. An RFID field detector may thus be provided to detect an RFID field.

Emulator 2351 may be able to generate electromagnetic fields of different frequencies and magnitudes, and operate in different manners, depending on drive signals provided by processor 2360. Accordingly, emulator 2351 may be driven to electrically couple with an RFID receiver and emulator 2351 may also be driven to electrically couple with a magnetic stripe reader. Accordingly, processor 2360 may drive emulator 2351 to communicate information (e.g., payment information that includes dynamic information) to an RFID receiver when an RFID field is present and to a magnetic stripe reader when a magnetic stripe is present. Accordingly, for example, a multi-purpose emulator is provided. In instances where, for example, both an RFID field and a magnetic stripe reader is detected, processor 2360 may select a default communications methodology (e.g., an RFID or magnetic stripe methodology). Processor 2360 may be operable to communicate at least two different drive signals to emulator 2351 (e.g., signals 2391 and 2392).

Card 2400 shows card 2400 that may include processor 2400, emulator 2401, read-heads 2402 and 2403, and magnetic stripe encoding receiver 2420. Magnetic stripe encoding receiver 2420 may be a coil such that a current is induced in the coil when a magnetic stripe encoder attempts to provide a signal that would encode a static magnetic track. Accordingly, receiver 2420 may receive information via an encoder such that bi-directional communication can be established with a magnetic stripe reader that includes an encoding capability. Persons skilled in the art will appreciate that a magnetic emulator may be provided that can both transmit data to a read-head of a magnetic stripe reader as well as receive data from an encoding-head of a magnetic stripe reader.

Card 2450 includes emulator 2451 that includes active region 2454 operable to communicate data serially to a magnetic stripe reader. Similarly, for example, emulator 2451 may receive information for a magnetic stripe encoder. Persons skilled in the art will appreciate that emulator 2451 includes a tail that is spread-out. Such a tail may include the return lines of emulator 2451 and may be spaced such that a magnetic reader is not able to pick up the electromagnetic fields generated by such a tail. Accordingly, active region 2454 may be spaced close together such that a magnetic stripe reader is able to pick up the cumulative electromagnetic field generated by such an active region. Processor 2453 may drive emulator 2451 via switching circuitry 2452. Switching circuitry 2452 may include, for example, one or more transistors that may be utilized to control the direction of current via emulator 2451 (e.g., the polarity of voltage(s) across a drive resistor).

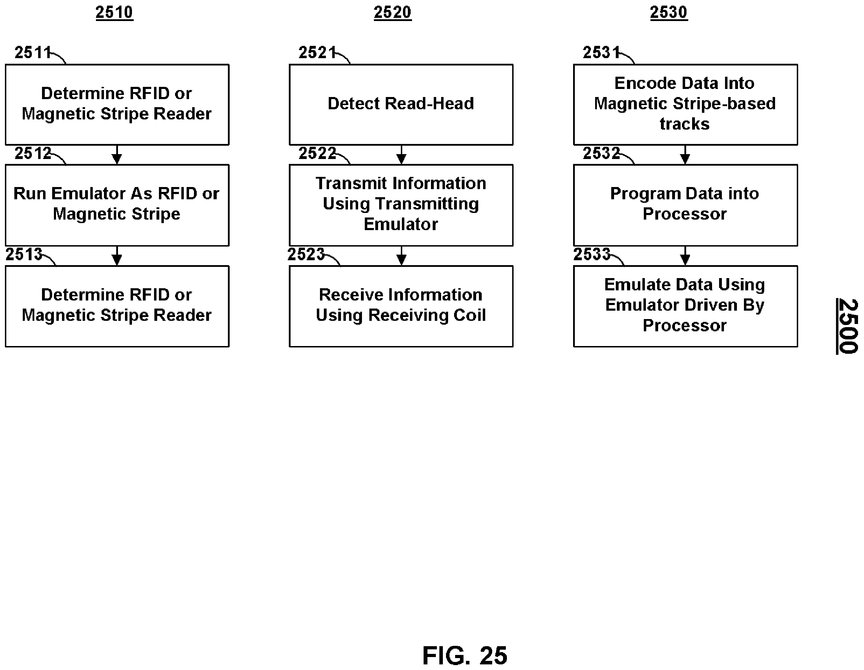

FIG. 25 shows flow chart 2510 that may includes steps 2511-513. Step 2511 may be utilized to determine, of example, whether an RFID or a magnetic stripe reader is within the proximity of a card (or other device). Step 2512 may be utilized to run an emulator as an RFID or magnetic stripe in response to step 2511. Step 2513 may be utilized to determine an RFID and magnetic stripe reader such that the process may be repeated.

Process 2520 may be included and may include step 2521 to detect a read-head. Step 2522 may be included to transmit information using an emulator in a transmitting mode. Step 2523 may be utilized to receive information from an emulator (or receiving coil) in a receiving mode. Persons skilled in the art will appreciate that an emulator may be operating in a receiving mode and a transmitting mode at the same time.

Process 2530 may be included and may include step 2531 to encode data into static magnetic tracks fabricated from a magnetic material. Step 2532 may be provided to program data into a processor to be utilized in a subsequent step (e.g., step 2533). Step 533 may be utilized to emulate data using an emulator driven by the data programmed in the processor.

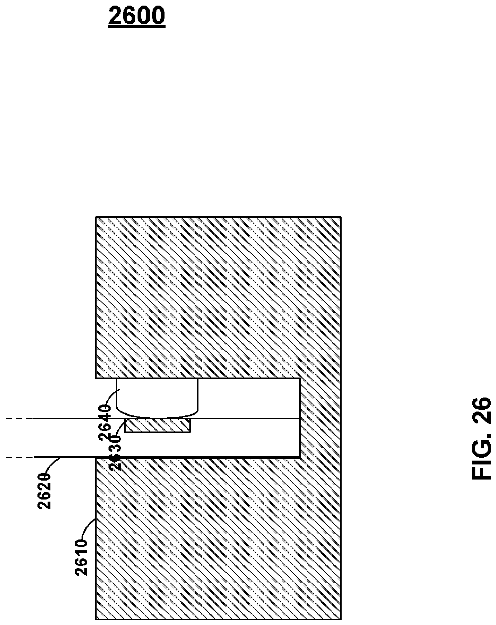

FIG. 26 shows environment 2600 that may include magnetic stripe reader 2610, read-head housing 2640, card 2620, and magnetic emulator 2630. Read-head housing 2640 may include any number of read-head's such as, for example, one, two, or three read-heads. Each read-head may independently receive magnetic fields from magnetic emulator 2630 (or a magnetic stripe, such as a magnetic stripe encoded on-card by card 2620). Emulator 2630 may be positioned to be adjacent to any one or more read-heads of read-head housing 2640 or may be positioned to communicate information to any one or more read-heads of read-head housing 2640. Persons skilled in the art will appreciate that emulators with longer lengths may be located within the proximity of one or more read-heads for a longer duration of time when a card is swiped. In doing so, for example, more information may be transmitted from an emulator to a read-head when a card is being swiped.

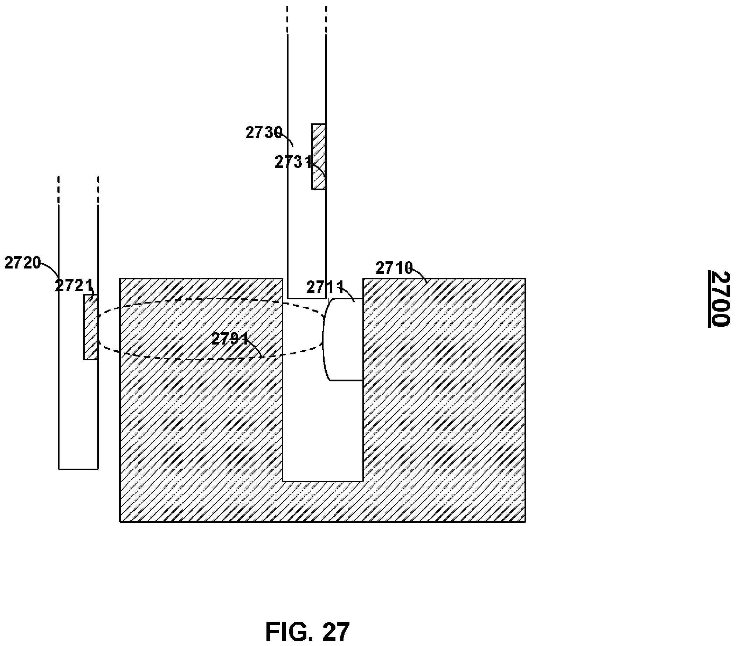

FIG. 27 includes environment 2700 that may include cards 2720 and 2730 as well as magnetic stripe reader 2710. Read-head housing 2711 may be included on a wall of a trough of magnetic stripe reader 2710. The trough may be sized to accept cards (e.g., credit cards).

Card 2720 may include emulator 2721. Emulator 2721 may provide electromagnetic field 2791 that may transmit through a portion of the housing of magnetic stripe reader 2710 (e.g., through a wall of a trough to get to read-head housing 2711). Accordingly, card 2720 may be located outside of a reader - yet still be operable to communicate information to a magnetic stripe reader. A reader may be provided with an outer wall, for example, with a thickness of a quarter of an inch or more. Emulator 2721 can provide electromagnetic field 2791 over a distance of, for example, a quarter of an inch or more.

Persons skilled in the art will appreciate that card 2720 may be coupled to a device via a permanent or removable cable. Such a device may provide power to card 2720 as well as control information - such as control information for emulator 2730. An external source of power may be utilized, for example, to provide a larger amount of electrical energy to emulator 2721 than from a source of power located within card 2720. Persons skilled in the art will appreciate that a car having an internal battery may still be able to receive a cable from a device having its own source of electrical energy.

Card 2730 may be provided with emulator 2731 and may electrically couple with a read-head of magnetic stripe reader 2710. Any number of emulators may be provided in card 2730 in any number of orientations such that the appropriate electromagnetic field may couple with a read head of read-head housing 2711 regardless of the orientation of card 2720 with respect to read-head 2711. More particularly, for example, additional read-head housings may be provided in magnetic stripe reader 2710 at different locations about the reader to electrically couple with a emulators in a number of different configurations. A sticker and/or guide-structures may be provided on a magnetic stripe reader to, for example, direct a user on how to position his/her card (or other device) for contactless transmission of data (e.g., credit card data) to a read-head housing without using the trough that includes that read-head housing.

Persons skilled in the art will appreciate that a magnetic stripe reader may include a trough that includes two (or more) read-head housings 2711 located in approximately the same vertical position on a card-swiping trough, but at different horizontal locations on opposite walls of the trough. In doing so, for example, a magnetic stripe may be read regardless of the direction that a card having the magnetic stripe is facing when the card is swiped. Magnetic emulator 2721 may, for example, communicate magnetic fields outside both the front and read surfaces of a card. Accordingly, a single emulator 2721 may, for example, couple with a single read-head regardless of the direction the card was facing when swiped. In doing so, for example, the costs of readers may be reduced as only a single read-head may be need to receive information regardless of the direction a card is facing when swiped. Accordingly, magnetic readers do not need stickers and/or indicia to show a user the correct orientation to swipe a card through a magnetic stripe reader. An adapter may be provided that coupled directly to a read-head that allows a device not operable to fit in a trough to electrically couple with a read-head.

An dynamic magnetic communications device, such as a emulator, may be positioned about a surface of a card (or other device), beneath a surface of a device, or centered within a card. The orientation of a magnetic emulator in a card may provide different magnetic fields (e.g., different strength's of magnetic fields) outside different surfaces of a card. Persons skilled in the art will appreciate that a magnetic emulator may be printed via PCB printing. A card may include multiple flexible PCB layers (e.g., FR4 layers) and may be laminated to form a card. Portions of an electronic ink display may also be fabricated on a layer during a PCB printing process.

Magnetic shielding may be provided to limit an electromagnetic field of an emulator. For example, layer 2810 may include magnetic shielding 2811 (which may be a magnetic material). Magnetic shielding may block magnetic fields from emulator 2851 on layer 2820. Accordingly, for example, a card may not interact with read-heads blocked from emulator 2851 from magnetic shielding 2811. In doing so, for example, a magnetic stripe reader may receive information from a single read-head housing at any given time. Layer 2830 may be provided, for example, with magnetic shielding 2831 that includes an active-region space 2832. Accordingly, layer 2830 may block magnetic fields from emulator 2851 except for those fields generated by active portion 2854 (e.g., if space 2832 is aligned with active potion 2854).

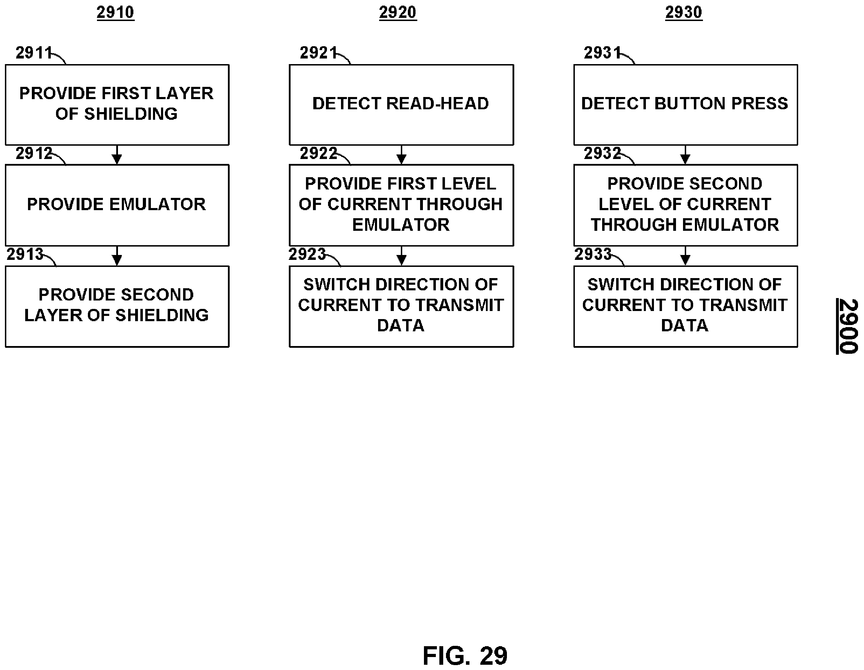

FIG. 29 shows processes 2900 that may include flow chart 2910. Flow chart 2910 may include step 2911, in which a first layer of magnetic shielding may be provided (e.g., printed). Step 2912 may be provided such that, for example, an emulator is provided (e.g., printed). Step 2913 may be included such that, for example, a second layer of shielding may be provided (e.g., printed).

Flow chart 2920 may be included. Step 2921 may be included in flow chart 2920. A read-head may be detected in step 2921, a first level of current may be provided through an emulator in step 2922, and the direction of the current through the emulator may be switched in step 2923 in order to transmit data.

Flow chart 2930 may be included. Step 2931 may be included in flow chart 2930. A button press may be detected in step 2931, a second level of current may be provided through an emulator in step 2932, and the direction of the current through the emulator may be switched in step 2933 in order to transmit data. Flow chart 2921 and 2931 may be utilized together, for example, to provide a multifunction emulator. For example, an emulator may provide a magnetic-stripe signal to a magnetic stripe reader in flow chart 2920 and may provide an RFID signal to an RFID receiver in flow chart 2930.

Persons skilled in the art will appreciate that a number does not need to, for example, change with time. Information can change, for example, based on manual input (e.g., a button press or combination of button presses). Additionally, a credit card number may be a static display number and may be wholly or partially displayed by a display. Such a static credit card number may result in the reduction of fraud if, for example, a personal identification code is required to be entered on a manual input entry system to activate the display. Additionally, fraud associated with card cloning may be minimized with the use of a magnetic emulator activated by the correct entry on a manual input entry system.

Person skilled in the art will also appreciate that a card may be cloned by a thief, for example, when the thief puts a illegitimate credit card reader before a legitimate credit card reader and disguising the illegitimate credit card reader. Thus, a read-head detector may detect a read-head housing and then, if a second read-head housing is detected on the same side of the credit card, the reader may transmit information to the second read-head that signifies that two read-head housings were detected. In doing so, for example, a bank, or the police, may be notified of the possibility of the presence of a disguised cloning device. The information representative of multiple read-heads may be included with information that would allow a credit card number to be validated. As such, a server may keep track of the number of read-head housings at each reader and, if more read-head housings are detected than expected, the server may contact an administrator (or the police). The server may also cause the credit card transaction to process or may reject the credit card transaction. If the number of read-head housings (or read-heads) is the number expected by the server, the server can validate the payment transaction.

A payment system using dynamic numbers may, for example, be operable with numbers that are stored outside of the period in which those numbers would otherwise be valid. A server may be included, for example, that accepts a dynamic credit card number, information representative of a past credit card number, and the merchant that is requesting payment. The server may register that merchant for that saved number. The number may be decrypted (or otherwise validated) for that past period of time. Accordingly, the credit card transaction may be validated. Additionally, the merchant identification information may be linked to the stored dynamic credit card number for that past period of time. If the server receives a transaction from a different merchant with that same dynamic credit card number for that same period of time, the server may reject the transaction. In doing so, a merchant may be protected from having credit card numbers stolen from its various storage devices. If a thief steals a number from a merchant's server that is associated with a past period of time, that number cannot be used, for example, anywhere else. Furthermore, such a topology may, for example, allow merchants to provide a one-click shopping, periodic billing, or any other type of feature that may utilize dynamic numbers that are stored and used outside of the period in which the dynamic numbers were generated.

Persons skilled in the art will appreciate that different emulators may be controlled by different switching circuitry (e.g., different transistors). Opto-isolators may be included to protect the processor from any voltage swings driving a magnetic emulator.

Persons skilled in the art will appreciate that multiple buttons may be coupled together to form a single-bit bus. If any button is pressed, the bus may change states and signal to the processor to utilize different ports to determine what button was pressed. In this manner, buttons may be coupled to non-triggerable ports of a processor. Each button (or a subset of buttons) may be coupled to one or more triggerable ports of a processor. A port on a microprocessor may be utilized to drive an emulator in addition to, for example, receiving information from a button. For example, once an appropriate personal identification code is received by a processor, the processor may utilize one or more ports that receive information from one or more buttons to drive an emulator (e.g., for a period of time). Alternatively, for example, a magnetic emulator may be coupled to its own triggerable or non-triggerable processor port. A card may also include a voltage regulator to, for example, regulate power received from an internal or external source of power.

Persons skilled in the art will appreciate that any type of device may be utilized to provide dynamic magnetic information on a card to a magnetic stripe reader. As discussed above, a magnetic encoder may be provided that can change information on a magnetic medium where the changed information can be detected by a magnetic stripe reader.

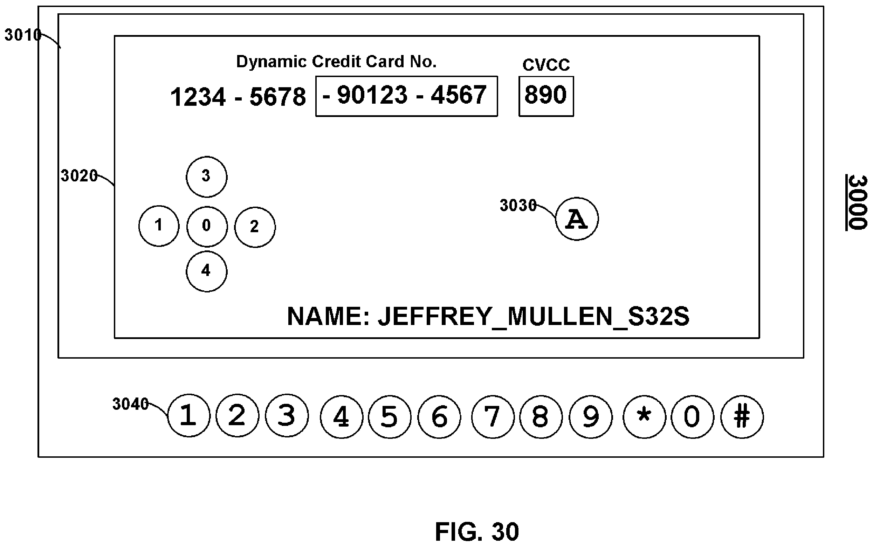

FIG. 30 shows personal electronic device 3000 which may be, for example, a portable telephonic device, portable media player, or any type of electronic device. Persons skilled in the art will appreciate that the functionality of a card may be provided on a personal device and displayed through a graphical user interface. Personal electronic device 3000 may include, for example, user inputs 3040 and display 3010. Virtual card 3020 may be displayed on display 3020. Display 3020 may be a touch-sensitive display such that, for example, virtual button 3030 may be provided on virtual card 3020. Persons skilled in the art will appreciate that cards may be provided as virtual cards and a user may interact with such virtual cards in order to provide a variety of functions. Personal electronic device 3000 may communicate to a card reader such as, for example, an RFID reader.

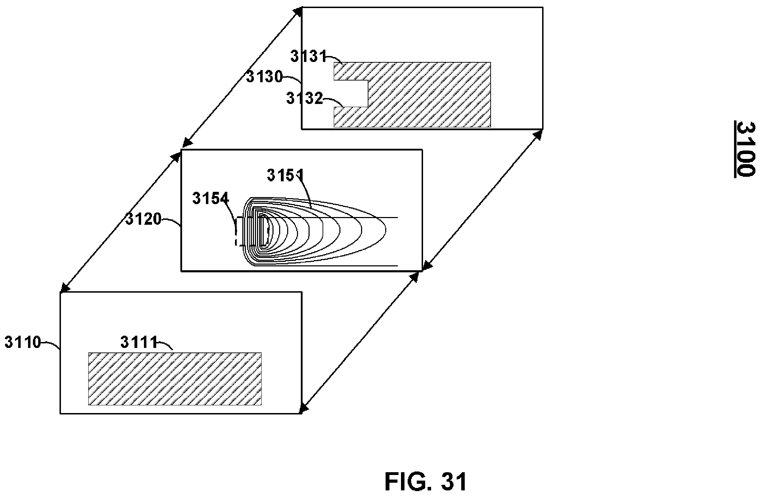

Magnetic shielding may be provided to limit an electromagnetic field of an emulator. For example, layer 3110 of FIG. 31 may include magnetic shielding 311 (which may be a magnetic material). Magnetic shielding may block magnetic fields from emulator 3151 on layer 3120. Accordingly, for example, a card may not interact with read-heads blocked from emulator 3151 from magnetic shielding 3111. In doing so, for example, a magnetic stripe reader may receive information from a single read-head of a read-head housing at any given time. Layer 3130 may be provided, for example, with magnetic shielding 3131 that includes an active-region space 3132. Accordingly, layer 3130 may block magnetic fields from emulator 3151 except for those fields generated by active portion 3154 (e.g., if space 3132 is aligned with active potion 3154).

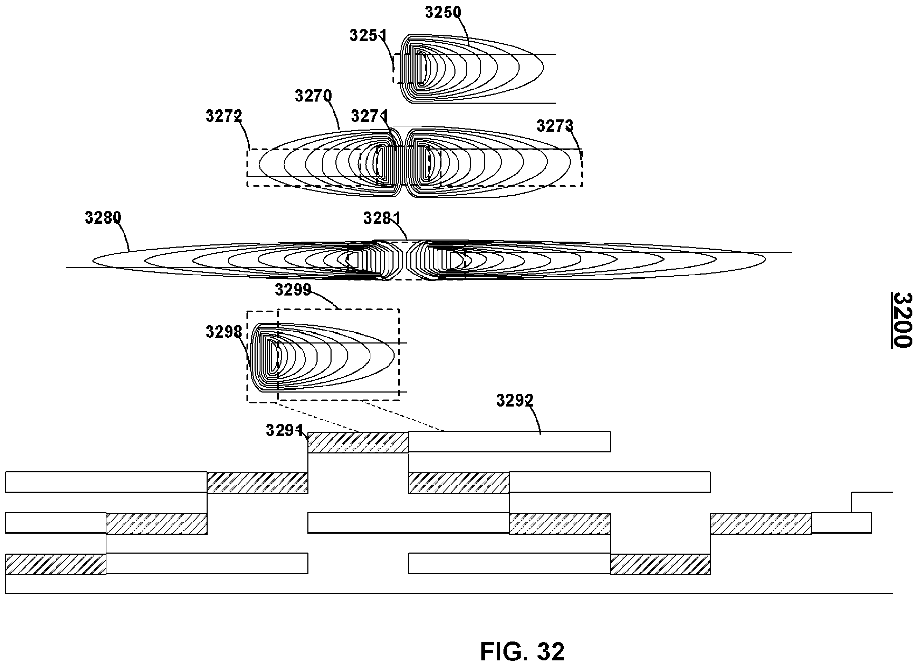

FIG. 32 includes circuits 3200 that may include magnetic emulator 3250 that includes active region 3251. Magnetic emulator 3250 may be, for example, a coil. Current may be provided through magnetic emulator 3250 such that the magnetic emulator generates an electromagnetic signal. Active region 3251 may include a dense section of coil segments where current runs through those coil segments in the same direction. Accordingly, the electromagnetic field is intensified in active region 3251 compared to the area of the coil with coil segments that are widely spaced. Accordingly, a current may be placed through the coil such that a magnetic stripe reader is operable to receive information from active region 3251 but not the region outside active region 3251. Persons skilled in the art will appreciate that the direction of current through magnetic circuit 3250 may be reversed in a pattern that is representative of magnetic stripe data. Particularly, a processor may, for example, transmit information through a coil by changing the direction of the electromagnetic field generated from emulator circuit 3250 at particular times. A change in the frequency of field reversals may be representative of, for example, a particular bit of information (e.g., "1" or "0"). Magnetic emulation circuit 3250 may include a dense active region and a less dense tail region.

Magnetic emulation circuit 3270 may be included that includes active region 3271 and non-active regions 3272 and 3273. Active region 3271 may include coil segments in which current flows through the segments in the same direction. Non-active regions 3272 and 3273 may include, for example, coil segment spacing that is wider than the coil segment spacing of active regions 3271. Persons skilled in the art will appreciate, for example, that non-active regions 3272 and 3273 may be utilized to communicate information to a magnetic stripe reader. However, active region 3271 may include a stronger electromagnetic signal then non-active regions 3272 and 3273. Persons skilled in the art will also appreciate that a read-head may travel through non-active region 3272, through active region 3271, and through non-active region 3273 when reading information communicated from emulator circuit 3270. Persons skilled in the art will appreciate that the level of current provided to emulator circuit 3270 may be configured such that a magnetic read-head received information from active region 3271 but does not receive information from non-active regions 3272 and 3273. Emulator 3270 may, for example, be tall enough such that more than one read-head passes over emulator 3270 at any one time. For example, one read-head from a magnetic stripe reader may pass over active region 3271 and another read-head from a magnetic stripe reader may pass under active region 3271. Person skilled in the art will appreciate that coil segments may be configured in an orientation such that they produce electromagnetic fields that are invisible to such read-heads. For example, active region 3271 may include coil segments that are parallel to one another. Coil segments above and below active region 3271 may be configured to be perpendicular to the coil segments of active region 3271 or approximately oriented at a 45 degree angle from the coil segments of active region 3271.

Magnetic emulation circuit 3280 may be provided that includes active region 3281. Persons skilled in the art will appreciate, for example, that the height of magnetic emulation circuit 3280 may be approximately equal to the height of a read-head of a magnetic stripe reader such that, for example, only one track read-head, of a multiple track read-head housing, passes over magnetic emulation circuit 3280.

Multiple magnetic emulation circuits may be provided on a multiple layer PCB. For example, a magnetic emulation circuit may be provided with active region 3291 (e.g., active region 3298) and non-active region 3292 (e.g., non-active region 3299). Emulators may be provided on different layers such that active layers align with non-active layers. Accordingly, for example, a read-head may pick up a continuous stream of active regions. Accordingly, the active regions may be controlled through a common switching circuit such that current flows in the same direction through the active regions of the multiple layer PCB at a given time. Accordingly, the active regions may, for example, provide electromagnetic fields in the same direction. Persons skilled in the art will appreciate that the widths of coil segments of non-active regions may be widened to decrease the effect of those non-active regions when multiple non-active regions are stacked. Additionally, for example, the coil segments of vertically stacked non-active regions may be aligned with one another or may be staggered from one another. Furthermore, for example, current may flow through vertically stacked non-active regions in the same or different directions.

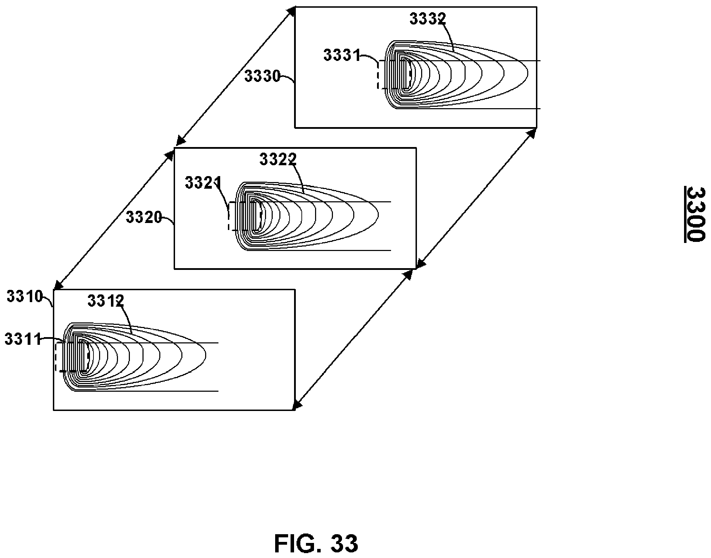

FIG. 33 shows card 3300 that may include layers 3310 , 3320, and 3330 fabricated as, for example, flexible PCB layers. Layer 3310 may include, for example, magnetic emulation circuit 3312 that includes active region 3311. Layer 3320 may include, for example, magnetic emulation circuit 3322 that may include active region 3321. Layer 3330 may include magnetic emulation circuit 3332 that may include active region 3331.

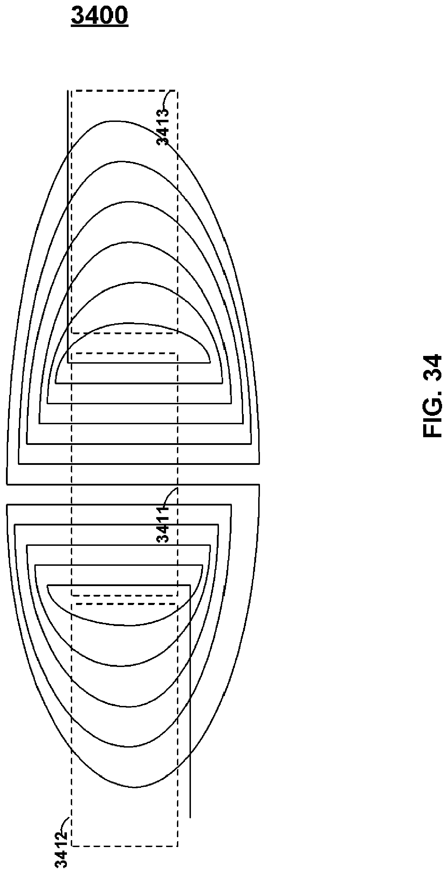

FIG. 34 includes circuit 3400 that may include, for example, active-region 3411 and non-active regions 3412 and 3413.

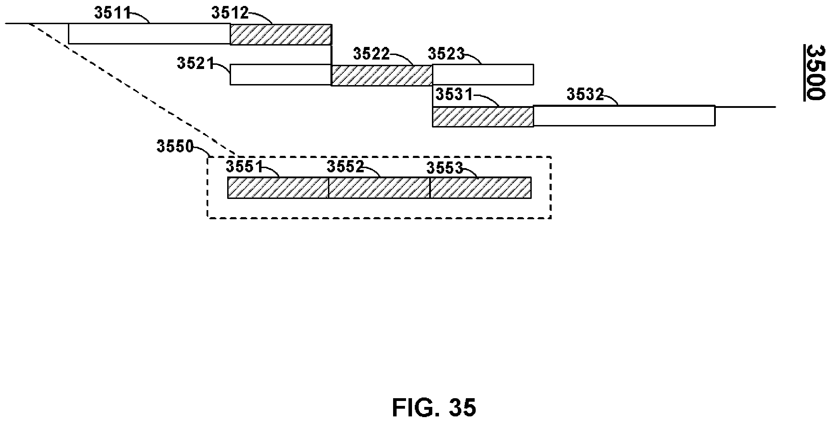

Persons skilled in the art will appreciate that magnetic emulation circuits with one non-active region may be vertically stacked with magnetic emulation circuits with two non-active regions. Magnetic emulation circuits with one non-active region may have non-active regions that are wider than the non-active regions of a magnetic emulation circuit with two non-active regions. FIG. 35 shows topology 3500 that may include active region 3512 (e.g., active region 3551), active region 3522 (e.g., active region 3552), and active region 3531 (e.g., active region 3553). Non-active regions 3511, 3521, 3523, and 3532 may also be included.

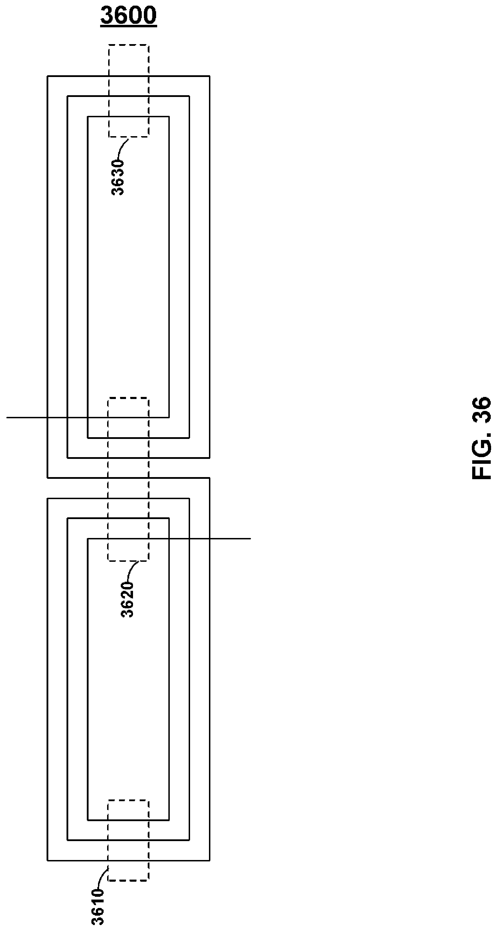

FIG. 36 shows magnetic emulation circuit 3600 that may include region 3610, 3620, and 3630. Regions 3610, 3620, and 3630 may include approximately the same spacing between coil segments or may provide different spacing between coil segments.

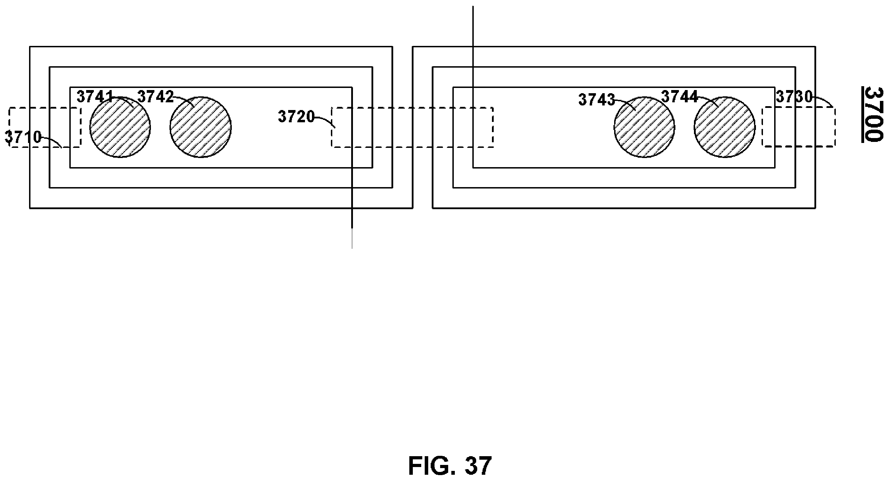

FIG. 37 shows circuit 3700 that may include region 3710, 3720, and 3730. Circuit 3700 may also include read-head detectors 3741, 3742, 3743, and 3744. Read-head detectors 3741, 3742, 3743, and 3744 may detect a read-head and/or a read-head housing of a magnetic stripe reader. A read-head detector may determine the presence of a read-head and/or read-head housing by detecting, for example, physical contact with a read-head and/or housing. Capacitive coupling, sonar, optical, or any other technique may be utilized to determine the presence of a read-head or read-head detector. Accordingly, for example, region 3620 may be utilized to communicate information to a magnetic stripe reader upon the detection of a read-head by a read-head detector. Accordingly, for example, no current may be provided through regions 3610 and 3630 when a read-head passes over regions 3610 and 3630. Multiple read-heads may be utilized, for example, to determine the direction that a read-head is moving as well as the read-heads velocity and/or acceleration. Such information may be utilized, for example, to provide information in different ways. For example, a processor may transmit information at a first rate when a user swipes a card including circuit 3700 at a first velocity and the processor may transmit the same (or different) information at a second rate when that user swipes the card at a second velocity.

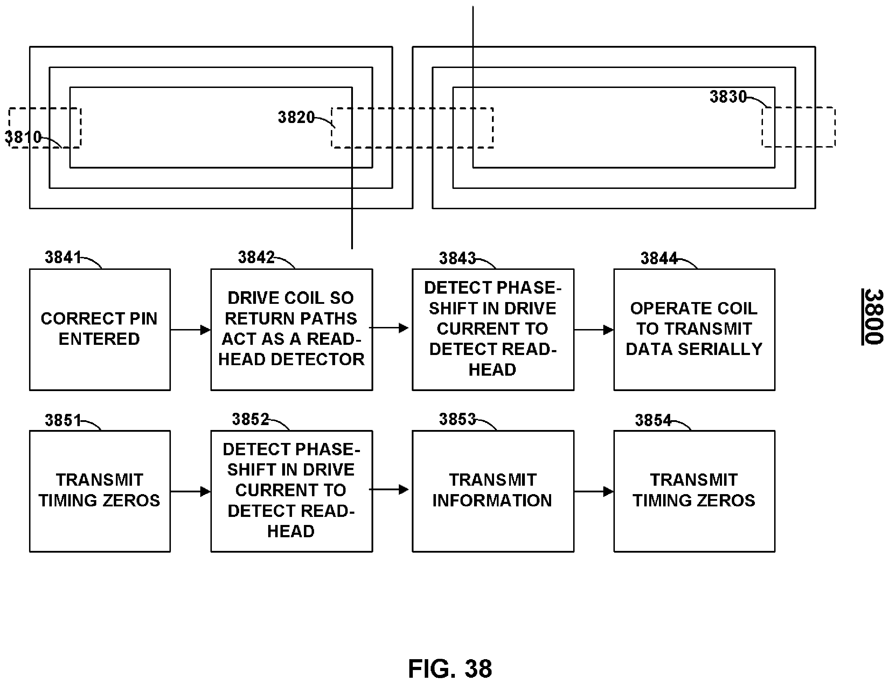

FIG. 38 shows topology 3800 that may include a magnetic emulation circuit having regions 3810, 3820, and 3830. Persons skilled in the art will appreciate that a magnetic emulation circuit may act as a read-head detector as well as a magnetic information transmitter. For example, a magnetic emulator may be driven according to a process that includes step 3841, in which a correct Personal Identification Code (e.g., a PIN) is determined to have been entered on a card. Accordingly, step 3842 may activate, in which a coil is driven such that its return paths act as a read-head detector. This may be done in numerous ways. For example, the current providing an electromagnetic field may undergo a phase-shift when a magnetic and/or conductive material is placed in the electromagnetic field. Accordingly, a phase-shift may be determined in step 3843. When such a phase-shift is determined, step 3844 may initiate and a magnetic emulation circuit may be driven to communicate data serially. Accordingly, regions 3810 and 3830 may be utilized to detect a read-head and region 3820 may be utilized to communicate information to that read-head. Persons skilled in the art will appreciate that a magnetic emulation circuit may not be supplied current until an appropriate Personal Identification Code (PIC) is entered into manual interfaces located on the card. Such a scheme, for example, provides for power savings as well as prevents card cloning. Accordingly, a magnetic emulator may be driven into a read-head detector mode upon receiving an appropriate manual input and then into a data transmission mode after determining the presence of a read-head.

Persons skilled in the art will appreciate that timing zeros may be provided before and after data such that a magnetic stripe reader can utilize such timing zeros to perform synchronization activities. Accordingly, a magnetic stripe emulator may transmit zeros while in a read-head detector mode in step 3851 and upon the detection of a current phase-shift in step 3852 the magnetic emulation circuit may transmit information in step 3853. After the data is transmitted, timing zeros may be transmitted again in step 3854. Step 3853 may also detect the presence of a read-head to determine, for example, whether all of the information was received. If a read-head was not detected after data transmission, the processor may increase the rate of data transmission for future transmissions until, for example, read-heads are detected at the beginning and the end of the transmission of data.

Persons skilled in the art will appreciate that a card may include multiple magnetic emulation and read-head detectors circuits. In this manner, a card may include multiple circuits that can operate as both a data transmitter and a read-head detector.

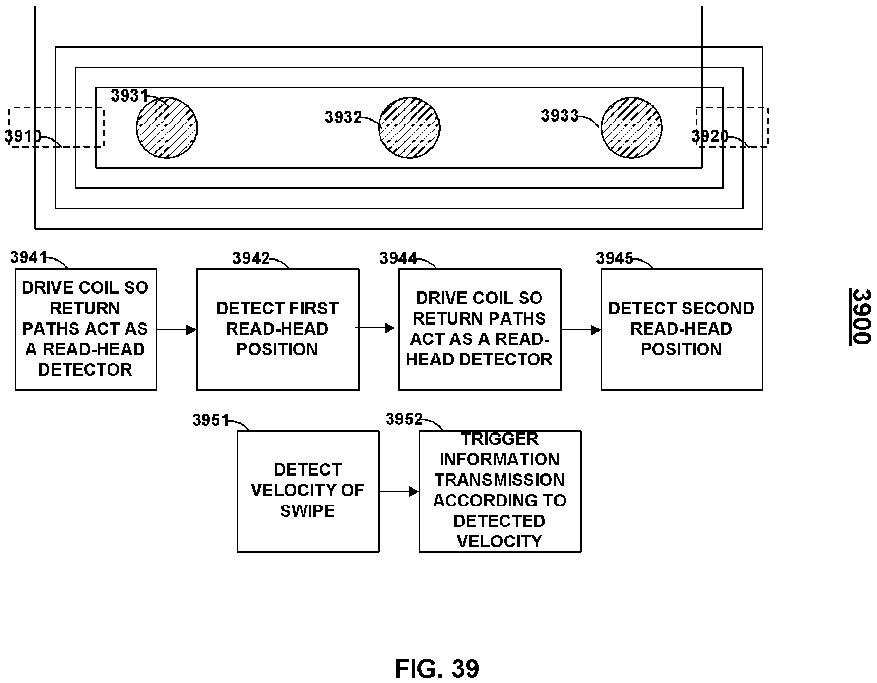

FIG. 39 includes topology 3900 that may include, for example, a magnetic emulation circuit that includes regions 3910 and 3920. Read-head detectors 3931, 3932, and 3932 may also be included.

A region (e.g., region 3910) may be utilized to determine the velocity of a read-head. For example, step 3941 may be included in which a region is driven such that the region acts as a read-head detector. A read-head may be detected in 3942, but the region may continue to drive the region to act as a read-head detector in step 3944. A second read-head position may then be determined in step 3945. The number of times that a read-head is sensed by a detector may correlate to a velocity. Information may then be transmitted (e.g., via another region) depending on the determined velocity. For example, the velocity of a swipe may be detected in step 3951 and information may be transmitted according to this velocity in step 3952. Read-head detectors 3931, 3932, and 3933 may also be utilized, for example, to determine the velocity of a read-head as well as the direction of movement of the read-head. Persons skilled in the art will appreciate that a magnetic stripe reader may be motorized and may read a card at a pre-determined speed. Such a card may determine that the card is being read by a motorized reader and may utilize this information to transmit different information and/or transmit information in a different manner.

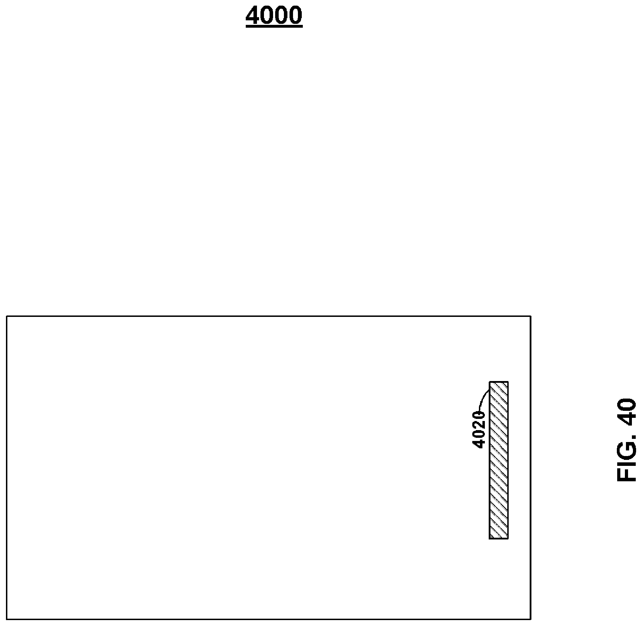

FIG. 40 shows card 4000 that may include magnetic emulation circuit 4020. Magnetic emulation circuit 4020 may be included on a short side of a rectangle-shaped card - yet may transmit serially all of the information on a track of financial payment data (e.g., credit and/or debit data). Such transmitted financial payment data may be utilized to validate a financial payment (e.g., a credit card purchase). Any number of magnetic emulation circuits may be included on any side of card 4000. For example, a magnetic emulation circuit may be provided on a long side of a rectangle-shaped card and another magnetic emulation circuit may be provided on the other long side of a rectangle-shaped card. Each magnetic emulation circuit may be utilized to transmit different data or the same data.

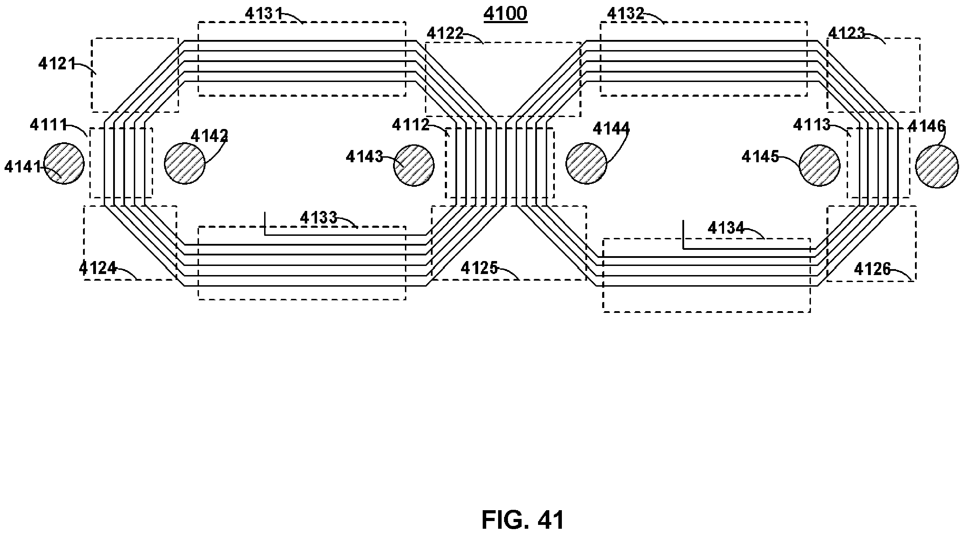

FIG. 41 includes circuit 4100 that may include read-head detectors 4141, 4142, 4143, 4144, 4145, and 4146. Circuit 4100 may include a magnetic emulation circuit that includes regions 4111, 4112, 4113, 4121, 4131, 4122, 4132, 4123, 4124, 4133, 4125, 4134, and 4126. Regions 4111-4113 may be operable to communicate with a read-head that passes through regions 4111-4113. The magnetic emulation circuit may include a coil. Regions 4111-4113 may include, for example, coil segments that are parallel to one another. The coil segments may produce an electromagnetic field operable to communicate data to a read-head of a magnetic stripe reader. The coil segments of regions 4121-4126 may be oriented approximately at a 45 degree angle from the coil segments of regions 4111-4113. Accordingly, for example, the orientation offset of regions 4121-4126 from regions 4111-4113 may allow regions 4121-4126 to produce an electromagnetic field that cannot communicate with a read-head of a magnetic stripe reader when regions 4111-4113 are communicating data to a read-head of a magnetic stripe reader. Similarly, the coil segments of regions 4131-4134 may be oriented perpendicular to the coil segments of regions 4111-4113. Accordingly, for example, the orientation offset of regions 4131-4134 from regions 4111-4113 may allow regions 4131-4134 to produce an electromagnetic field that cannot communicate with a read-head of a magnetic stripe reader when regions 4111-4113 are communicating data to a read-head of a magnetic stripe reader.

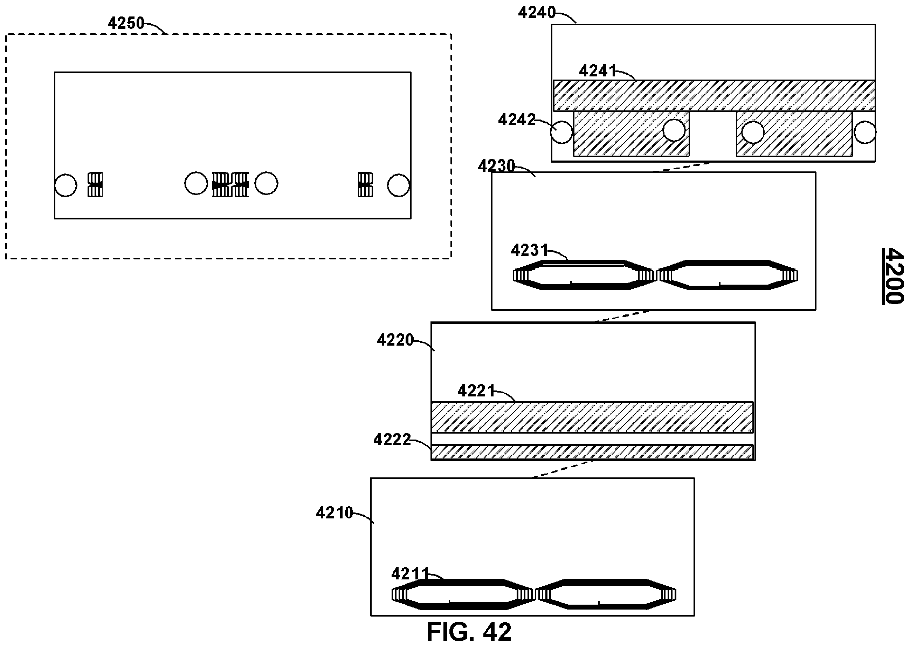

FIG. 42 shows card layout 4200 that may include flexible PCB layers 4210, 4220, 4230, and 4240 to form card 4250. Layer 4210 may include magnetic emulation circuit 4211. Layer 4220 may include magnetic shielding 4221 and 4222. Layer 4230 may include magnetic emulation circuit 4231. Layer 4240 may include read-head detectors (e.g., read-head detector 4242) and magnetic shielding (e.g., shielding 1641).

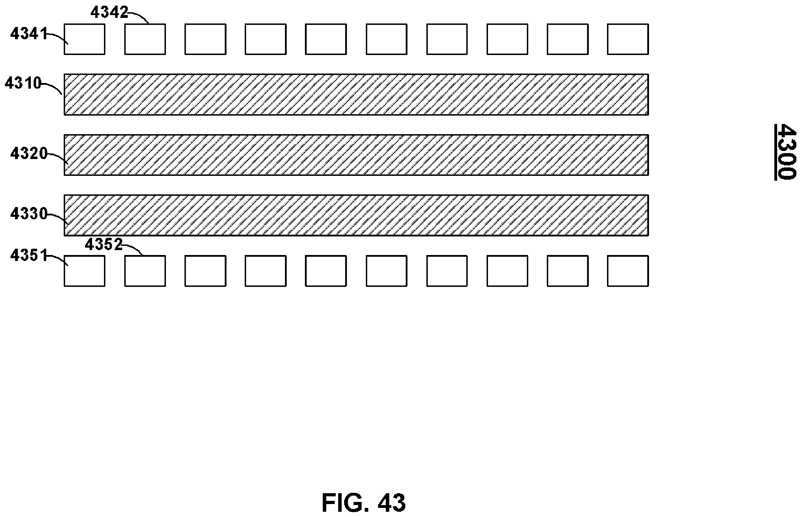

FIG. 43 includes circuit 4300 that may include, for example, read-head detectors 4341, 4342, 4351, and 4352. Circuit 4300 may also include, for example, magnetic emulation circuits 4310, 4320, and 4330. Read-head detectors 4341, 4342, 4351, and 4352 may be utilized as read-head detectors and may provide the capability for a read-head to be detected when the read-head is over a particular magnetic emulation circuit or portion of a magnetic emulation circuit. Read-head detectors 4341, 4342, 4351, and 4352 may detect a read-head by sensing, for example, capacitive coupling of a read-head. Alternatively, for example, a read-head contact may be, for example, pressed against another contact when pressure is applied via a read-head.

Persons skilled in the art will appreciate that a display on the card may display a credit card number that does not change with time. Additionally, for example, a magnetic emulator (or multiple magnetic emulators) may magnetically communicate financial data that does not change with time. Such a card may reduce, for example, the effects of physical card theft and card cloning.

One or more light generation devices, such as a Light Emitting Diode (LED), may be provided as part of a card (or other device). Such an LED may produce light, for example, upon a manual input such as a button press, the correct entry of a PIC such as a PIN, and/or the incorrect entry of a PIC. A light emitting device may be operable to produce different colors of light. For example, the incorrect entry of a PIC may produce a red light and the correct entry of a PIC may produce a green light. A PIC may take any form such as a numerical code or a code that include alphabet letters and/or symbols. For example, a PIC may be "A-B-B-B-A" and an "A" button may be provided on a card in addition to a "B" button (as well as other buttons such as a "C," "D," and/or "E" buttons).

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipts may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

A website may be provided where a user enters in his/her credit card number, pays a fee, and a new card is programmed and sent to the user. The new card may include a display to display a portion of the users credit/debit card number in a static form upon entry of an appropriate PIC. Such a card may also include one or more magnetic emulation circuits to transmit the information to a reader. Such a card may or may not, for example, include a portion of a magnetic stripe. For example, three tracks of magnetic stripe data may be communicated via three different emulation circuits, more than three different emulation circuits, one emulation circuits (e.g., tracks communicated serially to all read-heads), or one or more tracks may be represented by magnetic stripe(s) while one or more other tracks may be represented by a magnetic emulation circuit. A track of data may also be partially represented by a magnetic emulation circuit and partially represented by a magnetic stripe.

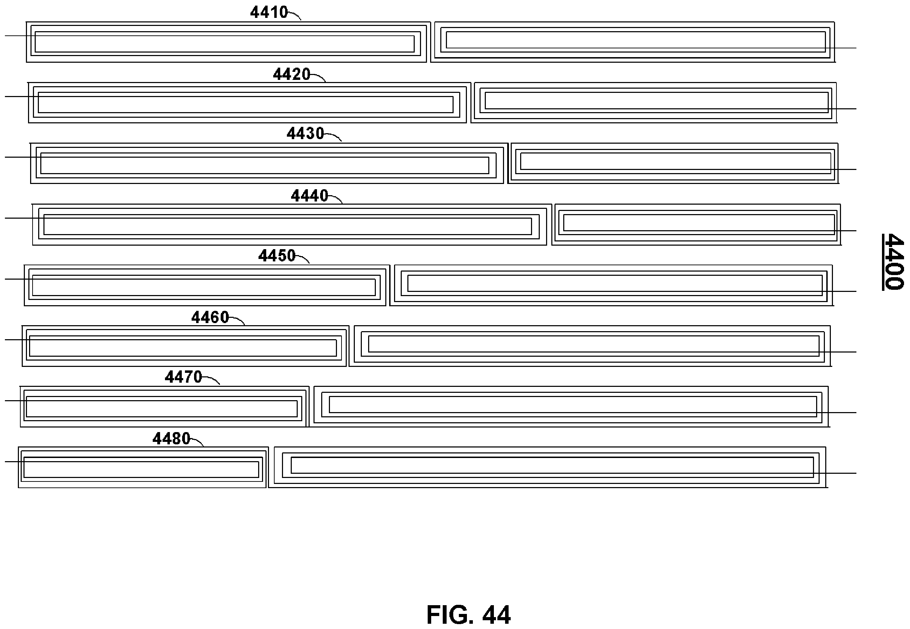

FIG. 44 includes topology 4400 that may be utilized to communicate information to a read-head. Topology 4400 may include magnetic emulators 6610, 4420, 4430, 4440, 4450, 4460, 4470, and 4480. Each of these magnetic emulators may be provided on a different layer and aligned such that the primary paths are seen, from the perspective of a read-head as one set of paths. The return paths may be aligned to minimize the amount of time a read-head is located above the return paths of the magnetic emulators. The magnetic emulators may be coupled in series or operated independently. Additional layers may be included to include additional routing between the magnetic emulators. A magnetic emulator may be, for example, a flat coil placed on a single layer of material. Current may be provided through a magnetic emulator such that the magnetic emulator generates an electromagnetic signal. A primary path of a coil may include a dense section of coil segments where current runs through those coil segments in the same direction. Accordingly, the electromagnetic field is intensified in active region compared to the area of the coil with coil segments that are widely spaced or not configured in an orientation where the magnetic fields from these coil segments can be read. Accordingly, a current may be placed through the coil such that a magnetic stripe reader is operable to receive information from active region 4451 but not the region outside active region 4451. Persons skilled in the art will appreciate that the direction of current through magnetic circuit 4450 may be changed and controlled in a pattern that is representative of magnetic stripe data. Particularly, a processor may, for example, transmit information through a coil by changing the direction of the electromagnetic field generated from emulator circuit at particular times. A change in the frequency of field reversals may be representative of, for example, a particular bit of information (e.g., "1" or "0"). Magnetic emulation circuit 4450 may include a dense active region and a less dense return paths. A magnetic emulation circuit may include return paths that also can transmit information to a read-head, but that provides an electromagnetic field in an opposite direction to that of a primary path. Accordingly, read-head sensors may be placed, for example, such that no current is provided through a magnetic emulator while a read-head is over a return path, but the emulator is utilized to communicate information when the read-head (or read-heads are located over the primary path). Additionally, a processor may change how an emulator is controlled in order to accommodate the different electromagnetic fields produced by the two regions such that a portion of a set of data can be transmitted by a return path and a portion of the set of data can be transmitted by a primary path. If zones, for example, are implemented, the return path may be utilized to communicate a portion of information when that zone associated with the return path is utilized and primary paths may be utilized to communicate another portion of information the zone associated with the primary path is activated. Accordingly, return paths may be staggered in order to increase their total visibility to a read-head. Magnetic emulators 4410, 4420, 4430, 4440, 4450, 4460, 4470, 4480, and 4480 may be sized to fit, for example, within the footprint of a track of data for a payment card. The primary paths of these magnetic emulators may be staggered such that, from above, a continuous set of primary path segments is seen. Accordingly, the emulators may be controlled in the same way such that, for example, a continuous electromagnetic field is produced and can be controlled. Accordingly, data can be communicated serially through this continuous electromagnetic field to a magnetic stripe reader. An instance of topology 4400 may, for example, be provided for each track of information on a magnetic card. Different magnetic emulator topologies may be utilized for different tracks. Different magnetic emulators in a magnetic emulator topology may be provided different amounts of current such that emulators at different depths can provide a sufficient electromagnetic field at the surface of topology 4400.

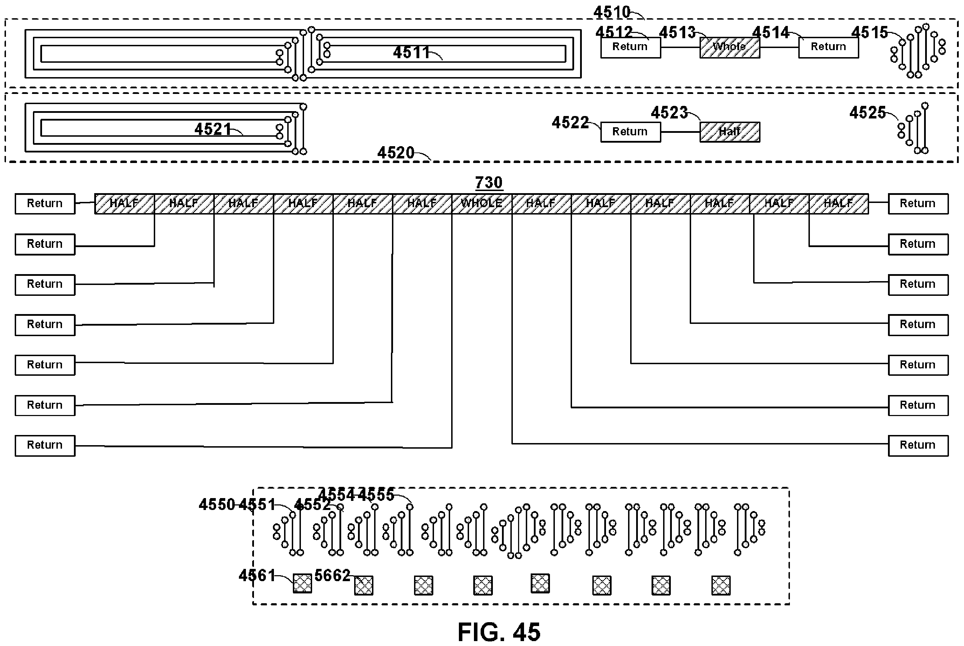

FIG. 45 shows topology 4500 where the primary paths of multiple emulators are provided on a layer of material and the return paths for the coils that are included in those emulators are provided on different layers. Accordingly, for example, each emulator may be provided with the same amount of current to produce a sufficient electromagnetic field. Such coils may be, for example, flat coils that are bent onto multiple layers (e.g., utilizing vias).

Topology 4500 may include coil 4511 with two portions of return paths and one portion of primary paths that may be, for example, visualized as return portion 4512, primary portion 4513, and return portion 4514. The primary portion may be viewed from an eagle-eye perspective as portion 4515. Multiple emulator design 4530 is included that may include multiple emulators with primary paths located adjacent to one another to provide a continuous electromagnetic field. The emulators may be controlled independently such that different emulators are utilized to communicate information at different times. Accordingly, emulators may be utilized from left-to-right as a reader passes over the emulators from left-to-right. For example, read-head detector 4561 of circuit 4550 may detect the presence of a read-head (e.g., a housing that stores a read-head) and may signal the processor to communicate with emulators 4551 and 4552. When read-head detector 4562 detects the presence of the read-head, read-head detector 4562 may signal the processor to communicate with emulators 4552, 4554, and 4555. Read-head detectors 4561 and 4562 may be, for example, contacts coupled to a capacitive sensing circuit or different capacitive sensing circuits.

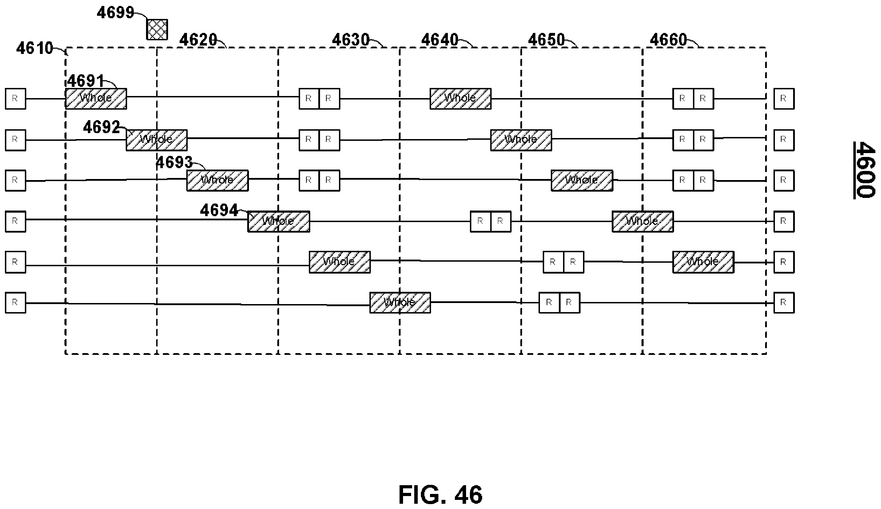

FIG. 46 shows topology 4600 that may include, for example, zones 4610, 4620, 4630, 4640, 4650, and 4660. Topology 4600 may be a cross-sectional perspective of a multiple layer card. Primary paths 4691 and 4692 may, for example, be located in zone 4610 and primary paths 4692, 4693, and 4694 may be located in zone 4620. Primary path 4692 may be utilized, for example, to produce a transitional electromagnetic field between zones 4610 and 4620. Read-head detector 4699, for example, may be utilized to signal the processor to change from operating zone 4610 to operating zone 4620 to communicate data. Primary portions may be divided into more than one primary portion and placed in different zones such that an emulator that includes a coil having two primary portion may cover more than one zone as well as non-adjacent zones. Similarly, return paths portions may be utilized to communicate data and primary portions may not be utilized (e.g., using a zone-based control methodology).

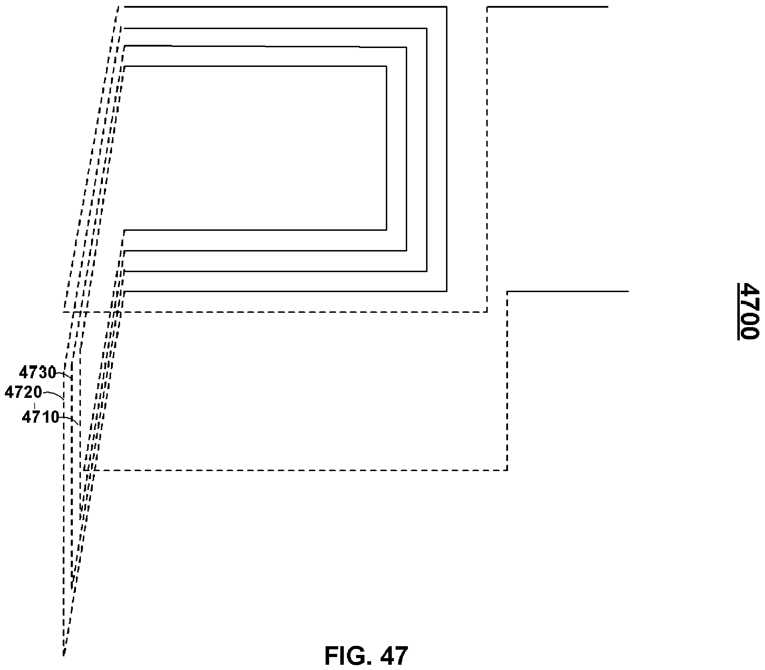

FIG. 47 shows circuit 4700 that may include primary paths on one layer, but that may place a different return path on different vertical layers such that the return paths are vertically stacked. In doing so, the area of the return paths (as viewed by a read-head) may be minimized. In a zone-based control topology, minimizing the read-head visibility to the return paths may, for example, allow for a layer margin of error when detecting the location of a read-head. Circuit 4700 may include return paths 4710, 4720, and 4730.

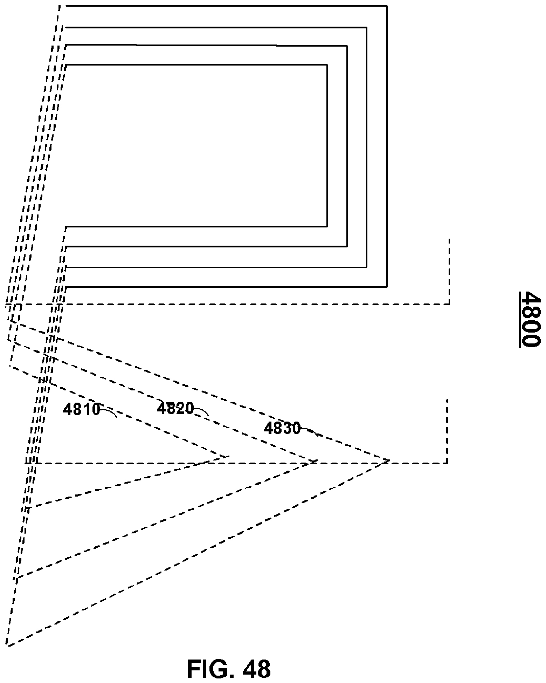

FIG. 48 includes circuit 4800 that may include, for example, a return portion that forms a triangle such that a return path includes two segments that are angled from one another. Each segment may be at a 45 degree angle from a path (e.g., a path it is vertically aligned with) and the two may be, for example, at a 90 degree angle from one another. One or more materials may be placed between the primary paths and the return paths either from the perspective of going into the page or spanning the length of the page (e.g., threading through the open loop created by the primary and return paths). Circuit 4800 may include return paths 4810, 4820, and 4830.

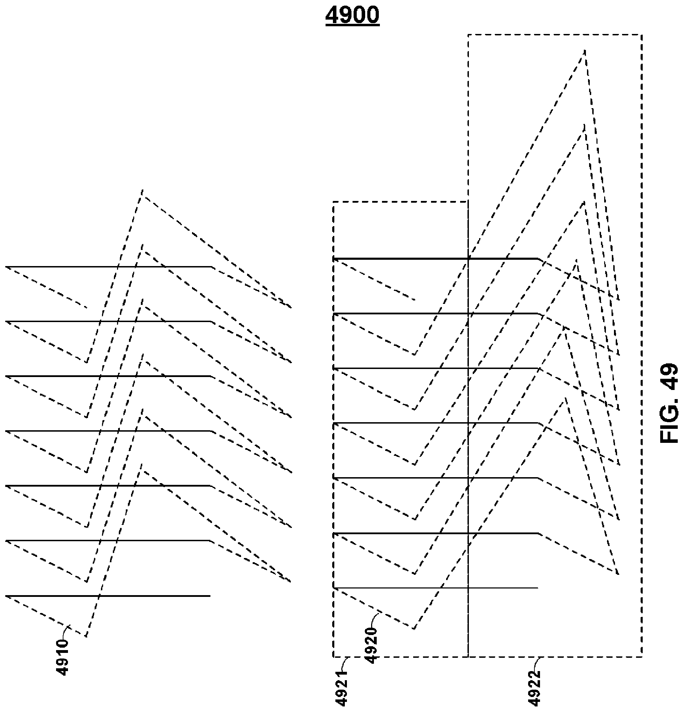

FIG. 49 includes circuit 4910 that may be, for example, a coil. Circuit 4920 may be included that includes two angled return path segments that are located in area 4922, which is outside track footprint 4921. Area 4922 may correspond to an area, for example, that is outside of where a read-head (or read-head housing) passes.

FIG. 5000 shows coil 5011 that may include interior material 5010. Multiple pieces of interior material 5010 may be provided inside of coil 5011. Interior material 5010 may be, for example, a magnetic, ferromagnetic, and/or a ferromagnetic material. A powder may be utilized. An alloy may be utilized. An interior material may extend various distances outside of a coil. An interior material may end at the last coil segment of a coil. Coil 5021 includes interior segment 5020.

A coil may be wrapped around interior materials of different shapes and sizes. A gap between a coil may be provided having various distances. A material may be provided between the interior material and the coil such that the interior material does not, for example, short the coil. An interior material may be, for example, rectangular (e.g., material 5030), square (e.g., material 5040), or cylindrical (e.g., material 5050).

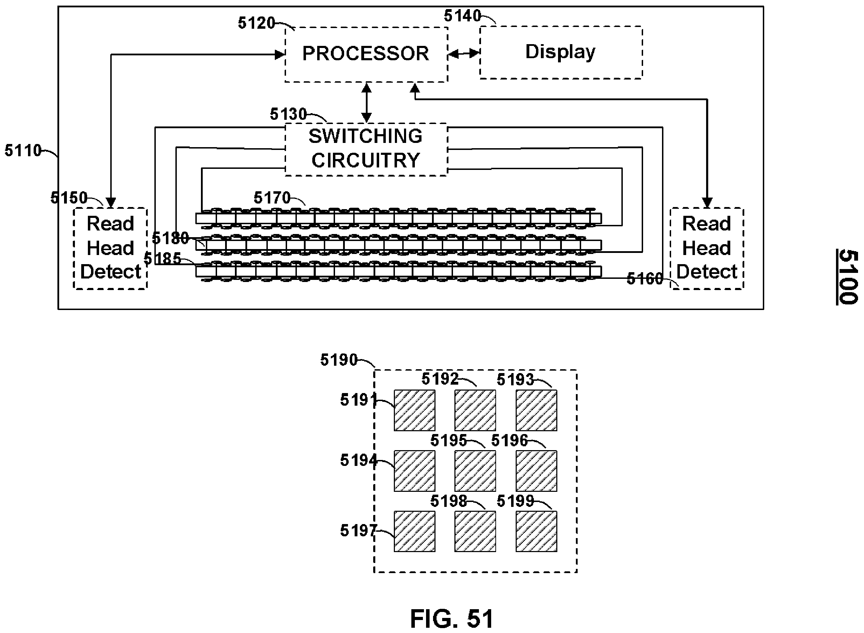

FIG. 51 shows card 5100 that may include, for example, processor 5120, display 5140, switching circuitry 5130, read-head detectors 5150 and 5160, and coils 5170, 5180, and 5185. Coils 5170, 5180, and 5185 may span the length of a track and may communicate information serially. Coils 5170, 5180, and 5185 may be spaced and controlled such that the electromagnetic field from one coil does not introduce interference in a read-head attempting to read information from another track (e.g., an adjacent track). An interior material may be provided to assist in manipulating the profile of the electromagnetic field provided by a coil.

A read head detector may include a cluster of read-head detectors such as cluster 5190. Cluster 5190 may include read-head detectors 5191-5199. Each read-head detector may be, for example, a capacitive sensor. The read-head detectors may thus, for example, be conductive areas coupled to a capacitive sensing circuit. Cluster 5190 may be coupled to a multi-channel capacitive sensing circuit. Each of the contacts of a cluster may be coupled to their own capacitive sensing circuit. A microprocessor may be utilized in capacitive sensing. Any type of read-head detector may be utilized in cluster 5190. For example, a read-head detector may include a physical contact, proximity, optical, or other detector. A cluster may include multiple different types of read-head detectors. Cluster 5190 may be utilized to discern, for example, between different objects. For example, a processor may determine that a user swiped the card through a reader if, for example, 1) only read-head detector 5196 detects an object; 2) after read-head detector 5196 detects an object, only read-head detector 5195 detects an object; and 3) after read-head detector 5195 detects an object, only read-head detector 5194 detects an object. Accordingly, different detection profiles can be associated with readers such that if other types of objects are detected, the processor can discern between these objects and read-heads. Different profiles may be associated with different readers (e.g., motorized, insertion-swipe, and user-swipe). Such different profiles may be utilized to better combat card cloning as, for example, a fake reader overlaid on top of a legitimate ATM machine may have a different profile.

Persons skilled in the art will appreciate that a magnetic emulation circuit may act as a read-head detector as well as a magnetic information transmitter. For example, a magnetic emulator may be driven according to a process that includes a step in which a correct Personal Identification Code (e.g., a PIN) is determined to have been entered on a card. Accordingly, another step may activate, in which a coil is driven such that its return paths act as a read-head detector. This may be done in numerous ways. For example, the current providing an electromagnetic field may undergo a phase-shift when a magnetic and/or conductive material is placed in the electromagnetic field. Accordingly, a phase-shift may be determined. When such a phase-shift is determined, a step may initiate and a magnetic emulation circuit may be driven to communicate data serially. Accordingly, some region(s) may be utilized to detect a read-head and other region(s) 1220 may be utilized to communicate information to that read-head. Persons skilled in the art will appreciate that a magnetic emulation circuit may not be supplied current until an appropriate Personal Identification Code (PIC) is entered into manual interfaces located on the card. Such a scheme, for example, provides for power savings as well as prevents card cloning. Accordingly, a magnetic emulator may be driven into a read-head detector mode upon receiving an appropriate manual input and then into a data transmission mode after determining the presence of a read-head.

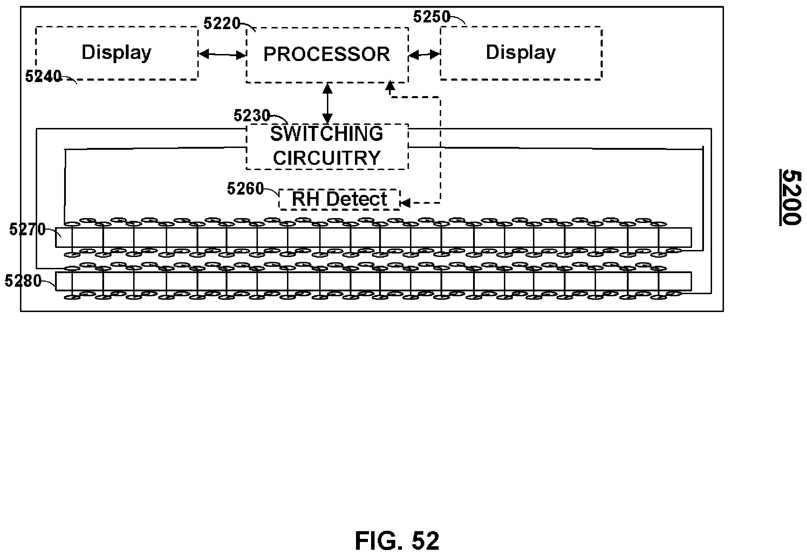

FIG. 52 shows card 5200 that may include display 5240, processor 5220, display 5250, switching circuitry 5230, emulators 5270 and 5280, and read-head detector 5260. Read-head detector 5260 may be located such that emulators are located between read-head detector 5260 and an edge of the card. Accordingly, for example, a read-head detector may be triggered when a read-head is in the middle of the card such that a single read-head detector can be utilized. Data may be communicated quickly such that all data is transmitted serially before a read-head (or read-heads) travel past emulators 470 and 480. Additionally, a capacitive sensing circuit may be coupled to detector 5260 without having the interconnection fall within the path of a moving read-head. In doing so, capacitive changes in the interconnection due to a read-head moving over an interconnection may not mis-trigger a capacitive sensing circuit.

Persons skilled in the art will appreciate that a user's payment card number (e.g., credit card or debit card number) does not have to change. A display may hide this payment card number until an appropriate unlocking code is entered into buttons of the card. Similarly, a magnetic emulator may not be provided current until the proper unlocking code is entered - thus keeping magnetic information private and not allowing undesirable readers to read a card. A security code may be displayed on the same or a different display. A button may be provided representative of an online purchase (or a user may utilize buttons to instruct the processor that an online purchase is desirable). For such an online purchase, the credit card number and the security code may be displayed - but the magnetic emulator may not be activated. In doing so, the level of security of the card is increased. Furthermore, for example, a button may be provided representative of in-store purchases (or a user may utilize buttons to instruct the processor that an in-store purchase is desirable). Accordingly, a processor may be signaled that an in-store purchase is desired. A different operation may be associated with different types of purchases (e.g., online or in-store). Accordingly, for example, magnetic emulators may be activated for an in-store environment - but not the displays. Accordingly, for example, a restaurant cashier may not be able to read the credit card number from the card, but may still be able to swipe the card. If a reader is down or a cashier requires reading particular information (e.g., a security code or credit card number information) then controls may be utilized to communicate this information. A record of the types of transactions may be stored and may be communicated in discretionary fields of data within a transmitted data track. Such record information may be utilized, for example, to further increase security and/or introduce a variety of additional functionality.

Different types of cards may be provided on a card. For example, a security ID number and a credit card number may both be provided on the same card. A button may be utilized to allow a user to provide instruction to a processor such that the processor can display (e.g., visually and/or magnetically) the desired information. For example, a user may determine to use one of a variety of payment accounts (e.g., credit and/or debit) for a purchase. An entire payment number (e.g., credit or debit) may be changed and/or hidden visually and/or magnetically. A portion of a payment card number (e.g., credit or debit) may be changed and/or hidden visually and/or magnetically.

Persons skilled in the art will appreciate that a display on the card may display a credit card number that does not change with time. Additionally, for example, a magnetic emulator (or multiple magnetic emulators) may magnetically communicate financial data that does not change with time. Such a card may reduce, for example, the effects of physical card theft and card cloning.

One or more light generation devices, such as a Light Emitting Diode (LED), may be provided as part of a card (or other device). Such an LED may produce light, for example, upon a manual input such as a button press, the correct entry of a PIC such as a PIN, and/or the incorrect entry of a PIC. A light emitting device may be operable to produce different colors of light. For example, the incorrect entry of a PIC may produce a red light and the correct entry of a PIC may produce a green light. A PIC may take any form such as a numerical code or a code that include alphabet letters and/or symbols. For example, a PIC may be "A-B-B-B-A" and an "A" button may be provided on a card in addition to a "B" button (as well as other buttons such as a "C," "D," and/or "E" buttons).

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipt may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

A website may be provided where a user enters in his/her credit card number, pays a fee, and a new card is programmed and sent to the user. The new card may include a display to display a portion of the users credit/debit card number in a static form upon entry of an appropriate PIC. Such a card may also include one or more magnetic emulation circuits to transmit the information to a reader. Such a card may or may not, for example, include a portion of a magnetic stripe. For example, three tracks of magnetic stripe data may be communicated via three different emulation circuits, more than three different emulation circuits, one emulation circuits (e.g., tracks communicated serially to all read-heads), or one or more tracks may be represented by magnetic stripe(s) while one or more other tracks may be represented by a magnetic emulation circuit. A track of data may also be partially represented by a magnetic emulation circuit and partially represented by a magnetic stripe.

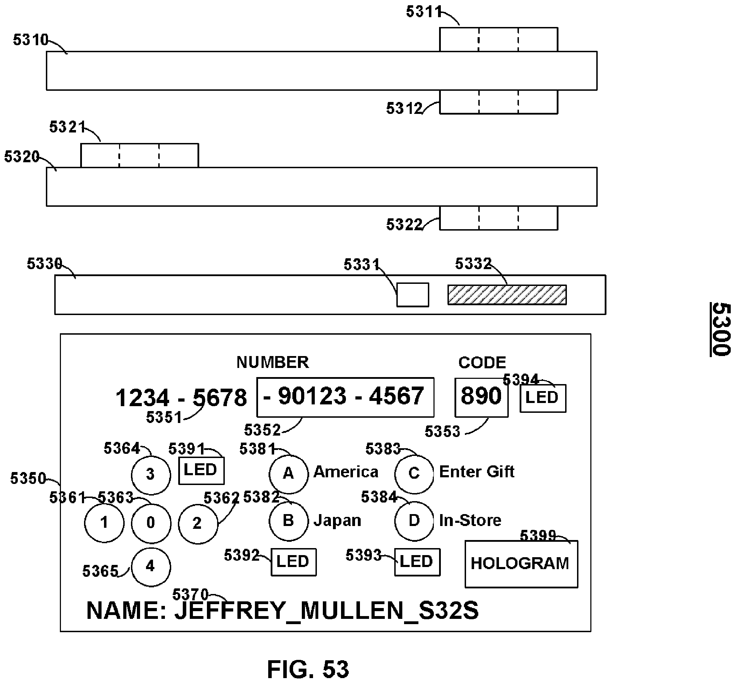

FIG. 53 shows card 5310, which may include magnetic stripe 5311 and magnetic stripe 5313. A magnetic stripe may, for example, have multiple tracks. Each track may be fabricated from, for example, a different magnetic stripe material. Magnetic stripe 5311 may include data structured to be received by the infrastructure for one country. Magnetic stripe 5312 may include data structured to be received by the infrastructure for another company. Magnetic Stripe 5312 and 5311 may be aligned vertically. Accordingly, a user may place card 5300 into a reader from one country in one direction and the user may place card 300 into a different reader from a different country in a different direction. The information included on magnetic stripe 5311 and 5312 may be associated with a user's payment card account such that purchase to both territories posts to the same account. Card 5320 may include, for example, magnetic stripe 5321 and 5323 that includes payment card data operable for use in different territories. Magnetic stripe 5321 and 5323 may be provided such that magnetic stripe 5321 and 5323 are not vertically aligned with one another. Card 5330 may be included that may include direction detector 5331. A card may determine the direction the card is facing via direction detector 5331. Accordingly, the card may determine which territory the user of the card believes he/she is located in. Accordingly, magnetic emulator 5332 (or a magnetic encoder) may be utilized to communicate the appropriate territory-based payment information through magnetic emulator 5332. Persons skilled in the art will appreciate that direction detector 5331 may be, for example, a read-head detector. Accordingly, read-head detector 5331 may be utilized to determine the side facing a read-head detector such that the payment information associated with that side's territory is communicated. An RFID antenna and/or smart-chip may be provided with information in a similar manner by detecting the orientation of a card with respect to a reader. Persons skilled in the art will appreciate that multiple emulators (or encoders) may be utilized instead (e.g., one for each track). Similarly, for example, two arrays of magnetic emulators may be utilized instead of the two magnetic stripes - each array located on a different end of a card (e.g., at the ends where magnetic stripe 5321 and 5322 of card 5320 are located).

Card 5350 includes buttons 5351-5364, light sources 5391-694, displays 5552-5353, permanent information 5351 and 5370, buttons 5381-5384, and hologram 5399. A user may be provided with a payment number. Such a payment number may be comprised of permanent data, dynamic data, or a combination of permanent and dynamic data. Dynamic data may be provided, for example, on display 5352. Display 5353 may be utilized to provide a code, which may be dynamic. Such a code may be utilized in authorize a transaction. Persons skilled in the art will appreciate that displays may display a code, payment number, or any type of data that changes based on time or based on use (e.g., utilizes one-time use data). Similarly, data may be static and may not change. Accordingly, for example, a display may be utilized to display the same data when desired such that the data may be hidden when the data is not desired to be displayed. Buttons 5351-5364, 5381-5382, and/or 5383-5384 may be utilized to signal a processor to display information on display 5352, display 5343, or display 5352 and display 5353.

A Personal Identification Code (PAC) may be entered to utilize to display data, as well as instruct a processor to provide particular data. For example, a particular PAC may provide one payment number (e.g., a credit card number) while a different PAC may provide a different payment number (e.g., a debit card number). A PAC may include a sequence of button presses (e.g., 5 particular button presses). Furthermore, a PAC may be utilized to unlock a card so that the card may be utilized. For example, buttons 5381, 5382, 5383, and 5384 may not be utilized by a user until an appropriate PAC has been entered via buttons 5351-5365. A number may be changed based on time (e.g., via display 5352, display 5353, or display 5352 and display 5353). Accordingly, a PAC may be entered such that the particular number associated with a particular button (e.g., a number associated with button 5351) for a particular time period (e.g., a particular day) may be displayed. One PAC may activate display 5352 while another PAC may activate display 5353.

Light source 5391 may be an LED or other source of light. Light source 5391 may display light each time a button associated to light source 5391 is pressed (e.g., buttons 5361-5362). Similarly, light source 5392 may display light each time a button associated with light source 5392 is pressed (e.g., button 5381 or 5382). Light source 5393 may display light each time a button associated with light source 5393 is pressed (e.g., light source 5383 or 5384). Light source 5394 may be associated to a component and may display light each time that component is activated (e.g., display 5353 or 5352 is activated). Light sources may emit light having different colors. For example, a processor may determine that a PAC provided to the processor via buttons 5361-5365 matches a valid PAC for performing an operation. Each button press may cause light source 5391 to emit light of a first color (e.g., YELLOW). The last button press to complete the PAC, however, may cause light source 5391 to emit a different color if the PAC is VALID (e.g., emit GREEN) yet emit another color if the PAC is INVALID (e.g., emit RED). Particular areas of a laminated card may be transparent such that light from a light-source illuminates the transparent area.

Button 5381 may be associated with a card of a particular country. Persons skilled in the art will appreciate that a card may be provided with a default number. Such a default number may include, for example, permanent data 5351 and data displayed on display 5352. Accordingly, a particular PAC may display the default data on display 5352.

Persons skilled in the art will appreciate that other default data may be provided to other components of a card upon entry of a PAC. For example, particular default data (e.g., payment card number and discretionary data) may be communicated to a magnetic emulator (or magnetic encoder) such that the information may be communicated to a magnetic stripe read-head. Similarly, default data (e.g., payment card number and discretionary data) may be communicated to an RFID antenna, an IC chip, or an RFID antenna and an IC chip. Such default data may be different for each component (e.g., magnetic encoder/emulator, RFID antenna, IC Chip) and may be in different formats (e.g., one track of payment data for one magnetic emulator and another track of payment data for another magnetic emulator).

Button 5381 may cause, for example, display 5352, display 5353, or display 5352 and 5353 to display data associated to button 5381. Similarly, data associated to button 5381 for other components of card 5350 (e.g., a magnetic emulator, magnetic encoder, RFID antenna, and IC chip) may be communicated through those components. Button 5381 may be associated with, for example a particular territory (e.g., America). Accordingly, for example, information communicated via card 5350 may be associated with a default country upon entry of a particular PAC until, for example, a button is pressed associated with a different country. At this time, for example, the information communicated by card 5350 may change to the information associated with the particular button pressed. Button 5392 may be provided for a country different than, for example, a default country and a country associated with another button (e.g., button 5381). A card may not be associated with a default country such that, for example, a button is pressed to determine the type of information communicated by a card.

A card, or other device, may autonomously determine, for example, the location of the card such that the appropriate information is communicated. For example, a GPS receiver (or another type of antenna) may be utilized to determine the location of the card, or other device (e.g., mobile telephone), such that the appropriate information is transmitted. Accordingly, a card, or other device, may send American payment data when the card, or other device, is located in America. However, the card may send Japanese payment data, or other data, when the card is located in Japan. Display 5352 and/or display 5353 may display different amounts of information based on the country scheme utilized. Similarly, other components such as magnetic emulators, magnetic encoders, RFID antennas, and IC chips may utilize different amounts of information based on the type of country utilized.

Button 5383 may be utilized to provide instructions to a processor that a gift card is desired to be utilized via card 5350. A gift code may be entered (e.g., via buttons 5361-5365) after button 5383 is pressed such that a user may, for example, associate a gift card to card 5350. Accordingly, card 5350 may be utilized to make a gift purchase such that the original gift card may be thrown out (or left at home). The code entered into card 5350 may be utilized, for example, to provide a processor with a number to transmit via the card (e.g., next time button 5383 is utilized). Such a number (as well as associated data such as associated discretionary data) may be communicated by card 5350 via one or more displays, magnetic emulators, magnetic encoders, RFID antennas, and IC chips. A code may alternatively, for example, transmit a flag (e.g., discretionary data) that a gift card is being utilized (e.g., upon another use of button 5383) such that a server may look at a seller ID number and check if there are any gift cards associated to a particular payment card number for that seller ID number. Accordingly, for example, a user may obtain a gift card (e.g., Target gift card) and may link that gift card to his/her payment card account (e.g., credit card account) and may utilize a button (e.g., 5383) to send a flag that a gift card is desired to be utilized. A code may be entered to provide a particular flag (e.g., a flag associated with a particular seller). Alternatively, no code may be entered and button 5383 may just be utilized to generate a generic flag (e.g., causing a server to check if there are any linked gift cards for the account associated with the seller associated with the utilized point-of-sale reader). A user may be provided with a particular code to be entered when utilize the gift card at an online store (e.g., Target's online store). The online store may, for example, allow a user to enter his/her payment information (e.g., credit card number, expiration date, name on card, zip code associated with card) and allow the user to select whether a gift card should be utilized associated with that card (e.g., via a radio button or other webpage input structure).

Button 5384 may be provided. Button 5384 may be utilized, for example, to make an in-store purchase. Button 5384 may activate, for example, display 5352 but not display 5353. Code 5353 may be utilized, for example, to at least complete a particular online transaction. In not activating display 5353, for example, a user that is provided with a card during an in-store purchase may not gain access to information displayed on display 5353. Persons skilled in the art will appreciate, for example, that the information on display 5353 may be transmitted via a component (e.g., emulator) even though the information is not displayed. Moreover, for example, display 5352 and 5353 may be the same display but that a particular interface (e.g., button) may display information on different portions of the display.

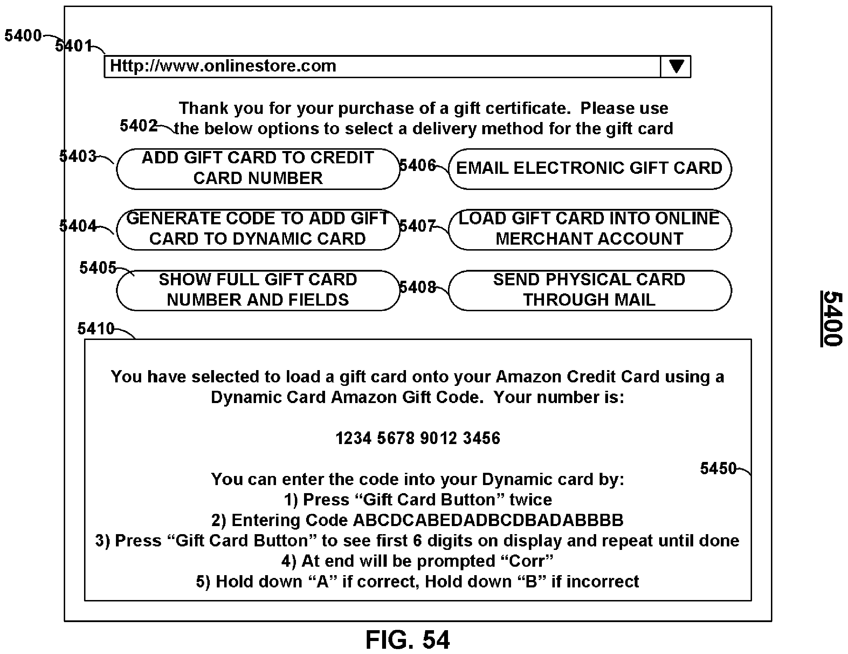

FIG. 54 includes webpage 5400 that may include, for example, navigational tool 5401 that may be utilize to navigate through different pages of a website or different websites of an intranet or internet. Information 5402 may be displayed, for example, when a user purchases a gift certificate online or purchases a gift certificate in a store and attaches that gift certificate to the users account online. Interface 5403 may be utilized by a user, for example, to add the particular gift card to their payment account. Such a payment account may be a credit account, debit account, check account, or any other type of payment account. A gift card may be linked to such an account such that a user does not have to, for example, carry around multiple cards. The user may, for example, carry around a single card, but a server may recognize whether a user has a gift card associated with a particular seller (e.g., based on a seller ID communicated from the point-of-sale device or other server). A user may add, modify, delete, or transfer gift cards associated to a payment account. Thus, for example, a user may receive a gift card for Christmas, may link the gift card to his/her payment account (e.g., credit card account), and may utilize the gift card by utilizing his/her card associated with his/her payment account. Interface 5406 may be utilized, for example, to email an electronic gift card to a user. Interface 5408 may be utilized to send a physical card through the mail to a user such as the user that purchased the gift card or a friend of the user that purchased the gift card. Interface 5407 may be utilized to load the gift card onto a merchant account. For example, a gift card that is purchased in a store (e.g., Walmart) may be entered into a website associated with that user's payment card (e.g., Bank of America) and then transferred to a merchant's website (e.g., Walmart). Persons skilled in the art will appreciate, for example, that a gift card may not be active until purchased. Activation may occur in a variety of ways. For example, a card may be activated by a cashier that completes a purchase of a gift card - thus communicating data to a server that activates that gift card. Similarly, a gift card number generated online may be activated upon generation. Persons skilled in the art will appreciate that pre-paid payment cards such as pre-paid debit cards may be utilized as gift cards are utilized.

Interface 5404 may be utilized, for example, to generate a code that may be utilized to add a gift card to a dynamic card. Interface 5405 may be utilized to show a full string of data that a credit card may utilize to communicate to an input device. For example, the full string of data may be the data that may be displayed and entered into a website to use the gift card. Alternatively, the data may be one or more tracks of magnetic stripe data (e.g., card number, name, and discretionary data). Alternatively still, for example, the data may be RFID antenna data or IC Chip data.

Persons skilled in the art will appreciate that a payment card may be associated with a company. For example, TJX may issue a TJX credit card. Such a card may offer incentives if the card is utilized within the company (e.g., within a TJX store). The gift cards of such a store may have data that is the same from gift card to gift card (e.g., the first digit or digits of a gift card number may be the same as they may be utilized to route data to a particular network). Accordingly, such a payment card may include a gift card functionality that accepts TJX gift cards. A user may be able to, for example, load in the control portions of the data such that the user does not have to enter in data that may be the same for all TJX cards. In doing so, for example, the amount of time utilized to enter information may be minimized.

Persons skilled in the art will appreciate that data may be transferred, such as gift card and/or pre-paid card data, to a card in a variety of ways. For example, a card may be swiped a second time through a magnetic stripe reader that includes a magnetic stripe encoder. A coil on the card may be utilized to receive the information and provide the received information to a processor. In doing so, information may be loaded into the card. Similarly, an IC chip may be utilized to receive data as well as a passive or active RFID. Additionally, one or more microphones may be included to receive audio information that may be representative of data. Accordingly, for example, a user may hold his/her card, or other device, next to a device that is operable to transmit audio via a speaker (e.g., laptop, stationary computer, or mobile telephonic device). The audio information may be discerned by the card and utilized to load information into the card (e.g., a gift card or pre-paid card. An application may also be loaded that enhances the functionality of the card. Such an application may include, for example, a user's medical information such that medical information can be displayed via the card (or other device) during a medical emergency. Accordingly, applications and/or payment cards may be purchased online and a speaker may communicate information to a card. Similarly, the card may include a speaker for transmitting information such that bi-directional communications are established. A light detector may be provided on a card that may receive light pulses indicative of data. Accordingly, for example, a user may hold a card up to a display - such as the screen of a laptop, stationary computer, or mobile phone - and information may be communicated from the display to the card via the light detector. Similarly, a light source may be utilized to communicate information from one device to another. For example, a light source (e.g., LED) may be utilized to communicate information from one card to another. Similarly, a magnetic stripe reader may include a light source. A card may be positioned over the light source such that a light detector of the card is aligned with the light source to receive light. Accordingly, the light of a magnetic stripe reader (or other type of reader) may be utilized to communicate information back to a card. A user may utilize interfaces on the card (e.g., buttons) to initiate a transfer of data from one card to another card or from a device to a card. A variety of types of data may be communicated. For example, money may be communicated from one debit card to another debit card such that payments may occur between the cards. Accordingly, for example, the next time a card is utilized via a reader (e.g., a magnetic stripe reader) information of the transfer may be communicated to a server for processing. Light may be utilized to transfer data from a card to a computer using, for example, a camera (e.g., webcam) on the computer. Sound may be utilized to transfer data from a card to a computer using, for example, a microphone on the computer.

A interface (e.g., a button) may be activated (e.g., pressed) for a period of time and different actions may be associated with different durations of activation. Different combinations of interfaces (e.g., different buttons) may be held together at the same time to perform a variety of functions. For example, a master unlocking code may be provided in case a card becomes locked (e.g., too many incorrect PACs were entered in a row). To enter a master unlocking code, a particular set of buttons may first need to be pressed in combination.

Information 5410 may be displayed on webpage 5400. Information 5410 may, for example, describe that a selection occurred as a result of user input that was indicative of requesting that a gift card be loaded onto a credit card using a code. The information may include instructions for loading the code. For example, a button may be requested to be pressed once. Then, a code may be requested to be entered into the card. Next, a button may be requested to be pressed to display a particular portion of the code to confirm that the particular portion was correctly entered. This process may repeat until the entire code is confirmed as having been correctly entered. The information may include how a card may respond (e.g., via displaying information, providing light in a particular manner, vibrating in a particular manner, or providing sound in a particular manner). To confirm an entry is correct, the user may be requested to perform a particular action such as, for example, holding down a particular button if the entry was correct.

A display may also be utilized as an interface. For example, a display may include a contact and an electronic ink. The electronic ink may change colors in response to, for example, a particular electrical signal being supplied to the contact. A capacitive sensor may be coupled to such a contact, however, such that a user interaction with the contact may be sensed by the capacitive sensor. Accordingly, a card may include a display that can also receive user input. Persons skilled in the art will appreciate that a display may include multiple contacts. For example, a display may include multiple 54-segment (e.g., to display digits) or 11-segment, 14-segment, or 16-segment (e.g., to display alphanumerics) regions where each segment may be coupled to a capacitive sensor.

A biometric sensor may be placed on a card or other device. Such a biometric sensor may be, for example, a fingerprint reader. Accordingly, one or more fingerprints may be stored in the memory of a card and compared to scanned fingerprints. Different fingerprints may activate the card differently (e.g., utilize a different user's payment card info).

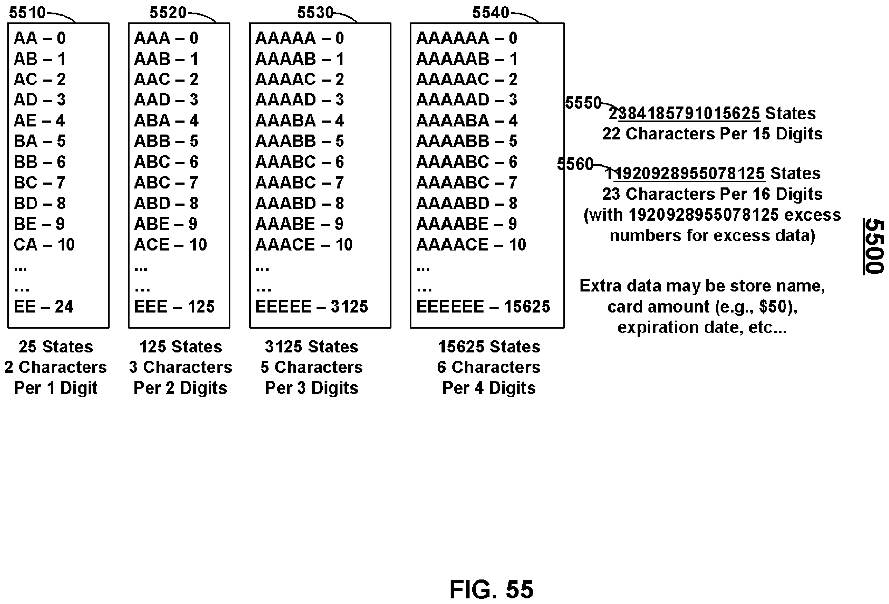

FIG. 55 shows mapping scheme 5500. Mapping scheme 5510 may be utilized. Any number of interfaces (e.g., buttons) may be placed on a card. For example, four or five buttons may be placed on a card. A button may be associated with, for example, a color, number, letter, or symbol. Payment card numbers, however, may have a digit base. Accordingly, mapping may be utilized such that different combinations of colors, numbers, or symbols may be associated with a digit. For example, suppose five buttons are provided and each button is associated with a letter between A and E. Mapping scheme 5510 may be utilized to allow a user to input digits. Accordingly, for example, a processor may include such a table such that the processor may be able to translate incoming information from a user such that digits may be communicated (e.g., via a display, encoder, emulator, RFID, and IC chip). Two characters from a five-button combination may, for example, provide for 25 different states. Scheme 5520 may be provided in which 3 character sets (where each character has 5 possibilities) provides 125 states. Accordingly, a 3 character set may provide 2 digits of information. Scheme 5530 may be utilized, for example, to provide 3 digits of information with a 5 character set. Scheme 5540 may be utilized, for example, to provide 4 digits of information with a 6 character set. Scheme 5550 may be utilized to provide 15 digits of information with a 22 character set. Scheme 5560 may be utilized to provide, for example, 16 digits of information with a 23 character set (where each character has one of five possibilities).

Persons skilled in the art will appreciate that numerous numbers may not be utilized by a company. For example TJX may only be provided with 15,000 pre-paid debit card numbers. Such numbers may be, for example, 19 digits in length. Suppose, for example, that TJX issued a payment card that allowed for TJX pre-paid debit cards to be entered into a the TJX payment card. Accordingly, albeit a number is 19 digits, a mapping may allow a user to enter a card number with only 6 characters. Accordingly, for example, a 19 digit number may be entered into a card using less than 19 button activations. Numbers may be encrypted based on time to increase security as these numbers are communicated from a card (e.g., via an emulator, encoder, display, RFID, and IC chip).

Persons skilled in the art will also appreciate that additional information may be communicated with a code. For example, the type of gift card (e.g., a TJX gift card) may be communicated with the code. Additionally, the amount of the gift card, the balance of the gift card upon code generation, the expiration date of a gift card, as well as a message provided by the purchaser of the gift card may be communicated via a code.

Persons skilled in the art will appreciate that a user's payment card number (e.g., credit card or debit card number) does not have to change. A display may hide this payment card number until an appropriate unlocking code is entered into buttons of the card. Similarly, a magnetic emulator may not be provided current until the proper unlocking code is entered - thus keeping magnetic information private and not allowing undesirable readers to read a card. A security code may be displayed on the same or a different display. A button may be provided representative of an online purchase (or a user may utilize buttons to instruct the processor that an online purchase is desirable). For such an online purchase, the credit card number and the security code may be displayed - but the magnetic emulator may not be activated. In doing so, the level of security of the card is increased. Furthermore, for example, a button may be provided representative of in-store purchases (or a user may utilize buttons to instruct the processor that an in-store purchase is desirable). Accordingly, a processor may be signaled that an in-store purchase is desired. A different operation may be associated with different types of purchases (e.g., online or in-store). Accordingly, for example, magnetic emulators may be activated for an in-store environment - but not the displays. Accordingly, for example, a restaurant cashier may not be able to read the credit card number from the card, but may still be able to swipe the card. If a reader is down or a cashier requires reading particular information (e.g., a security code or credit card number information) then controls may be utilized to communicate this information. A record of the types of transactions may be stored and may be communicated in discretionary fields of data within a transmitted data track. Such record information may be utilized, for example, to further increase security and/or introduce a variety of additional functionality.

Different types of cards may be provided on a card. For example, a security ID number and a credit card number may both be provided on the same card. A button may be utilized to allow a user to provide instruction to a processor such that the processor can display (e.g., visually and/or magnetically) the desired information. For example, a user may determine to use one of a variety of payment accounts (e.g., credit and/or debit) for a purchase. An entire payment number (e.g., credit or debit) may be changed and/or hidden visually and/or magnetically. A portion of a payment card number (e.g., credit or debit) may be changed and/or hidden visually and/or magnetically.

Persons skilled in the art will appreciate that a display on the card may display a credit card number that does not change with time (or transaction or button press). Additionally, for example, a magnetic emulator (or multiple magnetic emulators) may magnetically communicate financial data that does not change with time. Such a card may reduce, for example, the effects of physical card theft and card cloning.

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipt may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

A website may be provided where a user enters in his/her credit card number, pays a fee, and a new card is programmed and sent to the user. The new card may include a display to display a portion of the users credit/debit card number in a static form upon entry of an appropriate PIC. Such a card may also include one or more magnetic emulation circuits to transmit the information to a reader. Such a card may or may not, for example, include a portion of a magnetic stripe. For example, three tracks of magnetic stripe data may be communicated via three different emulation circuits, more than three different emulation circuits, one emulation circuits (e.g., tracks communicated serially to all read-heads), or one or more tracks may be represented by magnetic stripe(s) while one or more other tracks may be represented by a magnetic emulation circuit. A track of data may also be partially represented by a magnetic emulation circuit and partially represented by a magnetic stripe.

An LED may blink in a pattern to provide a number of functionalities. For example, an LED may blink to denote that a particular action is occurring (e.g., a magnetic emulator is ON). An LED may blink to communicate information to a card or other device (e.g., the video camera of a mobile telephone).

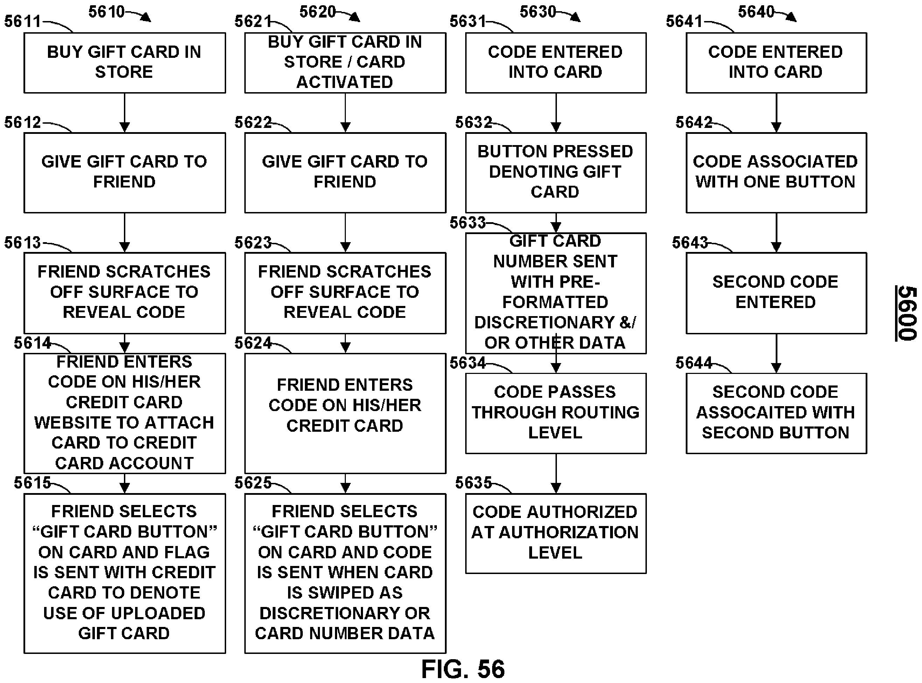

FIG. 56 shows process flow charts 5600. Flow chart 5610 may be included that includes step 5611, in which a gift card is purchased in a store. Step 5612 may then occur, in which the gift card is transferred to a friend or relative. Step 5613 may ensue, in which the friend or relative scratches off a surface of the card to reveal a code. Step 5614 may occur, in which the friend or relative may enter the code on a website associated with his/her card to attach the card to his/her payment card account. Step 5615 may be included in which the friend or relative presses a button on his/her payment card and a flag is sent with the payment card information (e.g., payment card number, name, discretionary data) to denote that an uploaded card is desired to be utilized. As such, a server may utilize the gift card number linked to the payment card instead of, for example, the credit card number.

Flow chart 5620 may be included. Step 5621 may be included in flow chart 5620, in which a gift card is purchased in a store and activated as a result of the card being purchased. Step 5622 may occur, in which the gift card is given to a friend or relative. Step 5623 may then occur, in which the friend or relative scratches off the surface of the card to reveal a code. Step 5624 may be included in which the friend or relative enters the code on his/her payment card. Step 5625 may be included in which the friend presses a button such that the code is transmitted when the card is utilized by a reader (e.g., a magnetic stripe reader) as discretionary or other data (e.g., credit card number data).

Flow chart 5630 may be included. Step 5631 may be included in flow chart 5630, in which a code is entered into a card (or other device). A button may be pressed denoting use of a gift card function in step 5632. Step 5633 may be utilized such that the gift card data may be sent with pre-formatted discretionary and/or other data. Step 5634 may be included such that the code passes through a routing server. Persons skilled in the art will appreciate that particular digits (e.g., the first six digits) of a gift card number may be utilized by a server as a routing address. Step 5635 may be included, for example, such that the code is authorized at an authentication server such that the gift card may be verified and utilized to pay for a purchase.

Flow chart 5640 may be included. Step 5641 may be included in flow chart 5640 such that a code is entered into a card. Step 5642 may be included such that the code is associated with one or more buttons on a card. Step 5643 may be utilized such that a second code is entered into a card. Step 5644 may be utilized such that a second code is associated with one or more second button. Accordingly, for example, one gift card may be entered and associated with one button while another gift card may be entered and associated with another button.

Persons skilled in the art will appreciate that a variety of accounting functions may be performed by a card (or other device). For example, buttons may be provided that are associated with different accounting categories (e.g., food, hotel, entertainment, gas, other). A user may press a button before a purchase in order to add information into the discretionary data associated with, for example, a category. Such information may be communicated to a magnetic emulator, encoder, RFID, and IC chip. Such information may be stripped out at a server and forwarded to a different server. Such a different server, may, for example, utilize such discretionary data to add information associated with the categories to a user's online bill statement for a particular payment card. Thus, for example, a user may select a business credit card button on a card and then a travel button. In doing so, for example, the user's business credit card number may be communicated to a magnetic stripe read-head (along with any associated information) as well as discretionary data representative of the purchase being for travel. Accordingly, a business person may save time in creating an expense report as information is communicated at time of purchase through a payment infrastructure at the demand of the user.

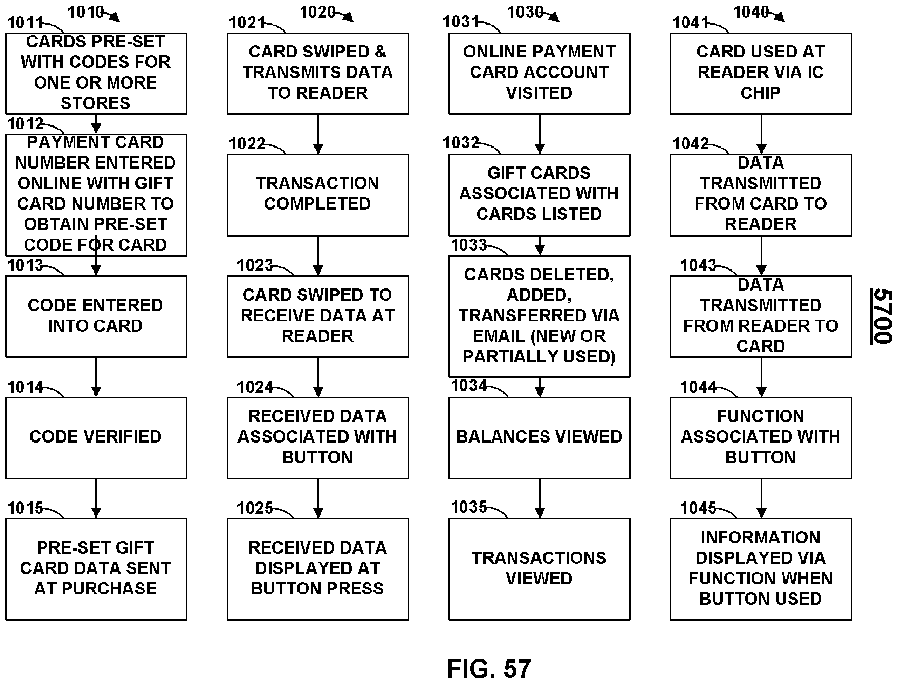

FIG. 57 may include process flow charts 5700. Flow chart 5710 may be included. Step 5711 may be included, in which a card is pre-set with codes for one or more stores. Step 5712 may occur, in which a payment card number is entered online with a gift card number to obtain a pre-set code for a store for a particular card (or for any card). Such a code may be entered into a card in step 5713. The code may be verified in step 5714 and pre-set gift card data may be sent at purchase in step 5715 (e.g., via a magnetic emulator, encoder, IC chip, or RFID).

Flow chart 5720 may be included that may include step 5721, in which a card may be swiped to transmit data to a reader (e.g., a magnetic stripe reader). Step 5722 may occur in which a transaction is completed. Step 5723 may then ensue, in which a card is swiped through the reader (e.g., a magnetic stripe reader) to receive data from the reader. The received data may be, at least in part, associated with a button in step 5724. Such received data may be displayed, at least in part, when the associated button is pressed in step 5725.

Flow chart 5730 may be included. Step 5731 may be included, in which an online card account maybe be visited by a user. Step 5732 may be included in which gift cards associated with the card account may be listed with the card. Additional cards may be listed such as, for example, additional credit, debit, check, or pre-paid cards. Cards may be added, deleted, or transferred electronically (e.g., via mail) in step 5733. A balance may be viewed for one or more cards in step 5734. Transactions associated with cards may be viewed in step 5735.

Flow chart 5740 may be included. Card may be used at a reader via an IC chip in step 5741. Step 5742 amy be included in which data may be transmitted from a card to a reader. Step 5743 may occur in which data may be transmitted from a reader to a card. A function may be associated with one or more buttons in step 5744. Information may be displayed via a function when the associated button is pressed in, for example, step 5745.

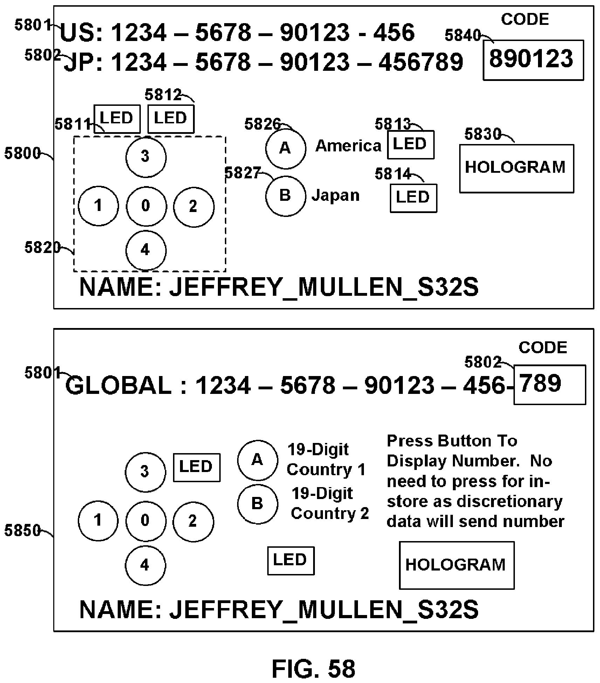

FIG. 58 shows card 5800 that may include, for example, button array 5820, light sources 5811-5814, hologram 5830, permanent information 5801 and 5802, display 5840, and buttons 5826 and 5827. Permanent information 5801 and 5802 may be, for example, printed and/or embossed. Permanent information 5801 may correspond to, for example, the card number for one territory while permanent information 5802 may include information for another territory. Display 5840 may include, for example, a code that may change based on transaction (via read-head detectors), button-press, or time. The code displayed on display 5840 may be associated with buttons 5826 and 5827 such that different codes are provided depending on the button that is pressed. A code may be different lengths. Persons skilled in the art will appreciate, for example, that one payment card may include a card number of one length (e.g., 15 digits) and a code of one length (e.g., 4 digits) while another payment card may include a card number of a different length (e.g., 16 digits) and a code of a different length (e.g., 3 digits). Light source 5811 may activate in certain circumstances while light source 5812 may activate in different circumstances. Each light source may be able to emit different colors of light. Each light source may, for example, emit a different color of light. For example, light source 5811 may emit GREEN while light source 5812 may emit RED. Accordingly, for example, an appropriate PAC may result in light source 5811 emitting GREEN while light source 5812 emits RED. Light source 5813 may be utilized to emit light after button 5826 is pressed so that a user can see that a button is active. Similarly, button 5827 may be utilized to emit light after button 5827 is pressed.

Card 5850 may be provided. Global number 5801 may be displayed permanently (or via a display). Display 5802 may be utilized to display information. For example, display 5802 may be utilized to display a code or a portion of a payment card number. Persons skilled in the art will appreciate that different territories may be have different lengths of different types of data (e.g., different lengths of payment card numbers). Accordingly, a user may be assigned numbers in each territory with overlapping digits (e.g., global portions). For example, a sixteen digit number for one territory may be the same as the first sixteen digits of a nineteen digit number in another territory. The remaining digits for that territory may be displayed, for example, on display 5802. Accordingly, for example, the card may, upon entry of an appropriate PAC default to not showing any information on a display and, upon sensing of a read-head, sending the default payment information through one or more magnetic emulators and/or encoders (or RFID or IC chip). Upon the utilization of a button signifying a nineteen digit scheme for a different territory is utilized, the extra digit information may be displayed on display 5802 and the payment information for that territory may be communicated via a magnetic emulator, magnetic encoder, RFID, or IC chip. Persons skilled in the art will appreciate that pressing a button for a particular territory (or other payment information) may be utilized to send that payment information through a communications device such as a magnetic emulator. However, for example, another button may be utilized to cause a display to display the appropriate corresponding payment information. Accordingly, for example, a user may protect information from being visible read by others in particular situations (e.g., when a card is handed to a bartender for processing).

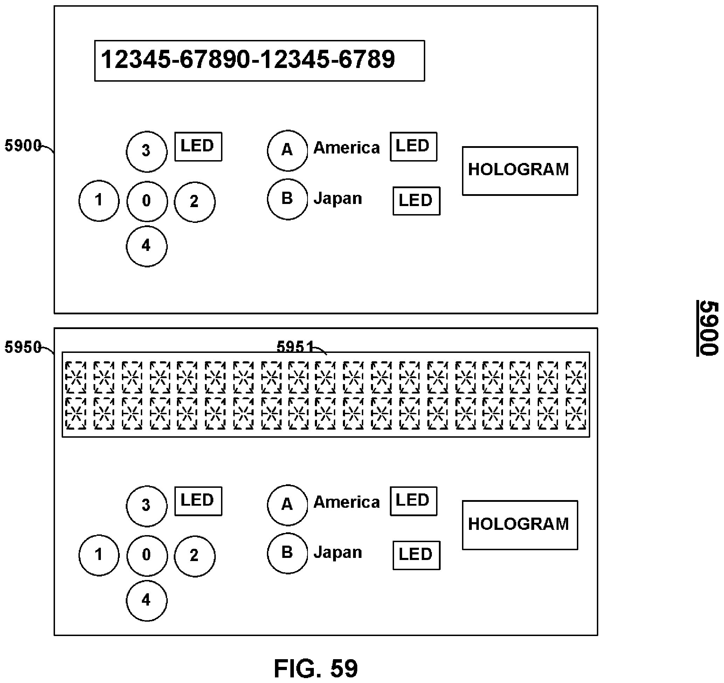

FIG. 59 may include card 5900 in which a payment card number may be displayed on a display. Persons skilled in the art will appreciate that a variety of information may be displayed on a display such as, for example, battery life, time until a number expires (e.g., in a time-based encryption scheme), the number of numbers left (e.g., in a multiple-factor one-time-use scheme). Card 5950 may be provided that may include display 5951 that may include, for example 11-segment character displays. A card may include any type of display such as, for example, a 7-segment digit display. A display may be one, two, three or more lines of characters. A display may include pixels that may be controlled to display data. A color or two-tone (e.g., black and white) display may be utilized).

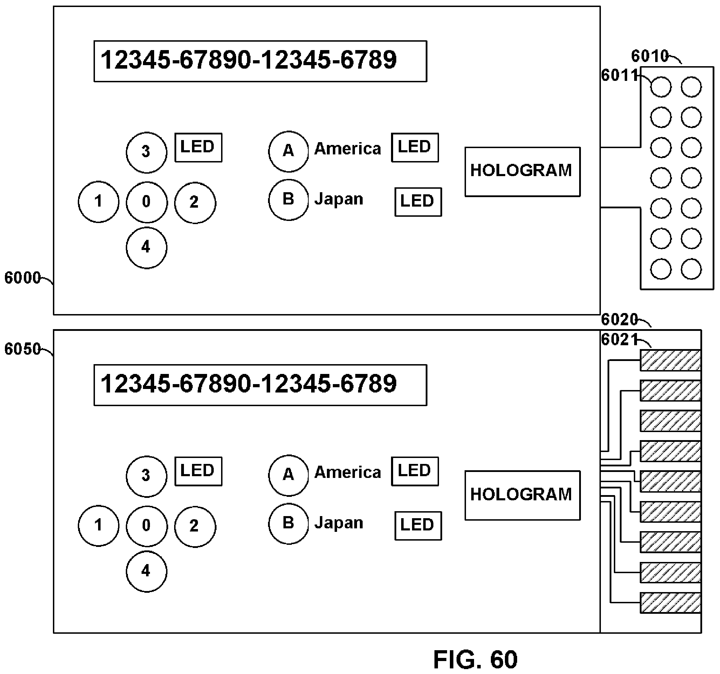

FIG. 60 shows card 300 which may be manufactured and laminated (e.g., via a HOT, WARM, or COLD lamination process). A card may be printed on a circuit board, such as a flexible circuit board. Components (e.g., microprocessor) may be attached to the board (e.g., soldered via hot air). The card may be laminated. For example, the board may be placed into a mold and warm laminate may be injected into the mold. The mold may be removed such that a card is provided. Molding may take place, for example, on the sheet level and then cut into cards. Programming portion 6010 may be a portion of the board that was not laminated. The board may have, for example, multiple layers. Accordingly, portion 6010 may be utilized to program a processor, or other components, via contacts 6010. Contacts 6010 may take the form of, for example, rings such that a male-programming connector may couple each ring. Accordingly, a perpendicular connection may occur. After a card is programmed, for example, portion 6010 may be cut-off such that a card is provided. Card 6050 may be included with portion 6020 and contacts 6021. Contacts 6021 may couple via a parallel connection with a programmer. A microprocessor may be instructed to ignore or burn-out programming ports after programming. Additional laminate may be placed on the edge where the board is cut such that, for example, no contacts are exposed. Furthermore, a processor may be programmed through laminate using, for example, capacitive programming.

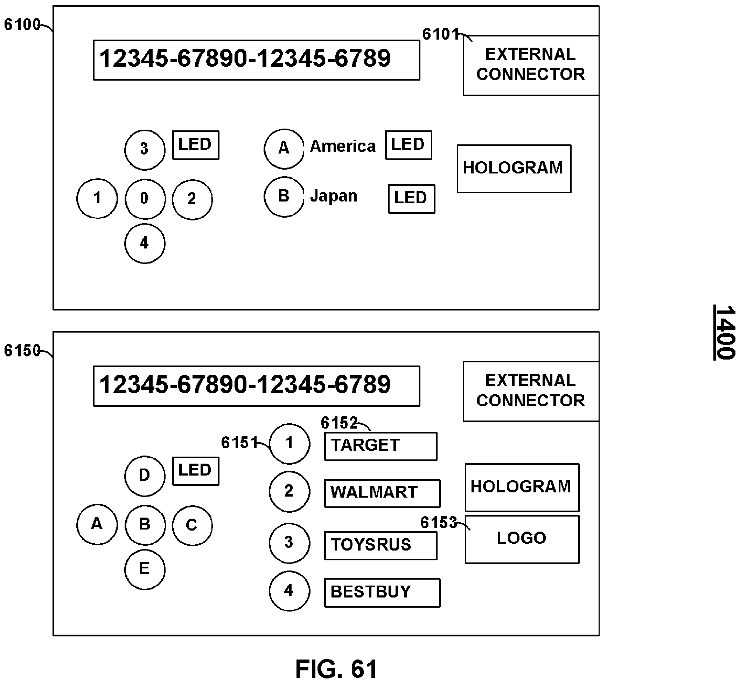

FIG. 61 may include external connector 6101 which may be, for example, a USB connector. Such a USB connector may, for example, fold out from card 6100. Such a connector may be, for example, USB, USB 2.0, mini-USB, or USB 3.0. Other external connectors may be utilized. Data may be transferred to and from a card, or other device, utilizing external connector 6101.

Card 6150 may be provided. Card 6150 may include, for example, button 6151 and associated display 6152. A code may be entered using a button array to cause a processor to display a particular number, character set (e.g., name), logo, or other indicia on display 6152 upon the utilization of button 6151. Accordingly, for example, a user may load a gift card onto a card and may be provided with customized visual indicia such that a user may not forget what card is associated with what button. A user may select what button a card (e.g., gift card) may be associated with or the card may autonomously associate a card to the next available button. A card may have a pre-set number of cards that are provided to buttons on a one card per button basis or multiple cards may be associated per button (e.g., a single button press of a button provides one gift card while two button presses of a button in tandem provides a different gift card). Balances, gift card amounts, expiration dates, or other information may also be provided via a display. Other information may include a message from the gift card purchaser. A display may scroll data autonomously such that information that would extend beyond the display capabilities of a display may be displayed by the display without further user interaction. Accordingly, for example, a user may unlock a card by entering a particular PAC on a button array, the user may press a button to see the gift card associated with that button, the user may then select a different button and see a different gift card associated with that different button, the user may then swipe a card and a read-head detector may detect a swipe, the processor may then drive one or more magnetic emulators to communicate data to one or more magnetic stripe read-heads that causes that different credit card to be utilized. Persons skilled in the art will appreciate that codes may be utilized to, for example, load cards or denote a particular flag be sent with particular payment card information (e.g., default credit card information) such that processing servers may utilized gift cards linked to a card. Similarly, for example, a user may load a gift card into a card and the card may send information along with the payment card information (e.g., in discretionary fields) such that a processing server notes that a gift card was added to the card and links the gift card to the user's account. Accordingly, a user may, for example, receive a gift card, enter the card into his card (e.g., by entering a unique code for that gift card), use the credit card, and then go online to his payment card account and see that the loaded gift card is associated to his account. The user then may, for example, utilize the loaded gift card online from his/her payment card account.

Permanent logo 6153 may be provided on a card. A card may include logos of multiple credit card companies, banks, or other entities. A logo may be provided on a display (e.g., an electronic ink display). Different logos may be provided on a display and a particular button press may cause a different logo to be displayed. Different logos of different stores may be displayed depending on, for example, the gift card that is utilized at a particular time.

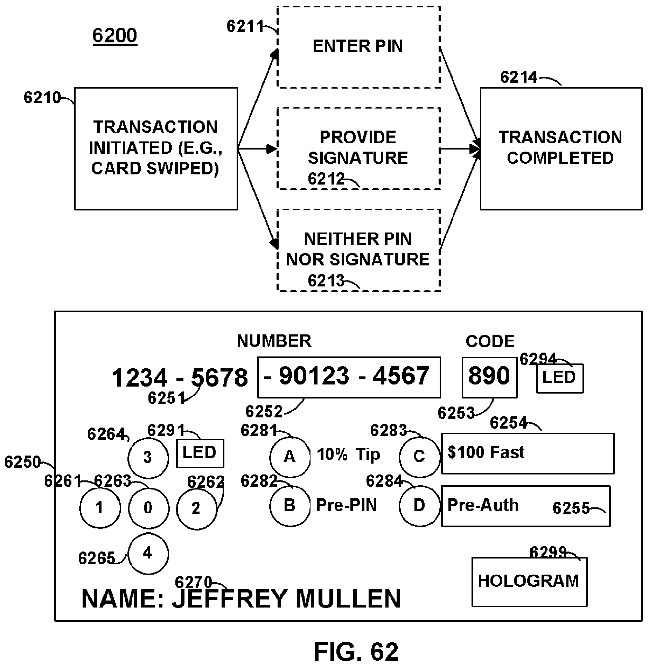

FIG. 62 shows card 6250 that includes buttons 6261-664, light sources 6291-694, displays 6252-6253, permanent information251 and 6270, buttons 6281-6284, and hologram 6299.

Button 6281 may be included on card 6250. Button 6281 may cause, for example, display 6252, display 6253, or display 6252 and 6253 to display data associated to button 6281. Similarly, data associated to button 6281 may be communicated through components of card 6250 (e.g., a magnetic emulator, magnetic encoder, RFID antenna, and IC chip).

Button 6281 may, for example, be associated with a particular amount of a tip (e.g., a 10% tip). Accordingly, for example, a user may interact with button 6281 to denote that the user desires to add a tip to a purchase. For example, a user in a restaurant may hand his/her payment card to a waitress and activate button 6281 (e.g., after entering in an appropriate PIC into card 6250 utilizing buttons 6261-6265). Accordingly, the waitress may swipe card 6250 through a magnetic stripe swipe reader. A read-head detection circuit on card 6250 may recognize that card 6250 is being swiped. Accordingly, for example, card 6250 may communicate information through a magnetic emulator. This information may include payment information (e.g., a payment card number and associated discretionary data). Included in the communicated data may be, for example, data representative of the desired tip amount. Such data may, for example, be a flag (e.g., a particular character in a particular location of communicated data). A system, such as a cash register or remote server, may recognize the flag and may authorize a payment transaction associated with the total amount of a purchase (e.g., the amount after the desired tip has been added).

Button 6282 may be associated to, for example, a pre-ATM activity. Such a pre-ATM activity may be, for example, a pre-PIN activity. For example, a user may activate button 6282 and utilize buttons 6261-665 to enter in the user's PIN. The user may then, for example, place card 6250 in the proximity of a card reader such that payment information and a user's PIN may be communicated through a magnetic emulator (or communicated through an RFID antenna and/or IC chip). Accordingly, a user may enter in his/her PIN into a payment card such that a user does not have to enter his/her PIN into an ATM. In doing so, for example, a user may more securely enter in his/her PIN (e.g., by hiding a card) as well as accelerate an ATM activity (e.g., by entering a PIN while waiting in line for an ATM). A user may enter his/her PIN into card 6250 utilizing buttons 6261-6265 and then, for example, press button 6282 to cause a processor to place the entered PIN information into data communicated from card 6250. Accordingly, for example, the sequence of buttons 6261-6265 that are pressed may be stored in a memory of card 6250 and utilized by a processor of card 6250.

Button 6283 may be associated to display 6254. Alternatively, for example, button 6283 may be associated with written and/or embossed information (not shown). For example, button 6283 may be associated to display 6254. Display 6254 may display data associated with a particular card function. For example, display 6254 may display a fast-cash function (e.g., $100 fast cash). A user may utilize interfaces on card 6250 (e.g., buttons 6251-6262) to set or change the information displayed on display 6283. A user may enter in a card configuration on a computer and receive information into the card from the computer, in a variety of ways, in order to configure the card (e.g., to display a particular function on display 6254). For example, a user may swipe card 6250 and receive information through a coil from a magnetic encoding head that generates an electromagnetic field. Alternatively, for example, a processor may receive configuration information via an RFID antenna and/or an IC chip.

Button 6283 may be associated with a fast-cash function. A user may interact with button 6283 to provide an instruction to a processor that a fast-cash functionality is desired. A user may, for example, enter his/her PIN into a card. After the user's PIN is verified by a processor on card 6250, a user may, for example, press button 6283 such that a flag is communicated through transmitted payment information representative of a fast-cash function. Accordingly, for example, an ATM (or other device) may receive payment information that may include a fast-cash flag. The machine may also receive PIN information from a card. The machine may utilize a payment card number in the payment information with the PIN number to verify the identity of the user. The machine may recognize the received flag and utilize the flag as control data to dispense cash to the user. Accordingly, for example, a user may perform ATM activities on a card while waiting in line for an ATM in order to minimize the amount of time a user is required to interact with that ATM.

Button 6284 may be associated with display 6284 and a pre-authorization functionality. For example, a pre-authorization functionality may be utilized to pre-authorize a particular amount or to complete a signature-based transaction without a signature. For example, a tip amount may be added to a total amount and may be pre-authorized. Accordingly, a user may receive a receipt that requires his/her signature with the pre-authorized total amount (that includes the tip). Additionally, for example, a PIN may be entered utilizing buttons 6261-6265 and button 6284 may be utilized to communicate the PIN as a pre-authorization. Accordingly, a remote server may receive payment information that may include at least a PIN, a payment number, discretionary data, and a flag associated that the PIN is desired to be utilized in lieu of a signature as a form of authorization for the transaction.

Flow chart 6200 may be utilized in conjunction with a payment card, such as payment card 6250. Step 6210 may be included in flow chart 6200. Particularly, for example, step 6210 may be initiated when information is communicated from a payment card, through a payment card reader, through a payment card routing server, to a payment card authorization server. Step 6211 may be included, in which a PIN is requested to be entered at a reader. Step 6212 may be included, in which a signature is requested to be entered in a reader. Step 6213 may be included, in which a PIN, for example, is communicated to a remote server via a card, thus not requiring either a PIN or a signature to be entered on a payment card reader. A payment transaction may be completed in step 6214

Persons skilled in the art will appreciate that a pre-authorization activity may include the pressing of a single button after an appropriate PIN has been entered into a card. Accordingly, the card may authorize a user and may communicate an appropriate flag when payment data is communicated from a card. Accordingly, a remote server may receive a flag. The remote server may authorize a payment transaction based on the received data that includes the flag, indicative of an appropriate PIN entered into a card and the activation of a button associated with a pre-authorization activity.

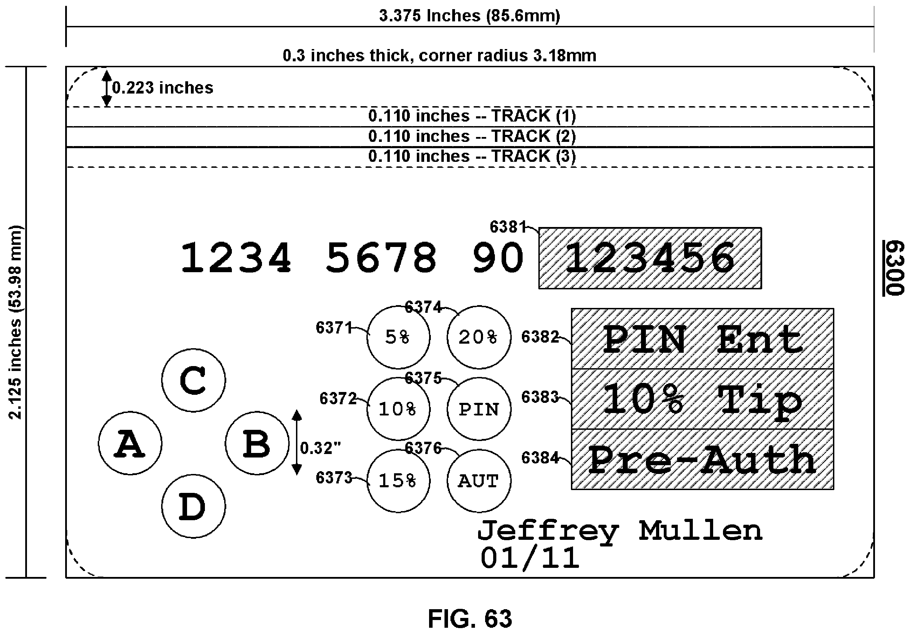

FIG. 63 shows card 6300. User interface 6371 may be included on card 6300 and may be associated to a particular tip percentage (e.g., 5%). User interface 6372 may be included on card 6300 and may be associated to a different tip percentage (e.g., 10%). User interface 6373 may be included on card 6300 and may be associated to another tip percentage (e.g., 15%). User interface 6374 may be included on card 6300 and may be associated to yet another tip percentage (e.g., 20%). User interface 6375 may be included on card 6300 and may be associated to the desire to enter a PIN into card 6300. User interface 6376 may be included on cad 6300 and may be associated to an authorization activity. Displays 6381-6384 may be utilized to display information. For example, display 6381 may display payment card information (e.g., after an appropriate PIN is entered into card 6300). Display 6382-6384 may be utilized to display selected combinations of activities. For example, if a user is in a restaurant, a user may enter a PIN, enter that the PIN should be utilized for payment authorization, and that a 10% tip is authorized. Persons skilled in the art will appreciate that a user may utilize buttons to enter in a PIN at any time (e.g., without pressing a button indicating a PIN is about to be entered) and a correct entry of a PIN may result in a display (e.g., display 6382) displaying indicia associated with the correct entry of a PIN.

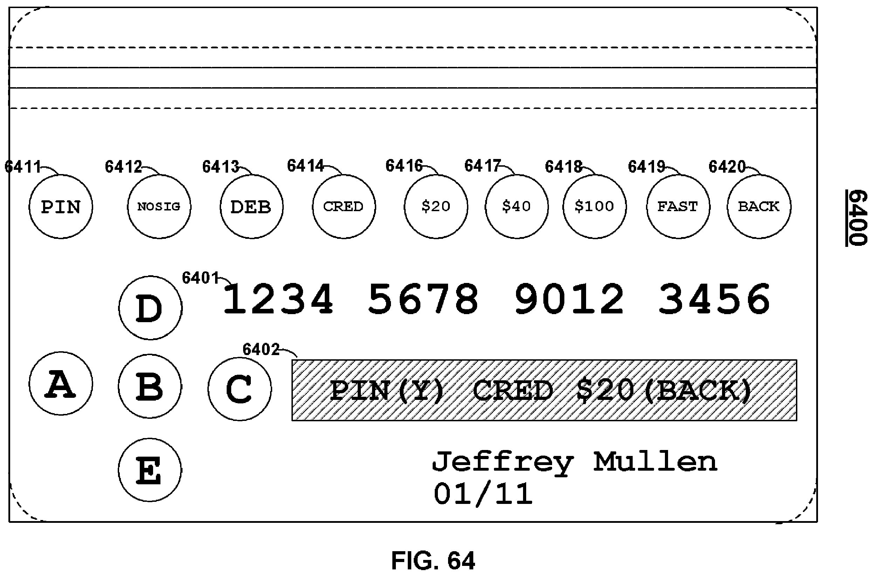

FIG. 64 shows card 6400 that may include, for example, user interfaces 6411-6420. User interfaces 6411-6420 may be a button such as a mechanical button or a capacitive button.

User interface 6411 may be pressed by a user to instruct a processor on card 6400 that a user desires to enter a PIN into card 6400 utilizing user interfaces located on card 6400. Accordingly, a user may interface with user interface 6411. The user may then enter his/her PIN into user interfaces of card 6400. The entered PIN may then be, for example, stored and verified by a processor of card 6400. Persons skilled in the art will appreciate, however, that a PIN entered by a user does not need to be verified by card 6400. An entered PIN may, for example, be stored and the entered PIN may be communicated to a remote device by card 6400. Accordingly, a PIN may be verified by remote devices A PIN may be verified by card 6400 in addition to being forwarded to a remote device for verification.

Card 6400 may include user interface 6412. A user may interface with user interface 6412 (e.g., press a mechanical button) and cause a processor of card 6400 to implement a functionality associated with user interface 6412. Such a functionality may include, for example, instructions to communicate information associated with the desired functionality when card 6400 communicates information to external devices (e.g., a payment card magnetic stripe reader, IC chip reader, or RFID reader). User interface 6412 may, for example, cause information to be sent indicative of a user's desire to complete a signature-based transaction without a signature. Accordingly, for example, card 6400 may communicate information that includes a payment card number, the PIN entered into card 6400 by a user, and a data indicative of the user's desire to utilize the entered PIN to complete a transaction instead of utilizing a signature.

User interface 6413 may be associated to a particular type of card. For example, user interface 6413 may be associated to a particular type of a payment card (e.g., credit payment, debit payment, gift payment). Accordingly, for example, a user may interact with user interface 6413 to instruct a processor that the user desires to utilize a particular type of payment for a purchase. Multiple interfaces may be included on card 6400 and each interface may be associated to a different type of payment. For example, user interface 6414 may be included on card 6400 and may be associated with a credit payment. A processor may receive a control signal from user interface 6413. The processor may then retrieve payment information associated with stored payment information for user interface 6413. Accordingly, for example, the processor may retrieve debit card information (e.g., a debit card account number and associated discretionary data). The processor may then communicate this retrieved information from card 6400. Persons skilled in the art will appreciate that the card may communicate payment information in different forms depending on the type of reader the card interfaces with. For example, card 6400 may detect that card 6400 is placed in a magnetic stripe swipe reader and may communicate the payment information desired by the user in the form of track 1 and track 2 magnetic stripe data. Card 6400 may alternatively, for example, detect that card 6400 is placed in an electromagnetic field and may communicate the appropriate payment information as an RFID signal from an RFID antenna located on card 6400.

User interfaces 6416-6418 may be associated to particular dollar amounts. User interfaces 6419 and 6420 may be associated to functions that are based on particular dollar amounts. Accordingly, for example, a user may select a user interface 6416-6418 as well as user interface 6419 or 6420 in order to provide a combinational instruction to a processor. For example, a user may utilize user interface 6419 and 6418 to instruct a processor that a fast-cash functionality is desired in the amount of $100. As per another example, a user may utilize user interface 6420 and 6418 to instruct a processor that a cash-back functionality is desired in the amount of $100. Accordingly, a user may utilize user interfaces to provide combinational logic in order to, for example, reduce the number of user interfaces on a card. In reducing the number of user interfaces on a card, for example, the cost of a card may be decreased. Persons skilled in the art will appreciate that a cash-back and a fast-cash functionality may be provided with a single button. A single flag may be placed in data outputted in a card indicative of the desire to utilize a fast-cash and cash-back functionality. A remote device, such as a cash register), may recognize the flag and may perform the desired operation if, for example, the machine is only capable of providing one of the two options (e.g., fast-cash or cash-back). A button may be pressed multiple times to toggle between different options. For example, a button may be pressed once to toggle to a fast-cash functionality while the same button may be pressed again to toggle to a cash-back functionality.

The results of user selections may be displayed, for example, on display 6402. Accordingly, for example, a user may visually verify that card 6400 has correctly received the user's desired selections. Permanent information 6401 may be provided. Permanent information 6401 may include, for example, a payment card number, a user's name, a verification code, an expiration date, instructions for destroying a card, and instructions for using a card. Person skilled in the art will appreciate that permanent information 6401 may include a default payment card number (e.g., a first credit card number). The use of user interface 6414 may, for example, cause a secondary credit card number to be displayed on display 6402. A card that is used with a reader without receiving any information from a user via user interfaces may, for example, communicate default information (e.g., default payment information or information indicative that a user has not entered any information into a card utilizing user interfaces located on the card).

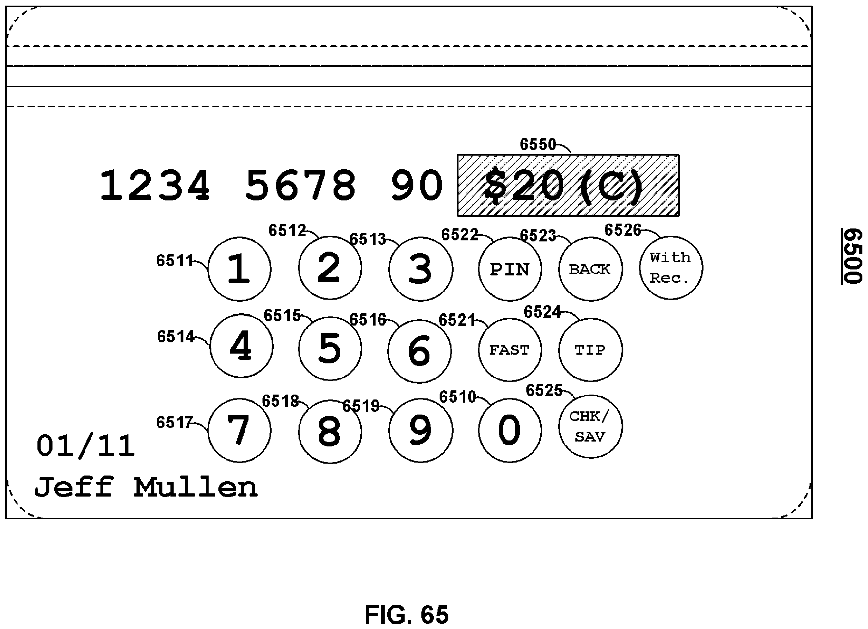

FIG. 65 shows card 6500. Card 6500 may include, for example, user interfaces 6511-6510 and 6522-6525. Card 6500 may also include, for example, display 6250. User interfaces 6510 to 6519 may each be associated to, for example, a digit. Accordingly, for example, user interfaces 6510-6519 may form a ten digit numeric keypad. This keypad may be utilized by a user to communicate numerical information to a processor. Multiple functions may, for example, utilize numerical information. For example, user interface 6522 may be associated with a PIN-entry function. Accordingly, a user may use interface 6522 and then the user may enter a PIN into card 6500 by utilizing interfaces 6511-6519. User interface 6521 may be associated with a fast-cash functionality. Accordingly, a user may select fast-cash by utilizing interface 6521 and then may enter in the desired amount of cash the user desires to withdrawal utilizing interfaces 6510-6519. A confirmation step may be utilized. For example, a user may interface with button 6521 before and after selecting a withdrawal amount. Display 6250 may, for example, display an updated selection status after, for example, a function button is pressed a second time (e.g., after numerical information is entered). Alternatively, for example, a user selection may be presented via display 6250 after the numerical information was entered by a user such that a user can press a function button a second time, after viewing the displayed selection, to confirm the selection. A user may reset the selection by, for example, entering a different numerical amount utilizing user interfaces 6510-6519. In doing so, for example, a user may easily correct a situation where the user entered the wrong numerical information into card 6500.

User interface 6523 may be associated with, for example, a cash-back functionality. Accordingly, a user waiting in line to purchase an item may select cash-back utilizing interface 6523 and may enter in an amount of desired cash-back. This request may be communicated from the card to a cash register such that the cashier is notified to provide the desired amount of cash-back to the user. Accordingly, a transaction may be authorized for the amount of the purchase as well as the amount of the cash withdrawal. User interface 6524 may be associated to a tip and user interfaces 6511-6519 may be utilized to enter in the desired tip. Persons skilled in the art will appreciate that a card, or a remote device, may utilize numerical information as a percentage (e.g., 10%) or as a number (e.g., $10). A user interface may be provided for a decimal place such that cents information may be entered into a card by a user. User interface 6525 may be utilized by a user to toggle between a checking account and a saving account. User interface 6526 may be utilized to notify the card that a user desires a receipt. Accordingly, information may be communicated by the card to a point-of-sale device to indicate that the user desires a receipt. In doing so, for example, the amount of user-to-cashier verbal interaction may be minimized. A user may utilize user interface 6526 to toggle between a state of desiring a receipt to a state of not desiring a receipt. Information indicative of a user's selection may be displayed, for example, on display 6550. For example, "$20(C)" may denote that a user selected to withdrawal $20 from his/her checking account.

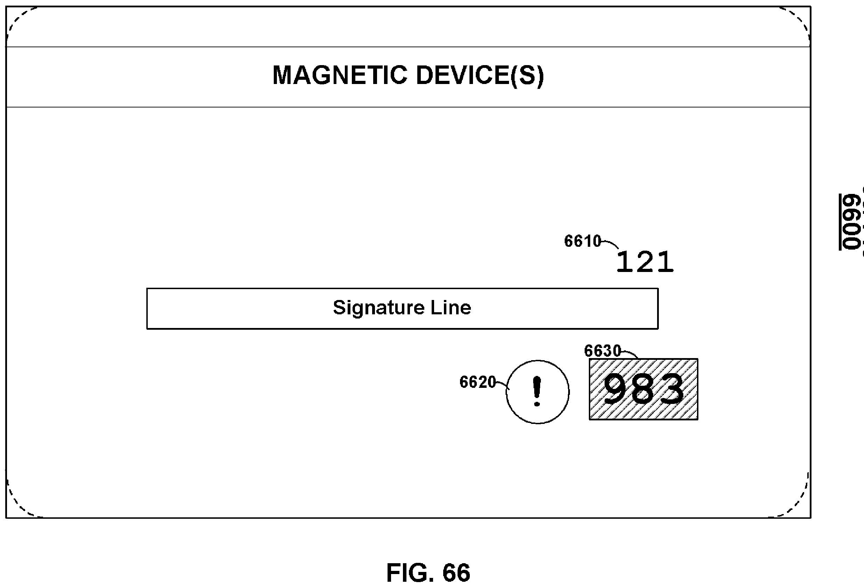

FIG. 66 shows card 6600. Card 6600 may include, for example, an reverse side that includes a material operable to receive a user's signature (e.g., a pen-based signature). The reverse side may also include code 6610. Code 6610 may be displayed on a display located on a reverse and/or obverse side. Code 6610 may be electronically communicated by a card (e.g., via an IC chip, a magnetic emulator/encoder, and/or an RFID antenna). Persons skilled in the art will appreciate that any interface, display, or other component of a card may be located on the reverse or obverse side of the card. Display 6630 may include a code that is displayed upon interaction with interface 6620. Display 6630 may not, for example, display information until, for example, a correct PIN has been entered into interfaces located on the obverse side of the card and interface 6620 has been utilized by a user. The code displayed on display 6630 may be communicated through a magnetic emulator/encoder in one or more tracks of magnetic stripe data.

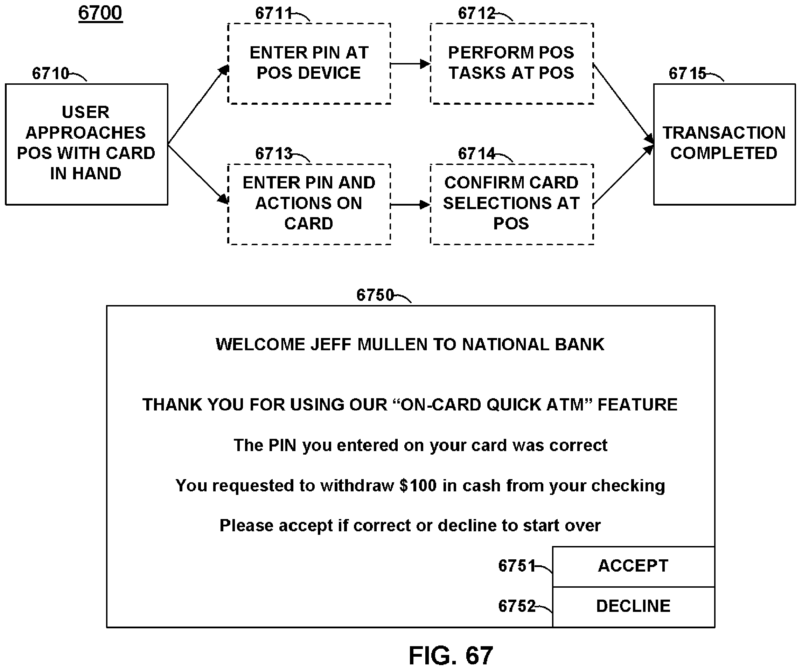

FIG. 67 shows flow chart 6700 and graphical user interface 6750. Flow chart 6700 may include, for example, step 6710, in which a user approaches a point-of-sale device with a payment card. Step 6711 may be provided that includes a user entering his/her PIN on the point-of-sale device. Accordingly, a user may perform associated tasks at the point-of-sale device in step 6712. The transaction may be completed at step 6715. Alternatively, for example, the user may enter his/her PIN and/or other point-of-sale decisions on his/her card in step 6713. A user may confirm the selections the user entered into his/her card at the point-of-sale in step 6714 after the card communicates the user's decisions to the point-of-sale device. The transaction may be completed in step 6715.

Graphical user interface 6750 may be provided on a display of a point-of-sale device. A point-of-sale device may include, for example, a cash-register, a payment card reader, and an ATM. Graphical user interface may include interfaces 6751 and 6752 for receiving manual input. Buttons may be provided on a point-of-sale device to receive user input. A card may display a graphical user interface and may include, for example, a capacitive touch screen such that a user may interact with interface areas of the touch screen in order to enter manual input into the touch screen. An ATM may include a graphical user interface to, for example, display a user's decisions that were entered into a card and communicated to the ATM via an output communications component (e.g., a magnetic emulator, RFID antenna, or IC chip).

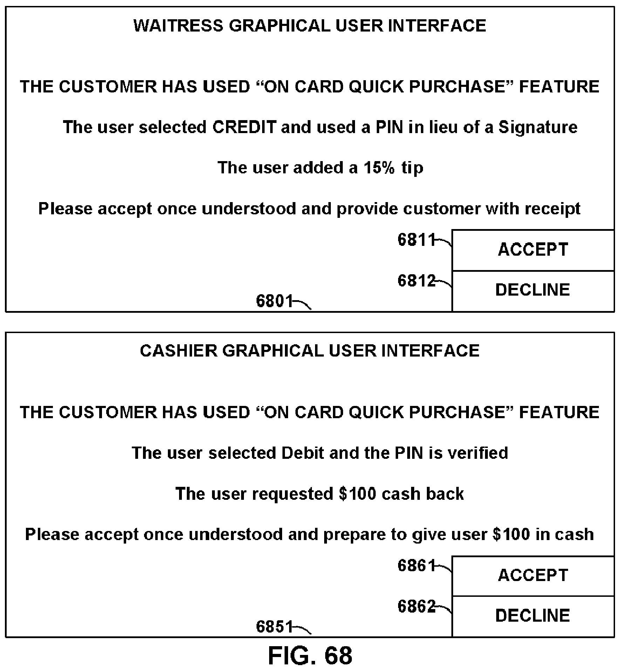

FIG. 68 shows graphical user interface 6801 that may include manual input interface 6811 and 6812 and graphical user interface 6851 that may include manual input interface 6861 and 6862. Graphical user interface 6801 may be provided on a display of a point-of-sale devices such that an operator of the point-of-sale device may be provided with the decisions of a the user of a card. The operator may acknowledge that the operator understands the user's selection by utilizing manual input interfaces 6811 and 6812. A point-of-sale device, such as a cash-register may perform a number of functions after an operator acknowledges understanding of a user's decisions. For example, a cash-register may cause a cash drawer to open such that an operator may remove cash from the drawer and hand the cash to a user (e.g., to complete a cash-back transaction).

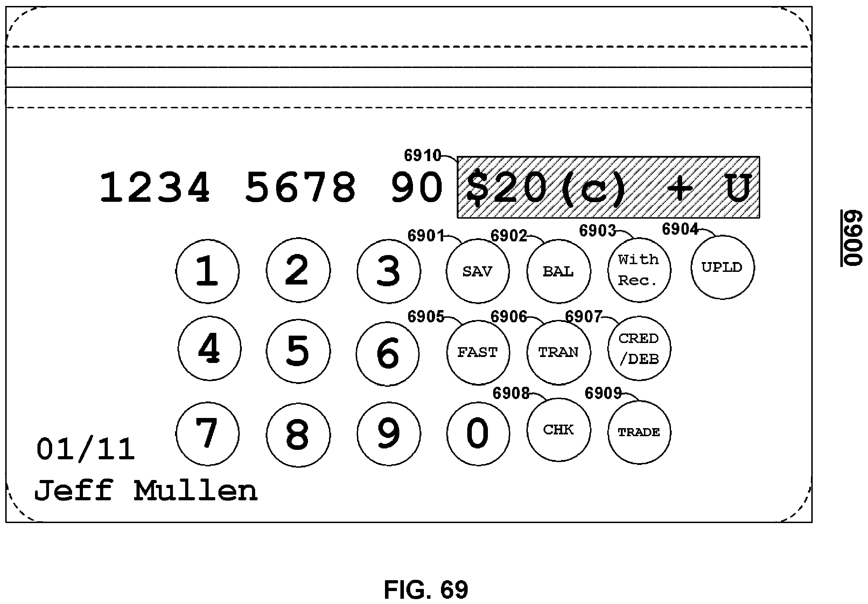

FIG. 69 shows card 6900 that may include user interfaces 6901-6909 and display 6910. A user may utilize interface 6902 to utilize a savings account. A user may utilize interface 6905 to utilize a fast-cash withdrawal function. A user may utilize interface 6902 to obtain a balance receipt. A user may utilize interface 6906 to conduct a transfer operation. A user may utilize interface 6908 to utilize a checking account. A user may utilize interface 6903 to note that a receipt is desired. A user may utilize interface 6907 to toggle between a credit and debit account. A user may utilize interface 6909 to instruct an ATM, for example, to provide the first graphical user interface as a stock-trading interface. User may utilize interface 6904 to initiate an upload feature. Display 6910 may note a user's selections (e.g., $20 withdrawal from a checking account followed by an upload operation).

A card may receive information in a variety of ways. For example, a card may receive information from an RFID antenna, an IC chip, or a magnetic emulator. For example, a magnetic stripe encoder on a point-of-sale device may communicate information to a coil located on a card. Thus, information may be communicated from a point-of-sale device to a card. A user may utilize interface 6904 to instruct a card to prepare for an upload function (e.g., a card operating a contact or coil in a receive mode instead of a transmit mode). Balance information, latest transaction information, or any other type of information may be communicated to a card and displayed on display 6910. Information may be uploaded to a card via audio signals received by an on-card microphone or light signals received by an on-card light sensor. A user may utilize a combination of buttons to provide a combinational decision. For example, a user may utilize interface 6901, then interface 6906, then numerical interfaces to enter in the number "100," then interface 1308 to instruct the card that the user desires an ATM machine to transfer $100 from the user's savings account to the user's checking account.

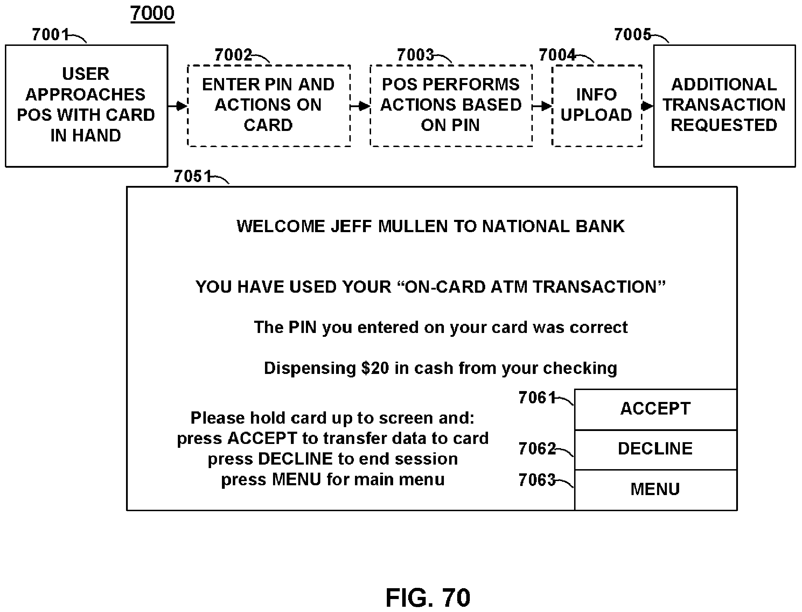

FIG. 70 shows flow chart 7000 and graphical user interface 7051. Flow chart 7000 may include step 7001, in which a user approaches a point-of-sale with a card. The user enters his/her decisions into a card in step 7002. Step 7003 occurs, in which the point-of-sale performs actions based on the actions and PIN received from the user's card. Step 7004 may then occur, in which information is uploaded from the point-of-sale to the card. An additional transaction may occur in step 7005. Graphical user interface 7051 may be included with manual input interfaces 7061, 7062, and 7063. Interface 7063 may take a user to an options menu. A display screen may communicate information from a point-of-sale device (e.g., an ATM) to a card.

A light detector may be provided on a card that may receive light pulses indicative of data. Accordingly, for example, a user may hold a card up to a display - such as the screen of a laptop, stationary computer, or mobile phone - and information may be communicated from the display to the card via the light detector. Similarly, a light source may be utilized to communicate information from one device to another. For example, a light source (e.g., LED) may be utilized to communicate information from one card to another. Similarly, a magnetic stripe reader may include a light source. A card may be positioned over the light source such that a light detector of the card is aligned with the light source to receive light. Accordingly, the light of a magnetic stripe reader (or other type of reader) may be utilized to communicate information back to a card. A user may utilize interfaces on the card (e.g., buttons) to initiate a transfer of data from one card to another card or from a device to a card. A variety of types of data may be communicated. For example, money may be communicated from one debit card to another debit card such that payments may occur between the cards. Accordingly, for example, the next time a card is utilized via a reader (e.g., a magnetic stripe reader) information of the transfer may be communicated to a server for processing. Light may be utilized to transfer data from a card to a computer using, for example, a camera (e.g., webcam) on the computer.

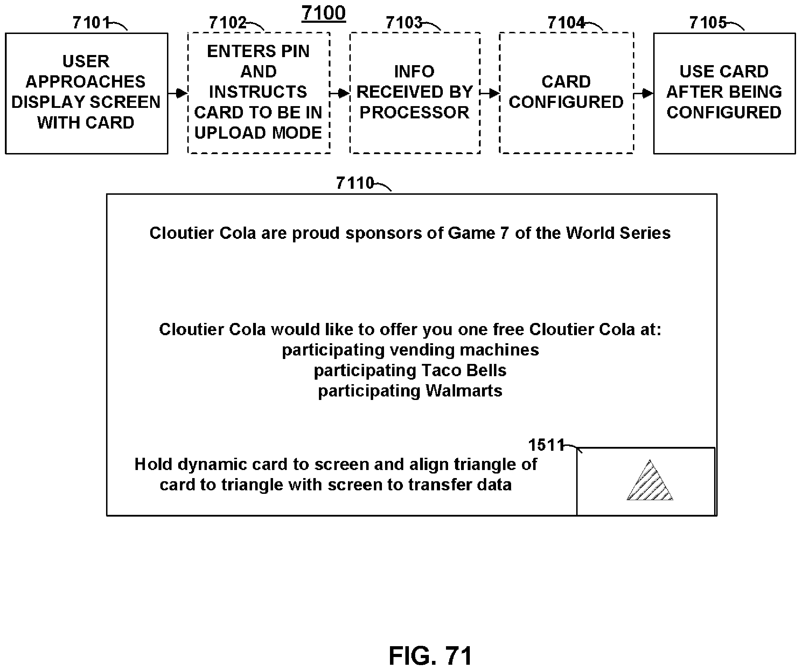

FIG. 71 shows flow chart 7100 and graphical user interface 7110. Flow chart 7100 may include step 7101, in which a user approaches a card-reader having a display with a card. The user may enter his/her PIN into the card and provide instructions to the card in step 7102. Particularly, for example, a user can instruct the card to operate in an upload mode. Step 7103 may initiate in which information is received by the processor of a card from a point-of-sale device. The card may configure itself depending on the received information in step 7104. Step 7105 may occur in which the card is used by a user after the card is configured.

Display screen 7110 may be provided. For example, display screen 7110 may be provided to communicate information to a card. For example, area 7111 may communicate information to a light sensor located on a card by providing light pulses that may be understood as information by a processor located on a card. Area 7111 may be provided on any type of display. For example, area 7111 may be provided during a commercial or during a television show. Alternatively, for example, area 7111 may be provided on a webpage. Information that may be communicated through area 7111 may include, for example, coupons that may be utilized at various point-of-sale devices. For example, a cola commercial may communicate a coupon for a free bottle of cola.

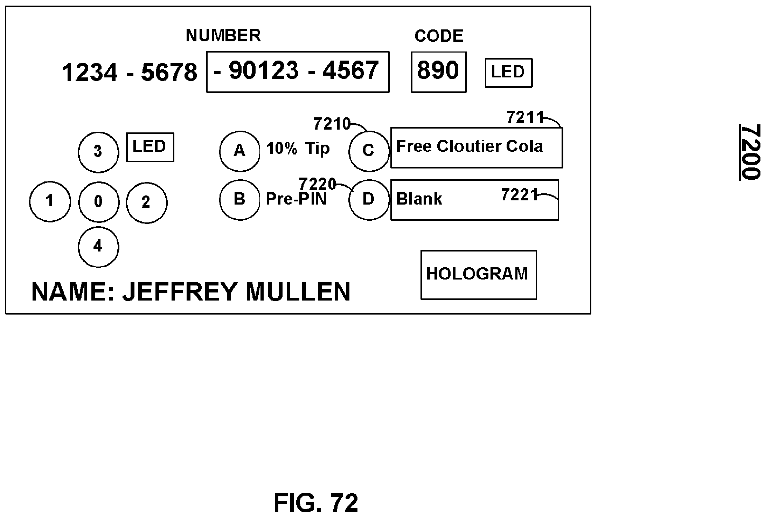

FIG. 72 shows card 7200 that may include, for example, button 7210, display 7211, button 7220, and display 7221. Persons skilled in the art will appreciate that a card may receive coupon information (e.g., from a light-emitting area on a commercial or webpage). The coupon information may include information to display on a display of a card as well as information to communicate to a reader when the coupon is desired to be used (e.g., via activation of button 7210). For example, a coupon code may be communicate via a magnetic emulator. Received coupons may expire after a period of time and may be erased from a card's memory. The time may be set by the coupon issuer and communicated to a card. A card may keep a list of displays that do not have a coupon associated with them and may, for example, display a newly received coupon in the next available display. Displays may be kept OFF until, for example, a user enters a PIN into a card and the card verifies the PIN. Multiple coupons may be associated with a display and a user may toggle through the coupons by pressing a button associated with the display. A user may select a coupon by, for example, holding a button down for a period of time (e.g., more than 2 seconds).

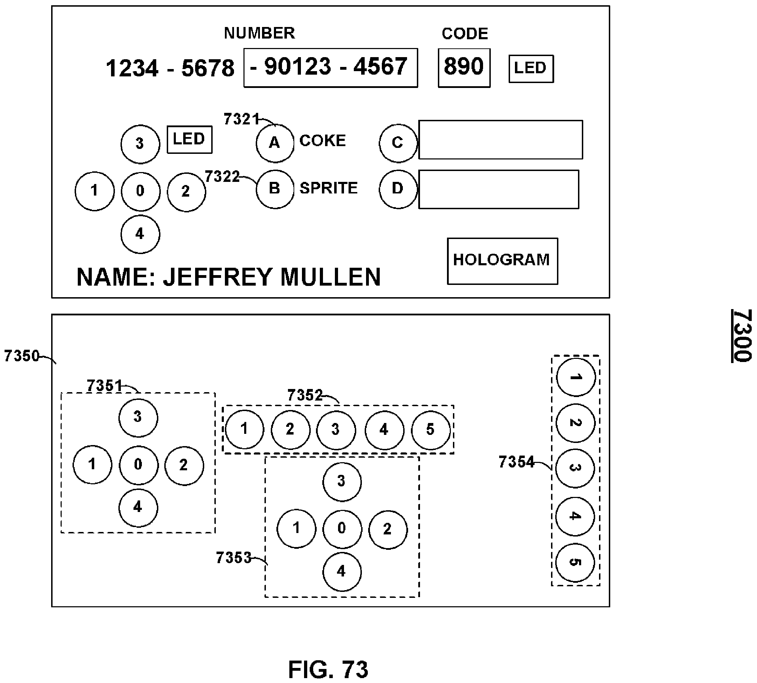

FIG. 73 shows card 7300 that may include buttons 7321 and 7322. A card may include buttons associated with, for example, particular items. A user may select such items and utilize the card at a vending machine. The vending machine may receive both payment information and ordering information. Accordingly, a user waiting in line to utilize a vending machine may select a button associated with cola and may simply swipe his/her card when the user approaches the vending machine. The vending machine may autonomously detect that the user desires a cola and may dispense a cola and charge the amount of the cola to the user's card.

FIG. 73 shows card 7350. Card 7350 may include areas 7351-7354. Areas 7351-7354 may include multiple user interfaces (e.g., mechanical or capacitive buttons). Persons skilled in the art will appreciate that the location of areas 7351-7354 may result in a different way that a user interacts with card 7350. For example, area 7353 may be located in the proximity of the center of the bottom of card 7350 such that left and right handed users may decide to utilize area 7353 in a similar manner. Area 7351 may be located within the proximity of the center of the left side of a side of card 6350 (e.g., the front side of card 7350). Accordingly, a right-handed user may find it easier to rotate the card such that user views the left-side of card 7350 as the top of card 7350. Accordingly, indicia located on an area may be provided in an orientation that can easily be read if the card was rotated and held in a different orientation than that shown in FIG. 73 (e.g., the card is rotated such that the indicia of area 7354 is oriented properly with respect to a user of card 7350). Interfaces within an area may be aligned in a line formation or in a different formation (e.g., a directional pad formation).

A display may also be utilized as an interface. For example, a display may include a contact and an electronic ink. The electronic ink may change colors in response to, for example, a particular electrical signal being supplied to the contact. A capacitive sensor may be coupled to such a contact, however, such that a user interaction with the contact may be sensed by the capacitive sensor. Accordingly, a card may include a display that can also receive user input. Persons skilled in the art will appreciate that a display may include multiple contacts. For example, a display may include multiple 7-segment (e.g., to display digits) or 11-segment, 14-segment, or 16-segment (e.g., to display alphanumerics) regions where each segment may be coupled to a capacitive sensor.

A biometric sensor may be placed on a card or other device. Such a biometric sensor may be, for example, a fingerprint reader. Accordingly, one or more fingerprints may be stored in the memory of a card and compared to scanned fingerprints. Different fingerprints may activate the card differently (e.g., utilize a different user's payment card info).

Persons skilled in the art will appreciate that a user's payment card number (e.g., credit card or debit card number) does not have to change. A display may hide this payment card number until an appropriate unlocking code is entered into buttons of the card. Similarly, a magnetic emulator may not be provided current until the proper unlocking code is entered - thus keeping magnetic information private and not allowing undesirable readers to read a card. A security code may be displayed on the same or a different display. A button may be provided representative of an online purchase (or a user may utilize buttons to instruct the processor that an online purchase is desirable). For such an online purchase, the credit card number and the security code may be displayed - but the magnetic emulator may not be activated. In doing so, the level of security of the card is increased. Furthermore, for example, a button may be provided representative of in-store purchases (or a user may utilize buttons to instruct the processor that an in-store purchase is desirable). Accordingly, a processor may be signaled that an in-store purchase is desired. A different operation may be associated with different types of purchases (e.g., online or in-store). Accordingly, for example, magnetic emulators may be activated for an in-store environment - but not the displays. Accordingly, for example, a restaurant cashier may not be able to read the credit card number from the card, but may still be able to swipe the card. If a reader is down or a cashier requires reading particular information (e.g., a security code or credit card number information) then controls may be utilized to communicate this information. A record of the types of transactions may be stored and may be communicated in discretionary fields of data within a transmitted data track. Such record information may be utilized, for example, to further increase security and/or introduce a variety of additional functionality.

Different types of cards may be provided on a card. For example, a security ID number and a credit card number may both be provided on the same card. A button may be utilized to allow a user to provide instruction to a processor such that the processor can display (e.g., visually and/or magnetically) the desired information. For example, a user may determine to use one of a variety of payment accounts (e.g., credit and/or debit) for a purchase. An entire payment number (e.g., credit or debit) may be changed and/or hidden visually and/or magnetically. A portion of a payment card number (e.g., credit or debit) may be changed and/or hidden visually and/or magnetically.

Persons skilled in the art will appreciate that a display on the card may display a credit card number that does not change with time (or transaction or button press). Additionally, for example, a magnetic emulator (or multiple magnetic emulators) may magnetically communicate financial data that does not change with time. Such a card may reduce, for example, the effects of physical card theft and card cloning.

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipt may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

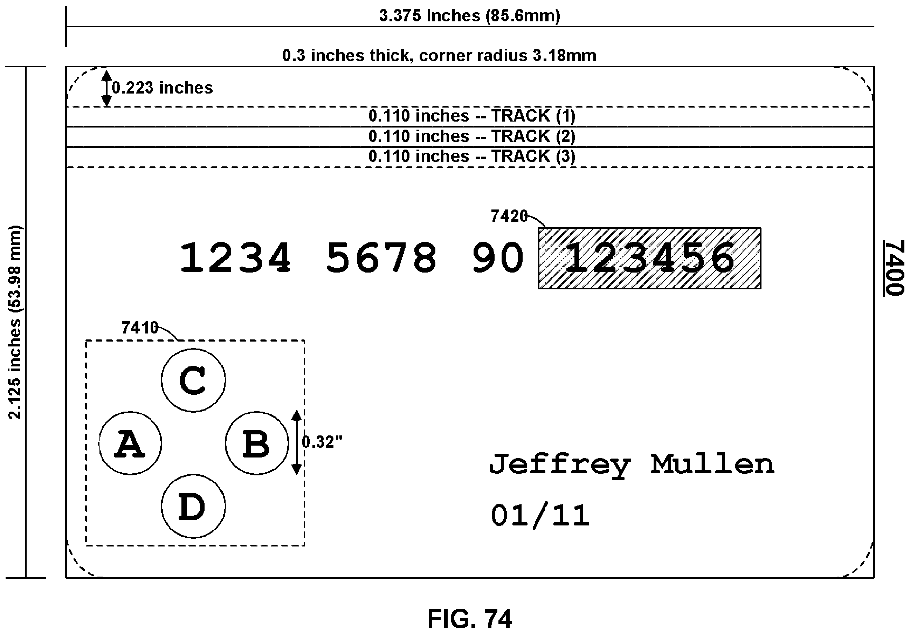

FIG. 74 shows card 7400. Card 7400 may include display 7420 and button array 7410. Button array 7410 may be located anywhere on a card. For example, one or more buttons of button array 7410 may be located over a magnetic emulator without interfering with data communicated from the magnetic emulator to a read-head of a magnetic stripe reader.

A button array may more generally be provided as a user interface array. A user interface may, for example, be able to receive manual input from a user. Any number of buttons (or other user interfaces) may be provided in an array. For example, four buttons may be provided. Alternatively, for example, five or six buttons may be provided. The buttons may be provided in one or more rows or columns. Alternatively, for example, the buttons may be included in another formation to decrease the distance between between the most remote buttons. For example, five buttons may be provided in the shape of a directional pad. In reducing the distance between the most remote buttons in an array configuration, a user may be provided with a configuration that allows for very fast, and possibly one-handed, user interaction.

Persons skilled in the art will appreciate that any card face may be located on the obverse or reverse of a card. Accordingly, for example, a magnetic emulator/encoder may be provided on any card face. Similarly, any permanent information (e.g., embossed card number) may be provided on any card face. Additionally, for example, persons skilled in the art will appreciate that a magnetic emulator may be provided inside of a card.

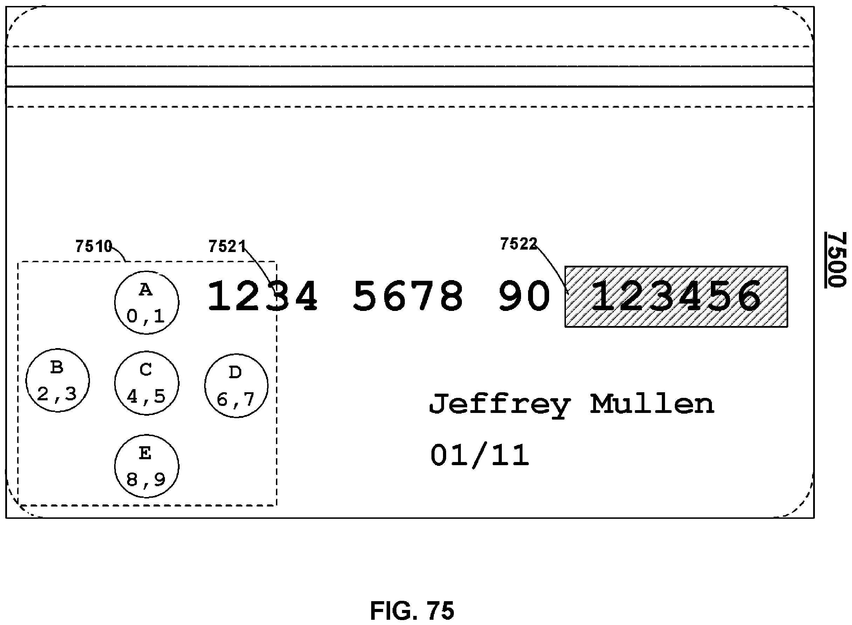

FIG. 75 shows card 7500 that may include button array 7510. Button array 7510 may include any number of buttons, or other user interfaces, such as five buttons. Indicia may be provided on the buttons in order to enhance the functionality of the buttons. For example, a button may include both a character and multiple digits. In doing so, for example, a user may be able to enter in both numerical and character based codes. Button array 7510 may be provided in a directional pad configuration. Accordingly, for example, a card may display on a display information that can be scrolled up and down as well as left and right. Accordingly, a directional pad configuration may facilitate a user when the user interfaces with such a display functionality. A directional pad configuration may also, for example, allow for spaces at the corners of the directional pad configuration. Permanent information may be provided in such spaces. For example, one or more digits of number 7521 may be provided in such spaces. Display 7522 may also be provided to display information such as, for example, payment card number information. Button array 7510 may be located in any area on the obverse or reverse side of a card. For example, button array 7510 may be located about the lower right, upper right, lower left, or upper left corner of a card.

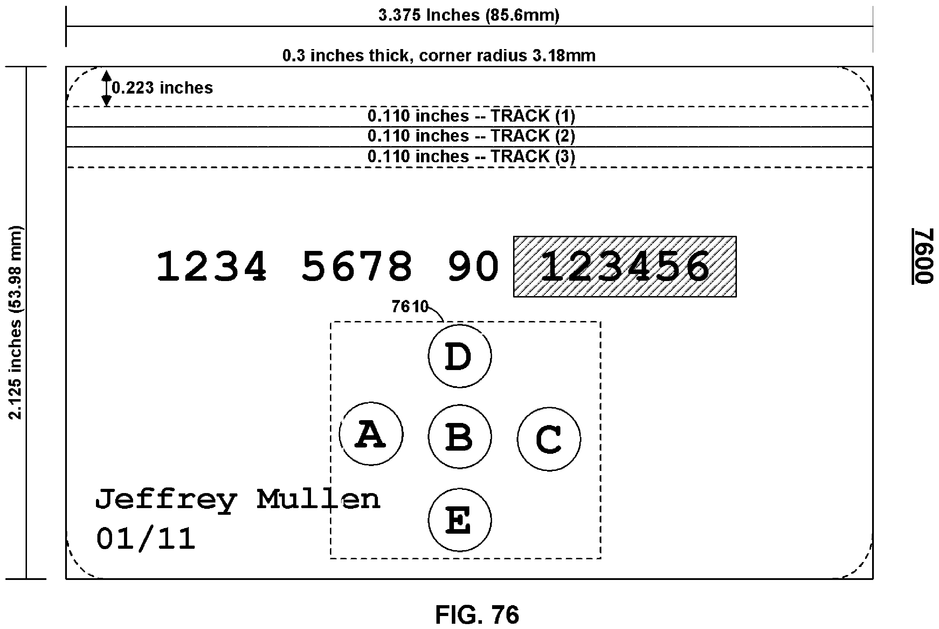

FIG. 76 shows card 7600 that may include button array 7610. Persons skilled in the art will appreciate that each button of a button array may be coupled to different ports of a microprocessor. Such ports may be, for example, triggerable ports. Additionally, for example, each button of button array 7610 may be coupled to intermediary circuitry such as a multiplexer. Button array 7610 may be located, for example, in the proximity of the middle of card 7600. For example, button array 7610 may be located in the middle, near the bottom of card 7600 or in the middle, near the top of card 7600. Alternatively, for example, button array 7610 may be located in the center of card 900.

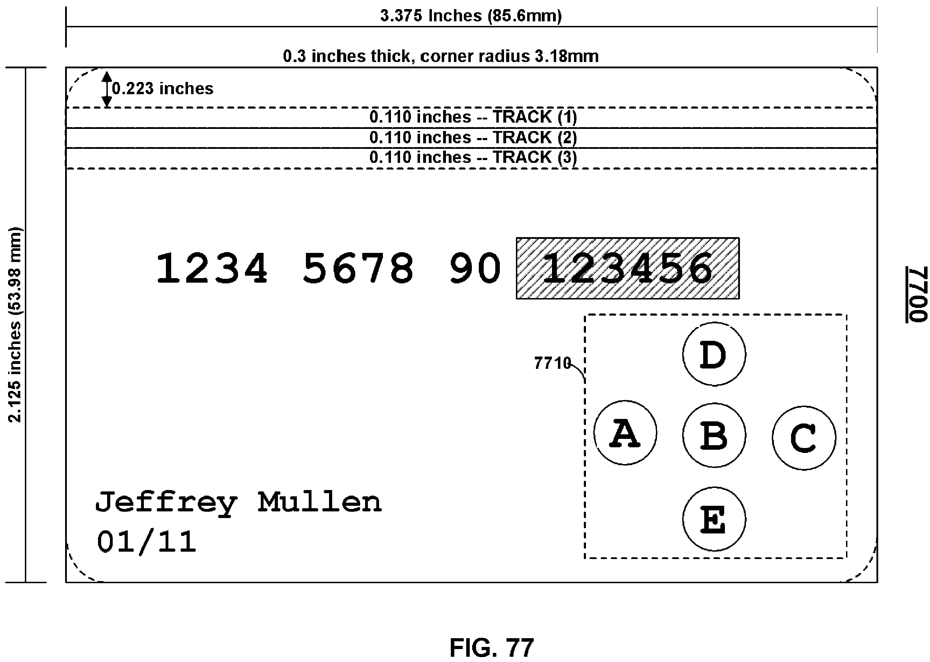

FIG. 77 shows card 7700 that may include button array 7710 located on the lower right corner of a surface of card 7700.

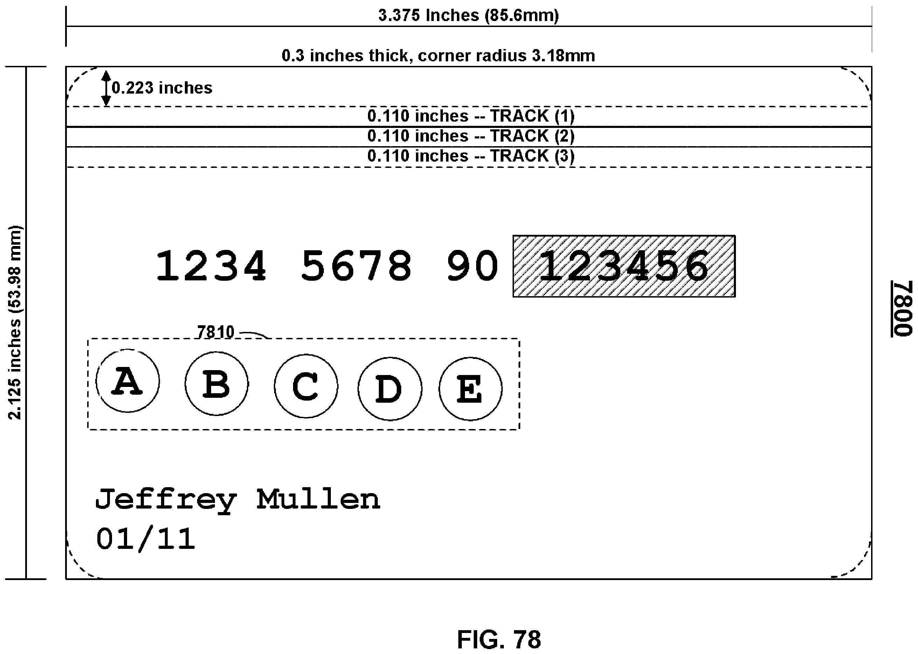

FIG. 78 shows card 7800 that may include button array 7800. Button array 7800 may include, for example, multiple buttons located in a horizontal configuration. By providing buttons in a horizontal configuration, for example, a user may easily understand any numbering scheme of the buttons in the array. For example, buttons may be numbered, or characters ordered, in ascending order from left to right (for users that read left to right) or right to left (for users that read right to left). Array 7810 may be located anywhere on the front or back of a card. For example, array 7810 may be located on the left-side or right-side of card 7800.

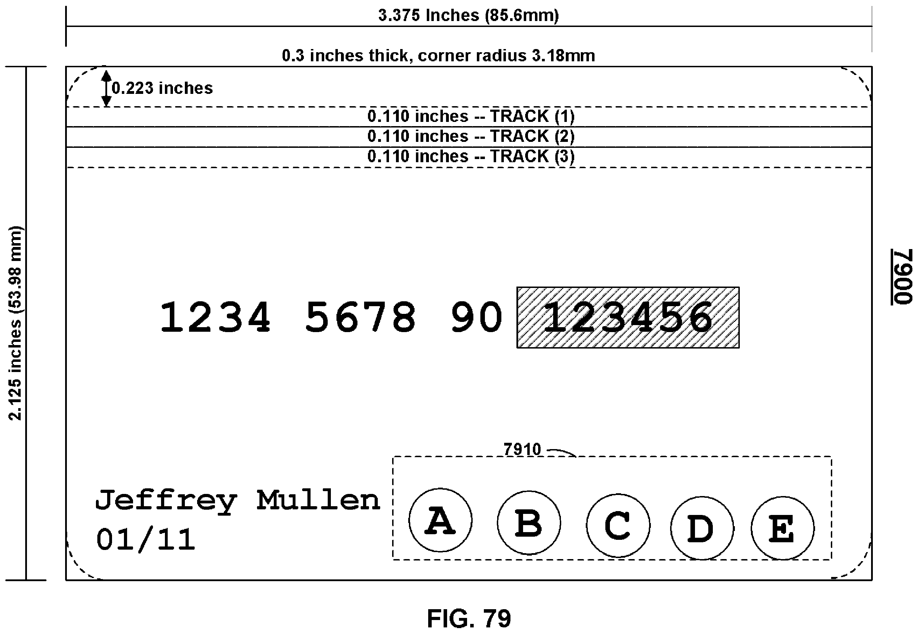

FIG. 79 shows card 7900 that may include button array 7910.

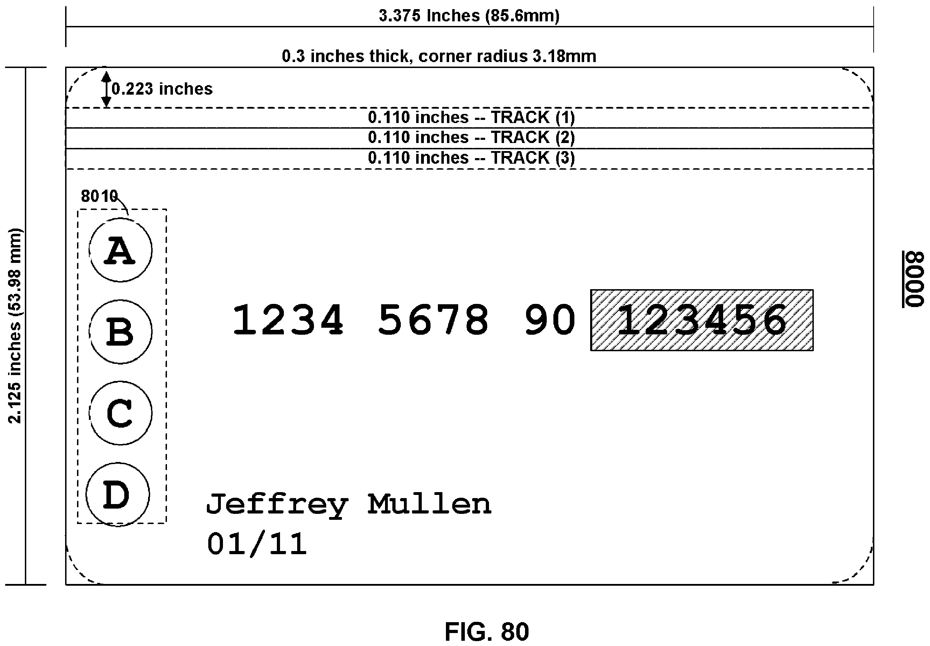

FIG. 80 shows card 8000 that may include button array 8010. Button array 8010 may include, for example, any number of buttons in a vertical configuration. Indicia located on the buttons may be oriented such that the indicia is properly read when the buttons are oriented vertically with respect to the user. Alternatively, for example, indicia located on the buttons may be oriented horizontally such that a user may rotate a card so that the vertical configuration is perceived as a horizontal configuration and the indicia is read horizontally.

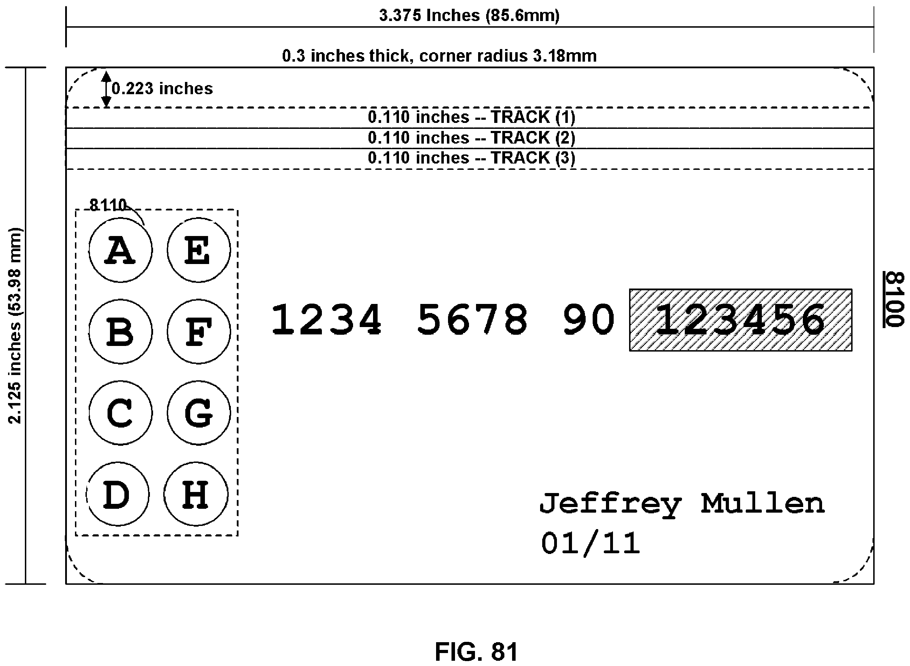

FIG. 81 shows card 8100 that may include, for example, button array 8110. Button array 8110 may include, for example, two columns of buttons. The buttons, liked any of the control interfaces provided, may include indicia such as, for example, numbers, characters, and symbols. Button array 8110 may include an even number of buttons such as, for example, eight buttons.

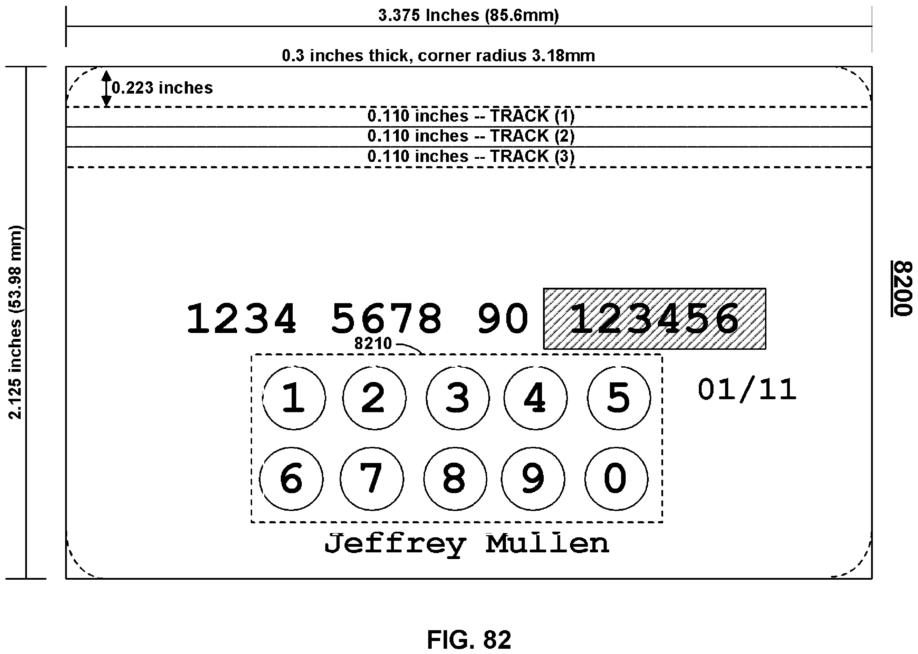

FIG. 82 shows card 8200 that may include, for example, button array 8210. Button array 8210 may include, for example, two rows of buttons. Button array 8210 may include, for example, an even number of buttons. Button array 8210 may include, for example, ten buttons. Persons skilled in the art will appreciate that additional buttons may be provided on a card outside of a button array. Buttons in a button array may, for example, be associated with a particular functionality or set of functionalities (e.g., PIC/PIN entry).

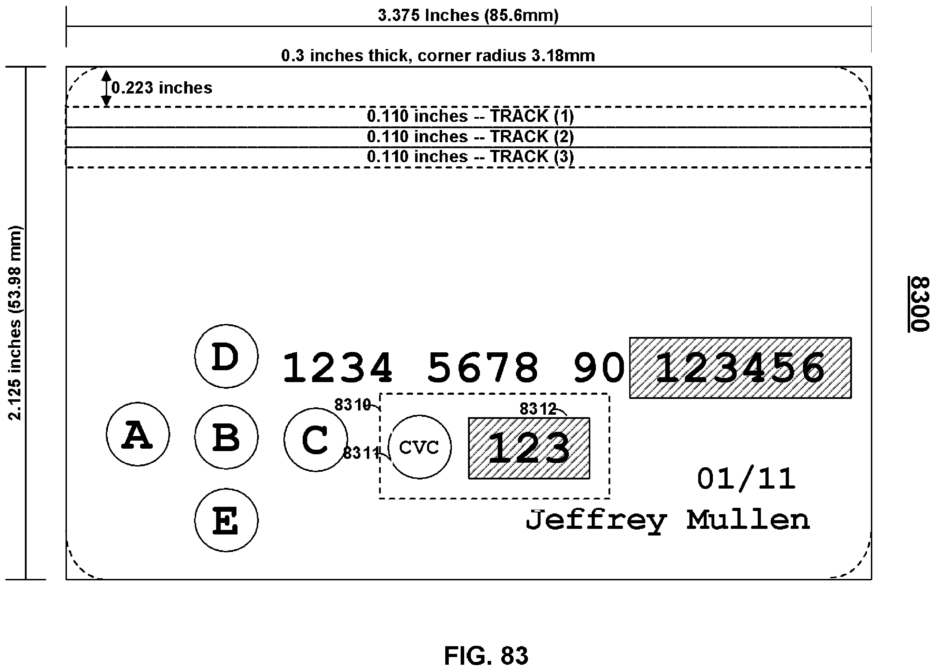

FIG. 83 shows card 8300 that may include button and display portion 8310. Portion 8310 may be located on any surface of any card. Button and display portion 8310 may include, for example, display 8312 and button 8311. Display 8312 may display particular information every time button 8311 is pressed. For example, display 8312 may display a code (e.g., CVC) every time button 8311 is pressed. Any display, such as display 8312 may be, for example, a bi-stable display such that power is utilized to change the state of the display - but is not utilized to maintain the state of the display. Accordingly, for example, a dynamic security code may be provided such that each time button 8311 is pressed, another security code may be displayed on display 8312. Such a security code may be, for example, a one-time security code or, alternatively, a code that is generated based on time. A bi-stable display may utilize power to change the number that is displayed but may not, for example, utilize power to maintain the display of any particular number. Alternatively, for example, a non bi-stable display may be utilized. A non bi-stable display may be utilized that utilizes power to maintain the display of indicia.

A card may include, for example, more than one display. A card may include, for example, both bi-stable and non bi-stable displays. In doing so, for example, a card may properly conserve energy from one or more on-board batteries. Particularly, a bi-stable display may be provided on a card that absorbs a large amount of power in order to change displayed indicia. Accordingly, a non bi-stable display may be provided on a card that absorbs a small amount of power in order to change displayed indicia and a small amount of power to maintain the displayed indicia. Accordingly, for example, a bi-stable display may be utilized to display information that is desired to be viewed for a relatively long period of time while a non bi-stable display may be utilized to display information that is desired to be viewed for a relatively short period of time. In providing both a bi-stable and a non bi-stable display, for example, electrical energy absorbed by a card may be minimized. A security code, for example, may be provided on a non bi-stable display. A portion of a card number may be provided, for example, on a bi-stable display. Alternatively, for example, a security code may be provided on a bi-stable display and a card number may be provided on a non bi-stable display. A non bi-stable display may also be advantageous, for example, when indicia is desired to be frequently changed on a display (e.g., once every few seconds).

Display 8312 may, for example, be provided as a non bi-stable display and may display a code that changes with time. Accordingly, for example, the activation of button 8311 by a user may cause a code to be displayed on display 8312 for a pre-determined period of time (e.g., approximately 10 seconds). Accordingly, for example, the number may change according to a longer period (e.g., an hour or a day). Accordingly, a user may press button 1611 multiple times during that period and the same number may be displayed on display 8312. A code may comprise, for example, three or four digits. Such a code may be provided magnetically through a magnetic emulator or encoder. Alternatively, a different three or four digit code may be provided through a magnetic emulator or encoder. A display may display indicia with two color tones (e.g., black and white) or may display indicia with several color tones.

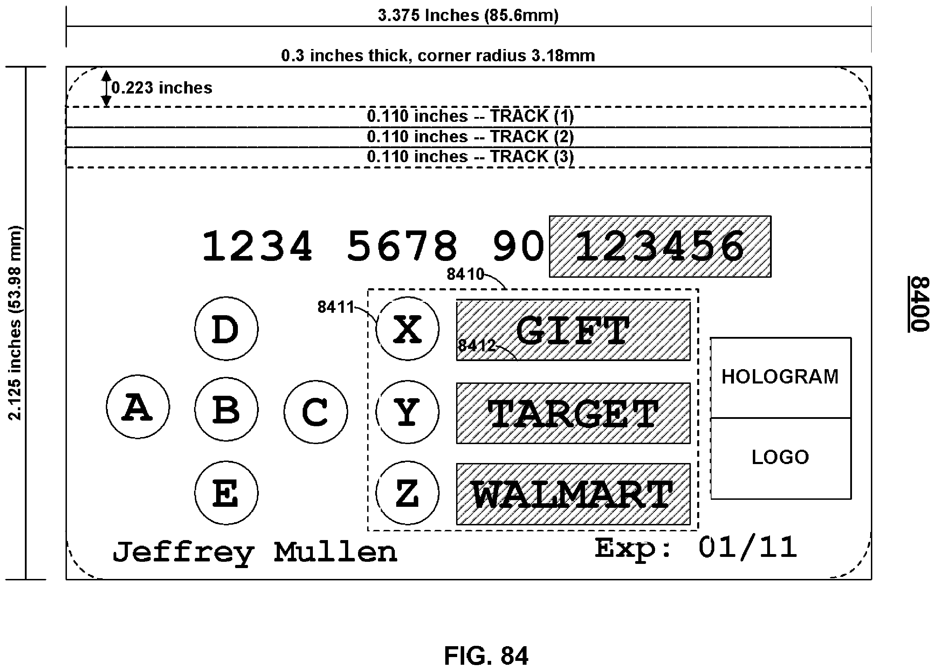

FIG. 84 shows card 8400 that may include portion 8410. Portion 8410 may include multiple buttons, each associated with a different display. Multiple buttons may be associated with a single display. An association may be functional in that interaction with a particular button causes an associated display to perform a particular function. Code executed by a microprocessor may, for example, define the association of a button and a display.

Card 8400 may be utilized as, for example, a programmable gift card. For example, a store may sell card 8400. A purchaser of the gift card may, for example, give the gift card to a friend or relative. The gift card may be associated with, for example, a pre-determined amount of money (e.g., $20, $50, $100, $200, $250, or $500). Alternatively, for example, the amount of the gift card may be determined at purchase by the purchaser. The recipient of the gift card may, for example, visit a webpage associated with the issuer of the gift card. The recipient may, for example, enter in an identification code that identifies the particular gift card. The user may then be provided with a number of stores (e.g., Target, Best Buy, and Walmart). The user may select any number of stores and may allocated the total amount of the gift card amongst the selected stores. The user may then be provided with a code (or several codes). The user may utilize controls (e.g., buttons) located on gift card 8400 to enter this code. The code may, for example, be received by a microprocessor located on card 8400. The microprocessor may utilize the code to identify the stores selected by the user and the associated gift amount allocations. Accordingly, for example, the gift card may associate the selected stores with a particular display and button. The gift card may automatically display the name of the store and the amount of each gift allocation on the display once the microprocessor processes the received code. Accordingly, a user may walk into a store and select an appropriate button to communicate information about the gift card to a card reader (e.g., via an IC chip, RFID antenna, or magnetic encoder/emulator). Persons skilled in the art will appreciate that a programmable gift card may be provided with a static magnetic stripe without a magnetic emulator or encoder. Instead, for example, the programmable gift card's identification may be sent through a reader and a remote server may determine whether the store associated with the reader was selected by the user and allocated a particular gift amount. Alternatively, for example, the card may display and electrically communicate different information depending on, for example, the particular gift a user selected on a programmable gift card.

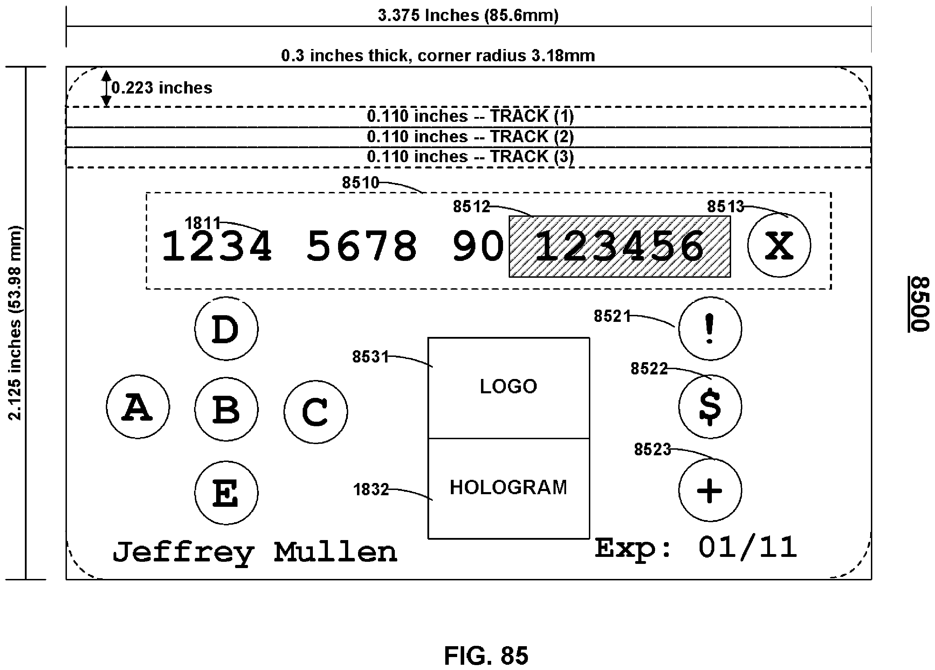

FIG. 85 shows card 8500. Card 8500 may include portion 1810 that may include permanent information 8511, display 8520, and button 8513. Buttons 8521, 8522, and 8523 may also be included. The activation of button 8513, 8521, 8522, and 8523 may cause different information to be displayed on display 1812. Hologram 8532 and logo 8531 may be provided on card 8500. Hologram 8532 and 8531 may be located about the middle of one of the surfaces of card 8500.

Card 8600 may include, for example, portion 8610. Portion 8610 may include, for example, multiple buttons and light sources. For example, portion 8600 may include a light source (e.g., LED) for every button (e.g., four light sources and four buttons). Alternatively, portion 8600 may include a different number of buttons and light sources. For example, portion 8600 may include five buttons and four light sources. The light sources may emit light in order to provide the user of card 8600 with valuable information. For example, the light sources may indicate to a user which button or buttons were pressed by a user. For example, a user pressing button 1910 may cause both light sources 8621 and 8622 to emit light. A user pressing button 8612 may cause all the light sources within portion 8610 to emit light. Sources of light may be provided that emit a single color or more than one color.

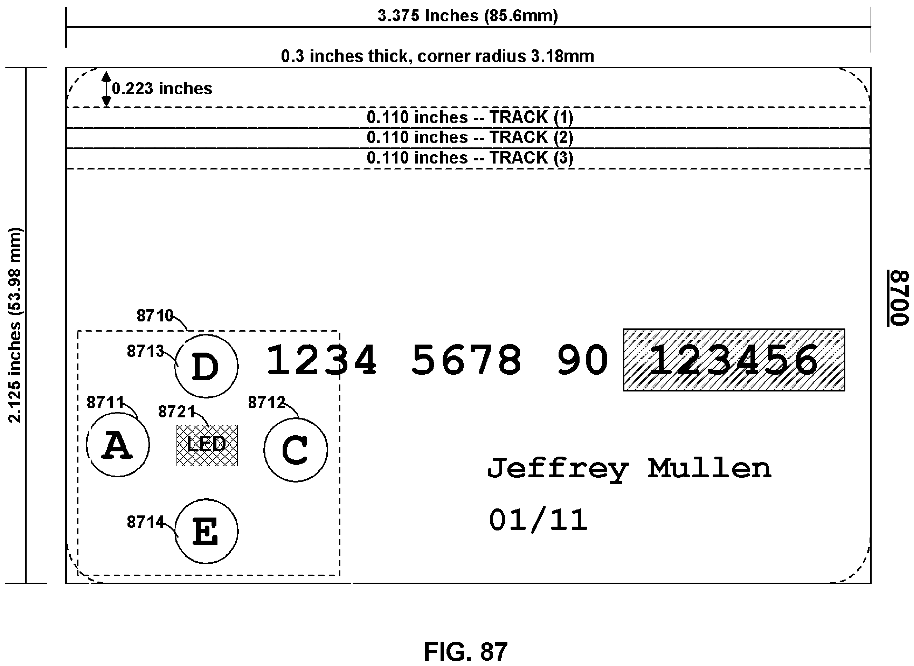

FIG. 87 shows card 8700 that may include portion 8710. Portion 8710 may include, for example, multiple buttons and a single light source. For example, portion 8710 may include buttons 8711-8714 and light source 8721. Portion 8710 may include any number of buttons (e.g., five, six, ten, twelve). Light source 8712 may emit light whenever a button is pressed. In doing so, for example, a user may be able to receive information indicative of a proper button press. Light source 8721 may emit light of two or more colors. For example, light source 8721 may emit three colors. The three colors may be, for example, GREEN, RED, and ORANGE. A button press may cause light source 8721 to emit light of the color ORANGE. The input of a proper PIC/PIN may cause light source 8721 to emit light of the color GREEN. The input of an improper PIC/PIN may cause light source 8721 to emit light of the color RED.

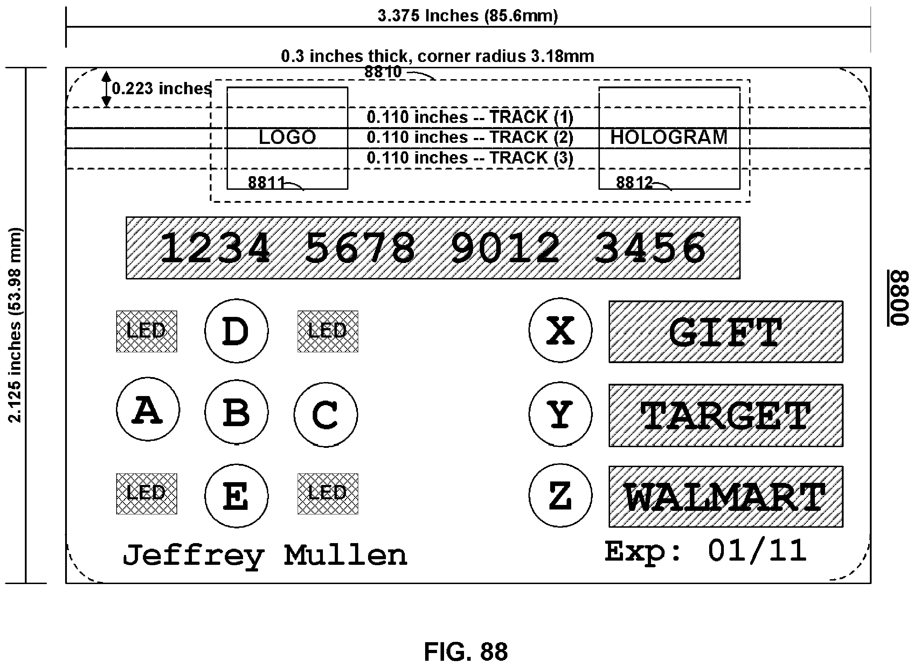

FIG. 88 shows card 8800 that may include, for example, portion 8810. Portion 8810 may be included, for example, over a magnetic emulator and/or encoder. For example, portion 8810 may include logo 8811 and hologram 8812. One or more magnetic emulators, for example, may be provided in card 8800 such that logo 8811 and hologram 8812 may be provided on, or printed upon, one or both surfaces of card 8800 about the magnetic emulator. In doing so, for example, the area of a card surface that a card issuer may personalize may be increased. Persons skilled in the art will appreciate that multiple tracks of data may be communicated to a magnetic stripe reader via one or more emulators. For example, two tracks of data may be communicated by two emulators. As per another example, three tracks of data may be communicated by three emulators.

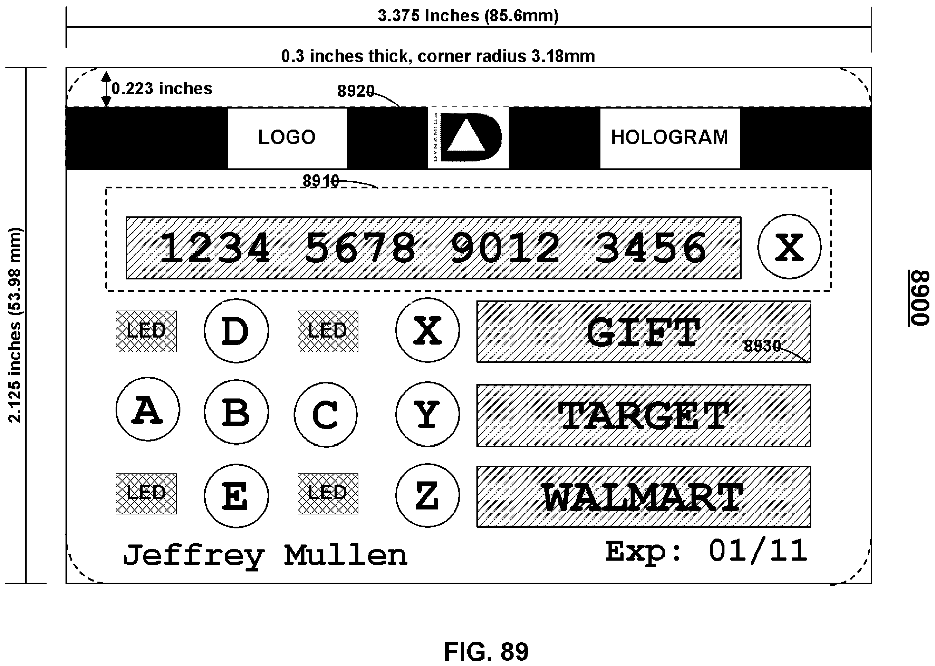

FIG. 89 shows card 8900. Card 8900 may include, for example, printed stripe indicia 8920. Stripe indicia 8920 may be printed on an area over one or more magnetic emulators. Stripe indicia 8920 may be printed such that a user recognizes how to properly swipe card 8920 in a reader. Card 2200 may also include portion 8910. Portion 8910 may include, for example, a display and a button. The display of portion 8910 may display a payment card number when an associated button is pressed or, for example, when an appropriate PIC/PIN is entered into a card. The display of portion 8910 may be longer than the length of 2/3rds the length of a card. Display 8920 may also be included and may have a length between 1/2 and 1/3 the length of card 8900.

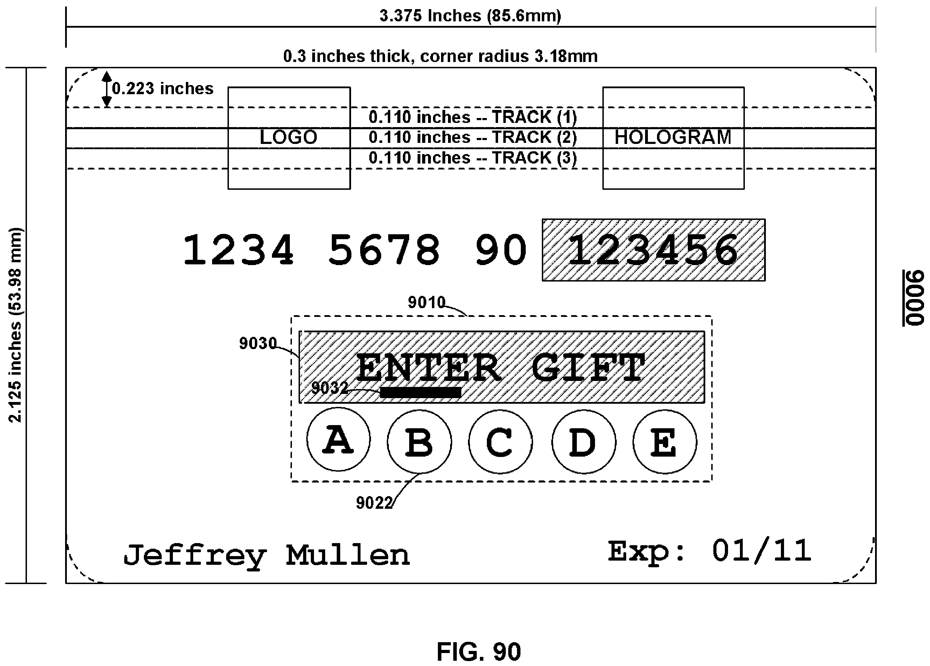

FIG. 90 shows card 9000. Card 9000 may include, for example, portion 9010. Portion 9010 may include, for example, display 9030 and multiple buttons. Button 9022 may be included. Each button of portion 9010 may be associated, for example, with different data that can be displayed on display 9030. When a particular button of portion 9010 is pressed, display 9030 may, for example, display indicia representative that a user has pressed that particular button. For example, the activation of button 9022 may cause, for example, indicia 9032 to be displayed in addition to information associated with button 9022. Accordingly, for example, a user may not only be acknowledged that a button was pressed, but also the particular button that was pressed. Furthermore, for example, power may be conserved as both the information associated with a button and the indicia indicative of a button press may be provided by the same display device. Portion 9010 may include buttons that are aligned along an edge of a display.

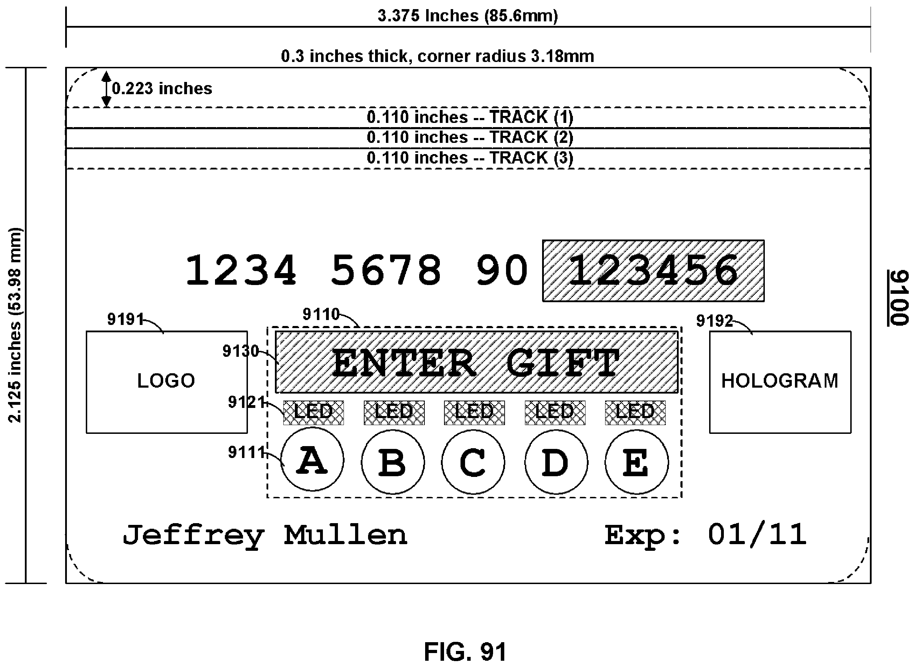

FIG. 91 shows card 9100 that may include portion 9110. Portion 9110 may include display 9130 and a multitude of buttons and light sources. For example, a light source may be provided for every button located in portion 9110. The activation of a button of portion 9110 may cause, for example, display 9130 to display particular information associated with the button activation. Additionally, the activation of button 9111 may cause associated light source 9121 to emit light. Light source 9121 may emit light for a particular period of time (e.g., less than a second, more than a second, more than five seconds). Light source 9121 may periodically emit light to indicate to a user that button 9111 has been activated by the user. Logo 9191 may be located on one side of portion 9110 and hologram 9192 may be located on the other side of portion 9110.

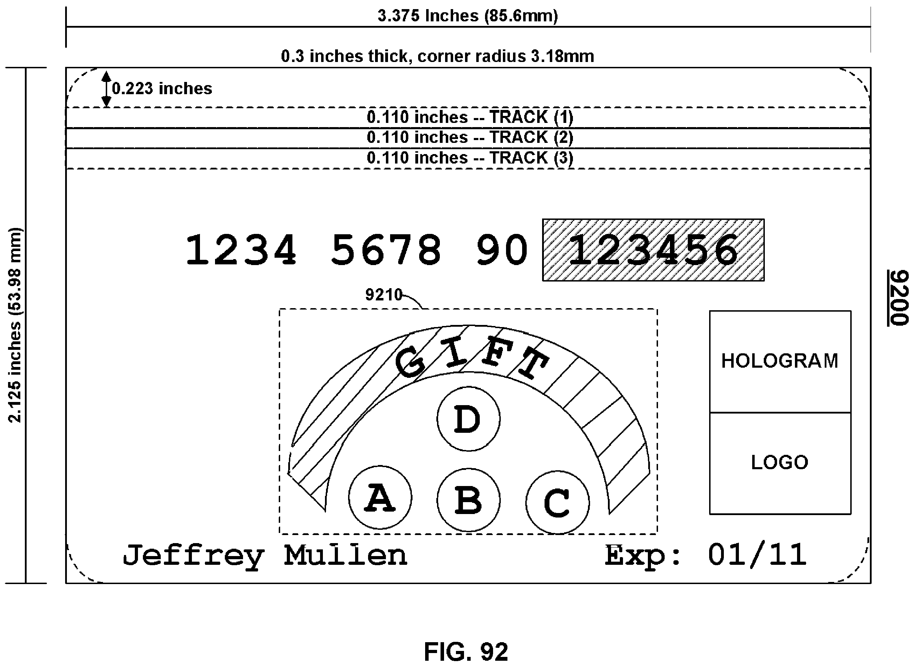

FIG. 92 shows card 9200 that may include portion 9210. Portion 9210 may include a display having one or more curved edges. Such displays may be, for example, LCD and/or electrochromic displays.

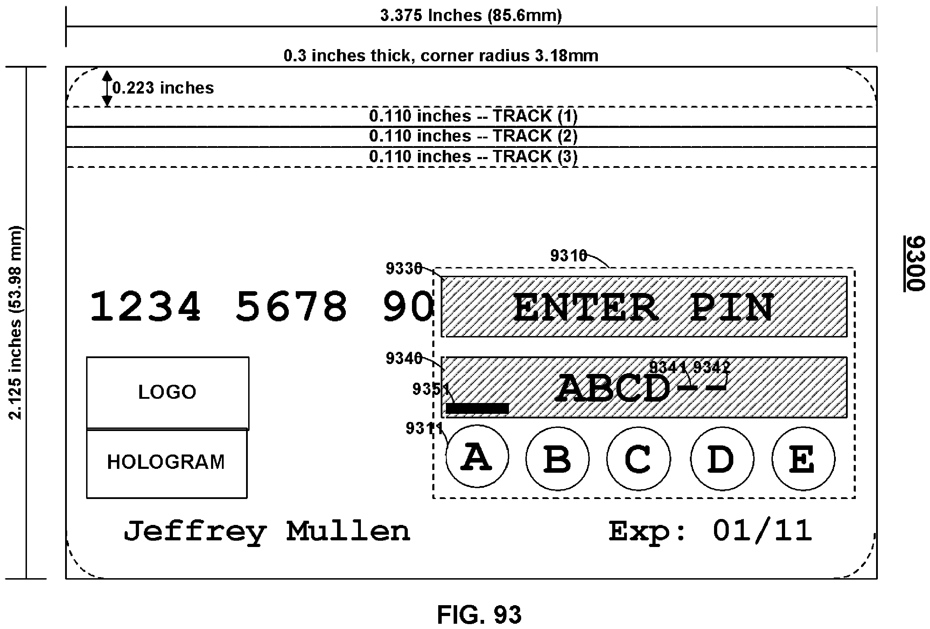

FIG. 93 shows card 9300 that may include portion 9310. Portion 9310 may include, for example, display 9330 and display 9340. A button press may, for example, result in information associated with the function of a button to be displayed in display 9330. Display 9340 may then be utilized, for example, to show the string of subsequent button presses. For example, a user may utilize button 9311 to indicate that a PIC is desired to be entered. Indicia 9351 may be displayed to denote that the function associated with button 9311 has been activated. Indicia 9341 and 9342 may be utilized to show the number of button entries needed to complete the function. As buttons are entered such indicia may change to indicia representative of the pressed buttons. After all buttons associated with a function have been entered, the function of the buttons of portion 2610 may be utilized to determine the next function (instead of, for example, information entry). Other functions may include, for example, amount desired for cash-back, amount desired for ATM withdrawal, tip percentage, amount to be charged to debit, and the amount to be charged to credit.

Persons skilled in the art will appreciate that a card may, for example, allow a user to enter in how much of a transaction is desired to be paid with a particular account. For example, a user may divide payment between a checking account and a savings account. Alternatively, for example, a user may divide payment between two or more credit accounts. Alternatively still, for example, a user may divide payment between a credit account and a debit account.

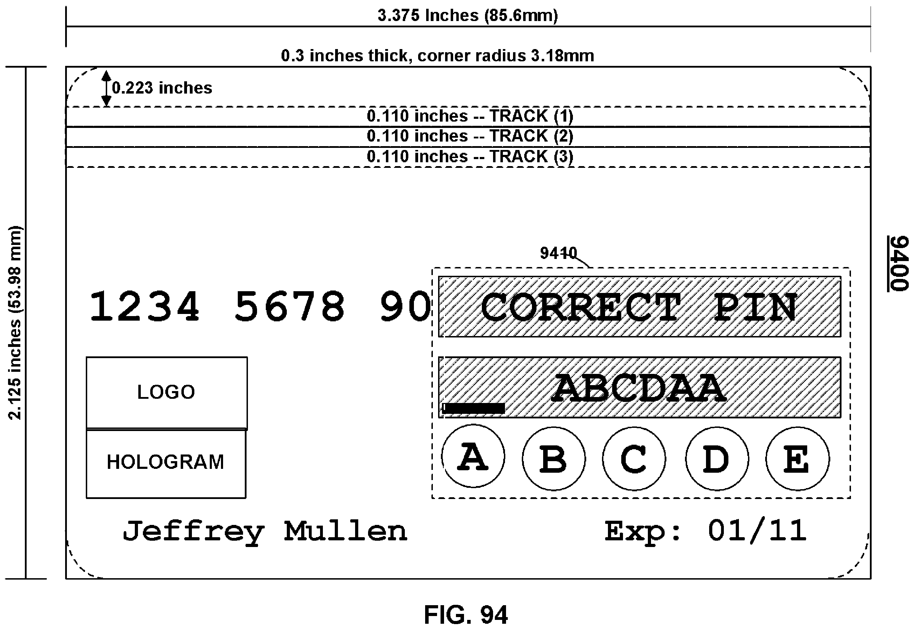

FIG 94 shows card 9400 that may include portion 9400. Portion 9400 may include a display that, for example, provides information indicative of whether an entered PIN/PIC was correct or incorrect.

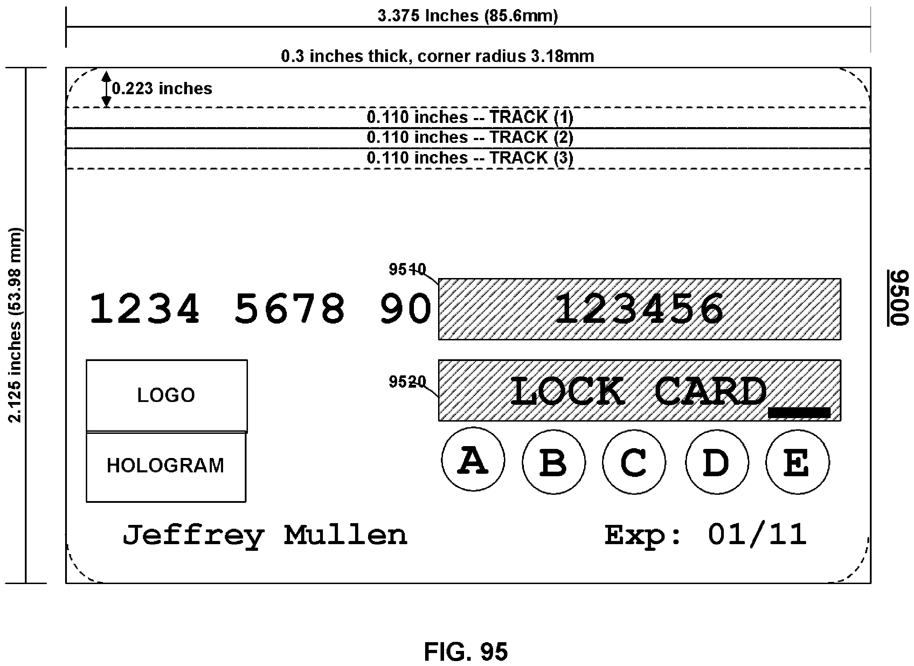

FIG. 95 shows card 9500 that may include, for example, display 9510 and display 9520. Display 9510 may, for example, be utilized to display a portion of a card number. Display 9520 may be utilized, for example, to display the various states of operation of a card. For example, display 9520 may be utilized to ask the user whether the user desires to lock a card.

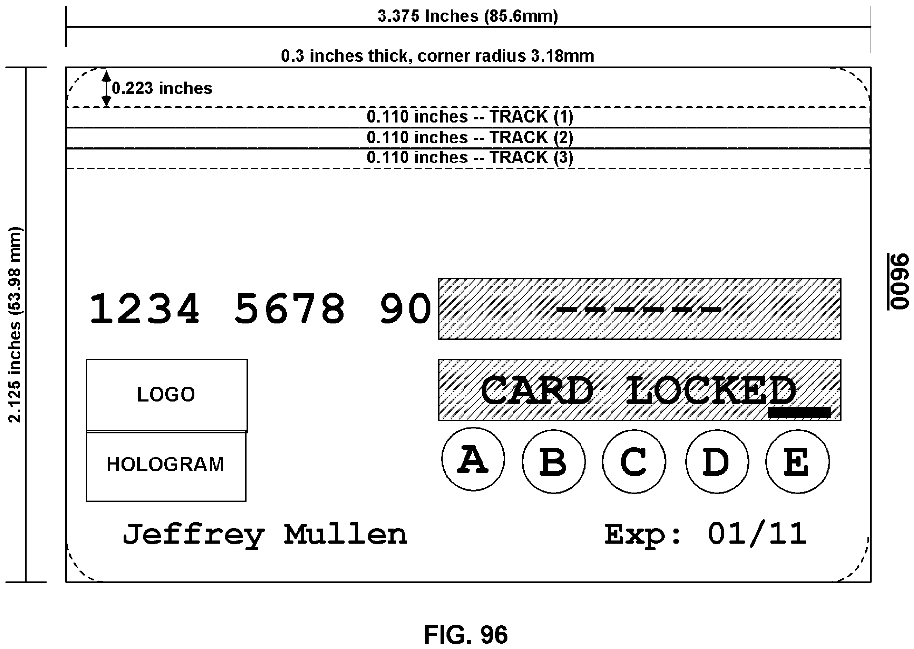

FIG. 96 shows card 9600 that may include, for example, display 9610 and display 9620. Display 9610 may, for example, be utilized to display a portion of a card number until a card is locked. Display 9620 may be utilized to indicate to a user that a card is locked and requires an appropriate PIC/PIN to unlock the card. A user may lock his/her card at anytime by providing the appropriate manual input to a card. A card may lock after a period of time of non-activity by a user. A card may lock and require an administrative unlock code if, for example, a particular number of consecutive incorrect PICs/PINs were entered into a card. Such an administrative unlock code may be obtained, for example, via a phone call to the appropriate card issuer or via a visit to a website of the appropriate card issuer.

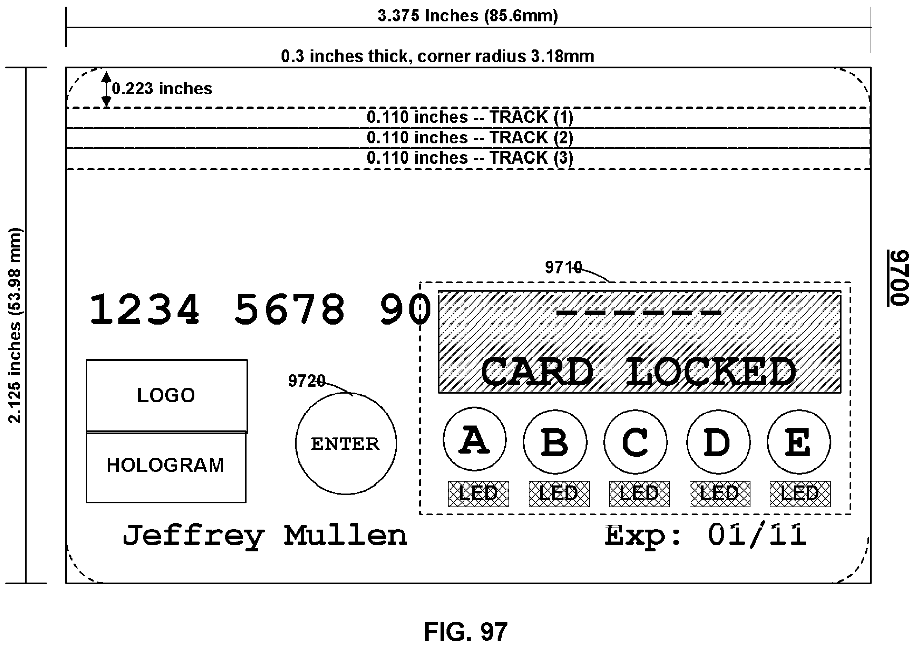

FIG. 97 shows card 9700 that may include portion 9710. Portion 9710 may include a multiple line display. Card 9700 may also include button 9720. Button 9720 may be, for example, larger than buttons in portion 9710. Such a button may be mechanical and/or capacitive. For example, button 9720 may be mechanical while other buttons located on card 9700 are capacitive. Accordingly, for example, more force may be needed to activate button 9720 than other buttons. Accordingly, for example, a user may press button 9720 in order to, for example, bring a processor of card 9700 out of hibernation such that a user may enter in a PIC/PIN. Accordingly, for example, card 9700 may automatically expect the entry of a PIN/PIC after activation of button 9720.

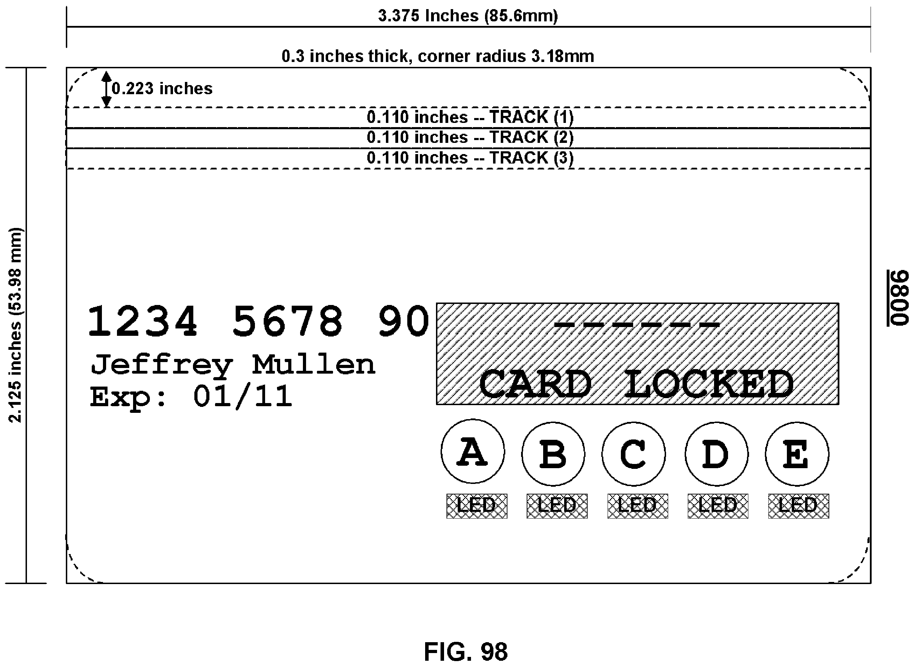

FIG. 98 shows card 9800 that may include, for example, a multiple line display where one of the lines of the display may display a portion of a payment card number.

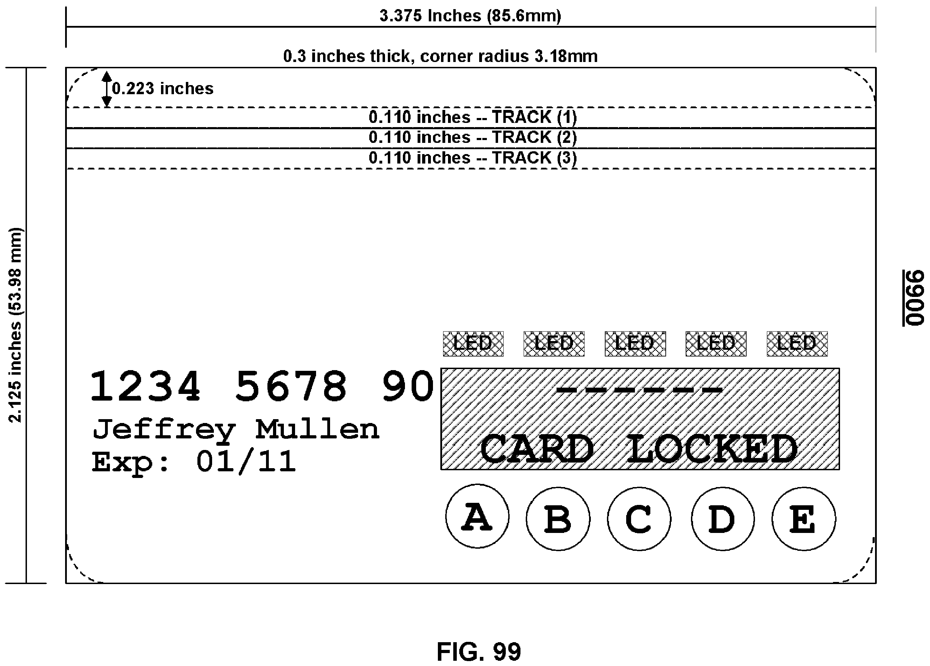

FIG. 99 shows card 900 that may include, for example, a row of light sources above a multiple line display. The multiple line display may be located above, for example, a row of buttons.

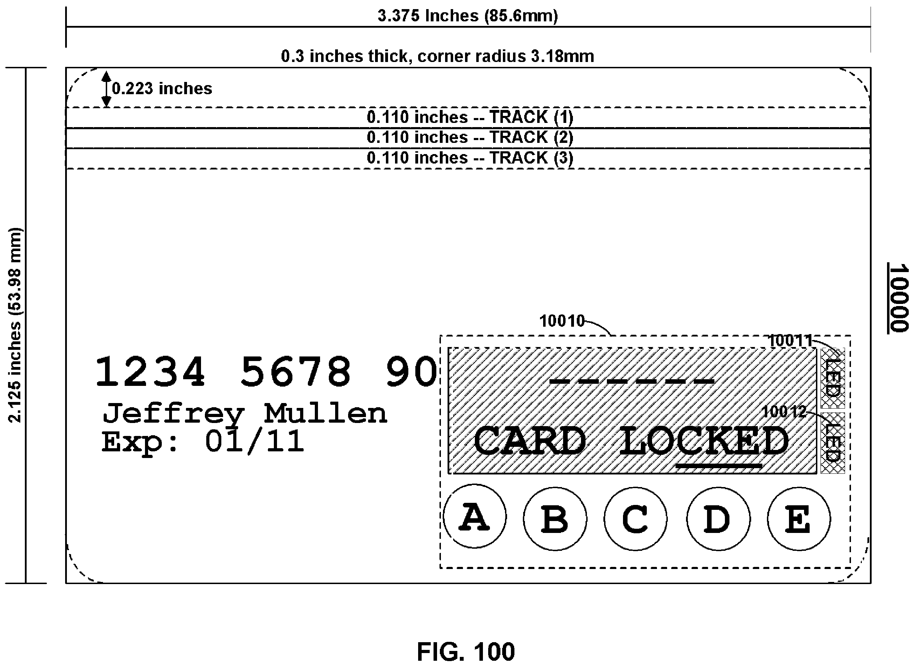

FIG. 100 shows card 10000 that may include, for example, portion 10010 that may include a multiple line display, buttons, and light sources 10011 and 10012. Light sources 10011 and 10012 may be aligned with, for example, a particular line of a multiple line display.

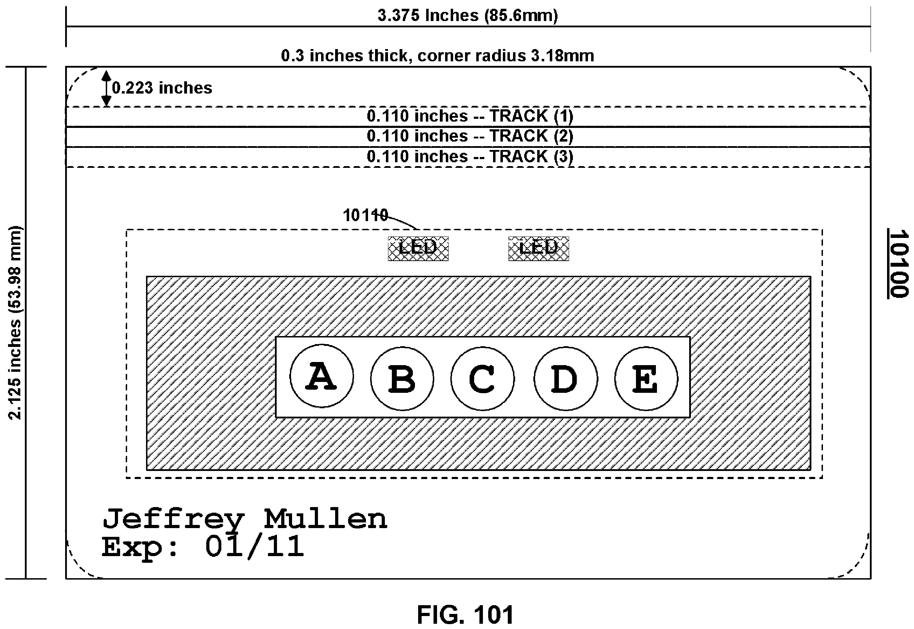

FIG. 101 shows card 10100 that may include, for example, portion 10110. Portion 10110 may include multiple buttons and light sources. Portion 10110 may also include a display with a non-display portion in its center. Buttons may be located in such a non-display portion.

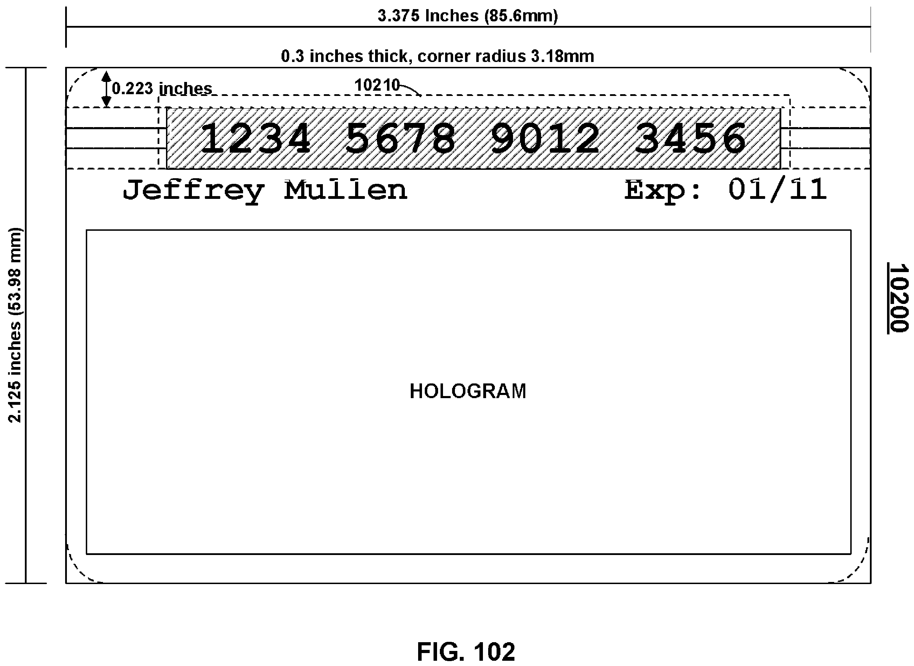

FIG. 102 shows card 10200. Card 10200 may include, for example, portion 10210 that may include a display (as well as buttons and light sources). A display may be provided, for example, over one or more magnetic emulators. For example, a magnetic emulator may be provided on one or more middle layers of a multiple layer PCB. A display may be provided, for example, on a surface layer of a multiple layer PCB or a different layer than the layers defining a magnetic emulator. By providing a display over a magnetic emulator, for example, the area that can be personalized may be increased. For example, a hologram or logo may be provided about more than 2/3rds the surface area of a surface of card 10200.

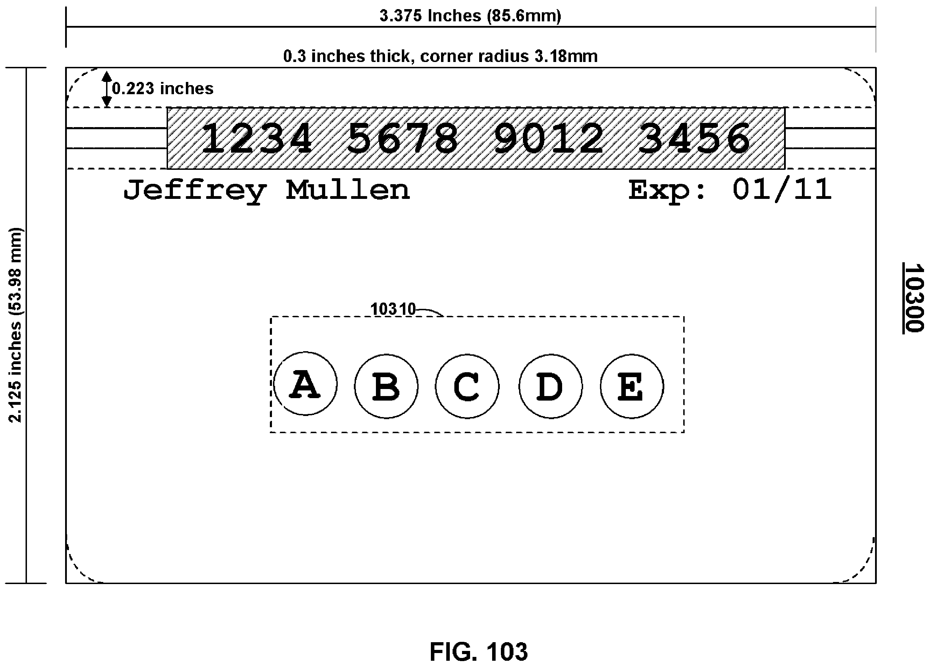

FIG. 103 shows card 10300 that may include portion 10310, which may include any number of buttons. For example, portion 10310 may include five buttons positioned in the center of card 10300 in a single row.

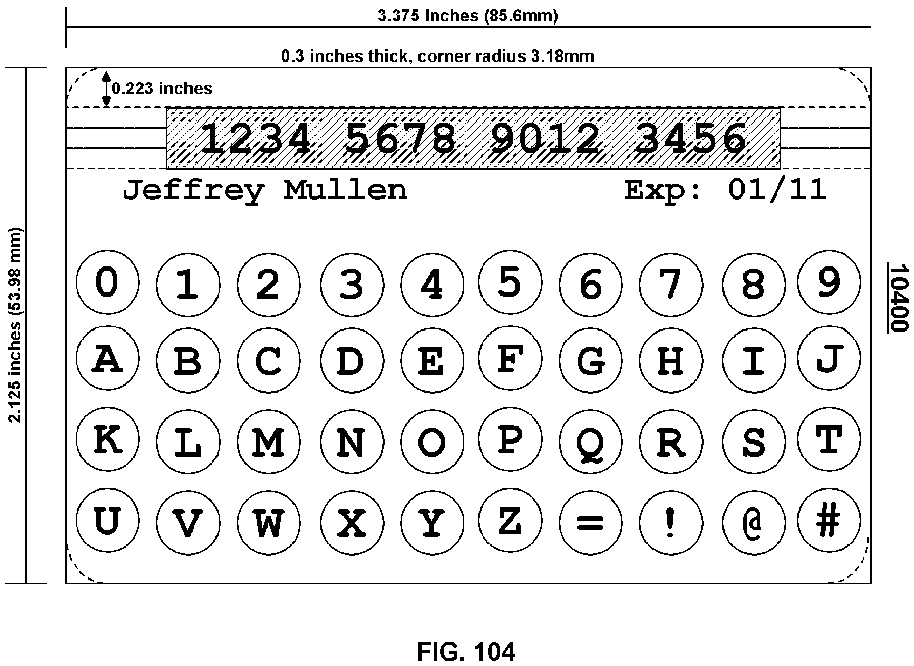

FIG. 104 shows card 10400 that may include over 26 buttons such that, for example, the entire alphabet may be represented by individual buttons. Buttons associated with a particular letter may also, for example, be associated with one or more numbers. Alternatively, for example, additional buttons may be provided for numbers as well as other characters.

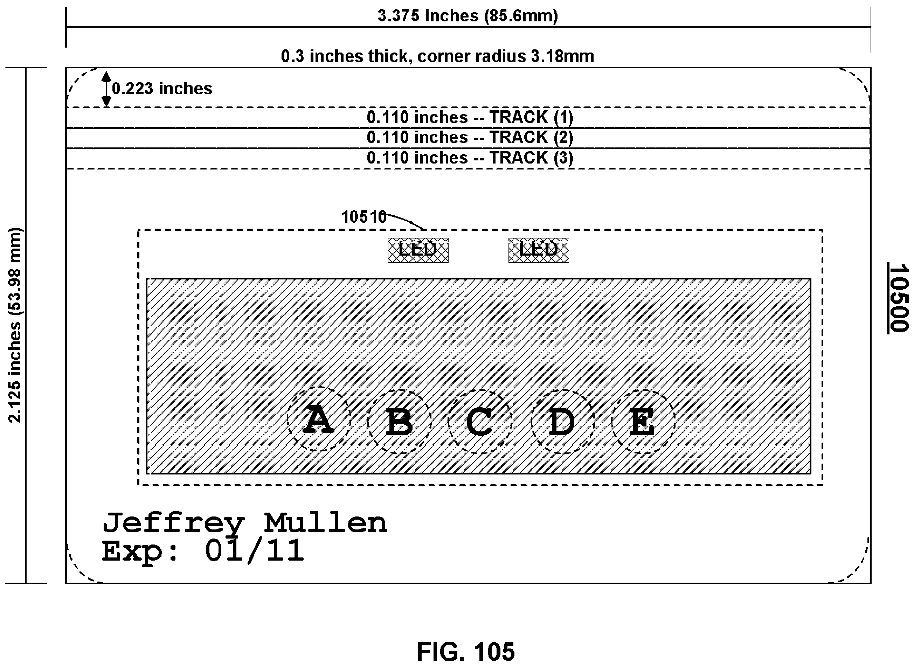

FIG. 105 shows card 10500 that may include portion 10510. Portion 10510 may include any number of buttons, light sources, or displays. Any display provided may, for example, be a touch-sensitive display. Accordingly, for example, such a touch-sensitive display may display indicia representative of areas of touch. Accordingly, each area of touch may be discerned as different manual input by a processor coupled to the touch-sensitive display. A touch-sensitive display may, for example, be provided as an electrochromic display with electrodes that are coupled to a capacitive sensor. Accordingly, for example, the electrodes may cause indicia to appear on the display in the shape of the electrodes, the capacitance of which may also change as a user's finger approaches the proximity of the electrode. A display may, for example, include five capacitive touch areas for receiving manual input.

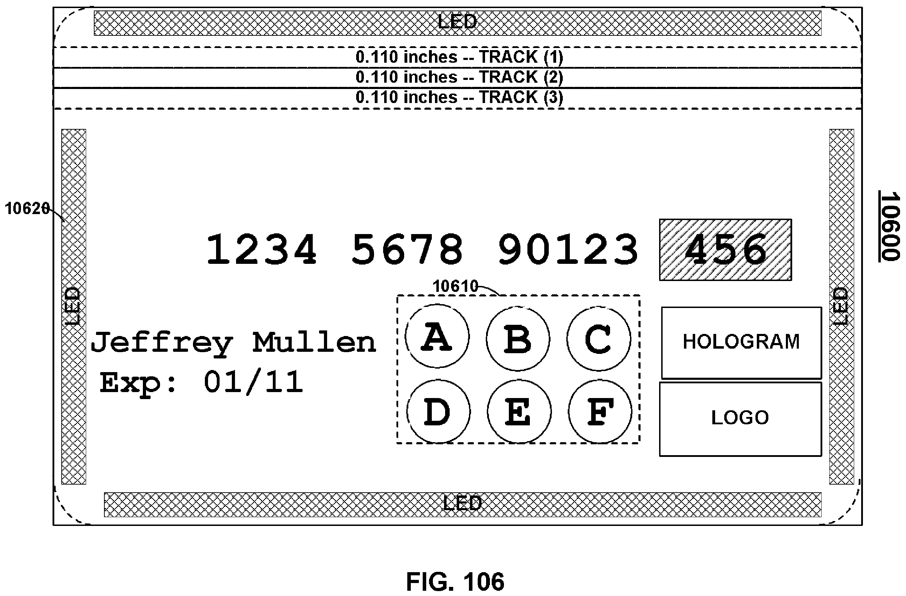

FIG. 106 shows card 10600 that may include, for example, button array 10610. Button array 10610 may include, for example, two rows of centered buttons. Each row may include, for example, three buttons. Light sources may be provided along each edge of a card. For example, light source 10620 may be provided along an edge of a card. A card may be laminated with transparent laminate.

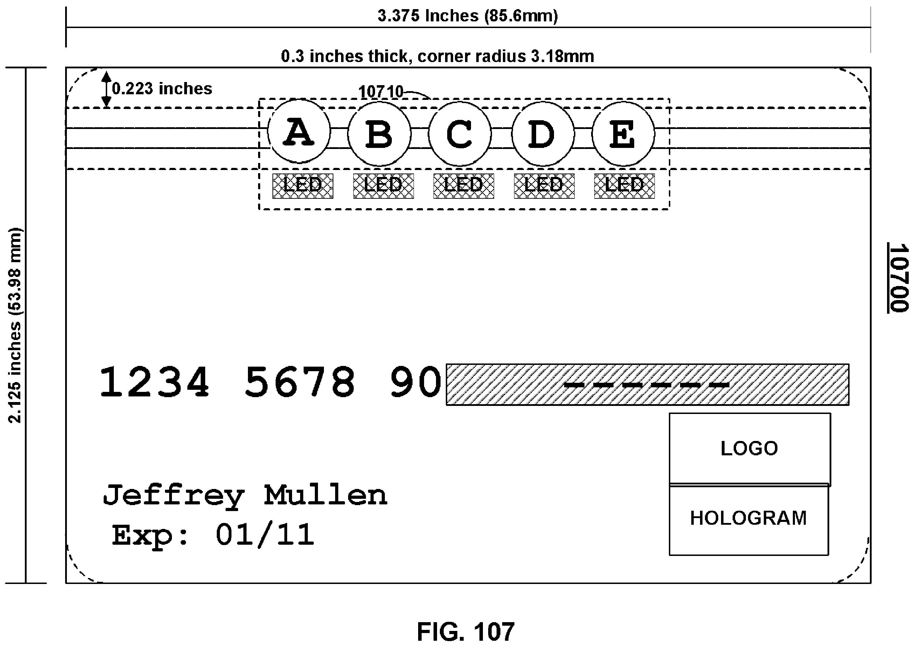

FIG. 107 shows card 10700 that may include, for example, portion 10710. Portion 10710 may include a row of buttons and light sources and may be provided over one or more magnetic emulators.

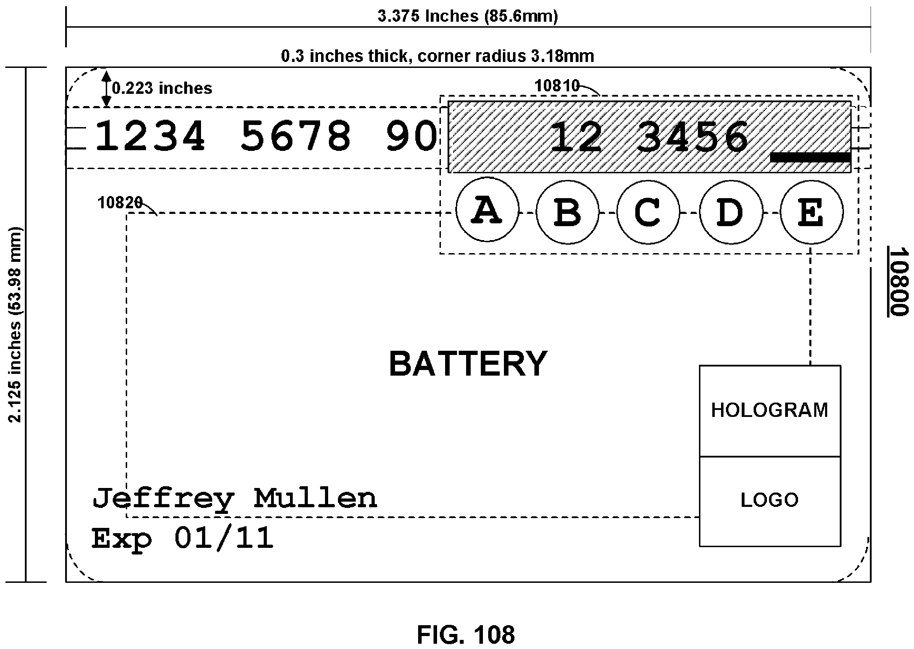

FIG. 108 shows card 10800 that may include portion 10810 at least partially located over one or more magnetic emulators or in line with a magnetic stripe read-head should card 10800 be swiped through a magnetic stripe reader such that card 10800 may transmit data to a magnetic read-head of the magnetic stripe reader through one or more magnetic emulators. Card 10800 may include, for example, battery 10820. Battery 10820 may, for example, span across a surface area of card 10800 that is greater than 2/3rds of the surface area of card 10800.

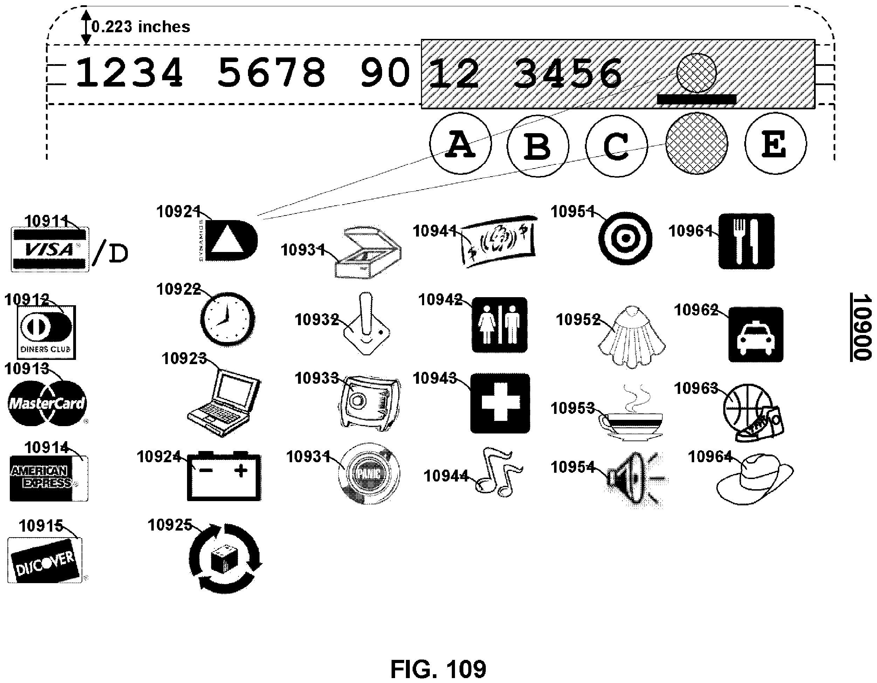

FIG. 109 shows card portion 10900 that may include a display. Various symbols may be provided on a display. Such various symbols may also be, for example, printed on a user interface (e.g., a button). Furthermore, the various symbols may be displayed on a display as part of a capacitive touch area.

Indicia 10911-10915 may be provided to signify different card issuers. Indicia 10911-10915 may take the form of corporate logos. Indicia 10921 may be utilized with a button, for example, to display a card number such as a dynamic card number. Indicia 10921 may take the form of a logo. A button may include both a symbol and one or more letters and/or numbers. Indicia 10922 may be utilized with a button, for example, to display the time. Indicia 10922 may take the form of, for example, a clock. Indicia 10923 may be utilized with a button, for example, a code for an online purchase. Indicia 10923 may take the form of, for example, a computer. Indicia 10924 may be utilized with a button, for example, to display the remaining battery power. Indicia 10924 may take the form of, for example, a battery. Indicia 10925 may be utilized by a microprocessor when a battery is low and the card or battery needs replacement. Indicia 10925 may take the form of a battery and another symbol (e.g., a recycling symbol). Alternatively, for example, indicia 10925 may take the form of an empty battery.

Indicia 10931 may be utilized with a button, for example, in order to signal to the microprocessor that a user desires to upload data. Indicia 10931 may take the form of, for example, a scanner or light reader. Accordingly, for example, a light sensor located on card 10900 may be utilized to receive light-based information. Indicia 10932 may be utilized with a button, for example, to initiate a game on the card. Indicia 10932 may take the form of, for example, a joystick. Indicia 10933 may be utilized with a button, for example, to lock a card (e.g., until an appropriate PIN/PIC is entered). Indicia 10933 may take the form of, for example, a safe. Indicia 10934 may be utilized with a button, for example, to add information in discretionary data indicative of a user warning. Such a warning may take many forms. For example, if a user is held at gunpoint at an ATM to withdraw money, the user may press a panic button on the card and the information may be communicated through the payment interchange to a server that may identify that a panic button was pressed. Accordingly, for example, the authorities (e.g., police) may be contacted and provided with the location of the reader from which the panic information was communicated. Indicia 10934 may take the form of, for example, a panic button.

Indicia 10941 may be utilized with a button, for example, to indicate that a user desires to withdraw cash. Indicia 10941 may take the form of, for example, money. Indicia 10942 may be utilized with a button, for example, to indicate the user of card 10900. For example, a card may be utilized by both a husband and a wife. A button may be utilized to toggle between the user such that the appropriate user's PIN/PIC can be recognized. Accordingly, for example, a card may operate different depending on the user of the card. For example, a husband may have one spending limit and a wife may have a different spending limit. Indicia 10942 may take the form of, for example, a man and a woman. Indicia 10943 may be utilized with a button, for example, to indicate that a user is in need of medical attention due to a medical emergency. Accordingly, for example, a card may communicate such information (e.g., via discretionary data) via a card reader. Indicia 10943 may take the form of, for example, a medical cross. Indicia 10944 may be utilized with a button, for example, to play a tone. Such a tone may be a song or a clip from a song. Such a tone may also be, for example, an identifying audible sound that may be forwarded to a remote server for identity recognition. Indicia 10944 may take the form of, for example, a musical note.

Indicia 10951-10953 may be utilized with a button, for example, to utilize a payment card associated with a particular store. Indicia 10951-10953 may take the form of, for example, a logo of a store such as a target, a shell, or a cup of coffee. Indicia 10954 may be utilized with a button, for example, to change the volume of a speaker of a card. Indicia 10954 may take the form of, for example, a speaker.

Indicia 10961-10964 may be utilized with a button to denote the type of purchase that was made. Such information may be communicated with payment data (e.g., via discretionary data) such that a user's bill may be organized based on the types of purchases a user indicated at the time of purchase. Indicia 10961-10964 take the form of, for example, a knife/form (e.g., for food), a car (e.g., for travel), a basketball (e.g., for entertainment), and a hat (e.g., for clothing).

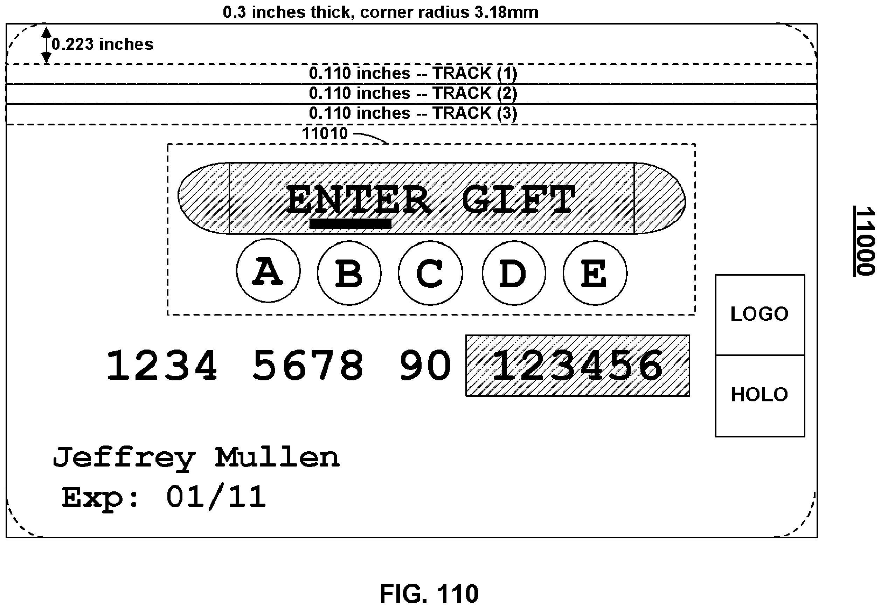

FIG. 110 shows card 11000 that may include portion 11010. Portion 11010 may include a display, for example, with rounded edges. Such a display may be, for example, in the shape of an oval.

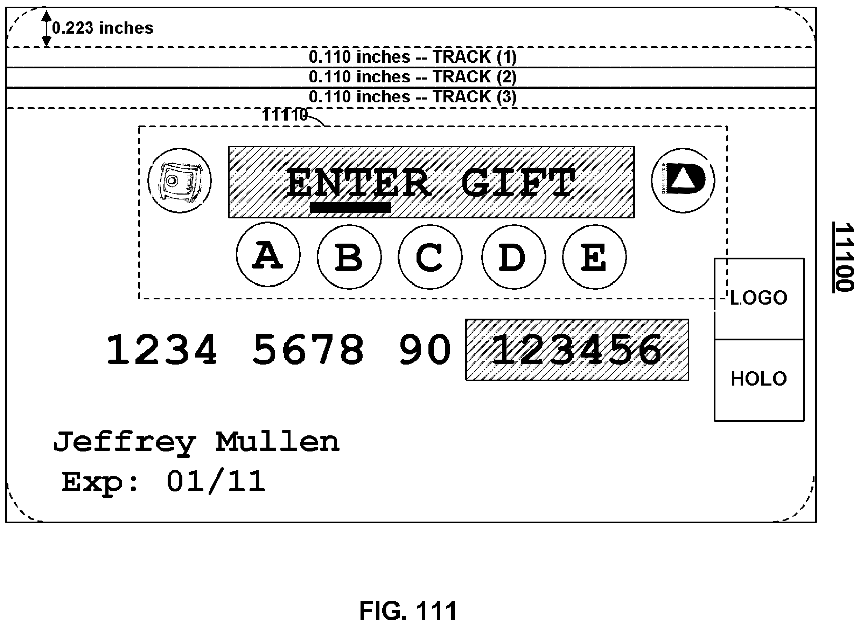

FIG. 111 shows card 11100 that may include portion 11110. Portion 11110 may include a display. Multiple buttons may be located below the display. A button may be located on each side of the display.

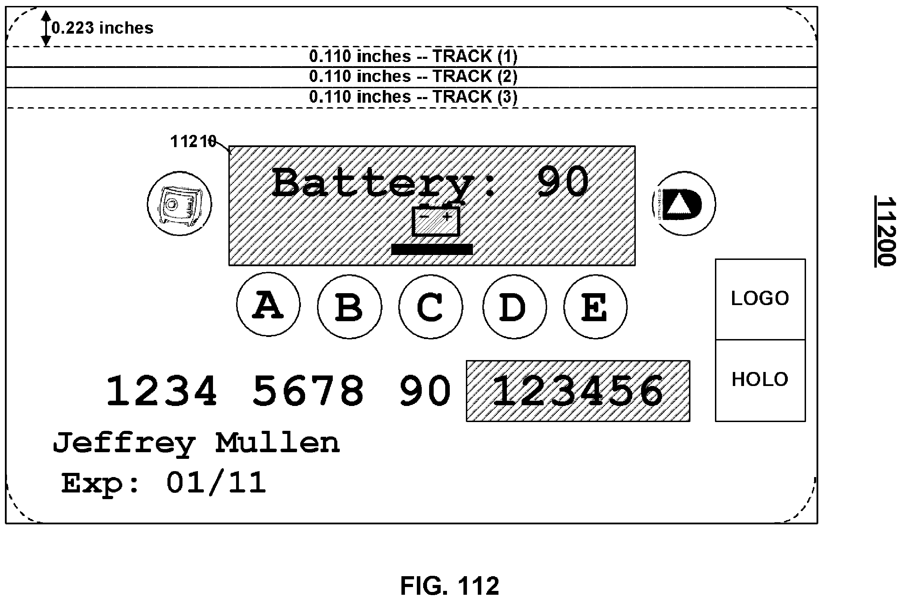

FIG. 112 shows card 11200 that may include display 11210. Display 11210 may include multiple lines of display. Display 11210 may display symbols. Display 11210 may display indicia representative of the activation of a button.

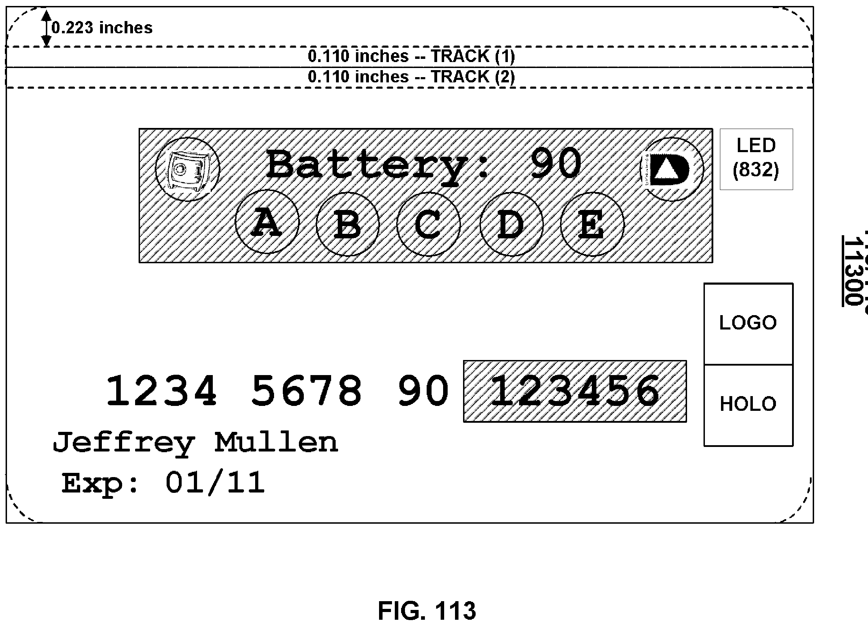

FIG. 113 shows card 11300 that may include a display having touch-sensitive display areas as well as non-touch sensitive display areas.

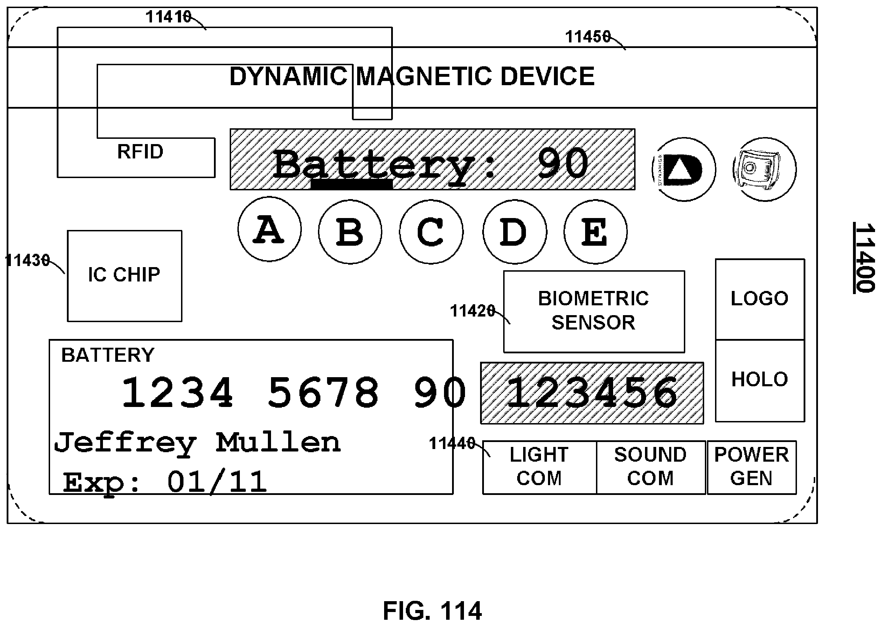

FIG. 114 shows card 11400 that may include any number of components. For example, card 11400 may include RFID antenna 11410. Card 11400 may also include a structure able to communicate dynamic magnetic stripe data (e.g., device 11450). Card 11400 may include IC chip 11430. Card 11400 may include biometric sensor 11420. Biometric sensor 11400 may be, for example, a fingerprint reader (e.g., a partial fingerprint reader) or a retina scanner. Biometric sensor 11400 may be utilized, for example, to unlock a card (e.g., used in lieu of a PIN/PAC). Light transmitter and receiver 11440 may be provided in order to transmit and receive light-based information signals. Card 11400 may include, for example, one or more batteries as well as a processor and memory. A processor may direct an IC chip, any number of magnetic emulators or encoders, and an RFID antenna to communicate information. IC chip 11430 may, alternatively, for example, direct an RFID antenna (e.g., a passive or active RFID antenna) and any number of magnetic emulators or encoder to communicate data. A sound receiver and transmitter may be provided in order for card 11400 to receive information via sound-based signals and send information via sound-based signals.

Card 11400 may also include, for example, power harvesting circuitry. Power harvesting circuitry may be utilized, for example, to repower a battery or provide charge to a capacitor to control a burst of data from an RFID antenna or one or more magnetic emulators and/or encoders. Power harvesting circuitry may include, for example, circuitry for harvesting electromagnetic fields such as fields utilized to power passive RFID antennas. Power harvesting circuitry may also include, for example, a kinetic-to-electrical energy converter that can convert mechanical energy to electrical energy. Alternatively, for example harvesting circuitry may include a thermal-to-electrical energy converter. Alternatively still, for example, harvesting circuitry may include an array of solar cells.

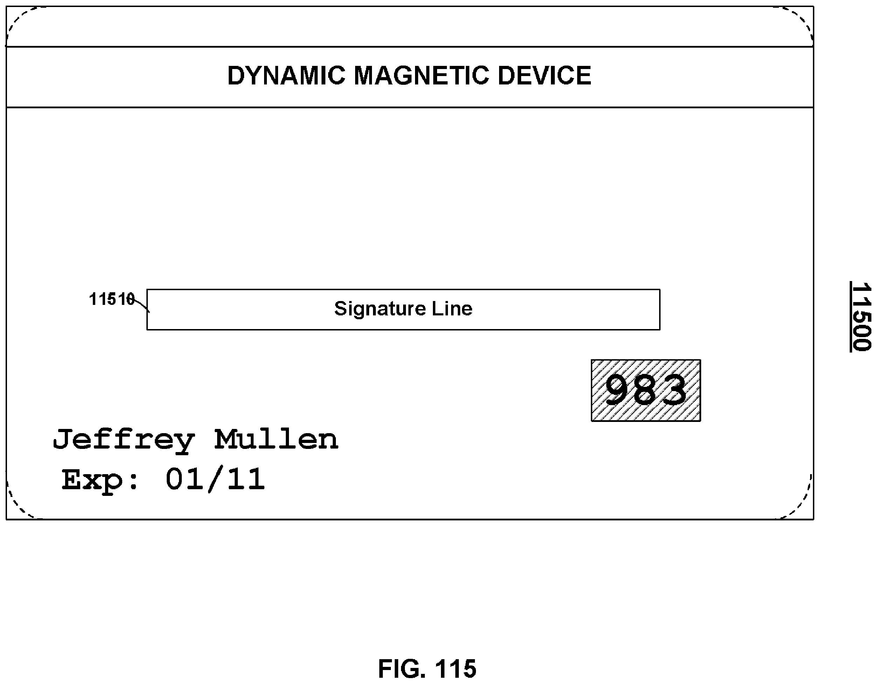

FIG. 115 shows card 11500. Signature-receivable portion 11510 may be provided. A display for a code (e.g., three or four digit code) may also be provided. Portion 11510 and a display for a code may be, for example, provided on the reverse side of any card.

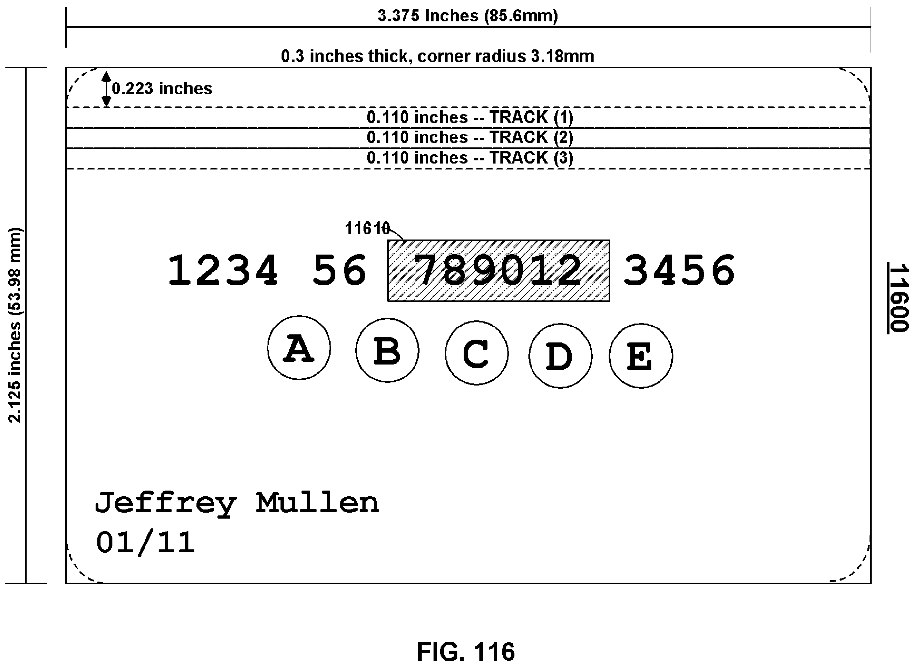

FIG. 116 shows card 11600 that may include a display to display a middle portion of a payment card number.

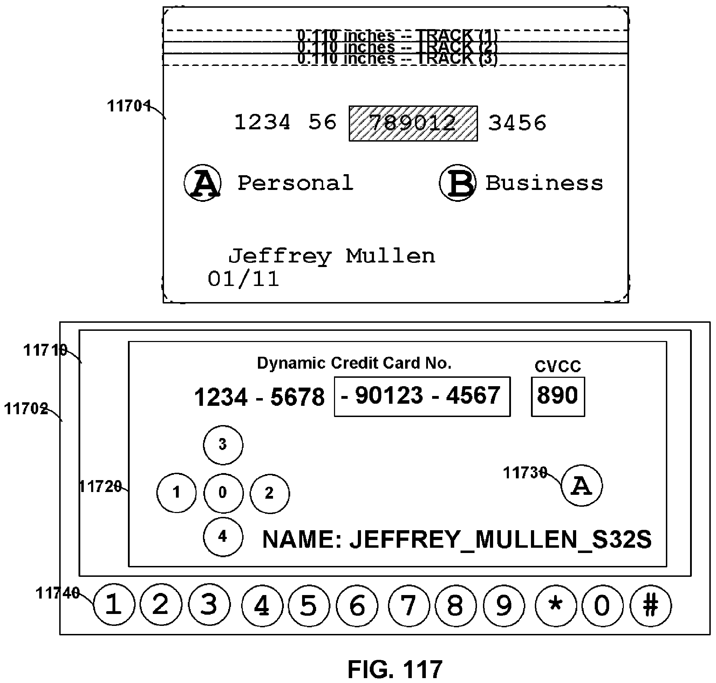

FIG. 117 shows card 11701 that may include a button associated with one account and another button associated with another account.

Persons skilled in the art will appreciate that data may be transferred, such as gift card and/or pre-paid card data, to a card in a variety of ways. For example, a card may be swiped a second time through a magnetic stripe reader that includes a magnetic stripe encoder. A coil on the card may be utilized to receive the information and provide the received information to a processor. In doing so, information may be loaded into the card. Similarly, an IC chip may be utilized to receive data as well as a passive or active RFID. Additionally, one or more microphones may be included to receive audio information that may be representative of data. Accordingly, for example, a user may hold his/her card, or other device, next to a device that is operable to transmit audio via a speaker (e.g., laptop, stationary computer, or mobile telephonic device). The audio information may be discerned by the card and utilized to load information into the card (e.g., a gift card or pre-paid card. An application may also be loaded that enhances the functionality of the card. Such an application may include, for example, a user's medical information such that medical information can be displayed via the card (or other device) during a medical emergency. Accordingly, applications and/or payment cards may be purchased online and a speaker may communicate information to a card. Similarly, the card may include a speaker for transmitting information such that bi-directional communications are established. A light detector may be provided on a card that may receive light pulses indicative of data. Accordingly, for example, a user may hold a card up to a display - such as the screen of a laptop, stationary computer, or mobile phone - and information may be communicated from the display to the card via the light detector. Similarly, a light source may be utilized to communicate information from one device to another. For example, a light source (e.g., LED) may be utilized to communicate information from one card to another. Similarly, a magnetic stripe reader may include a light source. A card may be positioned over the light source such that a light detector of the card is aligned with the light source to receive light. Accordingly, the light of a magnetic stripe reader (or other type of reader) may be utilized to communicate information back to a card. A user may utilize interfaces on the card (e.g., buttons) to initiate a transfer of data from one card to another card or from a device to a card. A variety of types of data may be communicated. For example, money may be communicated from one debit card to another debit card such that payments may occur between the cards. Accordingly, for example, the next time a card is utilized via a reader (e.g., a magnetic stripe reader) information of the transfer may be communicated to a server for processing. Light may be utilized to transfer data from a card to a computer using, for example, a camera (e.g., webcam) on the computer. Sound may be utilized to transfer data from a card to a computer using, for example, a microphone on the computer.

A display may also be utilized as an interface. For example, a display may include a contact and an electronic ink. The electronic ink may change colors in response to, for example, a particular electrical signal being supplied to the contact. A capacitive sensor may be coupled to such a contact, however, such that a user interaction with the contact may be sensed by the capacitive sensor. Accordingly, a card may include a display that can also receive user input. Persons skilled in the art will appreciate that a display may include multiple contacts. For example, a display may include multiple 7-segment (e.g., to display digits) or 11-segment, 14-segment, or 16-segment (e.g., to display alphanumerics) regions where each segment may be coupled to a capacitive sensor.

A biometric sensor may be placed on a card or other device. Such a biometric sensor may be, for example, a fingerprint reader. Accordingly, one or more fingerprints may be stored in the memory of a card and compared to scanned fingerprints. Different fingerprints may activate the card differently (e.g., utilize a different user's payment card info).

Persons skilled in the art will appreciate that a user's payment card number (e.g., credit card or debit card number) does not have to change. A display may hide this payment card number until an appropriate unlocking code is entered into buttons of the card. Similarly, a magnetic emulator may not be provided current until the proper unlocking code is entered - thus keeping magnetic information private and not allowing undesirable readers to read a card. A security code may be displayed on the same or a different display. A button may be provided representative of an online purchase (or a user may utilize buttons to instruct the processor that an online purchase is desirable). For such an online purchase, the credit card number and the security code may be displayed - but the magnetic emulator may not be activated. In doing so, the level of security of the card is increased. Furthermore, for example, a button may be provided representative of in-store purchases (or a user may utilize buttons to instruct the processor that an in-store purchase is desirable). Accordingly, a processor may be signaled that an in-store purchase is desired. A different operation may be associated with different types of purchases (e.g., online or in-store). Accordingly, for example, magnetic emulators may be activated for an in-store environment - but not the displays. Accordingly, for example, a restaurant cashier may not be able to read the credit card number from the card, but may still be able to swipe the card. If a reader is down or a cashier requires reading particular information (e.g., a security code or credit card number information) then controls may be utilized to communicate this information. A record of the types of transactions may be stored and may be communicated in discretionary fields of data within a transmitted data track. Such record information may be utilized, for example, to further increase security and/or introduce a variety of additional functionality.

Different types of cards may be provided on a card. For example, a security ID number and a credit card number may both be provided on the same card. A button may be utilized to allow a user to provide instruction to a processor such that the processor can display (e.g., visually and/or magnetically) the desired information. For example, a user may determine to use one of a variety of payment accounts (e.g., credit and/or debit) for a purchase. An entire payment number (e.g., credit or debit) may be changed and/or hidden visually and/or magnetically. A portion of a payment card number (e.g., credit or debit) may be changed and/or hidden visually and/or magnetically.

Persons skilled in the art will appreciate that a display on the card may display a credit card number that does not change with time (or transaction or button press). Additionally, for example, a magnetic emulator (or multiple magnetic emulators) may magnetically communicate financial data that does not change with time. Such a card may reduce, for example, the effects of physical card theft and card cloning.

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipt may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

FIG. 117 shows personal electronic device 11702 which may be, for example, a portable telephonic device, portable media player, or any type of electronic device. Persons skilled in the art will appreciate that the functionality of a card may be provided on a personal device and displayed through a graphical user interface. Personal electronic device 2000 may include, for example, user inputs 11740 and display 11710. Virtual card 11720 may be displayed on display 11720. Display 11720 may be a touch-sensitive display such that, for example, virtual button 5030 may be provided on virtual card 11720. Persons skilled in the art will appreciate that cards may be provided as virtual cards and a user may interact with such virtual cards in order to provide a variety of functions. Personal electronic device 11702 may communicate to a card reader such as, for example, an RFID reader.

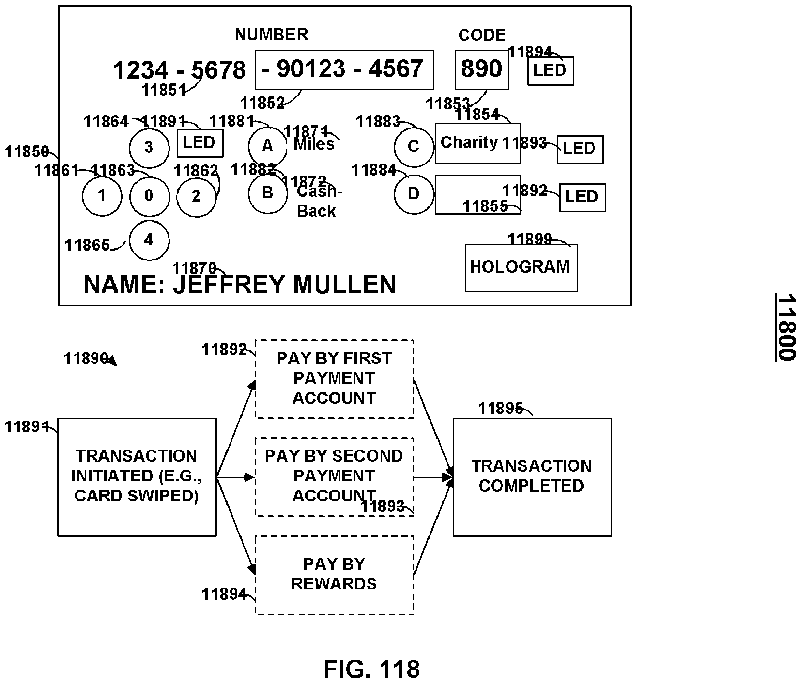

FIG. 118 shows card 11850 that includes buttons 11851-11864, light sources 11891-11894, displays 11852-11853, permanent information 11851 and 11870, buttons 11881-11884, and hologram 11899.

Buttons may be provided on a card that may be associated with, for example, different types of loyalty-based benefits. In this manner, a user may select the different type of reward the user desires to obtain for each purchase. In doing so, the user can select the type of reward that the user may found most useful for a particular period of time. For example, suppose a user is about to take an airplane trip and is only a few miles away from being awarded a free ticket. In such an instance, a user may find more utility in obtaining airline miles. Furthermore, allowing for multiple rewards on a card may, for example, provide a user with the ability to reduce the number of cards in his/her wallet while reducing card issuance costs at the card issuer.

Button 11881 may be provided and may be associated with permanently printed information 11871. Accordingly, for example, button 11881 may be utilized by a user to instruct a processor that a particular reward associated with permanently printed information 11871 is desired. Accordingly, the processor may communicate information through a reader communications component indicative of the user's desire to utilize a particular type of reward (e.g., RFID antenna, magnetic encoder, magnetic emulator, or IC chip). The processor may also display, for example, a code that may be entered online that is indicative of the type of reward a user desires. For example, a security code may be displayed on a display and a digit of this code may be associated to a particular reward. Alternatively, for example, a particular code may be representative of a particular reward. Alternatively still, for example, a particular code may take on a particular format such that an algorithm (e.g., a decryption algorithm) can both validate a code for security but also determine the type of reward that is desired.

For example, a security code may be generated based on one of a particular number (e.g., 4) of time-based encryption algorithms. Accordingly, a user may select a particular type of reward (or other feature) and an algorithm associated to that feature may be utilized to encrypt a private number. The code may be communicated through an online payment portion to a remote server. In turn, this remote server may decrypt the security code with all of the time-based encryption algorithms that could have been utilized. The algorithm that results in a particular private number may be determined to have been the algorithm selected by a user. In turn, the remote server may be able to determine the type of reward the user desires. In doing so, for example, information may be communicated through an online security code. Persons skilled in the art will appreciate that encryption algorithms may be chosen that, for example, do not result in the same encrypted number for any particular period of time.

Button 11882 may be associated with permanently printed information 11872. Button 11882 may be associated with, for example, a type of reward. For example, button 11882 may be associated with a cash-back reward. Accordingly, for example, a user may receive cash-back from a purchase if a cash-back reward is chosen. The cash-back may take many forms. For example, the user may receive a discount at the actual point-of-sale. Alternatively, the user's payment card account may be debited with the cash-back amount periodically (e.g., monthly or annually) or after a particular amount of cash-back has been accumulated (e.g., $100).

Button 11883 may be associated with display 11854. Display 11854 may show a type of reward (e.g., charity). Similarly, button 11883 may be associated with display 11855. Display 11855 may show a type of reward (e.g., reward points).

FIG. 118 also shows flow chart 11890. Step 11891 may be provided in flow chart 11890, in which a transaction may be initiated. A transaction may be initiated in a variety of ways. For example, a card may be swiped through a payment card terminal. A user may select one of a variety of payment types. For example, a user may select to pay by a particular type of payment account (e.g., a credit account). Accordingly, step 11892 may be initiated, in which a remote server determines from the received payment information that a user desired to pay with this particular payment account. Similarly, a user may select to pay via a different type of payment account (e.g., a debit account). Accordingly, step 11893 may be initiated, in which a remote server determines from the received payment information that a user desired to pay with this different payment account. A user may also select to pay via a particular type of rewards. Accordingly, step 11894 may be initiated, in which a remote server determines from the received payment information that a user desired to pay with this particular reward account. A user may pay for an item using one of multiple reward accounts. Step 11895 may initiate to complete a transaction.

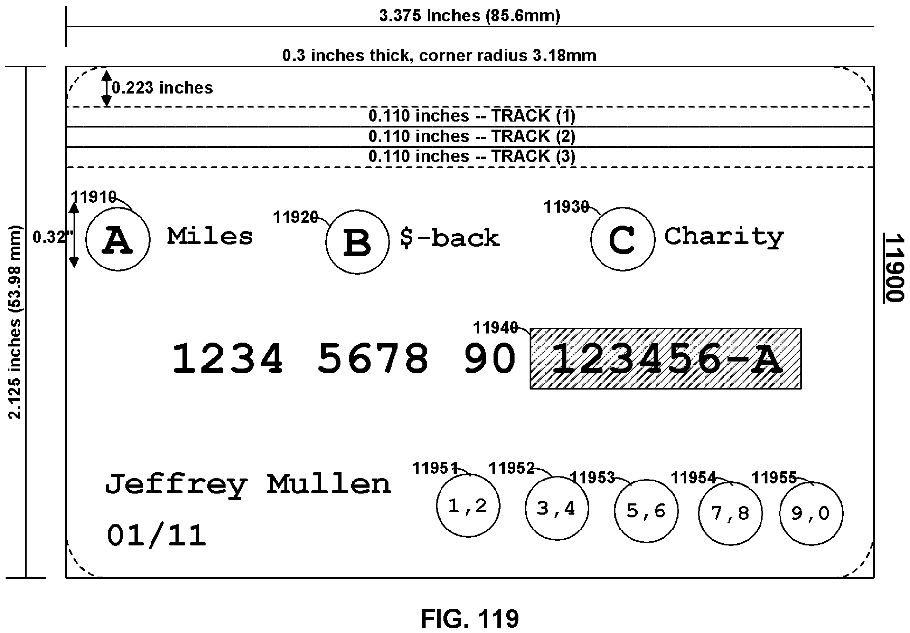

FIG. 119 shows card 11900 that may include buttons 11910, 11920, and 11930. A user may utilize buttons 11910, 11920, and 11930 to select a type of reward. For example, a user may select to purchase an item and have the purchase price utilized as part of a charity reward. In such a reward, for example, a particular percentage of the purchase price may be donated to charity. The user may be provided with an online or paper tax statement at the end of the year indicating how much money the user earned in rewards and provided to the charity.

Persons skilled in the art will appreciate that a user may be provided with a personalized webpage indicating to the user the types of rewards that were chosen for each transaction as well as summary information for the total amount of rewards earned during a period (e.g., a billing period) for each type of reward. A user may also be provided with the ability to transfer earned rewards to different types of rewards. Particular exchange rates may be provided for transferring rewards as well as transfer costs.

Display 11940 may be provided on card 11900. Display 11940 may display, for example, a portion of an account number as well as information indicative of any user selection.

Buttons 11951-11955 may be provided, for example, such that a user may enter in various types of codes (e.g., unlocking codes, gift codes, discount codes, programming codes for changing the types of rewards on a card). Persons skilled in the art will appreciate that buttons 11951-11955 may also be utilized, for example, to select a reward. Accordingly, a user may enter in his personal unlocking code and then, when prompted, pick a particular button for a particular type of reward.

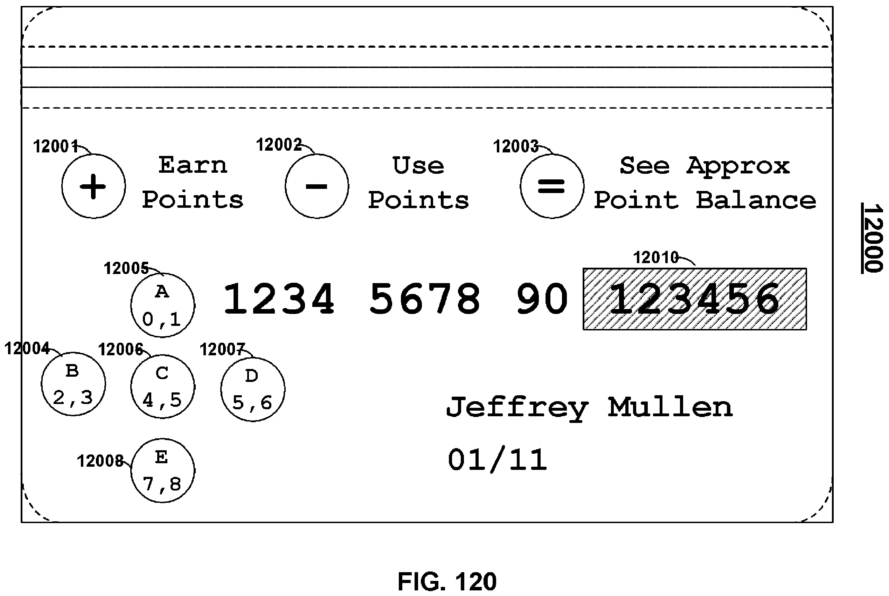

FIG. 120 shows card 12000 that may include buttons 12004-12008 for entering in various types of codes as well as making various types of user selections. Button 12001 may be provided to allow a user, for example, to select that the user desires to earn points for a purchase. Button 12002 may be provided for example, to allow a user to select that the user desires to spend points for a purchase. Persons skilled in the art will appreciate that a server may determine how many points are needed for a purchase, deduct that amount from a user's total, and, if applicable, deduct an amount of money from a particular payment account if the rewards points are exhausted with a remaining amount due.

Button 12003 may be utilized by a user to see the user's point balance on display 12010. Persons skilled in the art will appreciate that a card may receive balance information in a variety of ways. For example, a card may receive information via a magnetic emulator, IC chip, or an active RFID antenna.

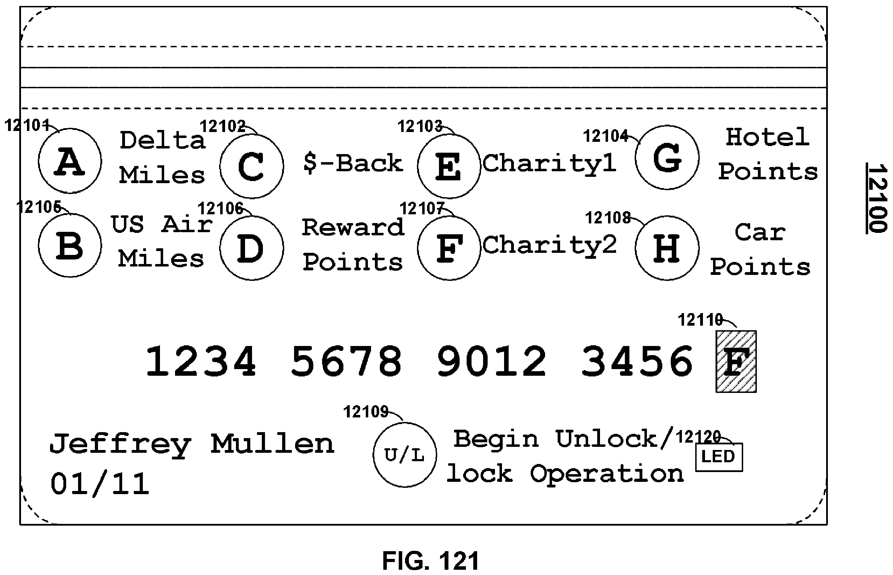

FIG. 121 shows card 12100 that may include buttons 12101-12108 for selecting various types of rewards. Display 12110 may be utilized to indicate to a user the type of reward that was selected. For example, display 12110 may be a one-character display. Button 12109 may be utilized to indicate that a user desires to lock or unlock a card. A user may then utilize buttons 12101-12108 to enter in an unlocking code. Person skilled in the art will appreciate that a user may lock a card simply by pressing, for example, button 12109. Light source 12120 may be utilized, for example, to indicate to a user whether the card is locked or unlocked. For example, light source 12120 may turn a particular color (e.g., GREEN) upon card 12100 receiving an appropriate unlocking code. Light source may then, for example, periodically blink that color while a card is unlocked and the card's reader communicating components are activated for communication by a processor. Light source 12120 may turn a different color (e.g., RED or ORANGE) if, for example, an incorrect code is entered. Light source 12120 may similarly flash the same color as an incorrect unlocking code when the card locks (e.g., automatically or as a result of user input). Persons skilled in the art will appreciate that a card provided in the United States may be programmed to include GREEN as an unlocking color and RED as a locking color and a card provided in a different country (e.g., a European country) may provide RED as an unlocking color and GREEN as a locking color.

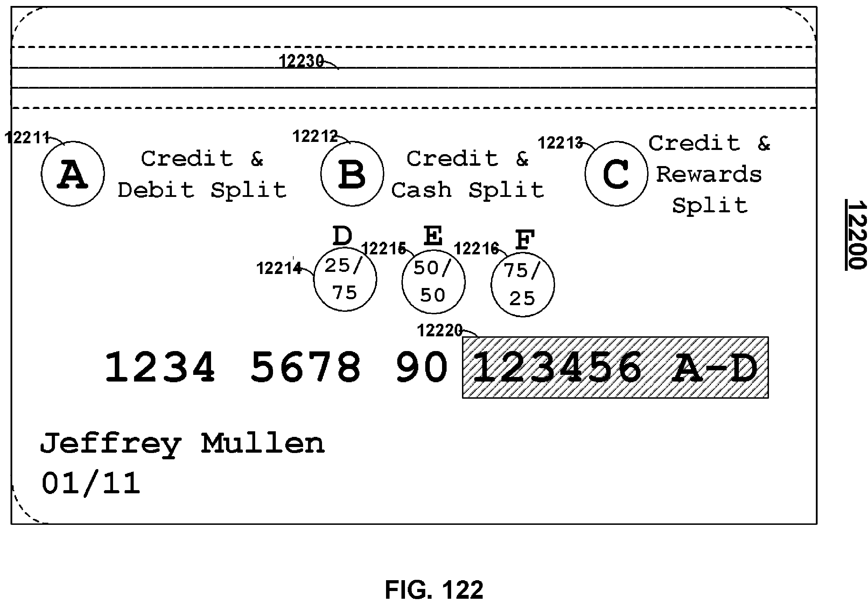

FIG. 122 shows card 12200 that may include buttons 12211-12213. Button 12211 may be utilized to indicate to a processor that a user desires to split a bill between two different payment accounts. Button 12212 may be utilized to indicate to a processor that the user desires to split a bill between a credit and cash payment. Button 12213 may be utilized to indicate to a processor that the user desires to split a bill between credit and rewards points. Buttons 12214-12216 may be utilized, for example, to indicate the proportions of the split. Display 12220 may be utilized to display a portion of a payment account number as well as indicate the types of splits and the proportions of the splits that were selected by a user.

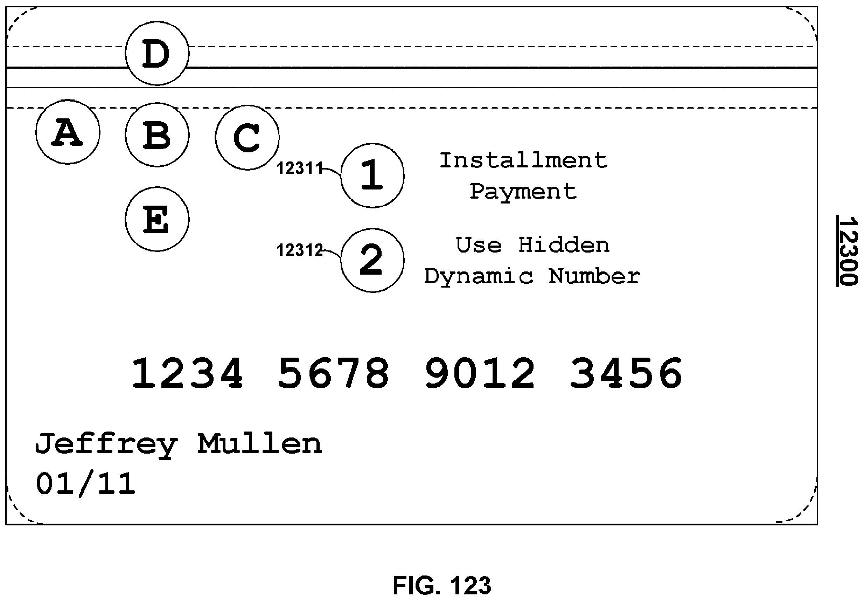

FIG. 123 shows card 12300 that may include buttons 12311 and 12312. Persons skilled in the art will appreciate that a card may include any components (e.g., buttons, display, and light source) on either side of a card. Similarly, a magnetic emulator or encoder may be provided on either side of a card or, alternatively, in substantially the middle of a card. Any card may include a display or may not include a display.

Button 12311 may be utilized to indicate a particular type of payment parameters - such as an installment payment. Additional buttons may be utilized to allow a user to select one of a variety of different installment payments. For example, numerical buttons may be included such that a user may enter in the amount of installments that are desired for a particular period of time. A button may be provided, for example, that allows a user to pay at his/her bonus time (e.g., a bonus paid by the user's employer). Button 1112 may be provided, for example, to indicate that a dynamic account number is desired to be used but that the number is desired to remain visually hidden. Accordingly, a dynamic account number may be provided via a magnetic emulator or stripe (e.g., or via an IC chip or RFID antenna).

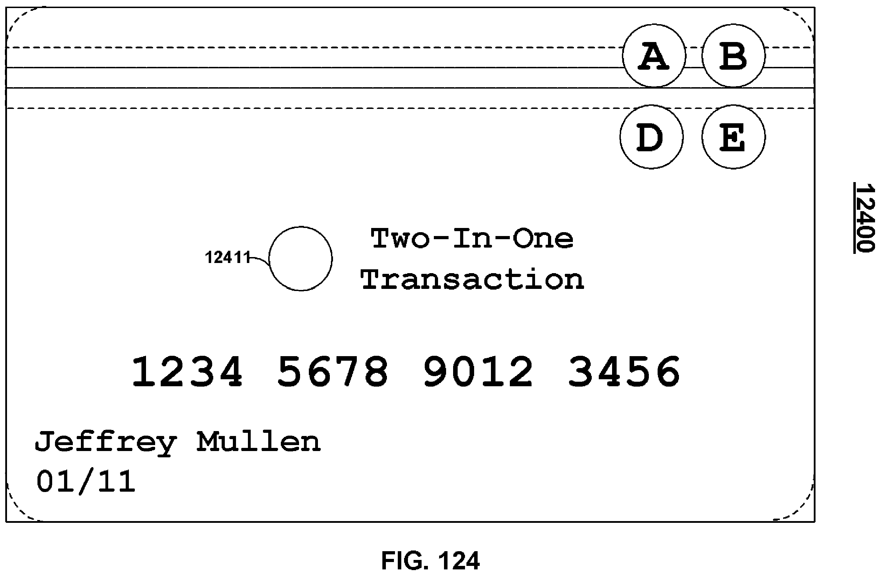

FIG. 124 shows card 12400 and may include button 12411. Button 12411 may be utilized, for example, to provide two bundles of payment information serially to a reader via a magnetic emulator or magnetic encoder. Person skilled in the art will appreciate that a magnetic encoder communicating data serially may communicate data faster than a static magnetic stripe (e.g., over 2, 10, 20, or 30 times as fast) to a magnetic stripe reader. Accordingly, additional data may be communicated. Readers that are coupled, for example, to computers (e.g., cash-registers) with programming operable to receive serial bundles of payment information may receive multiple bundles of payment information with a single swipe. For example, if a user desires a split order between credit and debit, two bundles of payment information (one for credit and one for debit) may be communicated. Information may be included in the discretionary fields of both bundles of payment information indicative of the user's desire for a split order.

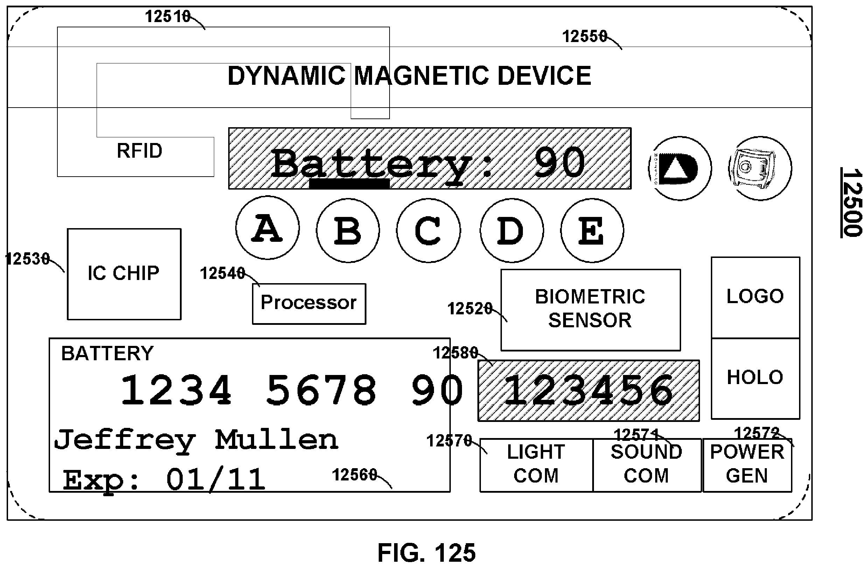

FIG. 125 shows card 12500. Card 12500 may include any number of card reader communication devices such as a magnetic stripe encoder, magnetic emulator, RFID antenna, or IC chip. Card 12500 may also include a magnetic stripe. Card 12500 may include, for example, a serial magnetic emulator for track 1 data, a different serial magnetic emulator for track 2 data, and a static magnetic stripe for track 3 data.

Processor 12540 may control the data transmitted and received from any RFID antenna, IC chip, or magnetic emulator or encoder. Additionally, for example, an IC chip located on the card may be utilized to transmit and receive information to other communications components (e.g., an RFID). In this manner, a processor may, for example, drive information through a magnetic emulator while an IC chip may drive information through an RFID antenna. One or more memories may be provided to store payment information that is utilized by, for example, a card reader communications device.

Any number of batteries 12560 may be included on card 12500. Such batteries may be lithium polymer batteries and may, for example, be coupled together in a series configuration. Such batteries may be stacked or may lie adjacent to one another in card 12550. Batteries may be recharged from power received via a reader (e.g., via a power signal supplied to an IC chip or an electromagnetic field supplied to an RFID antenna).

One or more displays may be provided on card 12550. For example, display 12580 may be provided. Such a display may take many forms. For example, display 12580 may be an electrochromic display or an LCD display. Various forms of user interfaces, such as mechanical or capacitive buttons, may be provided on card 12550.

IC chip 12530 may be provided on card 12550 such that IC chip 12530 may transmit information to, and receive information by, an IC chip reader. Similarly, card 12550 may include RFID antenna 12510 which may, in turn, transmit information to, and receive information by, an RFID reader.

Card 12550 may include dynamic magnetic device 12550 that may communicate different information to a magnetic stripe reader. For example, dynamic magnetic device 12550 may be provided as a magnetic emulator or a magnetic stripe encoder. Additionally, for example, a magnetic stripe reader having a magnetic encoder may communicate information to, for example, a magnetic stripe emulator.

Biometric sensor 12520 may be provided. A biometric sensor may take many forms such as, for example, a fingerprint reader. A fingerprint reader may capture and compare partial fingerprints or full-fingerprints. Images may be initially stored during a setup procedure in which a user is prompted (e.g., via a display) to scan in his/her fingerprint. Such images may be retrieved (e.g., from a memory) and compared to fingerprints as new fingerprints are scanned to confirm a user's identity.

Light communication device 12570 may be included on card 12550 and may, for example, transmit and receive light-based information signals. Sound based communication device 12571 may be included on card 12550 and may, for example, transmit and receive sound-based information signals. Power generator 12572 may be utilized, for example, to harvest power such that a rechargeable battery located on card 12550 may be recharged. For example, such a power generator may harvest kinetic, thermal, solar, or electromagnetic energy and convert this energy to an electrical energy.

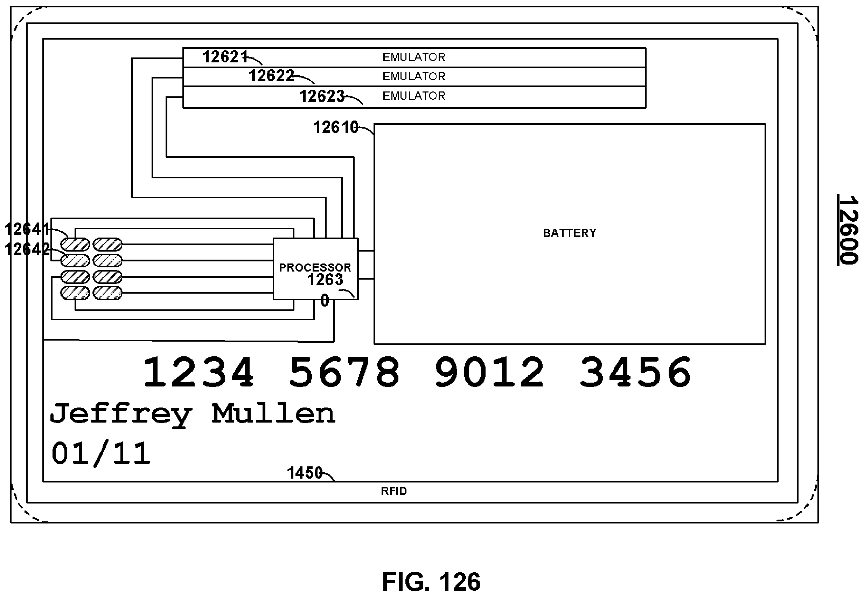

FIG. 126 shows card 12600 that may include single track emulators 12621-12623 that communicate information serially to a magnetic read-head at the direction of processor 12630. Battery 12610 may be included to power processor 12630 and the rest of the circuitry of card 12600. Processor 12630 may also perform the functions of a payment IC chip. Particularly, for example, contacts (such as contacts 12641 and 12642) may be provided that may be able to couple with an IC chip reader. The contacts may route information between the IC chip reader and processor 12630. In doing so, for example, the cost of card 12600 may be reduced. Processor 12630 may be coupled to additional reader communications devices such as, for example, one or more different types of RFID antennas (e.g., RFID antenna 12650).

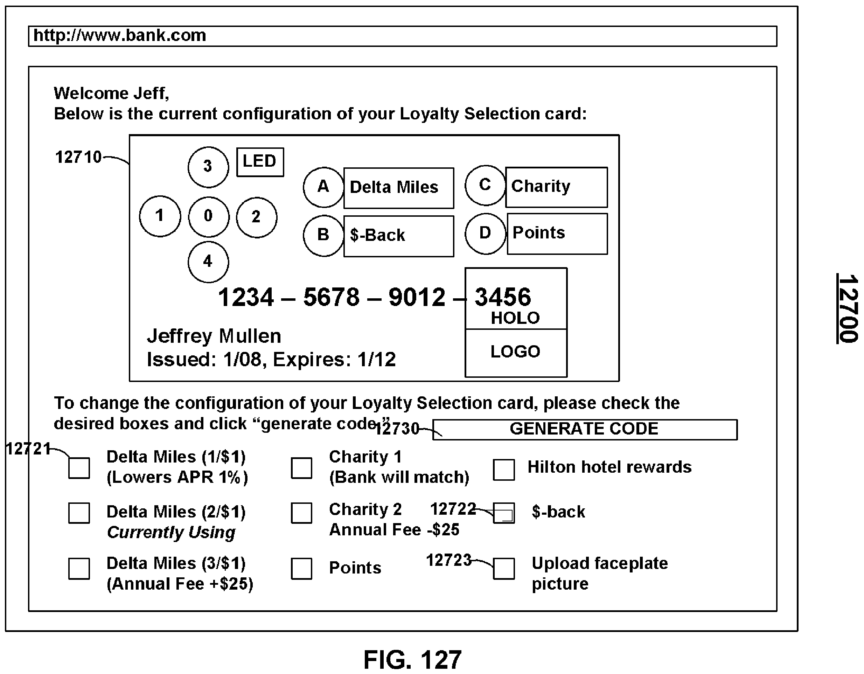

FIG. 127 shows website 12700. A card issuer may provide website 12700 to a user to allow that user to, for example, configure his/her payment card. For example, a user may select a number of features for a card using website 12700, be provided with a preview of a card that incorporates the selected features, and be provided with instructions on how to reconfigure the user's card. Website 12700 may also, for example, provide a user with a preview of the user's card as currently configured. For example, website 12700 may provide card layout 12710.

One type of feature that may be selected and configured by a user is that of a loyalty selection card. A user may select different types of rewards and generate a code, using virtual button 12730, for reconfiguring the user's card. Persons skilled in the art will appreciate that different rewards may change the general terms of a user's payment contract (e.g., credit contract). For example, a particular type of reward may raise or lower a user's APR, annual fee, late fees, or other costs. A user may also be charged a fee for reconfiguring a card (e.g., $1) or may be provided with a pre-determined number of reconfigurations before a cost is applied to a reconfiguration. A user may, for example, select reward 12721 and reward 12722. A user may upload a particular picture via upload 1523.

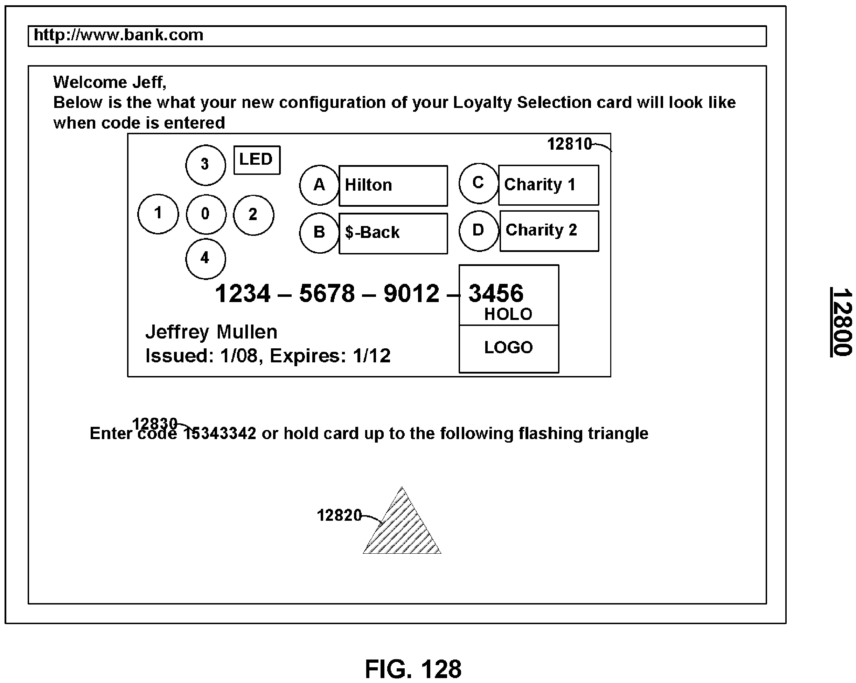

FIG. 128 includes webpage 12800. Persons skilled in the art will appreciate that the graphical user interface utilized by webpage 12800 may be utilized as a graphical user interface for a different medium (e.g., an application running on a mobile telephonic device). Similarly, card features may be provided on a mobile telephonic device either physically or embodied virtually. For example, a physical buttons may be provided as a virtual button on a graphical user interface displayed on a display screen of a mobile telephonic device.

Website 12800 may be initiated, for example, after a user has selected a configuration for his/her card. Such a configuration may associate, for example, different functionalities to different buttons as well as provide additional functionality. A user may be charged a monthly fee for particular features. For example, a user may be charged a periodic fee to introduce a dynamic account number capability to a card. As such, a user may configure a card to include both his/her personal and business accounts (e.g., via two buttons) and may configure a card to also include two types of rewards (e.g., miles and points). Accordingly, a user may select the type of card the user desires in a store as well as the type of rewards. If a dynamic account number capability was purchased (e.g., $5 or more per month or per year), then one or both of the accounts may be provided with time-based or use-based dynamic account numbers as well a time-based or use-based codes. Such codes may be displayed as well as communicated via a reader communications device (e.g., RFID, magnetic emulator or encoder, and IC chip). Displayed codes may be the same as codes communicated through a reader communications device or may be different codes.

Webpage 12800 may show an example of the reconfigured card via preview image 12810. Similarly, webpage 12800 may include either reconfiguration code 12830, virtual light transmission objet 12820, or both. Persons skilled in the art will appreciate that a card may have a table of possible configurations. Each entry of the table may correspond to a reconfiguration code. Accordingly, for example, a processor may reconfigure itself based on previously stored reconfiguration data. Alternatively, for example, the code for reconfiguration may be structured into a code that a user can enter manually or that can be wirelessly communicated to a card via signals (e.g., light-based signals from object 12820). Persons skilled in the art will appreciate that object 12820 may communicate information as light pulses and that a large amount of information may be communicated via object 12820. For example, a user may be directed to hold a card up to object 12820 for a particular period of time (e.g., approximately at least 10 seconds, 30 seconds, or at least 60 seconds or more). A user may be provided with a virtual button on the graphical user interface to initiate data transfer. A user may be provided with a virtual object (e.g., a red light may be replaced with a green light) on webpage 12800 after data is communicated. A card may provide a signal indicative that data was properly received (e.g., an LED may blink a particular color, such as GREEN, or a display may display indicia representative of successful receipt of data. Objects for communicating light pulses may be provided by any number of physical structures (e.g., an LED on a different payment card for card-to-card communications) or any number of virtual objects (e.g., on a television commercial).

Persons skilled in the art will appreciate that a reconfiguration may change the type of information that is communicated through reader communications devices as well as the functionality of any component (e.g., when an LED provides a particular color of light). For example, suppose a reconfiguration provides particular a particular buttons with a miles-based reward and provides a different button with a points-based reward. A magnetic encoder, magnetic emulator, IC chip (e.g., an EMV chip), or an RFID antenna may communicate a chosen reward by sending different data through the communications device.

The card may provide information indicative of the type of reward or functionality selected (e.g., a miles-based reward is desired). The card may provide information indicative of the button that was pressed. In this manner, a remote serve may have knowledge of the reconfiguration, receive the data indicative of the button that was pressed, utilize a look-up table to determine the functionality associated with the selected button for the particular reconfiguration, and utilize this retrieved information or forward this retrieved information to a different server.

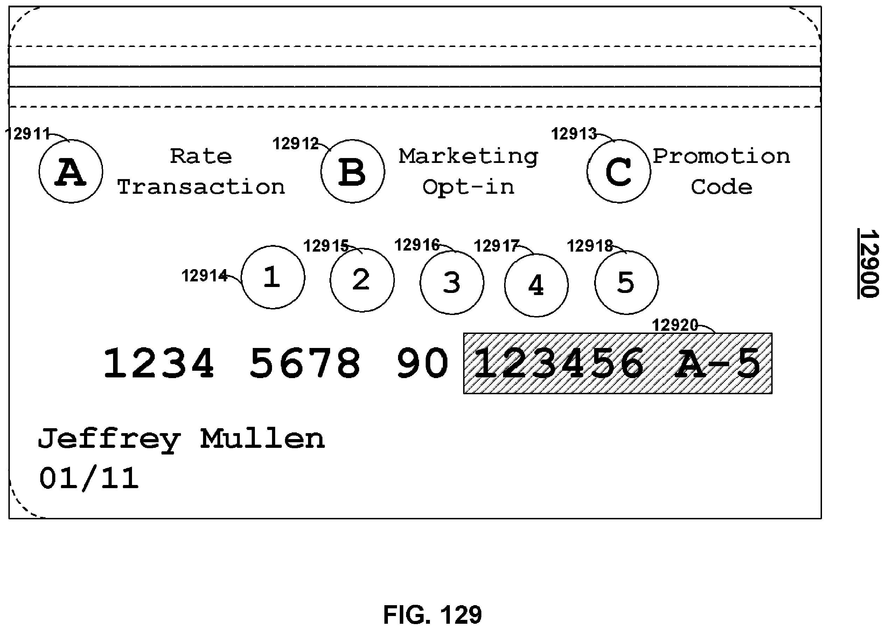

FIG. 129 shows card 12900 that may include buttons 12914-12918 for entering data (e.g., a Personal Identification Number) and display 12920. Additional buttons may be included. For example, button 12911 may be included. A user may utilize button 12911 in order to rate a transaction. Accordingly, a user may press button 12911 and then provide a rating (e.g., a 1-5 rating) using buttons 12914. The rating may be indicative of the waitress, cashier, or purchase experience in general. Different buttons may be provided to rate different attributes of a store or purchase. A user may receive a promotional code on his/her receipt as a result of rating a transaction. Alternatively, for example, a user may be provided with a display (e.g., on a cash-register) for communicating the data wirelessly (e.g., via light-based signals). Alternatively, for example, a user may receive additional rewards for rating a purchase or store attribute (e.g., a waitress, cook, meal, wait lines, customer service). Such additional rewards may include additional points or miles such as a set amount of additional points or miles, points or miles associated with a cost of a purchase, or a multiplier of points or miles (e.g., double, tripe, or quadruple points or miles). A rating may also provide an immediate discount on a purchase (e.g., 20% or less than 20%). A user may view his/her ratings on a website associated with a user's account. A rating may be utilized by a card issuer to further prove that the appropriate user was in possession of a card at the time of a particular purchase. Display 12920 may indicate a selection of a functionality as well as any additional entered data (e.g., button "A" was pressed before button "5" was pressed).

Button 12912 may be included such that a user may select a marketing opt-in 12912. The selection of marketing op-in 12912 may result in personal information being provided to a merchant that, for example, completes an associated transaction. In exchange for the personal information, a merchant may provide the user with a number of benefits - such as rewards, discounts, or promotional codes. Personal information (e.g., telephone number, email address, mailing address, annual income, shopping history, age) may be pre-loaded onto card 12900 and communicated via a reader communications device separately from, or with, a payment account number and associated data for completing a payment purchase. For example, a cashier may be directed by the card to swipe the card twice - once to communicate personal information and a second time to communicate payment information. Alternatively, for example, information may be sent indicative of a user's desire to execute an opt-in marketing functionality. Accordingly, a remote server may recognize this received information, retrieve associated personal data, and forward this personal data to the appropriate location(s) (e.g., a remote server of the merchant). An opt-in marketing functionality may result in, for example, the emailing of a coupon to a user or the inclusion of a coupon on a webpage associated with the user's payment account.

Button 12913 may be included such that a user may select, for example, the entry or use of a promotional code. For example, a user may be provided with a promotional code on a receipt at time of checkout (e.g., as a result of using an opt-in marketing functionality). The promotional code may be entered into the card using buttons (or via wireless light-based signals) and may be communicated via a reader communications device (e.g., a dynamic magnetic stripe communications device comprising two magnetic emulators that simultaneously serially communicate different tracks of data to a magnetic stripe reader). The promotional code may be displayed on a display and the result of the promotional code may be displayed on a display. For example, the result of the promotional code (e.g., "Walmart -10%") may be displayed on a display next to a button. A user may press the associated button to provide the promotional code in the data communicated via a reader communications device. Software on the merchant-side may recognize the code and apply the code to a purchase. Such codes may automatically expire after a period of time, expire after a number of uses (e.g, via button presses, light-based signals acknowledging completion of a purchase, or detection of a data communication to a reader such as a detection of a read-head), or expire after a particular number of new codes are received, and a processor may delete the code from its memory. A display may be a bistable display or a non-bistable display.

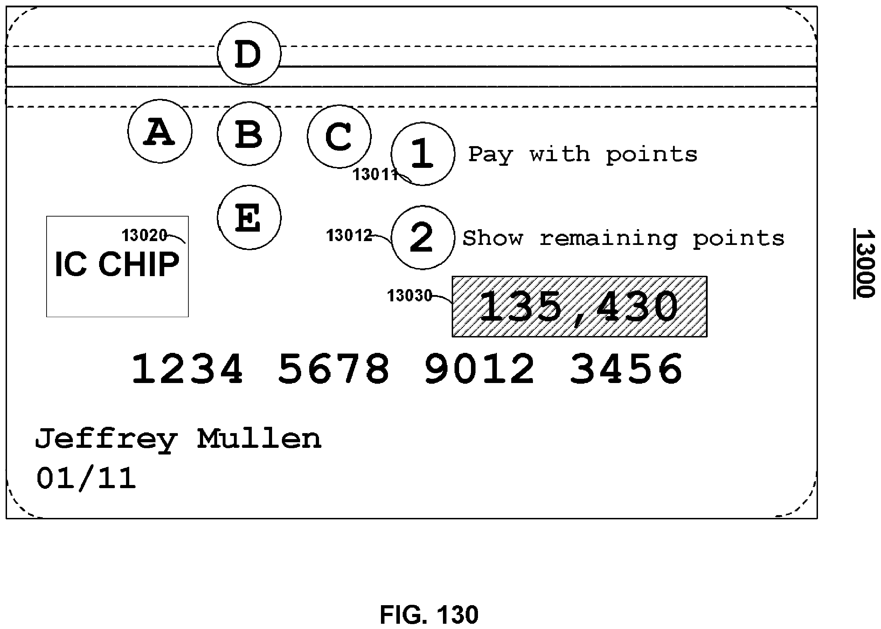

FIG. 130 shows card 13000 that may include IC chip 13020 (e.g., an EMV chip), buttons 13011 and 13012, and display 13030. Button 13011 may be utilized to pay for a purchase with points. Button 13012 may be utilized to show the remaining number of points a user has on display 13030. A card may receive information from a variety of devices such as light sensors, IC chip 13020, an RFID antenna, or a dynamic magnetic device such as a magnetic emulator or a magnetic encoder. Persons skilled in the art will appreciate that IC chip 13020 may have conductive physical contacts on the surface of card 13000. IC chip 13020 may be, for example, approximately 3mm x 5mm and may be located in the proximity of the center of the left side of the front of card 13000.

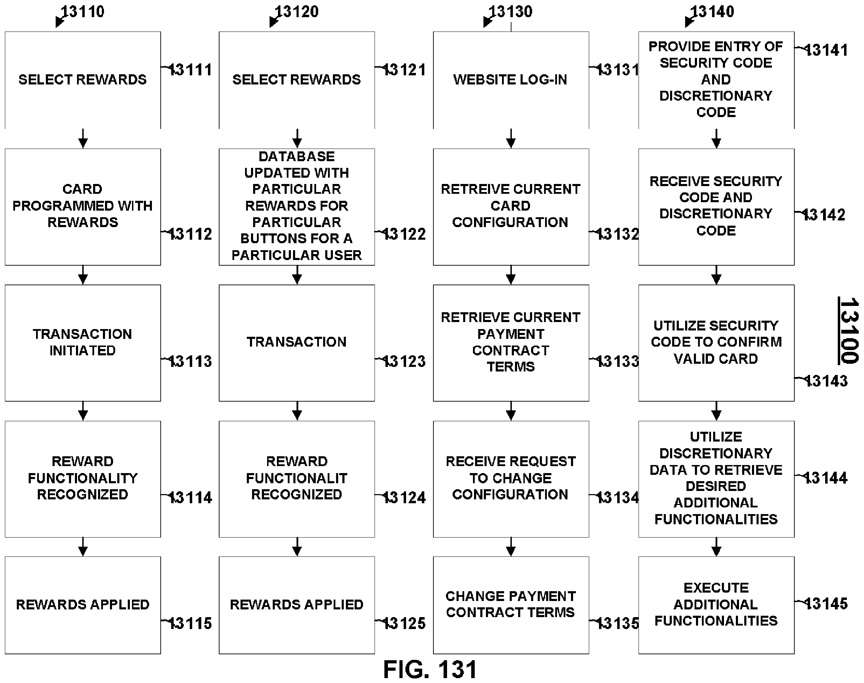

FIG. 131 shows flow charts 13110, 13120, 13130, and 13140. Flow chart 13110 may be initiated with step 13110, in which one or more rewards (or other functionalities) are selected by a user. Such a selection may occur at the time the card is originally requested or when the card is in the passion of the user. Step 13120 may commence, in which the card is programmed with the various rewards (or other functionalities). Such programming may occur via a programming machine at a manufacturing or programming facility or by the user through the use of configuration codes or light-based signals.

Persons skilled in the art will appreciate that a card may be manufactured at a printed circuit board manufacturer. The board may then be send to an assembler. In, the assembler may put various components onto the board (e.g., solder on a display, chip, LEDs and buttons). Particular components may be fabricated at the printed circuit board manufacturer. For example, displays and buttons (e.g., capacitive buttons) may be fabricated as the board is fabricated. Additionally, reader communication devices (e.g., a magnetic emulator or RFID antenna) may be fabricated at the printed circuit board manufacturer. Microprocessors may be, for example, pre-programmed with the appropriate software before being sent to the assembler. Alternatively, software may be programmed into a card at the assembler. A card may be laminated before or after programming. A card may be partially programmed with certain data and later programmed with additional data. The different programming steps may occur at different locations. For example, an assembler may program code into a card so that the card can later receive personalization data from programming at a personalization facility. A personalization facility may also print indicia onto the surfaces of a card, provide holograms and static magnetic stripes onto a card. Lamination may also occur at a personalization facility. A card may be, for example, laminated and personalized except for programming of the card. Such a card may then be programmed at a different facility. Such programming may occur capacitively through the laminant to programming contacts of a microprocessor. The cards may then be mailed in envelopes with personalized letters to users. The card issuer may be notified that the cards were mailed. A user may utilize his/her card in a, POS, ATM, or call an activation telephone number to activate his/her card.

A transaction may be initiated in step 13113. Such an initiation may occur, for example, via interaction with a card reader or interaction with an online payment portal. Step 13114 may initiate when, for example, a remote server recognizes data indicative of a user's desire to earn a particular reward. For in-store purchases, such data may take the form of data communicated through a card reader. For online purchase or other manual entry purchases, the data may be provided in an account number, security code, or another code such as a discretionary data code. An online portal may request multiple codes for purchases. For example, an online portal may request entry of a security code or a discretionary data code. A card may thus display a security code and a discretionary data code. Such codes may, for example, change based on use or based on time. Rewards may be applied to the purchase in step 13125 (e.g., certain rewards may be earned for a particular purchase).

Flow chart 13120 may be provided in which step 13121 is initiated when, for example, a user selects a particular reward. Step 13122 may commence, in which a database may associated the selected rewards (or other functionalities) to particular buttons. A user may select which buttons are associated to which rewards. A user may also select a default application that does not require a button to initiate. For example, a user may select a default type of payment (e.g., a credit card number) as well as a default type of reward (e.g., miles for a particular airline). A database may store the configuration of a card. A transaction may be initiated in step 13123. Step 13124 may receive information of which button(s) were pressed and retrieve the current configuration of the card to determine the functionalities desired by the user. The remote server may apply those functionalities in step 13125.

Flow-chart 13130 may be included, in which a user logs into a website associated with the user's account in step 13131. Persons skilled in the art will appreciate that a payment account may include multiple statements for various types of payment a user includes on one or more payment cards. A payment account may include a combined statement for all types of payment (e.g., personal credit, business credit, and personal debit).

Step 13132 may commence in which the current configuration of a card is retrieved from a server in step 13132. Step 13133 may also retrieve the current payment terms for a user. Step 13134 may be initiated via the reception of a request to change the configuration of a card (or other device). Step 13135 may change the payment contract terms according to the configuration changes. A user may be provided with a confirmation screen to manually confirm the changes.

Flow chart 13140 may be included. Step 13141 may be initiated and a graphical user interface may be provided with text boxes for the entry of data. Such data may include, for example, payment card number, expiration date, address with zip code, name on card, a security code, and a discretionary data code. Step 13142 may commence when a remote server receives the data including the security code and discretionary data code. Such a graphical user interface may be provided on a website as part of an online payment process. Alternatively, for example, such a graphical user interface may be provided on a cash register application, portable telephonic device, or other device. Step 13143 may commence in which the card is validated as authentic using the security code. Step 13144 may commence, in which the discretionary data code is utilized to retrieve a variety of associated additional functionalities. These functionalities are executed in step 13145. Person skilled in the art will appreciate that discretionary data (e.g., such as discretionary data communicated through a reader communications device) may be embedded into a payment card number, address information, name information, as well as a security code. Accordingly, a remote server may remove the discretionary data from this information. Alternatively, for example, the use of the above data may be replaced to be that of a discretionary data use. For example, a user's name may be replaced by a code that does not include information associated with a user's name. A server may accordingly utilize this data to determine the discretionary or other data. For example, a security code may be utilized as discretionary data. Furthermore, such codes may include a parity bit or character. Moreover, for example, a limited number of operable codes may be utilized in order to reduce, for example, the mistaken entry and execution of a non-desired code. A confirmation screen may be provided to confirm correct entry of data. Such a confirmation screen may exist via a webpage or on a display of a card.

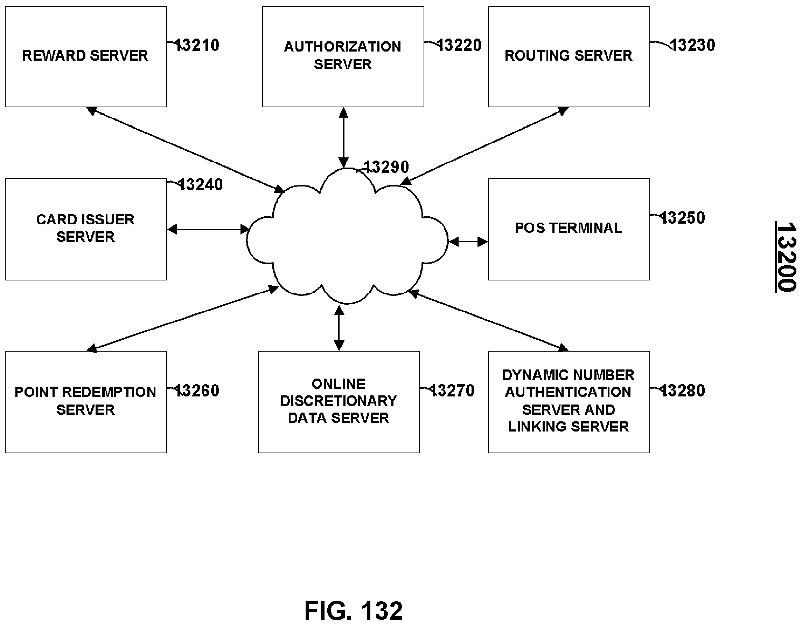

FIG. 132 shows network topology 13200. Network topology 13200 may include communications network 13290. Communications network 13290 may include any number of communications servers, transmitters, and receivers. Communications network 13290 may also include, for example, any type of communication medium or multiple types of communication mediums. Communication mediums may include, for example, wireless or wire-based communications.

Reward server 13210 may be included in topology 13200. Reward server 13210 may perform a variety of functions. For example, reward server 13210 may receive information that was extract from a received information packet from a card reader. Reward server 13210 may determine the type of reward that was requested based on the extracted data. Reward server 13210 may execute a reward-based function such as point/reward management, point redemption/usage. For example, server 13210 may keep a running total of the amount of a particular reward (e.g., miles or points). Server 13210 may provide information regarding the total number of rewards, reward acquisition history, and reward usage history to other facilities. For example, this information may be communicated to a server at a facility managed by a card issuer such that the card issuer can incorporate this data into a webpage customized for a particular user.

Authorization server 13210 may be included in topology 13200. Authorization server 13210 may authorize an event - such as a payment or other transaction. Authorization server 13210 may receive card information (e.g., payment number and zip code) and may authorize this data. Routing server 13230 may be included. Routing server 13230 may route information based on the contents of the information. For example, routing server 13230 may receive payment information from point-of-sale device 13250 and look at the beginning digits of a payment number to determine which facility of a list of facilities to send the payment data. Such a routing server may transmit all of the information that was received or may extract information such that a smaller amount of information is forwarded to other facilities (e.g., extract the portions of the payment number used to route the payment information). The information may be forwarded to a variety of facilities such as, for example, a facility housing another routing server, an authorization sever, or a card issuer's server.

Card issuer server 13240 may be managed by the issuer of a particular payment card. Card issuer server 13240 may, for example, issue a webpage for a user or may perform particular functions such as online access verification (e.g., using an access code communicated via a display). Persons skilled in the art will appreciate that a card issuer may manage, for example, their own routing and authorization servers.

POS terminal 13250 may communicate information received from a card. POS terminal 13250 may take many forms such as, for example, a cash-register having a display and a magnetic stripe reader. POS terminal 13250 may receive information from topology 13200. For example, POS terminal may receive a signal indicative of the result of a transaction authorization (e.g., failed, verified, destroy card, or hold card and customer until a representative or authority arrives). Additional information may be communicated to the POS terminal such as, for example, a point or reward balance and information associated with a functionality of a card. Such information may be printed on a receipt directly or in a code form or communicated to the card (e.g., via light-based signals).

Point redemption server 13260 may be utilized to redeem rewards such as miles or points. Online discretionary data server 13270 may be included and may, for example, receive discretionary data and perform functions based on the received discretionary data. Such discretionary data may be received from multiple tracks of magnetic data where each track includes discretionary data. Such discretionary data may be received from an online purchase application that includes a window for receiving manually input discretionary data.

Dynamic number authentication and linking server 13280 may be utilized to authorize a dynamic payment card number (as well as dynamic codes). Such dynamic card numbers and codes may be based on use or on time. Server 13280 may also keep track of merchants that have utilized a number such that those merchants can utilize the number again at a future time (e.g., for period billing and one-click shopping).

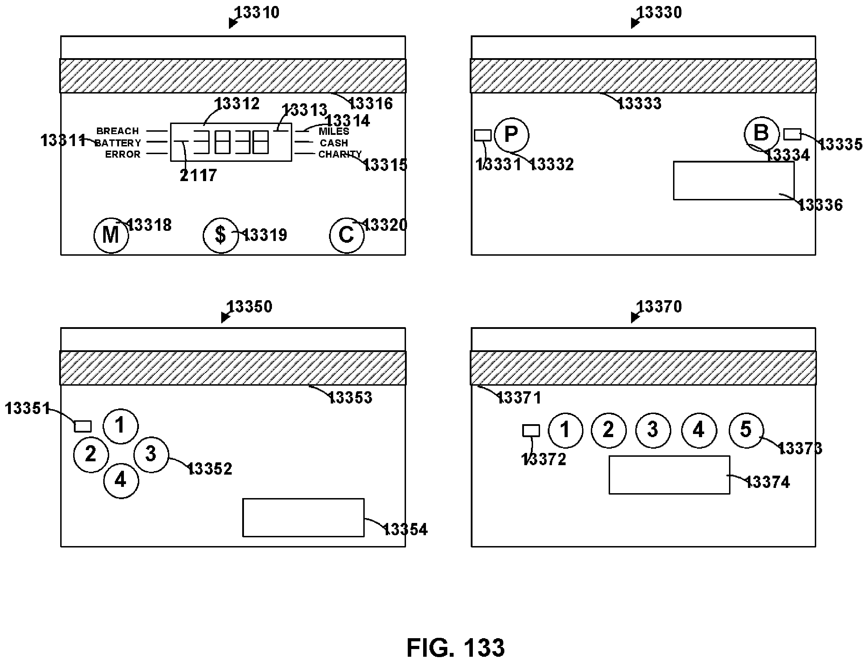

FIG. 133 shows cards 13310, 13330, 13350, and 13370. Card 13310 may be included. Dynamic magnetic communications device 13314 may be provided on the front, back, or middle of a card. Dynamic magnetic communications device 13314 may be configured to provide electromagnetic signals, operable to be read by a magnetic stripe reader, to any one or both sides of card 13310. Display 13312 may be provided on the front or back of card 13310. Display 13322 may display discretionary data for use with online transactions. Data displayed on display 13310 may be entered into a security code input text box of an online purchase application or a discretionary data text box of an online purchase application. Card 13310 may include additional displays for a separate code (e.g., a security code) or for displaying other information such as a dynamic payment number.

Persons skilled in the art will appreciate that display 13312 may be a six-digit seven segment display. Accordingly, display 13312 may display 13312 may display four digits and may have fourteen segments to display. Such segments can be utilized to display information other than numbers. For example, segment 13313 may be utilized to indicate that a particular functionality is activated. Such a functionality may be associated with permanently written data 13314. For example, a user may activate button 13318 and segment 13313 may be displayed. Persons skilled in the art will appreciate that multiple segments on a digit display may be utilized at any given time. For example, a user may be provided with the ability to split the rewards earned by a purchase between two different types of rewards. For example, a user may press button 13318 to turn segment 13313 ON and press button 13318 again to turn segment 13314 OFF (and the associated functionality). Accordingly, a user may press button 13319 and an associated segment may be displayed or a user may press button 13320 and an associated segment may be displayed. A user may thus press buttons 13318 and 13320 and rewards earned may be split (e.g., 50/50 between miles and charity. A user may select all of the buttons and may evenly split rewards earned between those types. A user may be provided with the ability to select the distribution of rewards for every purchase (e.g., via additional buttons). Segments may also be utilized to display status information. For example, segment 13317 may be displayed to indicate that a battery is low or that a battery is not low. Permanent information 13311 may be printed in the proximity of segment 13317 to aide a user in understanding the functionality associated with segment 13317.

Card 13330 may be provided with, for example, display 13336, light source (e.g., LED) 13331, light source 13335, dynamic magnetic communications device 13333, button 13332 and button 13334. Display 13336 may display a partial payment number or a full payment number. Additional data may be displayed. For example, display 13336 may display a dynamic payment number and a static security code. Such a static security code may, for example, be constructed such that each number is printed as a single segment such that the number cannot be changed. Alternatively, the security code may be changed (e.g., based on time or based on use). A user may utilize button 13332 to display a particular payment number while utilizing button 13334 to display a different payment number. Such selected information may be communicated via magnetic communications device 13333 (along with other discretionary data). Discretionary data may also be displayed via a display for online use. Light source 13331 may be utilized to indicate that button 13332 has been activated. Light source 13334 may be utilized to indicate that button 13335 has been activated. Persons skilled in the art will appreciate that cards may show payment numbers (e.g., credit and debit card numbers) or other types of data. Such other types of data may include, for example, serial numbers for authorizing brokerage trades, video gaming numbers for accessing access to particular video games or video game characters, gambling numbers for different gambling accounts, or identification numbers for different loyalty programs (e.g., a grocery store chain's discount card and an electronic store's reward and discount card). Card 13330 may be pre-programmed with various numbers that are associated with particular buttons. Card 13330 may include buttons such that a user can reconfigure a card with different numbers either through manual input via buttons or wireless signals (e.g., light-based signals).

Card 13350 may be included that includes button 13352, light source 13351 and display 13354. A number may be hidden on display 13353 until an appropriate code is entered via buttons, which may be indicated via light source 13351.

Card 13370 may be included that includes button 13373, light source 13372 and display 13374. A number may be hidden on display 13353 until an appropriate code is entered via buttons, which may be indicated via light source 13351. Such a number may change based on time such that the number associated with the time period in which a correct PIN was entered is displayed on display 13374.

Persons skilled in the art will appreciate that any communications device may be added to a card. For example, an IC chip (e.g, EMV chip) may be added to card 13330 and may be utilized to provide information to an RFID antenna.

Persons skilled in the art will appreciate that data may be transferred, such as gift card and/or pre-paid card data, to a card in a variety of ways. For example, a card may be swiped a second time through a magnetic stripe reader that includes a magnetic stripe encoder. A coil on the card may be utilized to receive the information and provide the received information to a processor. In doing so, information may be loaded into the card. Similarly, an IC chip may be utilized to receive data as well as a passive or active RFID. Additionally, one or more microphones may be included to receive audio information that may be representative of data. Accordingly, for example, a user may hold his/her card, or other device, next to a device that is operable to transmit audio via a speaker (e.g., laptop, stationary computer, or mobile telephonic device). The audio information may be discerned by the card and utilized to load information into the card (e.g., a gift card or pre-paid card. An application may also be loaded that enhances the functionality of the card. Such an application may include, for example, a user's medical information such that medical information can be displayed via the card (or other device) during a medical emergency. Accordingly, applications and/or payment cards may be purchased online and a speaker may communicate information to a card. Similarly, the card may include a speaker for transmitting information such that bi-directional communications are established. A light detector may be provided on a card that may receive light pulses indicative of data. Accordingly, for example, a user may hold a card up to a display - such as the screen of a laptop, stationary computer, or mobile phone - and information may be communicated from the display to the card via the light detector. Similarly, a light source may be utilized to communicate information from one device to another. For example, a light source (e.g., LED) may be utilized to communicate information from one card to another. Similarly, a magnetic stripe reader may include a light source. A card may be positioned over the light source such that a light detector of the card is aligned with the light source to receive light. Accordingly, the light of a magnetic stripe reader (or other type of reader) may be utilized to communicate information back to a card. A user may utilize interfaces on the card (e.g., buttons) to initiate a transfer of data from one card to another card or from a device to a card. A variety of types of data may be communicated. For example, money may be communicated from one debit card to another debit card such that payments may occur between the cards. Accordingly, for example, the next time a card is utilized via a reader (e.g., a magnetic stripe reader) information of the transfer may be communicated to a server for processing. Light may be utilized to transfer data from a card to a computer using, for example, a camera (e.g., webcam) on the computer. Sound may be utilized to transfer data from a card to a computer using, for example, a microphone on the computer.

A display may also be utilized as an interface. For example, a display may include a contact and an electronic ink. The electronic ink may change colors in response to, for example, a particular electrical signal being supplied to the contact. A capacitive sensor may be coupled to such a contact, however, such that a user interaction with the contact may be sensed by the capacitive sensor. Accordingly, a card may include a display that can also receive user input. Persons skilled in the art will appreciate that a display may include multiple contacts. For example, a display may include multiple 7-segment (e.g., to display digits) or 11-segment, 14-segment, or 16-segment (e.g., to display alphanumerics) regions where each segment may be coupled to a capacitive sensor.

A biometric sensor may be placed on a card or other device. Such a biometric sensor may be, for example, a fingerprint reader. Accordingly, one or more fingerprints may be stored in the memory of a card and compared to scanned fingerprints. Different fingerprints may activate the card differently (e.g., utilize a different user's payment card info).

Persons skilled in the art will appreciate that a user's payment card number (e.g., credit card or debit card number) does not have to change. A display may hide this payment card number until an appropriate unlocking code is entered into buttons of the card. Similarly, a magnetic emulator may not be provided current until the proper unlocking code is entered - thus keeping magnetic information private and not allowing undesirable readers to read a card. A security code may be displayed on the same or a different display. A button may be provided representative of an online purchase (or a user may utilize buttons to instruct the processor that an online purchase is desirable). For such an online purchase, the credit card number and the security code may be displayed - but the magnetic emulator may not be activated. In doing so, the level of security of the card is increased. Furthermore, for example, a button may be provided representative of in-store purchases (or a user may utilize buttons to instruct the processor that an in-store purchase is desirable). Accordingly, a processor may be signaled that an in-store purchase is desired. A different operation may be associated with different types of purchases (e.g., online or in-store). Accordingly, for example, magnetic emulators may be activated for an in-store environment - but not the displays. Accordingly, for example, a restaurant cashier may not be able to read the credit card number from the card, but may still be able to swipe the card. If a reader is down or a cashier requires reading particular information (e.g., a security code or credit card number information) then controls may be utilized to communicate this information. A record of the types of transactions may be stored and may be communicated in discretionary fields of data within a transmitted data track. Such record information may be utilized, for example, to further increase security and/or introduce a variety of additional functionality.

Different types of cards may be provided on a card. For example, a security ID number and a credit card number may both be provided on the same card. A button may be utilized to allow a user to provide instruction to a processor such that the processor can display (e.g., visually and/or magnetically) the desired information. For example, a user may determine to use one of a variety of payment accounts (e.g., credit and/or debit) for a purchase. An entire payment number (e.g., credit or debit) may be changed and/or hidden visually and/or magnetically. A portion of a payment card number (e.g., credit or debit) may be changed and/or hidden visually and/or magnetically.

Persons skilled in the art will appreciate that a display on the card may display a credit card number that does not change with time (or transaction or button press). Additionally, for example, a magnetic emulator (or multiple magnetic emulators) may magnetically communicate financial data that does not change with time. Such a card may reduce, for example, the effects of physical card theft and card cloning.

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipt may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

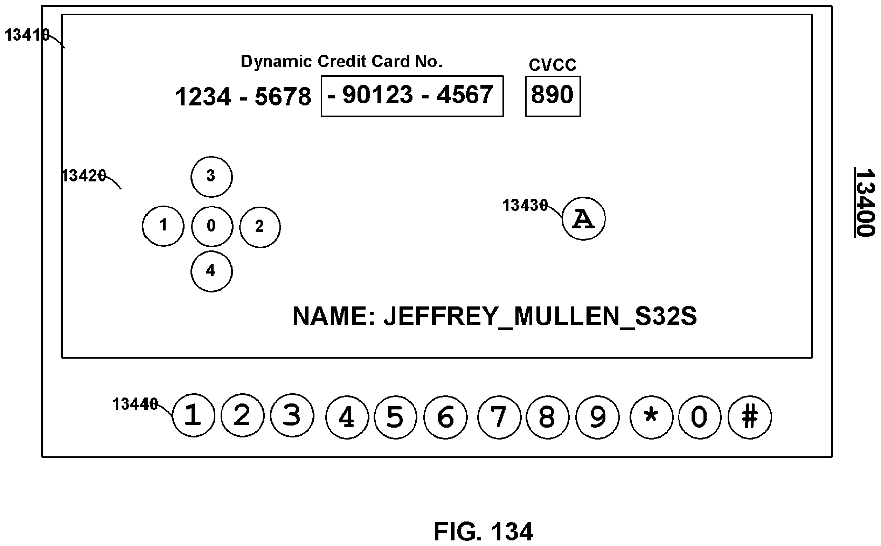

FIG. 134 shows personal electronic device 13400 which may be, for example, a portable telephonic device, portable media player, or any type of electronic device. Persons skilled in the art will appreciate that the functionality of a card may be provided on a personal device and displayed through a graphical user interface. Personal electronic device 13400 may include, for example, user inputs 13440 and display 13410. Virtual card 13420 may be displayed on display 13420. Display 13420 may be a touch-sensitive display such that, for example, virtual button 13430 may be provided on virtual card 13420. Persons skilled in the art will appreciate that cards may be provided as virtual cards and a user may interact with such virtual cards in order to provide a variety of functions. Personal electronic device 13400 may communicate to a card reader such as, for example, an RFID reader.

A display may be bi-stable or non bi-stable. A bi-stable display may consume electrical energy to change the information displayed on the bi-stable display but may not consume electrical energy to maintain the display of that information. A non bi-stable display may consume electrical energy to both change and maintain information on the non bi-stable display. A display driving circuit may be provided, for example, for a bi-stable display (or a non bi-stable display). Such a display driving circuit may step-up a supply voltage (e.g., 1-5 volts) to a larger voltage (e.g., 6-15 volts) such that a bi-stable display may change displayed information. A controller (e.g., a processor) may be utilized to control such a display driving circuit. Persons skilled in the art will appreciate that a display may be configured to display numerical data or alphanumerical data. A display may also be configured to display other indicia (e.g., the image of a battery and its remaining life).

A magnetic stripe reader may, for example, determine information on a magnetic stripe by detecting the frequency of changes in magnetic fields (e.g., flux transversals). A particular frequency of flux transversals may correlate to, for example, a particular information state (e.g., a logic "1" or a logic "0"). Accordingly, for example, a magnetic emulator may change the direction of an electromagnetic field at particular frequencies in order to communicate a different state of information (e.g., a logic "1" or a logic "0").

Persons skilled in the art will appreciate, for example, that a card may include an IC chip (e.g., EMV chip), RFID, and a dynamic magnetic communications device (e.g., a magnetic emulator or encoder). The same information may be communicated through, for example, any number of such devices (e.g., a dynamic magnetic communications device, RFID, and an EMV chip). A central processor may cause each device to communicate the information (in the same format or a different format). Each component may have its own processor or driving circuitry. Such individual processors or driving circuitry may be coupled to a central processor. An EMV chip may be utilized, for example, to provide control signals to other devices (e.g., circuitry driving a display as well as a dynamic magnetic communications device). Such an EMV chip may receive signals provided by one or more buttons to determine, for example, that a particular button, or sequence of buttons, was pressed by a user.

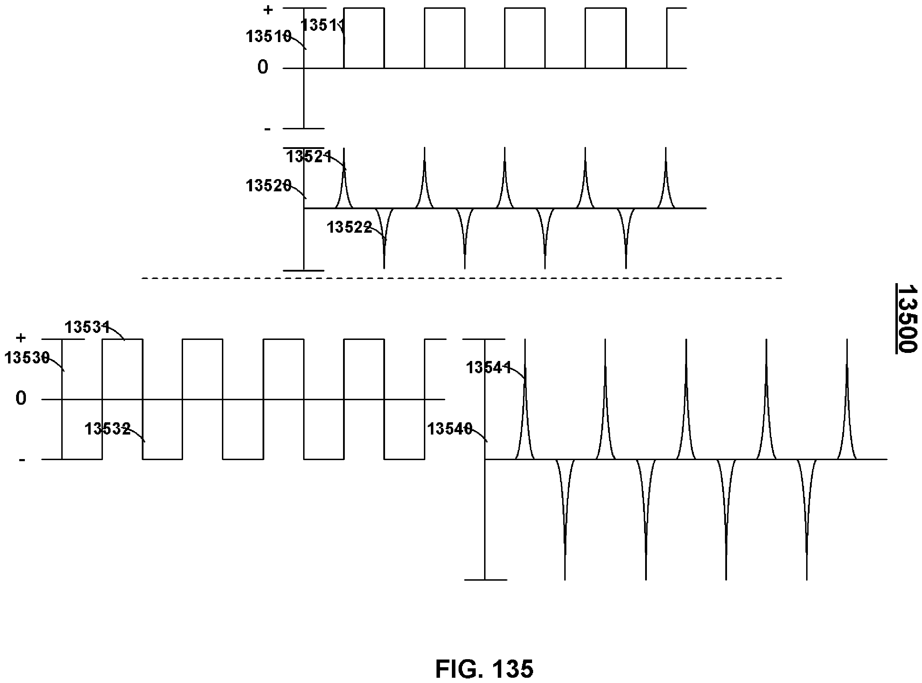

FIG. 135 shows control signals 13510 and 13520 and magnetic stripe reader sense signals 13530 and 13540. Control signals 13510 may be utilized to drive, for example, a magnetic emulator serially communicating data to a magnetic stripe reader. A magnetic emulator may be, for example, driven by a current having a positive polarity or being able to swing between a positive and a negative polarity. Such a current may be generated, for example, from a transistor providing a drive voltage over a drive resistor. An H-bridge may be utilized, for example, to drive current through a coil in both directions.

Range 13510 shows current signal 13511 that is provided in a single polarity. A magnetic stripe reader may read the timing of phase transversals to determine whether a one ("1") or a zero ("0") was received. For example, a short period of time before a phase transversal may be determined to be a particular bit of information while a long period of time before a phase transversal may be a different bit of information. Information may be encoded using, for example, F2F encoding such that a magnetic stripe reader may be configured to perform F2F decoding to extract information.

Range 13520 shows signal 13521, which may be the signal sensed by a magnetic stripe reader. Persons skilled in the art will appreciate that signal 13521. Signal 13521 may, for example, be provided as a series of pulses. An increase of current through an emulator may, for example, correspond to a positive pulse and a decrease of current through an emulator may, for example, correspond to a negative pulse. The magnitude of a magnetic stripe reader sense pulse may, for example, be correlated with the rate of change of the drive signal of a magnetic stripe emulator. Accordingly, for example, a larger rate of change may correlate to larger sense pulses. Information may be determined at the reader by, for example, F2F decoding. Persons skilled in the art will appreciate that, for example, a longer period of a pulse may lengthen the distance between pulses. Decoding may, for example, determine a swipe rate (e.g., by looking at a string of leading zero bits). A logic zero may provide pulses that define the beginning and end of a LONG period of time. Thus, a string of logic zeros may allow a reader to determine the LONG period for that swipe. A logic one may recognized, for example, when a pulse is provided in the middle of a LONG period of time such that the reader sees two SHORT periods of time. Accordingly, changing the time between when a current increase and decrease occurs may be utilized to communicate a logic one or logic zero. Similarly, the amount of time before a current decrease and increase occurs may be utilized to communicate a logic one or a logic zero.

Range 13530 may correlate to a current drive signal that swings between a positive and a negative polarity. As a result, the amplitude of sense signal 13541 on range 13540 may be increased with respect to signal 13531 having the same amplitude, but a single polarity.

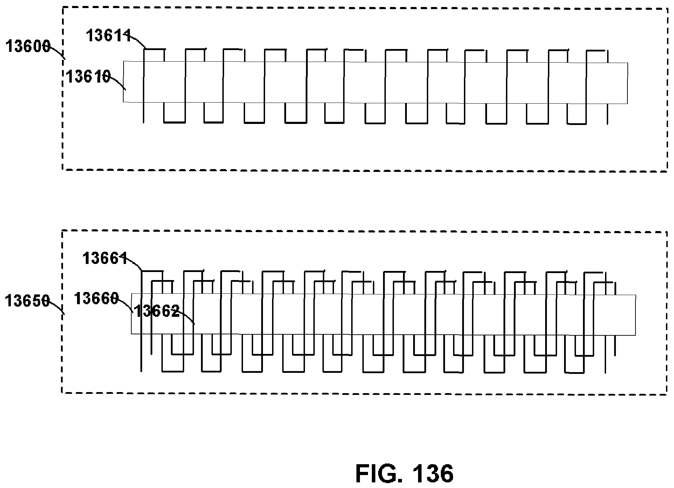

FIG. 136 shows magnetic emulator 13600 that may be utilized, for example, to communicate data serially to a magnetic stripe reader. Emulator 13600 may include coil 13611 around material 13610. Material 13610 may be, for example, a soft magnetic material.

Magnetic emulator 13650 may be utilized, for example, to communicate data serially to a magnetic stripe reader. Emulator 13650 may include coil 13661 and 13662 around material 13660. Material 13660 may be, for example, a soft magnetic material. Persons skilled in the art will appreciate that the increased number of coil turns may, for example, result in a larger electromagnetic field. Coil 13661 may be fabricated on one layer above and one layer below material 13660. Coil 13662 may be fabricated, for example, on a different layer above and a different layer below material 13660.

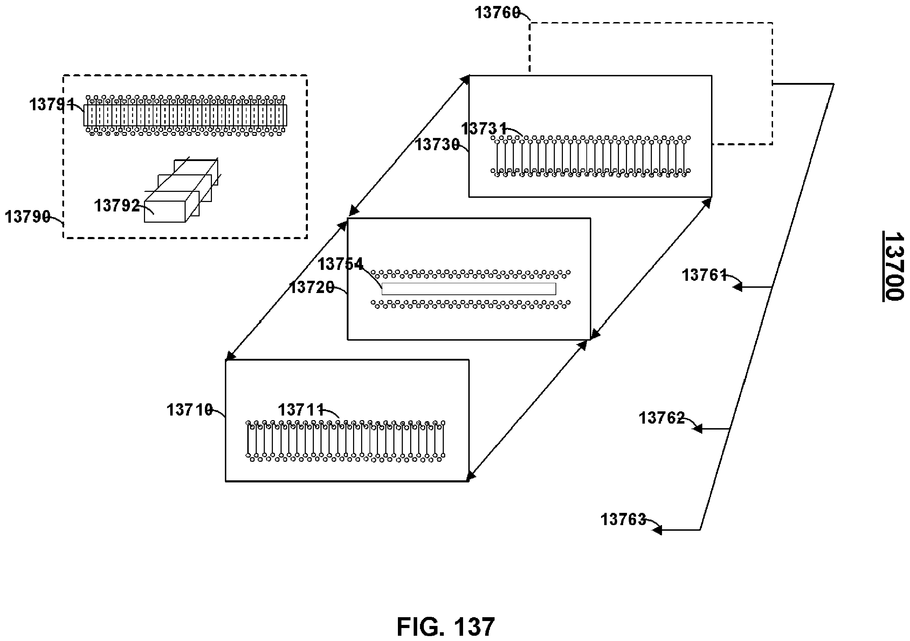

FIG. 137 shows manufacturing process 13700 that may be utilized, for example, to provide a card that includes a coil (e.g., coil 13790). Coil 13790 may take the form of birds-eye view perspective 13791 and angled cross-sectional perspective 13792.

Layer 13710 may be provided. Coil segments 13711 may be printed on, for example, layer 13710. Material 13753 may be provided between layer 13710 and layer 13730. Material 13754 maybe provided on layer 13720 (e.g., printed on layer 13720). Vias may be provided on layer 13720 to couple, for example, coil segments 13711 to coil segments 13731 of layer 13730. Additional layers may be provided. For example, layer 13710 may be provided and may include printed contacts for interconnecting circuitry. Layer 13760 may be provided, for example, at locations 13761-963. For example, layer 13760 may be provided as shown and at location 13763. Each such layer may include, for example, coil segments for a second coil. Layers 13710, 13720, and 13730 may, for example, include vias for coupling the coil segments for the second coil together. Any layer of FIG. 137 may include contacts to, and interconnections between, card components such as a processor, LEDs, buttons, battery, RFID, and IC chip (e.g., EMV chip).

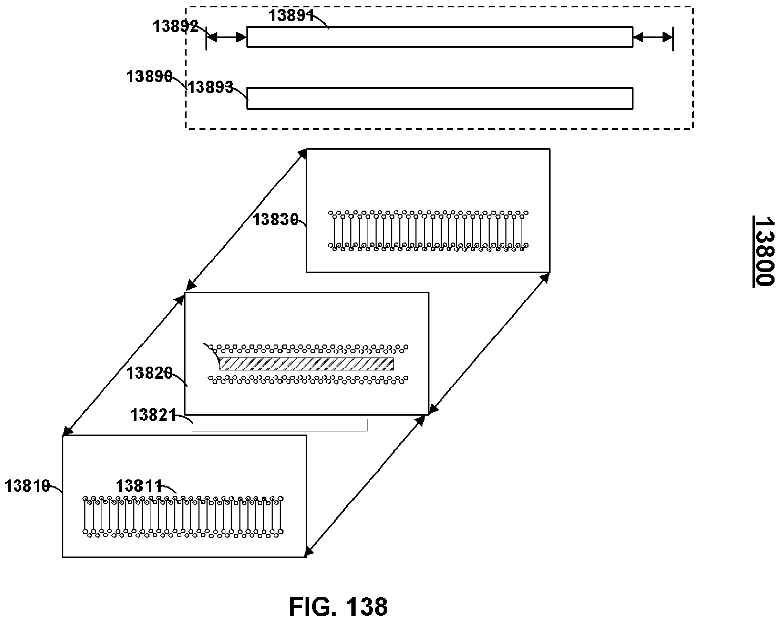

FIG. 138 shows materials 13890 that includes material 13891 and 13893. Material 13891 may be magnetostrictive such that, for example, material 13891 mechanically distorts in the presence of a magnetic field. For example, material 13891 may distort from a resting location to distorted location 13892. Material 13891 may affect an electromagnetic field as material 13891 distorts. Materials 13893 may, for example, be a soft-magnetic material with zero, or substantially zero, parts per million of magnetostriction. Material 13893 may be operable to affect an electromagnetic field without distorting.

Person skilled in the art will appreciate that a magnetostrictive material may, for example, have a better performance if allowed to mechanically distort. Accordingly, for example, layer 13820 may be provided with aperture 13854. Magnetostrictive material 13821 may be placed within cavity 13854 such that magnetostrictive material 13821 may mechanically distort within aperture 13854. Layer 13810 may be provided with coil segments 13811 coupled to vias on layer 13820 to couple the coil segments on layer 13810 to layer 13830.

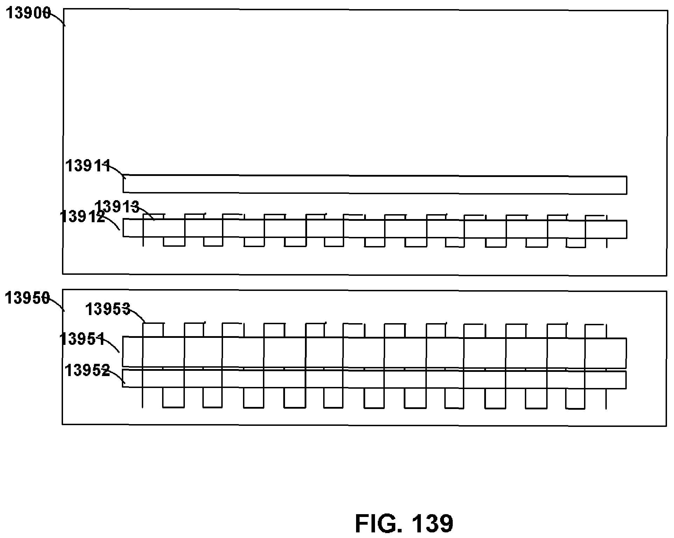

FIG. 139 shows card 13900 that may include, for example, permanent magnet 13911 located about coil 13913. Coil 13913 may be located around material 13912. Material 13912 may be, for example, a soft-magnetic material. Persons skilled in the art will appreciate that permanent magnet 13911 may provide a bias magnetic field that may increase the field located at the exterior of coil 13913 by providing bias to the field located at the exterior of coil 13913.

Cross-section 13950 may be, for example, a cross section of any type of card (e.g., payment card, gambling card, phone card, security access card). Coil 13953 may be provided, for example, to communicate data serially to a magnetic stripe reader. Soft-magnetic material 13951 may be provided within coil 13953. Similarly, for example, permanent magnet 13952 may be provided inside of coil 13953. Soft-magnetic material 13951 may be, for example, thicker (or thinner) than magnet 13952. Similarly, for example, soft magnetic material 13951 may be physically touching permanent magnet 13952 or may be separated (e.g., via an insulator).

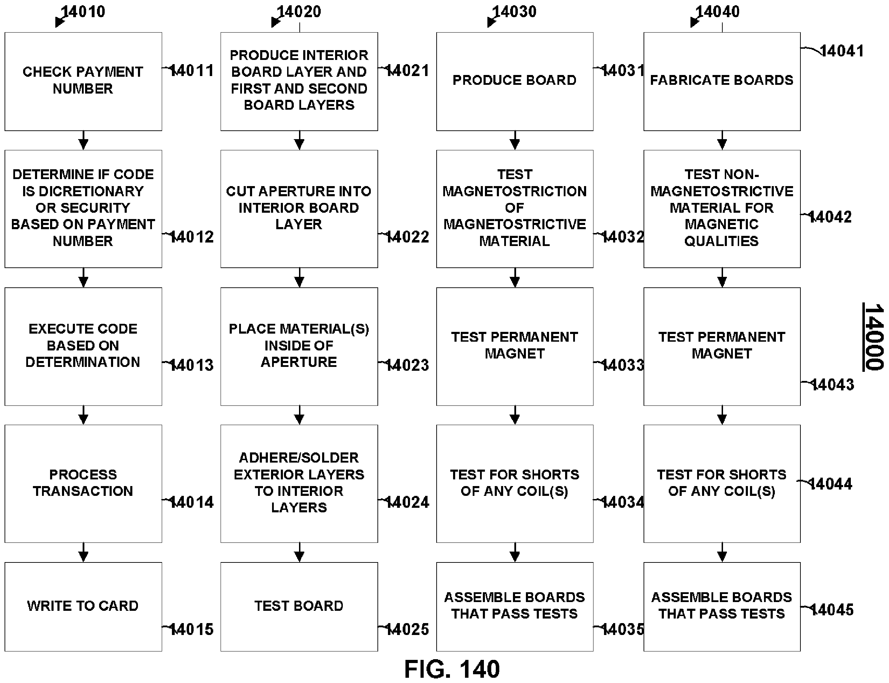

FIG. 140 shows flow charts 14000 that may include, for example, flow chart 14010, 14020, 14030, and 240. Flow chart 14010 may include step 14011, in which a number, such as a payment number, may be retrieved. Step 14013 may determine, for example, whether the payment number is associated with a security code, discretionary code, a code for both security and discretionary data, or no code. The code may be executed in step 14013. For example, if a security code is associated with a payment number then the security code may be, for example, validated. As per another example, if a discretionary code is associated with a payment number then the discretionary code may be, for example, validated. Persons skilled in the art will appreciate that no code may be associated to a payment number or only in particular instances (e.g., online purchases). Similarly, a card may have both a security component and a discretionary data portion. A transaction (e.g., a payment transaction) may be validated in step 14014. Step 14015 may, for example, be included to allow for a device to provide information to a card (e.g., via a magnetic stripe emulator, RFID antenna, or IC chip).

Process 14020 may be provided. Step 14021 may be included in process 14020 that may, for example, include producing an interior and two exterior layers. Such layers may be, for example, a printed circuit board layer (e.g., an FR4 layer). Step 14021 may include, for example, steps such as layer printing, cutting, and testing. Step 14022 may be included to cut, for example, an aperture into the interior board layer. Such an aperture may be sized to approximately the size of one or more materials that may be utilized in the interior of a coil of a magnetic emulator. The aperture may, for example, be larger than the one or more materials utilized in the interior of a coil of a magnetic emulator. Persons skilled in the art will appreciate that such a material may be a magnetostrictive material that distorts in the presence of a magnetic field in order to provide a particular influence on that magnetic field. Accordingly, a magnetostrictive material may be utilized with, for example, a two-dimensional coil (e.g., underneath or above) or another type of device (e.g., a magnetic encoder). Persons skilled in the art will also appreciate, for example, that a magnetostrictive material may distort if the material is placed in a cavity that is the same size as the magnetostrictive material. A magnetostrictive material may be configured to distort, for example, so long as the magnetostrictive material is not adhered to adjacent board layers.

Step 14022 may be utilized to place a material into the aperture. Person skilled in the art will appreciate that an aperture may be cut through a board layer and that a cut may be provided that only provides space partially through a board such that a trough is formed. Any material may be placed in a space that is cut for the material. For example, a permanent magnet or non-magnetostrictive material may be placed in such a space. In doing so, for example, a multiple layer board may be formed that has even exterior surfaces void of bulges. Without a space, for example, a bulge may appear. Bulges may be useful, however, in that apertures may increase the cost of making a multiple layer card. Apertures may also be cut into layers exterior to the layer housing the material (e.g., all layers) in order to reduce bulges as well as provide a thin card. For example, a permanent magnet may be placed outside of a coil and an aperture may be cut through all layers of the multiple layer circuit board such that a relatively thick permanent magnet may be provided. A material and board layer including an aperture may, for example, be configured to have substantially the same thickness. A machine may autonomously cut spaces and place materials into those spaces. Layers of a multiple-layer board may be adhered together in step 14024 and the board may be tested in step 14025.

Process 14030 may be provided in which a single or multiple-layer board (e.g., utilizing FR4) is produced in step 14031. The board may include a magnetostrictive material and the magnetostriction of the magnetostrictive material may be tested in step 14032. The magnetostriction of a material may be tested, for example, by providing a particular amount of current through a coil in which the magnetostrictive material resides and determining the amount of electromagnetic field produced by the magnetostrictive material.

The magnetism associated with a permanent magnetic included in a multiple layer board (if a permanent magnet is provided) may be tested in step 14033. Tests for any shorts, such as shorts in one or more coils, may be tested in step 14034. Boards that pass all tests may be, for example, assembled in step 14035 by placing electrical components (e.g., microprocessors, LEDs, oscillators, buttons, IC chips) on a board. Persons skilled in the art will appreciate that boards for cards may be fabricated in sheets and assembly and lamination may also occur in sheets. Programming may also be performed while cards are in sheet form as well as any additional personalization (e.g., printing and embossing). Sheets may be cut into cards at any time (e.g., after the cards are ready for mailing).

Process 14040 may be included. A single or multiple layer board may be fabricated in step 14041. Non-magnetostrictive material may be tested in step 14042. Permanent magnets may be tested in step 14043. Magnetostrictive material (if provided on a board) may also be tested. Shorts may be tested for in step 14044 and boards may be assembled or sent to an assembler in step 14045.

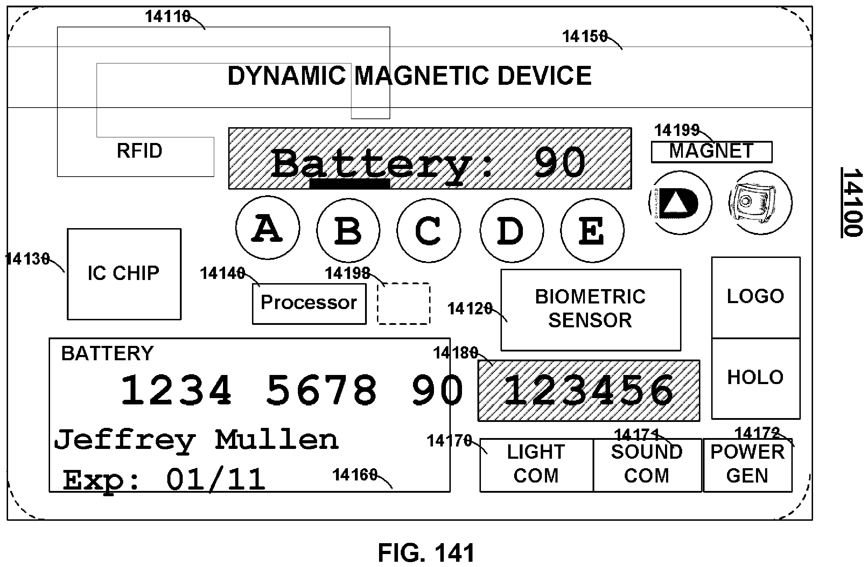

FIG. 141 shows card 14100 that may include a number of components for use in cards such as payment cards. Card 14100 may include RFID 14112, dynamic magnetic device 14150 (which may include one or more magnetic encoders or emulators), IC chip 14130, processor 14140, battery 14160, display 14180, biometric sensor 14120, permanent magnetic 14199, light communications device 14170 (for receiving and/or sending light-based information signals), sound communications device 14171 (for receiving and/or sending sound-based information signals), and power generator 14172 (for generating electrical energy to recharge battery 14160). Persons skilled in the art will appreciate that additional components may be provided on card 14100. For example, an oscillator may be provided as component 14198 such that time may be kept (e.g., to assist the deployment of time-based encryption).

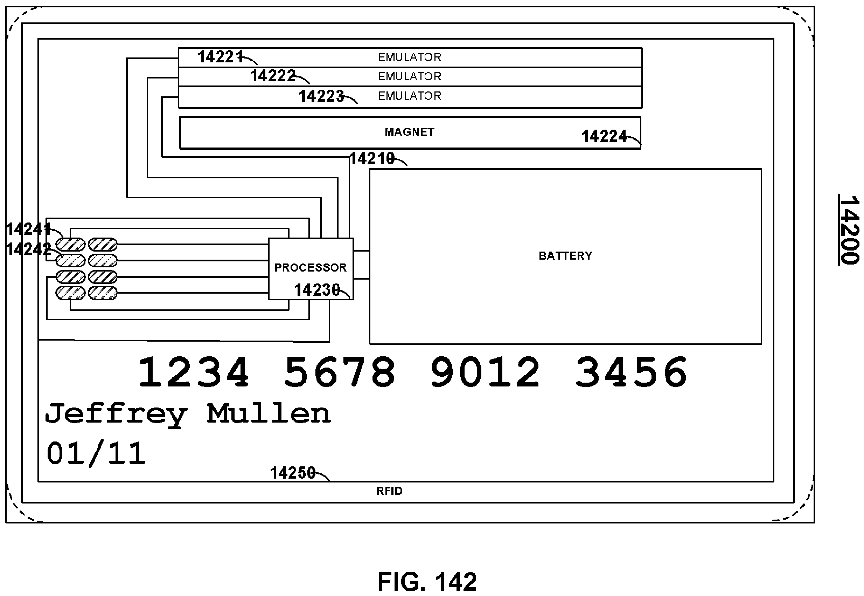

FIG. 142 shows card 14200 that may include, for example, magnetic emulator 14222 (e.g., for serially communicating track 1 information), magnetic emulator 14223 (e.g., for serially communicating track 2 information), and permanent magnet 14224. Persons skilled in the art will appreciate, for example, that a permanent magnet may be configured to provide a bias magnetic field that does not substantially emit from card 1300 such that the bias magnetic field may not substantially affect objects placed outside of card 14200 (e.g., a static magnetic stripe of a nearby payment card). Furthermore, permanent magnet 14224 may be configured such that permanent magnet 14224 is not strong enough to, for example, erase information of any nearby static magnetic stripes. Similarly, the coercivity of such a permanent magnet may be large such that the permanent magnet may have an expected lifespan of a relatively long period of time (e.g., over 10 years).

Card 14200 may include, for example, RFID 14250, battery 14210, processor 14230 and EMV chip contacts 14241 and 14242 such that processor 14230 may perform the processing of an EMV chip such that card 14200 may not include, for example, an EMV chip.

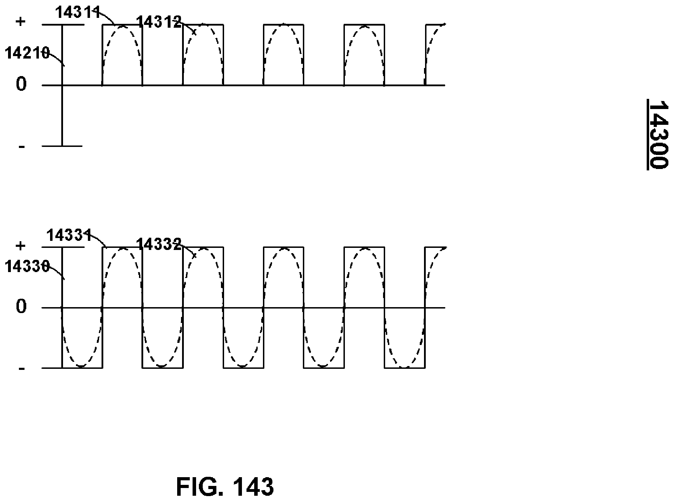

FIG. 143 shows control signals 14300. Range 14300 may include control signal 14312. Persons skilled in the art will appreciate that skewing control signal 14311 to produce curved signal 14312 may increase the ability for a magnetic stripe reader to recognize information. Similarly, range 14330 shows control signal 14331 skewed to provide a curved control signal in both a positive and negative polarity.

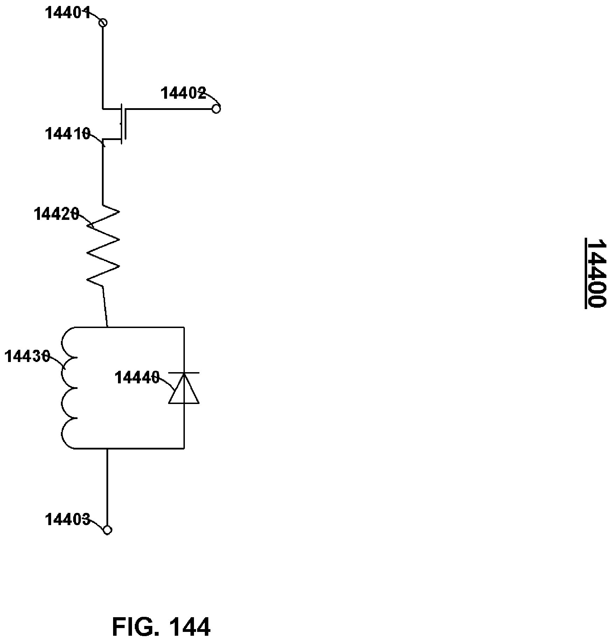

FIG. 144 shows driving circuit 14400. Driving circuit 14400 may include, for example, node 14401, node 14402, transistor 14410, resistor 14420, coil 14430, diode 14440, and node 14403. Persons skilled in the art will appreciate that a voltage may be coupled to note 14401. A processor may be coupled to, for example, node 14402. processor may control when the voltage coupled to node 14401 is coupled to resistor 14420 via transistor 14410. Resistor 14420 may be utilized, for example, to provide a drive current through coil 14430. Node 14403 may be coupled, for example, to ground. Diode 14440 may be coupled in parallel with coil 14430 in order to, for example, protect against voltage pulses that may be generated as the result of the inductance of coil 14430. Coil 14430 may be provided, for example, without diode 14440.

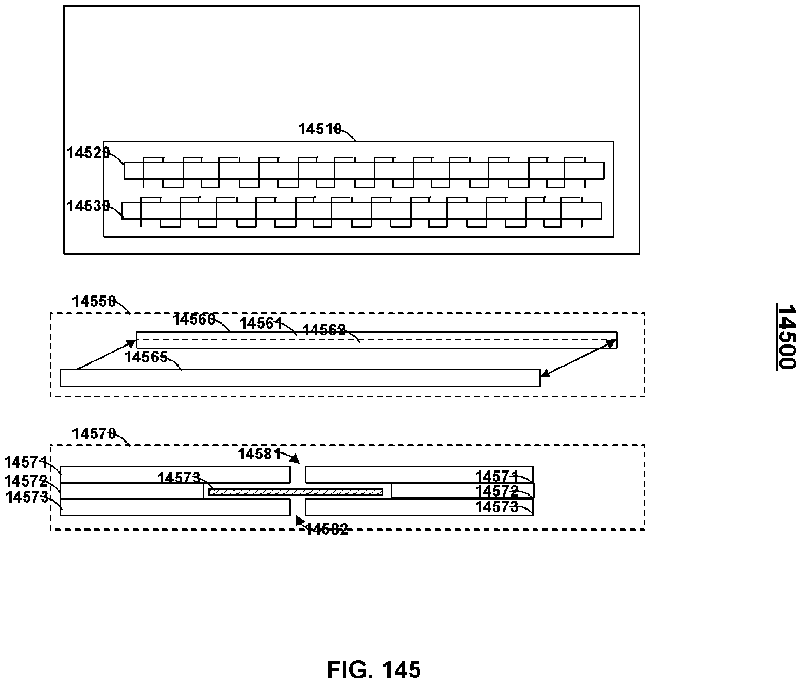

FIG. 145 shows card 14500 that may include emulators 14520 and 14530 located above a surface of permanent magnet 14510. Persons skilled in the art will appreciate that a permanent magnet may be provided above one or more coils and another permanent magnet may be provided below, for example, those one or more coils. Persons skilled in the art will appreciate that a permanent magnet may be polarized and oriented in a number of ways. For example, arrangement 14550 may be provided in which soft-magnetic material 14570 may be provide adjacent to permanent magnet 14560. Permanent magnet 14560 may be polarized such that region 14561 has one pole (e.g., a North pole) and region 14562 has another pole (e.g., a South pole). Persons skilled in the art will appreciate that materials may be polarized to form a permanent magnet. For example, a polarized metal, such as a magnetized steel allow, may be utilized.

Card portion 14570 shows layer 14571, 14572 and 14573. A cavity may be provided, for example, by cutting out a portion of layer 14572. A material (e.g., magnetostrictive material 14575 may be provided in the cavity. Persons skilled in the art will appreciate that, for example, apertures may be provided (e.g., aperture 14581 of layer 14571 and aperture 14582 of layer 14573). Such apertures may, for example, be provided before board layers are adhered together in order to, for example, prohibit a vacuum from forming inside of a cavity. Similarly, for example, apertures may be cut after a board is adhered to remove a vacuum from forming in a cavity. Such apertures may also be utilized to test characteristics of material 14581 as well as an associated device (e.g., a dynamic magnetic communications device). For example, testing probes may be placed in such apertures to determine, for example, if material 14581 shorted to other circuitry. Any number of apertures may be provided in any number of locations. A board having apertures may be, for example, laminated over after testing. For example, a board may be laminated via injection molding.

Persons skilled in the art will appreciate that data may be transferred, such as gift card and/or pre-paid card data, to a card in a variety of ways. For example, a card may be swiped a second time through a magnetic stripe reader that includes a magnetic stripe encoder. A coil on the card may be utilized to receive the information and provide the received information to a processor. In doing so, information may be loaded into the card. Similarly, an IC chip may be utilized to receive data as well as a passive or active RFID. Additionally, one or more microphones may be included to receive audio information that may be representative of data. Accordingly, for example, a user may hold his/her card, or other device, next to a device that is operable to transmit audio via a speaker (e.g., laptop, stationary computer, or mobile telephonic device). The audio information may be discerned by the card and utilized to load information into the card (e.g., a gift card or pre-paid card. An application may also be loaded that enhances the functionality of the card. Such an application may include, for example, a user's medical information such that medical information can be displayed via the card (or other device) during a medical emergency. Accordingly, applications and/or payment cards may be purchased online and a speaker may communicate information to a card. Similarly, the card may include a speaker for transmitting information such that bi-directional communications are established. A light detector may be provided on a card that may receive light pulses indicative of data. Accordingly, for example, a user may hold a card up to a display - such as the screen of a laptop, stationary computer, or mobile phone - and information may be communicated from the display to the card via the light detector. Similarly, a light source may be utilized to communicate information from one device to another. For example, a light source (e.g., LED) may be utilized to communicate information from one card to another. Similarly, a magnetic stripe reader may include a light source. A card may be positioned over the light source such that a light detector of the card is aligned with the light source to receive light. Accordingly, the light of a magnetic stripe reader (or other type of reader) may be utilized to communicate information back to a card. A user may utilize interfaces on the card (e.g., buttons) to initiate a transfer of data from one card to another card or from a device to a card. A variety of types of data may be communicated. For example, money may be communicated from one debit card to another debit card such that payments may occur between the cards. Accordingly, for example, the next time a card is utilized via a reader (e.g., a magnetic stripe reader) information of the transfer may be communicated to a server for processing. Light may be utilized to transfer data from a card to a computer using, for example, a camera (e.g., webcam) on the computer. Sound may be utilized to transfer data from a card to a computer using, for example, a microphone on the computer.

A display may also be utilized as an interface. For example, a display may include a contact and an electronic ink. The electronic ink may change colors in response to, for example, a particular electrical signal being supplied to the contact. A capacitive sensor may be coupled to such a contact, however, such that a user interaction with the contact may be sensed by the capacitive sensor. Accordingly, a card may include a display that can also receive user input. Persons skilled in the art will appreciate that a display may include multiple contacts. For example, a display may include multiple 7-segment (e.g., to display digits) or 11-segment, 14-segment, or 16-segment (e.g., to display alphanumerics) regions where each segment may be coupled to a capacitive sensor.

A biometric sensor may be placed on a card or other device. Such a biometric sensor may be, for example, a fingerprint reader. Accordingly, one or more fingerprints may be stored in the memory of a card and compared to scanned fingerprints. Different fingerprints may activate the card differently (e.g., utilize a different user's payment card info).

Persons skilled in the art will appreciate that a user's payment card number (e.g., credit card or debit card number) does not have to change. A display may hide this payment card number until an appropriate unlocking code is entered into buttons of the card. Similarly, a magnetic emulator may not be provided current until the proper unlocking code is entered - thus keeping magnetic information private and not allowing undesirable readers to read a card. A security code may be displayed on the same or a different display. A button may be provided representative of an online purchase (or a user may utilize buttons to instruct the processor that an online purchase is desirable). For such an online purchase, the credit card number and the security code may be displayed - but the magnetic emulator may not be activated. In doing so, the level of security of the card is increased. Furthermore, for example, a button may be provided representative of in-store purchases (or a user may utilize buttons to instruct the processor that an in-store purchase is desirable). Accordingly, a processor may be signaled that an in-store purchase is desired. A different operation may be associated with different types of purchases (e.g., online or in-store). Accordingly, for example, magnetic emulators may be activated for an in-store environment - but not the displays. Accordingly, for example, a restaurant cashier may not be able to read the credit card number from the card, but may still be able to swipe the card. If a reader is down or a cashier requires reading particular information (e.g., a security code or credit card number information) then controls may be utilized to communicate this information. A record of the types of transactions may be stored and may be communicated in discretionary fields of data within a transmitted data track. Such record information may be utilized, for example, to further increase security and/or introduce a variety of additional functionality.

Different types of cards may be provided on a card. For example, a security ID number and a credit card number may both be provided on the same card. A button may be utilized to allow a user to provide instruction to a processor such that the processor can display (e.g., visually and/or magnetically) the desired information. For example, a user may determine to use one of a variety of payment accounts (e.g., credit and/or debit) for a purchase. An entire payment number (e.g., credit or debit) may be changed and/or hidden visually and/or magnetically. A portion of a payment card number (e.g., credit or debit) may be changed and/or hidden visually and/or magnetically.

Persons skilled in the art will appreciate that a display on the card may display a credit card number that does not change with time (or transaction or button press). Additionally, for example, a magnetic emulator (or multiple magnetic emulators) may magnetically communicate financial data that does not change with time. Such a card may reduce, for example, the effects of physical card theft and card cloning.

Persons skilled in the art will appreciate that any numbers of a credit card number may remain static and/or change either with time or based off a transaction (e.g., by sensing a read-head "swipe"). Additionally, any static and/or dynamic numbers may be displayed via a display or printed on a card. For example, a middle 6 digits of a credit/debit card number may be static and may be displayed on a display. Such a middle 6 digits may be displayed, for example, upon the entry of a correct PIC. Similarly, a magnetic emulator may not communicate information until a correct PIC has been entered by a user. Doing so may, for example, reduce fraud associated with card cloning. Additionally, a receipt may be provided that includes masked credit card numbers except for the last few digits of credit card numbers. Accordingly, displaying a static middle 6 digits of credit card numbers may allow for such a receipt to be provided while still reducing credit card fraud from hiding numbers that are not displayed on such a receipt. Any amount of numbers and/or characters may be displayed through a display. For example, nineteen digits may be displayed as part of a credit/debit numbers and these numbers may also be communicated through one or more magnetic emulation circuits. The entry of particular PICs may provide different results. For example, a first PIC may only display a string of alphanumeric characters. A second PIC may only activate a magnetic emulation circuit to transmit information including that string of alphanumeric characters (or a different string). A third PIC may activate a magnetic emulation circuit and a display. A display and/or magnetic emulation circuit may be turned OFF, for example, upon entry of an incorrect PIC and/or after a period of time has passed since the entry of the PIC and/or after the detection of a particular number of swipes by a read-head detector (e.g., one or two).

Persons skilled in the art will appreciate that a credit/debit card number (or any other information) may remain static until an event occurs and then may become dynamic (e.g., change based on swipes and/or time). For example, a particular PIC may change from a static to a dynamic topology and/or a topology may be changed from static to dynamic after a pre-determined period of time. Additionally a card and/or device may include a wireless receiver and a topology may be changed from a static to a dynamic topology upon, for example, receiving an appropriate signal from the wireless receiver. Accordingly, a validation process may change at a validation server depending upon whether a card is utilizing a static and/or dynamic topology at any given time. Additionally, a static credit/debit card number may be printed on the face of a card and information (e.g., a security code) may be displayed via a display and remain static over time (or with use) or be provided dynamically.

A card or other device (e.g., a mobile telephone) may accept a pre-determined number of consecutive incorrect PICs before locking the card for a period of time or until an appropriate secondary PIC is entered. Accordingly, a user may enter in an incorrect PIC a number of times and then, after a card becomes locked, call a support center for a secondary one-time use PIC. A card may cycle through unlocking PICs based, for example, on time or the number of previous unlock attempts.

Persons skilled in the art will also appreciate that the present invention is not limited to only the embodiments described. Instead, the present invention more generally involves dynamic information. Persons skilled in the art will also appreciate that the apparatus of the present invention may be implemented in other ways then those described herein. All such modifications are within the scope of the present invention, which is limited only by the claims that follow.

### Additional Statements

The following numbered statements correspond to the claims originally filed on the parent patent application, number EP08865573.3.
1. A card comprising:
   a dynamic magnetic communications device for communicating a track of magnetic stripe data to a magnetic stripe reader, wherein said dynamic magnetic communications device communicates said track of magnetic stripe data serially and no static magnetic stripe is provided on said card.
2. The card of statement 1, wherein said dynamic magnetic communications device is fabricated in a single printed circuit board layer.
3. The card of statement 1, wherein said dynamic magnetic communications device is fabricated in a multiple layered printed circuit board.
4. The card of statement 1, wherein said dynamic magnetic communicated circuit is provided in an ASIC.
5. The card of statement 1, further comprising a bi-stable display.
6. The card of statement 1, further comprising a non bi-stable display.
7. The card of statement 1, further comprising at least one button.
8. The card of statement 1, further comprising a display and a plurality of buttons.
9. The card of statement 1, further comprising an RFID.
10. The card of statement 1, further comprising a processor and an EMV chip.
11. The card of statement 1, further comprising an RFID, a display, an EMV chip, and at least one button.
12. The card of statement 1, further comprising an RFID, a display, an EMV chip, a processor, and at least one button.
13. The card of statement 1, further comprising an RFID, a display, an EMV chip, a processor, a plurality of buttons.
14. A card comprising:
   a display;
   a magnetic stripe, wherein said magnetic stripe includes first information; and
   a first magnetic emulator about said magnetic stripe, wherein said first magnetic emulator is operable of communicating second information to a magnetic stripe card reader; and
   a second magnetic emulator about said magnetic stripe, wherein said second magnetic emulator is operable of communicating third information to said magnetic stripe card reader.
15. The card of statement 14, wherein said first magnetic emulator is located on a surface of said card above said second magnetic emulator, said card includes a first edge that is longer than a second edge, and at least a portion of said first and second emulators are aligned from the perspective of said first edge.
16. The card of statement 14, wherein said first magnetic emulator is located on a surface of said card above said second magnetic emulator, said card includes a first edge that is longer than a second edge, and at least a portion of said first and second emulators are staggered from the perspective of said first edge.
17. The card of statement 14, wherein said second information includes a payment account number and said third information includes said payment account number.
18. The card of statement 14, wherein said second information includes a payment account number, said third information includes said payment account number, and said payment account number is changed based on time.
19. The card of statement 14, wherein said second information includes a payment account number, said third information includes said payment account number, and said payment account number is changed based on use.
20. The card of statement 14, wherein said second information includes a payment account number and a security code.
21. The statement of statement 14, wherein said second information includes a payment account number and a security code, wherein said security code is changed based on time.
22. The card of statement 14, wherein said second information includes a payment account number and a security code, wherein said security code is changed based on use.
23. The card of statement 14, wherein said second information includes a payment account number and a security code, wherein said payment account number and said security code is changed based on time.
24. The card of statement 14, wherein said second information includes a payment account number and a security code, wherein said payment account number and said security code is changed based on use.
25. The card of statement 14, further comprising a display.
26. The card of statement 14, further comprising a plurality of buttons.
27. The card of statement 14, further comprising a button and a display.
28. A card comprising:
   a display;
   a magnetic stripe, wherein said magnetic stripe includes first information; and
   a magnetic emulator about said magnetic stripe, wherein said magnetic emulator is operable of communicating second information to said magnetic stripe card reader.
29. The card of statement 28, further comprising a plurality of buttons.
30. The card of statement 28, further comprising at least five buttons.
31. The card of statement 28, wherein said display is a bi-stable display.
32. The card of statement 28, wherein said display is a non bi-stable display.
33. The card of statement 28, wherein said second information includes a payment account number.
34. The card of statement 28, wherein said second information includes a payment account number that is changed based on time.
35. The card of statement 28, wherein said second information includes a payment account number that is changed based on use.
36. The card of statement 28, wherein said second information includes a payment account number and a security code.
37. A card comprising:
   a dynamic magnetic communications device for communicating a track of magnetic stripe data to a magnetic stripe reader, wherein said dynamic magnetic communications device communicates said track of magnetic stripe data serially and said dynamic magnetic communications device is fabricated on a single surface of a printed circuit board layer.
38. The card of statement 37, further comprising a display.
39. The card of statement 37, further comprising a display and at least one button.
40. The card of statement 37, further comprising a display and a plurality of buttons.
41. The card of statement 37, further comprising an EMV chip.
42. A card comprising:
   a dynamic magnetic communications device for communicating a track of magnetic stripe data to a magnetic stripe reader, wherein said dynamic magnetic communications device communicates said track of magnetic stripe data serials and said dynamic magnetic communications device is fabricated on multiple layers of material.
43. The card of statement 42, further comprising a display.
44. The card of statement 42, further comprising a bi-stable display.
45. The card of statement 42, further comprising a non bi-stable display and a plurality of buttons.
46. The card of statement 42, further comprising a display and at least one button.
47. The card of statement 42, further comprising a light sensor for sensing light-information pulses.
48. The card of statement 42, further comprising an EMV chip.
49. The card of statement 42, further comprising an EMV chip and a processor.
50. The card of statement 42, further comprising a rechargeable battery and a device for recharging said rechargeable battery.
51. A card comprising:
   a bi-stable display;
   a non bi-stable display, wherein data displayed on said bi-stable display is communicated to a magnetic stripe reader via a dynamic magnetic communications device.
52. The card of statement 51, wherein said bi-stable display is located on one surface of said card and said non bi-stable display is located on a second surface of said card.
53. The card of statement 51, further comprising a plurality of buttons.
54. The card of statement 51, further comprising an EMV chip.
55. The card of statement 51, further comprising an EMV chip and processor.
56. The card of statement 51, further comprising an EMV chip, processor, and RFID.
57. A card comprising:
   a bi-stable display located on a first surface of said card; and
   a non bi-stable display located on a second surface of said card.
58. The card of statement 57, wherein said bi-stable display displays a security code.
59. The card of statement 57, wherein said bi-stable display displays at least a portion of a payment account number.
60. The card of statement 57, further comprising a processor and a plurality of buttons.
61. The card of statement 57, further comprising a rechargeable battery.
62. The card of statement 57, further comprising a rechargeable battery and a device for recharging said rechargeable battery.
63. The card of statement 57, further comprising a light sensor for receiving light-based information pulses.
64. A card comprising:
   a bi-stable display, wherein data displayed on said bi-stable display is communicated serially to a magnetic stripe reader via a dynamic magnetic communications device and no static magnetic stripe is provided on said card.
65. The card of statement 64, further comprising a plurality of buttons and a processor.
66. The card of statement 64, further comprising an EMV chip and RFID.
67. The card of statement 64, further comprising a light sensor for receiving light-based information pulses.
68. A card comprising:
   a non bi-stable display, wherein data displayed on said non bi-stable display is communicated serially to a magnetic stripe reader via a dynamic magnetic communications device and no static magnetic stripe is provided on said card.
69. The card of statement 68, further comprising a plurality of buttons and a processor.
70. The card of statement 68, further comprising an EMV chip and RFID.
71. The card of statement 68, further comprising a light sensor for receiving light-based information pulses.
72. A card comprising:
   an RFID for communicating a first data;
   an EMV chip for communicating a second data;
   a magnetic emulator for communicating a third data to a magnetic stripe reader;
   a display;
   a plurality of buttons; and
   a central processor for controlling the communication of said third data.
73. The card of statement 42, further comprising a light sensor for receiving light-based information pulses.
74. The card of statement 42, wherein said display is a bi-stable display.
75. The card of statement 42, wherein said display is a non bi-stable display.
76. A card comprising:
   a display;
   a magnetic emulator for communicating data to magnetic stripe reader;
   a plurality of buttons, wherein said plurality of buttons accept manual input for a user; and
   a processor for determining whether said manual input is indicative of a valid unlocking code, wherein said determination of a valid unlocking code permits access to at least one additional function.
77. The card of statement 76, wherein each one of said buttons includes indicia representative of a letter of the alphabet.
78. The card of statement 76, wherein each one of said buttons includes indicia representative of a number.
79. The card of statement 76, wherein said at least one additional function includes utilizing a magnetic stripe reader read-head detector.
80. The card of statement 76, wherein said at least one additional function includes communicating data from said magnetic emulator.
81. The card of statement 76, wherein said at least one additional function includes communicating data from said magnetic emulator after a read-head detector determines the proximity of a magnetic stripe reader read-head.
82. A card comprising:
   a display;
   a magnetic emulator for communicating data to magnetic stripe reader;
   only five buttons for accepting an unlocking code, wherein a second one of said only five buttons is located above a first one of said only five buttons, a third one of said only five buttons is located to the left of said first one of said only five buttons, a fourth one of said only five buttons is located to the right of said first one of said only five buttons, a fifth one of said only five buttons is located below said first one of said only five buttons.
83. The card of statement 82, wherein each one of said five buttons includes indicia representative of a number.
84. The card of statement 82, wherein each one of said five buttons includes indicia representative of a first number and a second number.
85. The card of statement 82, wherein each one of said five buttons includes indicia representative of a letter.
86. The card of statement 82, wherein each one of said five buttons includes indicia representative of a first number, a second number, and a third number.
87. The card of statement 82, further comprising a processor for determining whether said unlocking code is valid, wherein said determination of a valid unlocking code permits access to at least one additional function.
88. The card of statement 82, wherein said at least one additional function includes utilizing a magnetic stripe reader read-head detector.
89. The card of statement 82, wherein said at least one additional function includes communicating data from said magnetic emulator.
90. The card of statement 82, wherein said at least one additional function includes communicating data from said magnetic emulator after a read-head detector determines the proximity of a magnetic stripe reader read-head.
91. A card comprising:
   a magnetic emulator, wherein said magnetic emulator communicates a track of magnetic stripe data, said magnetic emulator includes a coil and a soft-magnetic material placed in the interior of said coil.
92. The card of statement 91, wherein said soft-magnetic material comprises a permalloy.
93. The card of statement 91, further comprising a display and a plurality of buttons.
94. The card of statement 91, further comprising an EMV chip, a display, and a plurality of buttons.
95. The card of statement 91, further comprising an RFID.
96. The card of statement 91, further comprising a second magnetic emulator for communicating a second track of magnetic stripe information.
97. The card of statement 91, further comprising a second magnetic emulator for communicating a second track of magnetic stripe information and a third magnetic emulator for communicating a third track of magnetic stripe information.
98. The card of statement 91, wherein said magnetic emulator is fabricated in a multiple-layer printed circuit board.
99. A card comprising:
   a magnetic emulator, wherein said magnetic emulator communicates a track of magnetic stripe data, said magnetic emulator includes a coil and a soft-magnetic material placed about the exterior of said coil.
100. The card of statement 99, wherein said soft-magnetic material comprises a permalloy.
101. The card of statement 99, further comprising a display and a plurality of buttons.
102. The card of statement 99, further comprising an EMV chip, a display, and a plurality of buttons.
103. The card of statement 99, further comprising an RFID.
104. The card of statement 99, further comprising a second magnetic emulator for communicating a second track of magnetic stripe information.
105. The card of statement 99, further comprising a second magnetic emulator for communicating a second track of magnetic stripe information and a third magnetic emulator for communicating a third track of magnetic stripe information.
106. The card of statement 99, wherein said magnetic emulator is fabricated in a multiple-layer printed circuit board.
107. A card comprising:
   a magnetic emulator, wherein said magnetic emulator communicates a track of magnetic stripe data, said magnetic emulator includes a coil and a permanent magnet is placed about the exterior of said coil.
108. The card of statement 107, further comprising a display and a plurality of buttons.
109. The card of statement 107, further comprising an EMV chip, a display, and a plurality of buttons.
110. The card of statement 107, further comprising an RFID.
111. The card of statement 107, further comprising a second magnetic emulator for communicating a second track of magnetic stripe information.
112. The card of statement 107, further comprising a second magnetic emulator for communicating a second track of magnetic stripe information and a third magnetic emulator for communicating a third track of magnetic stripe information.
113. The card of statement 107, wherein said magnetic emulator is fabricated in a multiple-layer printed circuit board.
114. A card comprising:
   a magnetic emulator, wherein said magnetic emulator communicates a track of magnetic stripe data, said magnetic emulator includes a coil and a permanent magnet is placed in the interior of said coil.
115. The card of statement 114, further comprising a display and a plurality of buttons.
116. The card of statement 114, further comprising an EMV chip, a display, and a plurality of buttons.
117. The card of statement 114, further comprising an RFID.
118. The card of statement 114, further comprising a second magnetic emulator for communicating a second track of magnetic stripe information.
119. The card of statement 114, further comprising a second magnetic emulator for communicating a second track of magnetic stripe information and a third magnetic emulator for communicating a third track of magnetic stripe information.
120. The card of statement 114, wherein said magnetic emulator is fabricated in a multiple-layer printed circuit board.
121. A card comprising:
   a magnetic emulator, wherein said magnetic emulator communicates a track of magnetic stripe data, said magnetic emulator includes a coil and a magnetostrictive material that is placed in the interior of said coil.
122. The card of statement 121, further comprising a display and a plurality of buttons.
123. The card of statement 121, further comprising an EMV chip, a display, and a plurality of buttons.
124. The card of statement 121, further comprising an RFID.
125. The card of statement 121, further comprising a second magnetic emulator for communicating a second track of magnetic stripe information.
126. The card of statement 121, further comprising a second magnetic emulator for communicating a second track of magnetic stripe information and a third magnetic emulator for communicating a third track of magnetic stripe information.
127. The card of statement 121, wherein said magnetic emulator is fabricated in a multiple-layer printed circuit board.
128. A card comprising:
   a magnetic emulator, wherein said magnetic emulator communicates a track of magnetic stripe data, said magnetic emulator includes a coil and a non-magnetostrictive material that is placed in the interior of said coil.
129. The card of statement 128, further comprising a display and a plurality of buttons.
130. The card of statement 128, further comprising an EMV chip, a display, and a plurality of buttons.
131. The card of statement 128, further comprising an RFID.
132. The card of statement 128, further comprising a second magnetic emulator for communicating a second track of magnetic stripe information.
133. The card of statement 128, further comprising a second magnetic emulator for communicating a second track of magnetic stripe information and a third magnetic emulator for communicating a third track of magnetic stripe information.
134. The card of statement 128, wherein said magnetic emulator is fabricated in a multiple-layer printed circuit board.
135. A card comprising:
   a magnetic emulator, wherein said magnetic emulator communicates a track of magnetic stripe data, said magnetic emulator includes a coil and a magnetostrictive material that is placed about the exterior of said coil.
136. The card of statement 135, further comprising a display and a plurality of buttons.
137. The card of statement 135, further comprising an EMV chip, a display, and a plurality of buttons.
138. The card of statement 135, further comprising an RFID.
139. The card of statement 135, further comprising a second magnetic emulator for communicating a second track of magnetic stripe information.
140. The card of statement 135, further comprising a second magnetic emulator for communicating a second track of magnetic stripe information and a third magnetic emulator for communicating a third track of magnetic stripe information.
141. The card of statement 135, wherein said magnetic emulator is fabricated in a multiple-layer printed circuit board.
142. A card comprising:
   a magnetic emulator, wherein said magnetic emulator communicates a track of magnetic stripe data, said magnetic emulator includes a coil and a non-magnetostrictive material that is placed about the exterior of said coil.
143. The card of statement 142, further comprising a display and a plurality of buttons.
144. The card of statement 142, further comprising an EMV chip, a display, and a plurality of buttons.
145. The card of statement 142, further comprising an RFID.
146. The card of statement 142, further comprising a second magnetic emulator for communicating a second track of magnetic stripe information.
147. The card of statement 142, further comprising a second magnetic emulator for communicating a second track of magnetic stripe information and a third magnetic emulator for communicating a third track of magnetic stripe information.
148. The card of statement 142, wherein said magnetic emulator is fabricated in a multiple-layer printed circuit board.
149. A card comprising:
   a magnetic emulator, wherein said magnetic emulator communicates a track of magnetic stripe data, said magnetic emulator includes a coil and a material that has a relative permeability of 3 to 25,000 that of a vacuum.
150. The card of statement 149, further comprising a display and a plurality of buttons.
151. The card of statement 149, further comprising an EMV chip, a display, and a plurality of buttons.
152. The card of statement 149, further comprising an RFID.
153. The card of statement 149, further comprising a second magnetic emulator for communicating a second track of magnetic stripe information.
154. The card of statement 149, further comprising a second magnetic emulator for communicating a second track of magnetic stripe information and a third magnetic emulator for communicating a third track of magnetic stripe information.
155. The card of statement 149, wherein said magnetic emulator is fabricated in a multiple-layer printed circuit board.
156. A card comprising:
   a magnetic emulator, wherein said magnetic emulator communicates a track of magnetic stripe data, said magnetic emulator includes a coil and a material that comprises 75-85% nickel.
157. The card of statement 150, further comprising a display and a plurality of buttons.
158. The card of statement 150, further comprising an EMV chip, a display, and a plurality of buttons.
159. The card of statement 150, further comprising an RFID.
160. The card of statement 150, further comprising a second magnetic emulator for communicating a second track of magnetic stripe information.
161. The card of statement 150, further comprising a second magnetic emulator for communicating a second track of magnetic stripe information and a third magnetic emulator for communicating a third track of magnetic stripe information.
162. The card of statement 150, wherein said magnetic emulator is fabricated in a multiple-layer printed circuit board.
163. A card comprising:
   a display;
   a plurality of buttons;
   a magnetic emulator for communicating magnetic stripe data, wherein said plurality of buttons receive manual input indicative of a loyalty selection and information indicative of said loyalty selection is provided in said magnetic stripe data.
164. The card of statement 163, wherein said display is bi-stable.
165. The card of statement 163, wherein said plurality of buttons includes at least three buttons.
166. The card of statement 163, further comprising an EMV chip for communicating second data that includes said information indicative of said loyalty selection.
167. The card of statement 163, further comprising an RFID for communicating second data that includes said information indicative of said loyalty selection.
168. The card of statement 163, wherein said loyalty selection included a selection from loyalty points and loyalty cash-back.
169. The card of statement 163, further comprising a light source.
170. A card comprising:
   a display;
   a plurality of buttons;
   a magnetic emulator for communicating magnetic stripe data, wherein said plurality of buttons receive manual input indicative of a tip amount and information indicative of said tip amount is provided in said magnetic stripe data.
171. The card of statement 171, wherein said display is bi-stable.
172. The card of statement 171, wherein said plurality of buttons includes at least three buttons.
173. The card of statement 171, further comprising an EMV chip.
174. The card of statement 171, further comprising an RFID chip.
175. A card comprising:
   a display;
   a plurality of buttons;
   a magnetic emulator for communicating magnetic stripe data, wherein said plurality of buttons receive manual input indicative of a personal identification code and information indicative of said personal identification code is provided in said magnetic stripe data.
176. The card of statement 175, wherein said personal identification code is utilized by a remote server to authorize a transaction.
177. The card of statement 175, wherein said personal identification code is four characters in length.
178. The card of statement 175, wherein said personal identification code is more than four characters in length.
179. The card of statement 175, further comprising an EMV chip.
180. The card of statement 175, further comprising an EMV chip and a processor.
181. The card of statement 175, further comprising an EMV chip and an RFID.
182. A card comprising:
   a display;
   a plurality of buttons;
   a magnetic emulator for communicating magnetic stripe data, wherein said plurality of buttons receive manual input indicative of a request to purchase an object using loyalty rewards and information indicative of said request is provided in said magnetic stripe data.
183. The card of statement 182, further comprising an account number, wherein said purchase made without a request to purchase said object using said loyalty rewards includes associating new loyalty rewards for said purchase to said account number.
184. The card of statement 182, further comprising an account number, wherein a determination is made as to whether said loyalty rewards are enough for said purchase of said object.
185. The card of statement 182, wherein a balance of said loyalty rewards is printed on a receipt.
186. The card of statement 182, wherein a balance of said loyalty rewards is communicated to said card.
187. The card of statement 182, further comprising a light sensor for receiving light-based information pulses.
188. The card of statement 182, further comprising a biometric sensor.
189. The card of statement 182, further comprising a microphone for receiving sound-based data.
190. A card comprising:
   a bi-directional magnetic emulator, wherein said bi-directional magnetic emulator is operable to serially communicate data to and receiving information from a magnetic stripe reader.
191. The card of statement 190, further comprising a display.
192. The card of statement 190, further comprising a plurality of buttons.
193. The card of statement 190, further comprising a processor for driving said bi-directional magnetic emulator and an EMV chip.
194. The card of statement 190, further comprising an EMV chip and an RFID.
195. The card of statement 190, further comprising an EMV chip, display, and a plurality of buttons.
196. A card comprising:
   a processor; and
   a coil, wherein said processor drives said coil at a first frequency to communicate magnetic stripe data to a magnetic stripe reader and said processor drives said coil at a second frequency to communicate RFID data to an RFID reader.
197. The card of statement 196, further comprising a display.
198. The card of statement 196, further comprising a plurality of buttons.
199. The card of statement 196, further comprising a processor for driving said bi-directional magnetic emulator and an EMV chip.
200. The card of statement 196, further comprising an EMV chip and an RFID.
201. The card of statement 196, further comprising an EMV chip, display, and a plurality of buttons.
202. A card comprising:
   a display;
   a plurality of buttons;
   a magnetic emulator for communicating magnetic stripe data, wherein said plurality of buttons receive manual input indicative of a request to take part in opt-in marketing and information indicative of said request is provided in said magnetic stripe data.
203. The card of statement 202, wherein said display is non bi-stable.
204. The card of statement 202, further comprising a second display.
205. The card of statement 202, further comprising an EMV chip.
206. The card of statement 202, further comprising an RFID.
207. The card of statement 202, further comprising a second magnetic emulator.
208. A card comprising:
   a display;
   a plurality of buttons;
   a magnetic emulator for communicating magnetic stripe data; and
   a light sensor for receiving information from pulses of light.
209. The card of statement 208, wherein said pulses of light were received from a television commercial.
210. The card of statement 208, wherein said pulses of light were received from a website.
211. The card of statement 208, wherein said information includes a gift code.
212. The card of statement 208, wherein a function is added to said card as a result of said received information.
213. The card of statement 208, wherein a balance of an account is included in said information.
214. A card comprising:
   a processor running program code; and
   a light sensor for receiving light pulses representative of information, wherein said information updates said program code.
215. The card of statement 214, wherein said light pulses are received from a website.
216. The card of statement 214, wherein said light pulses are communicated through a television commercial.
217. The card of statement 214, further comprising a magnetic emulator for communicating magnetic stripe data.
218. The card of statement 214, further comprising a display and a plurality of buttons.
219. A card comprising:
   a display;
   a plurality of buttons;
   a magnetic emulator for communicating magnetic stripe data, wherein said plurality of buttons receive manual input indicative of a gift code and information indicative of said gift code is provided in said magnetic stripe data.
220. The card of statement 219, further comprising a second display.
221. The card of statement 219, further comprising a second display and a third display.
222. The card of statement 219, further comprising a second display, a third display, and a fourth display.
223. The card of statement 219, further comprising a light sensor for receiving light-based information pulses.
224. A card comprising:
   a magnetic emulator for communicating magnetic stripe data to a read-head of a magnetic stripe reader, wherein said magnetic emulator is operable to communicate said magnetic stripe data serially to said read-head across a distance of at least 0.25 inches.
225. The card of statement 224, further comprising a display.
226. The card of statement 224, further comprising a display and a plurality of buttons.
227. The card of statement 224, further comprising an EMV chip and processor.
228. The card of statement 224, further comprising a processor, EMV chip, and RFID.
229. The card of statement 224, wherein said magnetic stripe data includes a first track of magnetic stripe data followed by a second track of magnetic stripe data.
230. A card comprising:
   a display;
   an output device for communicating data to an external device;
   a processor, wherein a code is provided by a website representative of one or more select gift cards and said processor utilizes said code to display indicia representative of at least one of said one or more selected gift cards on said display.
231. The card of statement 230, wherein said output device comprises a magnetic emulator for communicating magnetic stripe data.
232. The card of statement 230, wherein said output device comprises an EMV chip.
233. The card of statement 230 wherein said code is manually input into said card via a plurality of buttons provided on said card.
234. The card of statement 230, further comprising a light sensor for receiving light-pulses representative of information, wherein said information includes said code.
235. The card of statement 230, further comprising a second display.
236. A card comprising:
   a display;
   a plurality of buttons;
   a rechargeable battery;
   a magnetic emulator for communicating magnetic stripe data; and
   a device for recharging said rechargeable battery.
237. The card of statement 236, wherein said device converts kinetic energy to electrical energy.
238. The card of statement 236, wherein said device converts an electromagnetic field into an electrical energy.
239. The card of statement 236, further comprising a biometric sensor.
240. The card of statement 236, further comprising a fingerprint reader.
241. A card comprising:
   a display;
   a plurality of buttons; and
   a magnetic emulator, wherein said magnetic emulator communicates, to a single read-head of a magnetic stripe reader, a first track of magnetic stripe data serially before a second track of magnetic stripe data is communicated serially.
242. The card of statement 241, wherein said display is a bi-stable display.
243. The card of statement 241, wherein said first track of data includes a payment account number and said second track of data includes said payment number.
244. The card of statement 241, wherein said first track of data includes less data than said second track of data.
245. A card comprising:
   a display;
   a plurality of buttons; and
   a magnetic emulator for communicating magnetic stripe data, wherein said magnetic emulator further comprises:
      a coil;
      a permanent magnet;
      a soft-magnetic material.
246. The card of statement 245, wherein said soft-magnetic material comprises a permalloy.
247. The card of statement 245, wherein said plurality of buttons comprises at least five buttons.
248. The card of statement 245, wherein said display includes a bi-stable display.
249. The card of statement 245, further comprising an EMV chip.
250. The card of statement 245, further comprising an RFID.
251. A card comprising:
   a first display;
   a second display;
   a first LED;
   a second LED;
   a plurality of buttons; and
   a magnetic emulator for communicating magnetic stripe data.
252. The card of statement 251, wherein a processor causes said first LED to blink.
253. The card of statement 251, wherein said first LED is operable to emit at least two colors of light.
254. The card of statement 251, further comprising an RFID.
255. The card of statement 251, further comprising an EMV chip.
256. The card of statement 251, further comprising an EMV chip and a processor.
257. A card comprising:
   a first display;
   a second display;
   a plurality of buttons;
   a first magnetic emulator for communicating a first track of magnetic stripe data; and
   a processor, wherein a code is entered into said plurality of buttons and said code causes said processor to display a first information on said first display.
258. The card of statement 257, further comprising an EMV chip.
259. The card of statement 257, further comprising an RFID.
260. The card of statement 257, wherein said first display is non bi-stable.
261. A card comprising:
   a first printed circuit board layer;
   a second printed circuit board layer; and
   a third printed circuit board layer, where a magnetic emulator, comprising a coil, for communicating magnetic stripe data includes a first set of coil segments is provided on a first surface of said first printed circuit board layer, a second set of coil segments is provided a second surface of said second printed circuit board layer, and a set of vias is cut into said second printed circuit board layer for coupling said first coil segments to said second coil segments.
262. The card of statement 261, wherein a material is provided about said magnetic emulator that increases an electromagnetic field generated outside of said magnetic emulator.
263. The card of statement 261 wherein said material is placed between at least a portion of said first coil segments and a portion of said second coil segments.
264. The card of statement 261 further comprising a permanent magnet.
265. The card of statement 261 further comprising a soft-magnetic material.
266. A card comprising:
   a display;
   a processor;
   a battery;
   a magnetic emulator for communicating magnetic stripe data; and
   a button, wherein a single segment of said display is displayed when said button is pressed.
267. The card of statement 266, wherein said display is a bi-stable display.
268. The card of statement 266, wherein a code is displayed on said display and said code is entered into an online checkout process in addition to a payment account number to validate a purchase transaction.
269. The card of statement 266, wherein a code is displayed on said display, said code is entered into an online checkout process in addition to a payment account number to validate a purchase transaction, and said code changes based on time..
270. The card of statement 266, wherein a code is displayed on said display, said code is entered into an online checkout process in addition to a payment account number to validate a purchase transaction, and said code changes based on use.
271. A card comprising:
   a first read-head detector;
   a second read-head detector; and
   a magnetic emulator for communicating a track of magnetic stripe data, wherein said first and second read-head detectors are utilized to determine a magnetic-stripe reader type.
272. The card of statement 271, wherein said magnetic-stripe reader type is a motorized reader.
273. The card of statement 271, wherein said magnetic-stripe reader type is a swipe reader.
274. The card of statement 271, wherein said magnetic-stripe reader is an insertion reader.
275. A card comprising:
   a capacitive touch screen; and
   a magnetic emulator for communicating magnetic stripe data.
276. The card of statement 275, wherein said magnetic emulator serially communicates said magnetic stripe data.
277. The card of statement 275, wherein indicia representative of a button is displayed on, and is operable to accept manual input from, said capacitive touch screen.
278. The card of statement 275, further comprising an EMV chip.
279. The card of statement 275, further comprising an RFID.
280. A card comprising:
   an output device for communicating data:
   a capacitive-touch display, wherein said capacitive touch display displays a plurality of user interfaces;
   a processor operable to receive signals indicative of manual input received by at least one of said plurality of user interfaces; and
   a battery.
281. The card of statement 280, wherein said display comprises a non bi-stable display.
282. The card of statement 280, wherein said output device comprises an EMV chip.
283. The card of statement 280, wherein said output device comprises an RFID.
284. The card of statement 280, wherein said output device comprises a magnetic emulator for communicating magnetic stripe data.
285. A device comprising:
   a magnetic emulator for serially communicating a first track of data to a read-head of magnetic stripe reader followed by serially communicating a second track of data, wherein said magnetic emulator communicates said data over a distance of 0.25 inches or more.
286. The device of statement 285, wherein said device comprises a mobile telephonic device.
287. The device of statement 285, wherein said magnetic emulator comprises a coil and a magnet is provided to affect an electromagnetic field generated by said coil.
288. The device of statement 285, wherein said magnetic emulator comprises a coil and a soft-magnetic material is provided to affect an electromagnetic field generated by said coil.
289. The device of statement 285, wherein said magnetic emulator comprises a coil, a material is provided to affect an electromagnetic field generated by said coil, and said material has a relative permeability to a vacuum of 2 to 25,000.
290. A method comprising:
   manufacturing a multiple layer printed circuit board;
   assembling electrical components to said multiple layer printed circuit board;
   laminating said assembled multiple layer printed circuit board to form a laminated payment card;
   programming application code into said laminated payment card at a first location;
   programming personal data into said laminated payment card at a second location.
291. The method of statement 290, wherein said second location embosses said laminated payment card.
292. The method of statement 290, wherein said second location adds printed indicia to said laminated payment card.
293. The method of statement 290, wherein a magnetic emulator for communicating magnetic stripe data is formed during said manufacturing.
294. The method of statement 290, wherein a display is formed during said manufacturing.
295. The method of statement 290, wherein a processor is added during said assembling.
296. The method of statement 209, wherein a magnetic emulator for communicating magnetic stripe data is formed during said manufacturing, a display is formed during said manufacturing, and a processor is added during said assembling.
297. A method comprising:
   manufacturing a multiple layer printed circuit board;
   assembling electrical components to said multiple layer printed circuit board;
   programming application code into said assembled card at a first location;
   laminating said assembled multiple layer printed circuit board to form a laminated payment card;
   programming personal data into said laminated payment card at a second location.
298. The method of statement 297, wherein said second location embosses said laminated payment card.
299. The method of statement 297, wherein said second location adds printed indicia to said laminated payment card.
300. The method of statement 297, wherein a magnetic emulator for communicating magnetic stripe data is formed during said manufacturing.
301. The method of statement 297, wherein a display is formed during said manufacturing.
302. The method of statement 297, wherein a processor is added during said assembling.
303. The method of statement 297, wherein a magnetic emulator for communicating magnetic stripe data is formed during said manufacturing, a display is formed during said manufacturing, and a processor is added during said assembling.
304. A card comprising:
   means for displaying display data;
   magnetic stripe means, wherein said magnetic stripe means includes first information; and
   means, about said magnetic stripe, for communicating second information to said magnetic stripe card reader; and
   means, about said magnetic stripe, for communicating third information to said magnetic stripe card reader.
305. A card comprising:
   display means;
   magnetic stripe means, wherein said magnetic includes first information; and
   means, about said magnetic stripe, for communicating second information to said magnetic stripe card reader.
306. A card comprising:
   means for communicating a track of magnetic stripe data to a magnetic stripe reader, wherein said means communicates said track of magnetic stripe data serially and no static magnetic stripe is provided on said card.
307. A card comprising:
   means for communicating a track of magnetic stripe data to a magnetic stripe reader, wherein said means communicates said track of magnetic stripe data serially and said means is fabricated on a single surface of a printed circuit board layer.
308. A card comprising:
   means for communicating a track of magnetic stripe data to a magnetic stripe reader, wherein said means communicates said track of magnetic stripe data serials and said means is fabricated on multiple layers of material.
309. A card comprising:
   a bi-stable display means;
   a non bi-stable display means wherein data displayed on said bi-stable display means is communicated via a dynamic magnetic communications device means.
310. A card comprising:
   a bi-stable display means located on a first surface of said card; and
   a non bi-stable display means located on a second surface of said card.
311. A card comprising:
   a bi-stable display means, wherein data displayed on said bi-stable display means is communicated serially to a magnetic stripe reader via dynamic magnetic communications device means and no static magnetic stripe means is provided on said card.
312. A card comprising:
   a non bi-stable display means, wherein data displayed on said non bi-stable display means is communicated serially to a magnetic stripe reader via a dynamic magnetic communications device means and no static magnetic stripe means is provided on said card.
313. A card comprising:
   RFID means for communicating a first data;
   EMV chip means for communicating a second data;
   means for communicating a third data to a magnetic stripe reader;
   a display means;
   means for accepting manual input; and
   means for controlling the communication of said third data.
314. A card comprising:
   a display means;
   means for communicating data to magnetic stripe reader;
   a plurality of button means, wherein said plurality of button means accept manual input for a user; and
   means for determining whether said manual input is indicative of a valid unlocking code, wherein said determination of a valid unlocking code permits access to at least one additional function.
315. A card comprising:
   a display means;
   means for communicating data to magnetic stripe reader;
   only five buttons means for accepting an unlocking code, wherein a second one of said only five buttons means is located above a first one of said only five button means, a third one of said only five button means is located to the left of said first one of said only five button means, a fourth one of said only five button means is located to the right of said first one of said only five button means, a fifth one of said only five button mea ns is located below said first one of said only five button mea ns.
316. A card comprising:
   magnetic emulator means for communicating a track of magnetic stripe data, said magnetic emulator means includes a coil and a soft-magnetic material placed in the interior of said coil.
317. A card comprising:
   magnetic emulator means for communicating a track of magnetic stripe data, said magnetic emulator means includes a coil and a soft-magnetic material placed about the exterior of said coil.
318. A card comprising:
   magnetic emulator means for communicating a track of magnetic stripe data, said magnetic emulator means includes a coil and a permanent magnet is placed about the exterior of said coil.
319. A card comprising:
   magnetic emulator means for communicating a track of magnetic stripe data, said magnetic emulator includes a coil and a permanent magnet is placed in the interior of said coil.
320. A card comprising:
   magnetic emulator means for communicating a track of magnetic stripe data, said magnetic emulator means includes a coil and a magnetostrictive material that is placed in the interior of said coil.
321. A card comprising:
   magnetic emulator means for communicating a track of magnetic stripe data, said magnetic emulator means includes a coil and a non-magnetostrictive material that is placed in the interior of said coil.
322. A card comprising:
   magnetic emulator means for communicating a track of magnetic stripe data, said magnetic emulator means includes a coil and a magnetostrictive material that is placed about the exterior of said coil.
323. A card comprising:
   magnetic emulator means for communicating a track of magnetic stripe data, said magnetic emulator means includes a coil and a non-magnetostrictive material that is placed about the exterior of said coil.
324. A card comprising:
   magnetic emulator means for communicating a track of magnetic stripe data, said magnetic emulator means includes a coil and a material that has a relative permeability of 3 to 25,000 that of a vacuum.
325. A card comprising:
   magnetic emulator means for communicating a track of magnetic stripe data, said magnetic emulator means includes a coil and a material that comprises 75-85% nickel.
326. A card comprising:
   a display means;
   a plurality of button means;
   means for communicating magnetic stripe data, wherein said plurality of button means receive manual input indicative of a loyalty selection and information indicative of said loyalty selection is provided in said magnetic stripe data.
327. A card comprising:
   a display means;
   a plurality of button means;
   means for communicating magnetic stripe data, wherein said plurality of button means receive manual input indicative of a tip amount and information indicative of said tip amount is provided in said magnetic stripe data.
328. A card comprising:
   a display means;
   a plurality of button means;
   means for communicating magnetic stripe data, wherein said plurality of button means receive manual input indicative of a personal identification code and information indicative of said personal identification code is provided in said magnetic stripe data.
329. A card comprising:
   a display means;
   a plurality of button means;
   means for communicating magnetic stripe data, wherein said plurality of button means receive manual input indicative of a request to purchase an object using loyalty rewards and information indicative of said request is provided in said magnetic stripe data.
330. A card comprising:
   means for serially communicating magnetic stripe data to and receiving magnetic stripe information from a magnetic stripe reader.
331. A card comprising:
   a processor means; and
   a coil means, wherein said processor means drives said coil means at a first frequency to communicate magnetic stripe data to a magnetic stripe reader and said processor means drives said coil means at a second frequency to communicate RFID data to an RFID reader.
332. A card comprising:
   means for displaying;
   a plurality of button meanss;
   means for communicating magnetic stripe data, wherein said plurality of button means receive manual input indicative of a request to take part in opt-in marketing and information indicative of said request is provided in said magnetic stripe data.
333. A card comprising:
   means for displaying;
   means for accepting user input;
   means for communicating magnetic stripe data; and
   means for receiving information from pulses of light.
334. A card comprising:
   means for running program code; and
   means for receiving light pulses representative of information, wherein said information updates said program code.
335. A card comprising:
   means for displaying;
   means for receiving manual input;
   means for communicating magnetic stripe data, wherein said means for receiving manual input receives manual input indicative of a gift code and information indicative of said gift code is provided in said magnetic stripe data.
336. A card comprising:
   means for communicating magnetic stripe data to a read-head of a magnetic stripe reader, wherein said magnetic emulator is operable to communicate said magnetic stripe data serially to said read-head across a distance of at least 0.25 inches.
337. A card comprising:
   means for displaying;
   means for communicating data to an external device;
   processing means, wherein a code is provided by a website representative of one or more select gift cards and said processing means utilizes said code to display indicia representative of at least one of said one or more selected gift cards on said display.
338. A card comprising:
   means for displaying;
   means for accepting user input;
   a rechargeable battery;
   a magnetic emulator for communicating magnetic stripe data; and
   a device for recharging said rechargeable battery.
339. A card comprising:
   means for displaying;
   a plurality of means for accepting user input; and
   means for communicating, to a single read-head of a magnetic stripe reader, a first track of magnetic stripe data serially before a second track of magnetic stripe data is communicated serially.
340. A card comprising:
   means for displaying data;
   a plurality of means for accepting user input; and
   means for communicating magnetic stripe data, wherein said magnetic emulator further comprises:
      a coil;
      a permanent magnet; and
      a soft-magnetic material.
341. A card comprising:
   first display means;
   second display means;
   first means for producing light;
   second means for producing light;
   a plurality of means for accepting manula input; and
   means for communicating magnetic stripe data.
342. A card comprising:
   first display means;
   a second display means;
   a plurality of means for accepting manual input;
   means for communicating a first track of magnetic stripe data; and
   processor means, wherein a code is entered into said plurality of user input means and said code causes said processor means to display a first information on said first display means.
343. A card comprising:
   a first printed circuit board layer;
   a second printed circuit board layer; and
   a third printed circuit board layer, wherein means, comprising a coil, for communicating magnetic stripe data includes a first set of coil segments is provided on a first surface of said first printed circuit board layer, a second set of coil segments is provided a second surface of said second printed circuit board layer, and a set of vias is cut into said second printed circuit board layer for coupling said first coil segments to said second coil segments.
344. A card comprising:
   a display means;
   a processor means;
   means for providing electrical energy;
   means for communicating magnetic stripe data; and
   means for accepting manual input, wherein a single segment of said display is displayed when said manual input is accepted.
345. A card comprising:
   a first read-head detector means;
   a second read-head detector means; and
   means for communicating a track of magnetic stripe data, wherein said first and second read-head detectors are utilized to determine a magnetic-stripe reader type.
346. A card comprising:
   a capacitive touch screen means; and
   means for communicating magnetic stripe data.
347. A card comprising:
   means for communicating data to an external device:
   a capacitive-touch display means, wherein said capacitive touch display means displays a plurality of user interfaces;
   a processor means operable to receive signals indicative of manual input received by at least one of said plurality of user interfaces; and
   means for providing electrical energy.
348. A device comprising:
   means for serially communicating a first track of data to a read-head of magnetic stripe reader followed by serially communicating a second track of data, wherein means communicates said data over a distance of 0.25 inches or more.

## Claims

1. A card (12400) comprising:
a dynamic magnetic communications device for communicating a track of magnetic stripe data to a magnetic stripe reader, wherein said dynamic magnetic communications device communicates said track of magnetic stripe data serially
a button (12411);
wherein the card and button are configured to provide two bundles of payment information serially to a magnetic stripe reader via the dynamic magnetic communications device, and information is included in the discretionary fields of both bundles of payment information indicative of a split order.

2. The card of claim 1, wherein said dynamic magnetic communications device is fabricated in one of: a single printed circuit board layer, or a multiple layered printed circuit board.

3. The card of any preceding claim, wherein said dynamic magnetic communicated circuit is provided in an ASIC.

4. The card of any preceding claim, further comprising a bi-stable display.

5. The card of any of claims 1 to 3, further comprising a non bi-stable display.

6. The card of any preceding claim, further comprising a plurality of buttons.

7. The card of any preceding claim, further comprising an RFID.

8. The card of any preceding claim, further comprising a processor and an EMV chip.

9. A card comprising:
a bi-directional magnetic emulator, wherein said bi-directional magnetic emulator is operable to serially communicate data to and receiving information from a magnetic stripe reader; and a display.

10. The card of claim 9, further comprising a plurality of buttons.

11. The card of any of claims 9 to 10, further comprising a processor for driving said bi-directional magnetic emulator and an EMV chip.

12. A card comprising:
a processor; and
a coil, wherein said processor drives said coil at a first frequency to communicate magnetic stripe data to a magnetic stripe reader and said processor drives said coil at a second frequency to communicate RFID data to an RFID reader.

13. A card comprising:
a display;
a plurality of buttons;
a rechargeable battery;
a magnetic emulator for communicating magnetic stripe data; and
a device for recharging said rechargeable battery.

14. The card of claim 13, wherein said device converts kinetic energy to electrical energy.

15. The card of claim 14, wherein said device converts an electromagnetic field into an electrical energy.
